# EUROPEAN PATENT APPLICATION

(11) **EP 2 471 920 A2**
(43) Date of publication of application: **04.07.2012**
(21) Application number: 12153915.9
(22) Date of filing: 03.03.2008
(51) Int. Cl.: C12N 15/11, A61K 31/713

(54) **Nucleic acid compounds for inhibiting WNT gene expression and uses thereof**

(30) Priority: 02.03.2007 US 934940 P; 16.03.2007 US 934930 P; 20.12.2007 US 15611 P
(62) Divisional of application: 08731244.3
(71) Applicant: Marina Biotech, Inc., Bothell WA 98021-7266 (US)
(72) Inventor: Quay, Steven C., Woodinville, WA 98072 (US); McSwiggen, James, Boulder, CO 80304 (US); Vaish, Narendra K., Kirkland, WA 98034 (US); Ahmadian, Mohammed, Carlsbad, CA 92009 (US)
(74) Representative: MacLean, Martin Robert

(57) **Abstract**

The present disclosure provides meroduplex ribonucleic acid molecules (mdRNA) capable of decreasing or silencing WNT1, WNT2, or WNT3A gene expression. An mdRNA of this disclosure comprises at least three strands that combine to form at least two non-overlapping double-stranded regions separated by a nick or gap wherein one strand is complementary to a WNT1, WNT2, or WNT3A mRNA. In addition, the meroduplex may have at least one uridine is a 5-methyluridine, a nucleoside is a locked nucleic acid, or optionally other modifications, and any combination thereof. Also provided are methods of decreasing expression of a WNT1, WNT2, or WNT3A gene in a cell or in a subject to treat a WNT1, WNT2, or WNT3A-related disease.

SEQUENCE LISTING

<110>  Marina Biotech, Inc.

<120>  NUCLEIC ACID COMPOUNDS FOR INHIBITING
       WNT GENE EXPRESSION AND USES THEREOF

<130>  P34126EP-D1-PCT

<150>  US 61/015,611
<151>  2007-12-20

<150>  US 60/934,930
<151>  2007-03-16

<150>  US 60/934,940
<151>  2007-03-02

<160>  1901

<170>  PatentIn version 3.4


<210> 1
<211> 21
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 1
cuacacaaau cagcgauuut t                                                21


<210> 2
<211> 21
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 2
aaaucgcuga uuuguguagt t                                                21


<210> 3
<211> 25
<212> DNA
<213> Artificial Sequence

<220>

<223> Description of Combined DNA/RNA Molecule:
      Synthetic oligonucleotide

<220>
<223> Description of Artificial Sequence: Synthetic oligonucleotide

<400> 3
cuacacaaau cagcgauuuc caugt                                          25


<210> 4
<211> 27
<212> RNA
<213> Artificial Sequence

<220>
<223> Description of Artificial Sequence: Synthetic oligonucleotide

<400> 4
acauggaaau cgcugauuug uguaguc                                        27


<210> 5
<211> 13
<212> RNA
<213> Artificial Sequence

<220>
<223> Description of Artificial Sequence: Synthetic oligonucleotide

<400> 5
cuacacaaau cag                                                       13


<210> 6
<211> 11
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 6
gauuuccaug t                                                         11


<210> 7
<211> 21
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 7
ggaucuuauu ucuucggagt t                                                21

<210> 8
<211> 21
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 8
cuccgaagaa auaagaucct t                                                21

<210> 9
<211> 11
<212> RNA
<213> Artificial Sequence

<220>
<223> Description of Artificial Sequence: Synthetic oligonucleotide

<400> 9
ggaucuuauu u                                                           11

<210> 10
<211> 10
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 10
cuucggagtt                                                             10

<210> 11
<211> 27
<212> RNA
<213> Artificial Sequence

<220>
<223> Description of Artificial Sequence: Synthetic oligonucleotide

<400> 11
cauugucucc gaagaaauaa gauccuu                                          27

<210> 12
<211> 8
<212> RNA
<213> Artificial Sequence

<220>
<223> Description of Artificial Sequence: Synthetic oligonucleotide

<220>
<221> modified_base
<222> (2)
<223> LNA-G

<400> 12
ggaucuua                                                                8


<210> 13
<211> 17
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 13
uuucuucgga gacaatg                                                      17


<210> 14
<211> 16
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 14
uucuucggag acaatg                                                       16


<210>  15
<211>  15
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<400>  15
ucuucggaga caatg                                                        15


<210>  16
<211>  14
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<400>  16
cuucggagac aatg                                                         14


<210>  17
<211>  13
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<400>  17
uucggagaca atg                                                          13


<210>  18
<211>  12
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<400>  18
ucggagacaa tg                                                           12


<210>  19
<211>  11
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<400>  19
cggagacaat g                                                          11


<210> 20
<211> 9
<212> RNA
<213> Artificial Sequence

<220>
<223> Description of Artificial Sequence: Synthetic oligonucleotide

<220>
<221> modified_base
<222> (2)
<223> LNA-G

<400> 20
ggaucuuau                                                             9


<210> 21
<211> 10
<212> RNA
<213> Artificial Sequence

<220>
<223> Description of Artificial Sequence: Synthetic oligonucleotide

<220>
<221> modified_base
<222> (2)
<223> LNA-G

<400> 21
ggaucuuauu                                                            10


<210> 22
<211> 11
<212> RNA
<213> Artificial Sequence

<220>
<223> Description of Artificial Sequence: Synthetic oligonucleotide

<220>
<221> modified_base
<222> (2)
<223> LNA-G

<400> 22
ggaucuuauu u                                                          11


<210> 23
<211> 12
<212> RNA

<213> Artificial Sequence

<220>
<223> Description of Artificial Sequence: Synthetic oligonucleotide

<220>
<221> modified_base
<222> (2)
<223> LNA-G

<400> 23
ggaucuuauu uc                                                          12


<210> 24
<211> 8
<212> RNA
<213> Artificial Sequence

<220>
<223> Description of Artificial Sequence: Synthetic oligonucleotide

<220>
<221> modified_base
<222> (3)
<223> LNA-A

<400> 24
cuacacaa                                                               8


<210> 25
<211> 13
<212> DNA
<213> Artificial Sequence

<220>
<223> Description of Artificial Sequence: Synthetic oligonucleotide

<220>
<221> modified_base
<222> (8)
<223> LNA-A

<400> 25
aucagcgauu utt                                                         13


<210> 26
<211> 12
<212> DNA
<213> Artificial Sequence

<220>
<223> Description of Artificial Sequence: Synthetic oligonucleotide

<220>
<221> modified_base
<222> (7)
<223> LNA-A

<400> 26
ucagcgauuu tt                                                          12


<210> 27
<211> 11
<212> DNA
<213> Artificial Sequence

<220>
<223> Description of Artificial Sequence: Synthetic oligonucleotide

<220>
<221> modified_base
<222> (6)
<223> LNA-A

<400> 27
cagcgauuut t                                                           11


<210> 28
<211> 10
<212> DNA
<213> Artificial Sequence

<220>
<223> Description of Artificial Sequence: Synthetic oligonucleotide

<220>
<221> modified_base
<222> (5)
<223> LNA-A

<400> 28
agcgauuutt                                                             10


<210> 29
<211> 9
<212> DNA
<213> Artificial Sequence

<220>
<223> Description of Artificial Sequence: Synthetic oligonucleotide

<220>
<221> modified_base
<222> (4)
<223> LNA-A

<400> 29
gcgauuutt                                                               9


<210> 30
<211> 8
<212> DNA
<213> Artificial Sequence

<220>

<223> Description of Artificial Sequence: Synthetic oligonucleotide

<220>
<221> modified_base
<222> (3)
<223> LNA-A

<400> 30
cgauuutt                                                                8


<210> 31
<211> 7
<212> DNA
<213> Artificial Sequence

<220>
<223> Description of Artificial Sequence: Synthetic oligonucleotide

<220>
<221> modified_base
<222> (2)
<223> LNA-A

<400> 31
gauuutt                                                                 7


<210> 32
<211> 9
<212> RNA
<213> Artificial Sequence

<220>
<223> Description of Artificial Sequence: Synthetic oligonucleotide

<220>
<221> modified_base
<222> (3)
<223> LNA-A

<400> 32
cuacacaaa                                                               9


<210> 33
<211> 10
<212> RNA
<213> Artificial Sequence

<220>
<223> Description of Artificial Sequence: Synthetic oligonucleotide

<220>
<221> modified_base
<222> (3)
<223> LNA-A

<400> 33
cuacacaaau                                                             10

<210> 34
<211> 11
<212> RNA
<213> Artificial Sequence

<220>
<223> Description of Artificial Sequence: Synthetic oligonucleotide

<220>
<221> modified_base
<222> (3)
<223> LNA-A

<400> 34
cuacacaaau c                                                            11


<210> 35
<211> 12
<212> RNA
<213> Artificial Sequence

<220>
<223> Description of Artificial Sequence: Synthetic oligonucleotide

<220>
<221> modified_base
<222> (3)
<223> LNA-A

<400> 35
cuacacaaau ca                                                           12


<210> 36
<211> 13
<212> RNA
<213> Artificial Sequence

<220>
<223> Description of Artificial Sequence: Synthetic oligonucleotide

<220>
<221> modified_base
<222> (3)
<223> LNA-A

<400> 36
cuacacaaau cag                                                          13


<210> 37
<211> 14
<212> RNA
<213> Artificial Sequence

<220>
<223> Description of Artificial Sequence: Synthetic oligonucleotide

<220>

<221> modified_base
<222> (3)
<223> LNA-A

<400> 37
cuacacaaau cagc                                                          14


<210> 38
<211> 13
<212> RNA
<213> Artificial Sequence

<220>
<223> Description of Artificial Sequence: Synthetic oligonucleotide

<220>
<221> modified_base
<222> (2)
<223> LNA-G

<400> 38
ggaucuuauu ucu                                                           13


<210> 39
<211> 14
<212> RNA
<213> Artificial Sequence

<220>
<223> Description of Artificial Sequence: Synthetic oligonucleotide

<220>
<221> modified_base
<222> (2)
<223> LNA-G

<400> 39
ggaucuuauu ucuu                                                          14


<210> 40
<211> 8
<212> RNA
<213> Artificial Sequence

<220>
<223> Description of Artificial Sequence: Synthetic oligonucleotide

<220>
<221> modified_base
<222> (2)
<223> LNA-G

<220>
<221> modified_base
<222> (8)
<223> LNA-A

<400> 40

ggaucuua                                                                8

<210> 41
<211> 9
<212> RNA
<213> Artificial Sequence

<220>
<223> Description of Artificial Sequence: Synthetic oligonucleotide

<220>
<221> modified_base
<222> (2)
<223> LNA-G

<220>
<221> modified_base
<222> (9)
<223> LNA-U

<400> 41
ggaucuuau                                                               9

<210> 42
<211> 10
<212> RNA
<213> Artificial Sequence

<220>
<223> Description of Artificial Sequence: Synthetic oligonucleotide

<220>
<221> modified_base
<222> (2)
<223> LNA-G

<220>
<221> modified_base
<222> (10)
<223> LNA-U

<400> 42
ggaucuuauu                                                              10

<210> 43
<211> 11
<212> RNA
<213> Artificial Sequence

<220>
<223> Description of Artificial Sequence: Synthetic oligonucleotide

<220>
<221> modified_base
<222> (2)
<223> LNA-G

<220>

<221> modified_base
<222> (11)
<223> LNA-U

<400> 43
ggaucuuauu u                                                            11


<210> 44
<211> 12
<212> RNA
<213> Artificial Sequence

<220>
<223> Description of Artificial Sequence: Synthetic oligonucleotide

<220>
<221> modified_base
<222> (2)
<223> LNA-G

<220>
<221> modified_base
<222> (12)
<223> LNA-C

<400> 44
ggaucuuauu uc                                                           12


<210> 45
<211> 13
<212> RNA
<213> Artificial Sequence

<220>
<223> Description of Artificial Sequence: Synthetic oligonucleotide

<220>
<221> modified_base
<222> (2)
<223> LNA-G

<220>
<221> modified_base
<222> (13)
<223> LNA-U

<400> 45
ggaucuuauu ucu                                                          13


<210> 46
<211> 14
<212> RNA
<213> Artificial Sequence

<220>
<223> Description of Artificial Sequence: Synthetic oligonucleotide

<220>

<221> modified_base
<222> (2)
<223> LNA-G

<220>
<221> modified_base
<222> (14)
<223> LNA-U

<400> 46
ggaucuuauu ucuu                                                    14


<210> 47
<211> 13
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<220>
<221> modified_base
<222> (8)
<223> LNA-G

<400> 47
uuucuucgga gtt                                                     13


<210> 48
<211> 12
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<220>
<221> modified_base
<222> (7)
<223> LNA-G

<400> 48
uucuucggag tt                                                      12


<210>  49
<211>  11
<212>  DNA
<213>  Artificial Sequence

<220>

<223> Description of Combined DNA/RNA Molecule:
      Synthetic oligonucleotide

<220>
<223>  Description of Artificial Sequence: Synthetic oligonucleotide

<220>
<221> modified_base
<222> (6)
<223> LNA-G

<400>  49
ucuucggagt t                                                          11


<210>  50
<211>  10
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<220>
<221> modified_base
<222> (5)
<223> LNA-G

<400>  50
cuucggagtt                                                            10


<210>  51
<211>  9
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<220>
<221> modified_base
<222> (4)
<223> LNA-G

<400>  51
uucggagtt                                                              9


<210>  52
<211>  8
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<220>
<221> modified_base
<222> (3)
<223> LNA-G

<400>  52
ucggagtt                                                                 8


<210>  53
<211>  7
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<220>
<221> modified_base
<222> (2)
<223> LNA-G

<400>  53
cggagtt                                                                  7


<210> 54
<211> 13
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<220>
<221> modified_base
<222> (1)
<223> LNA-U

<220>
<221> modified_base
<222> (8)
<223> LNA-G

<400> 54

uuucuucgga gtt                                                          13


<210> 55
<211> 12
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<220>
<221> modified_base
<222> (1)
<223> LNA-U

<220>
<221> modified_base
<222> (7)
<223> LNA-G

<400> 55
uucuucggag tt                                                           12


<210>  56
<211>  11
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<220>
<221> modified_base
<222> (1)
<223> LNA-U

<220>
<221> modified_base
<222> (6)
<223> LNA-G

<400>  56
ucuucggagt t                                                            11


<210>  57
<211>  10
<212>  DNA
<213>  Artificial Sequence

<220>

<223> Description of Combined DNA/RNA Molecule:
      Synthetic oligonucleotide

<220>
<223>  Description of Artificial Sequence: Synthetic oligonucleotide

<220>
<221> modified_base
<222> (1)
<223> LNA-C

<220>
<221> modified_base
<222> (5)
<223> LNA-G

<400>  57
cuucggagtt                                                              10


<210>  58
<211>  9
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<220>
<221> modified_base
<222> (1)
<223> LNA-U

<220>
<221> modified_base
<222> (4)
<223> LNA-G

<400>  58
uucggagtt                                                               9


<210>  59
<211>  8
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<220>
<221> modified_base
<222> (1)

<223> LNA-U

<220>
<221> modified_base
<222> (3)
<223> LNA-G

<400>  59
ucggagtt                                                                8


<210>  60
<211>  7
<212>  DNA
<213>  Artificial Sequence

<220>
<223> Description of Combined DNA/RNA Molecule:
      Synthetic oligonucleotide

<220>
<223>  Description of Artificial Sequence: Synthetic oligonucleotide

<220>
<221> modified_base
<222> (1)
<223> LNA-C

<220>
<221> modified_base
<222> (2)
<223> LNA-G

<400>  60
cggagtt                                                                 7


<210> 61
<211> 21
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<220>
<221> modified_base
<222> (2)
<223> LNA-G

<220>
<221> modified_base
<222> (16)
<223> LNA-G

<400> 61
ggaucuuauu ucuucggagt t                                                21

<210> 62
<211> 25
<212> DNA
<213> Artificial Sequence

<220>
<223> Description of Combined DNA/RNA Molecule:
      Synthetic oligonucleotide

<220>
<223> Description of Artificial Sequence: Synthetic oligonucleotide

<400> 62
ggaucuuauu ucuucggaga caatg                                          25


<210>  63
<211>  25
<212>  RNA
<213>  Artificial Sequence

<220>
<223>  Description of Artificial Sequence: Synthetic oligonucleotide

<400>  63
guauuuugau gaggaguuca cggcc                                          25


<210>  64
<211>  25
<212>  RNA
<213>  Artificial Sequence

<220>
<223>  Description of Artificial Sequence: Synthetic oligonucleotide

<400>  64
ggcccagaug aucaccauca cacca                                          25


<210>  65
<211>  25
<212>  RNA
<213>  Artificial Sequence

<220>
<223>  Description of Artificial Sequence: Synthetic oligonucleotide

<400>  65
gggaagaaaa cuauccugcg gguuu                                          25


<210>  66
<211>  25
<212>  RNA
<213>  Artificial Sequence

<220>
<223>  Description of Artificial Sequence: Synthetic oligonucleotide

<400>  66
guuuuaauuu auuucaucca guuug                                          25


<210>  67
<211>  25
<212>  RNA
<213>  Artificial Sequence

<220>
<223>  Description of Artificial Sequence: Synthetic oligonucleotide

<400>  67
acguagggaa auguuaagga cuucu                                          25


<210>  68
<211>  25
<212>  RNA
<213>  Artificial Sequence

<220>
<223>  Description of Artificial Sequence: Synthetic oligonucleotide

<400>  68
ccaggguuua cccaguggga cagag                                          25


<210>  69
<211>  25
<212>  RNA
<213>  Artificial Sequence

<220>
<223>  Description of Artificial Sequence: Synthetic oligonucleotide

<400>  69
agcaagguuu aaauuuguua uugug                                          25


<210>  70
<211>  25
<212>  RNA
<213>  Artificial Sequence

<220>
<223>  Description of Artificial Sequence: Synthetic oligonucleotide

<400>  70
uguauuaugu uguucaaaug cauuu                                          25


<210>  71
<211>  25
<212>  RNA
<213>  Artificial Sequence

<220>
<223>  Description of Artificial Sequence: Synthetic oligonucleotide

<400>  71
uuuuaaucuu ugugacagga aagcc                                          25

<210>  72
<211>  25
<212>  RNA
<213>  Artificial Sequence

<220>
<223>  Description of Artificial Sequence: Synthetic oligonucleotide

<400>  72
uuaaucuuug ugacaggaaa gcccu                                          25

<210>  73
<211>  25
<212>  RNA
<213>  Artificial Sequence

<220>
<223>  Description of Artificial Sequence: Synthetic oligonucleotide

<400>  73
gcugcuuaug ucucccagca uggcc                                          25

<210>  74
<211>  25
<212>  RNA
<213>  Artificial Sequence

<220>
<223>  Description of Artificial Sequence: Synthetic oligonucleotide

<400>  74
aaguguuuca gaagcuucuc ccuga                                          25

<210>  75
<211>  25
<212>  RNA
<213>  Artificial Sequence

<220>
<223>  Description of Artificial Sequence: Synthetic oligonucleotide

<400>  75
gaccaucaau aaggaagaag cccuu                                          25

<210>  76
<211>  25
<212>  RNA
<213>  Artificial Sequence

<220>
<223>  Description of Artificial Sequence: Synthetic oligonucleotide

<400>  76

ccaucaauaa ggaagaagcc cuuca 25

<210> 77
<211> 25
<212> RNA
<213> Artificial Sequence

<220>
<223> Description of Artificial Sequence: Synthetic oligonucleotide

<400> 77
ucaauaagga agaagcccuu cagcg 25

<210> 78
<211> 25
<212> RNA
<213> Artificial Sequence

<220>
<223> Description of Artificial Sequence: Synthetic oligonucleotide

<400> 78
aauaaggaag aagcccuuca gcggc 25

<210> 79
<211> 25
<212> RNA
<213> Artificial Sequence

<220>
<223> Description of Artificial Sequence: Synthetic oligonucleotide

<400> 79
aggaagaagc ccuucagcgg ccagu 25

<210> 80
<211> 25
<212> RNA
<213> Artificial Sequence

<220>
<223> Description of Artificial Sequence: Synthetic oligonucleotide

<400> 80
gcauaacuaa aggugaaaag cuccg 25

<210> 81
<211> 25
<212> RNA
<213> Artificial Sequence

<220>
<223> Description of Artificial Sequence: Synthetic oligonucleotide

<400> 81
cauaacuaaa ggugaaaagc uccgg 25

<210>  82
<211>  25
<212>  RNA
<213>  Artificial Sequence

<220>
<223>  Description of Artificial Sequence: Synthetic oligonucleotide

<400>  82
cuacaucacg ccagucaaca gucug                                          25


<210>  83
<211>  25
<212>  RNA
<213>  Artificial Sequence

<220>
<223>  Description of Artificial Sequence: Synthetic oligonucleotide

<400>  83
acuggauuua agcagaguuc aaaag                                          25


<210>  84
<211>  25
<212>  RNA
<213>  Artificial Sequence

<220>
<223>  Description of Artificial Sequence: Synthetic oligonucleotide

<400>  84
cuggauuuaa gcagaguuca aaagc                                          25


<210>  85
<211>  25
<212>  RNA
<213>  Artificial Sequence

<220>
<223>  Description of Artificial Sequence: Synthetic oligonucleotide

<400>  85
gauuuaagca gaguucaaaa gcccu                                          25


<210>  86
<211>  25
<212>  RNA
<213>  Artificial Sequence

<220>
<223>  Description of Artificial Sequence: Synthetic oligonucleotide

<400>  86
auuuaagcag aguucaaaag cccuu                                          25

<210>  87
<211>  25
<212>  RNA
<213>  Artificial Sequence

<220>
<223>  Description of Artificial Sequence: Synthetic oligonucleotide

<400>  87
uuaagcagag uucaaaagcc cuuca                                          25


<210>  88
<211>  25
<212>  RNA
<213>  Artificial Sequence

<220>
<223>  Description of Artificial Sequence: Synthetic oligonucleotide

<400>  88
uaagcagagu ucaaaagccc uucag                                          25


<210>  89
<211>  25
<212>  RNA
<213>  Artificial Sequence

<220>
<223>  Description of Artificial Sequence: Synthetic oligonucleotide

<400>  89
agcagaguuc aaaagcccuu cagcg                                          25


<210>  90
<211>  25
<212>  RNA
<213>  Artificial Sequence

<220>
<223>  Description of Artificial Sequence: Synthetic oligonucleotide

<400>  90
cucagggucu gagugaagcc gcucg                                          25


<210>  91
<211>  25
<212>  RNA
<213>  Artificial Sequence

<220>
<223>  Description of Artificial Sequence: Synthetic oligonucleotide

<400>  91
caagcaacua caucacgcca gucaa                                          25

<210>  92
<211>  25
<212>  RNA
<213>  Artificial Sequence

<220>
<223>  Description of Artificial Sequence: Synthetic oligonucleotide

<400>  92
aucaauggca gcuucuuggu gcgug                                          25


<210>  93
<211>  25
<212>  RNA
<213>  Artificial Sequence

<220>
<223>  Description of Artificial Sequence: Synthetic oligonucleotide

<400>  93
uuccagccca cauuggauuc aucag                                          25


<210>  94
<211>  25
<212>  RNA
<213>  Artificial Sequence

<220>
<223>  Description of Artificial Sequence: Synthetic oligonucleotide

<400>  94
cagcugagaa uguggaauac cuaag                                          25


<210>  95
<211>  25
<212>  RNA
<213>  Artificial Sequence

<220>
<223>  Description of Artificial Sequence: Synthetic oligonucleotide

<400>  95
aacguaucuc cuaauuugag gcuca                                          25


<210>  96
<211>  25
<212>  RNA
<213>  Artificial Sequence

<220>
<223>  Description of Artificial Sequence: Synthetic oligonucleotide

<400>  96
ccuaaaauaa uuucucuaca auugg                                          25


<210>  97

<211>  25
<212>  RNA
<213>  Artificial Sequence

<220>
<223>  Description of Artificial Sequence: Synthetic oligonucleotide

<400>  97
uggaagauuc agcuaguuag gagcc                                          25


<210>  98
<211>  25
<212>  RNA
<213>  Artificial Sequence

<220>
<223>  Description of Artificial Sequence: Synthetic oligonucleotide

<400>  98
uuaaacucuc cuagucaaua uccac                                          25


<210>  99
<211>  25
<212>  RNA
<213>  Artificial Sequence

<220>
<223>  Description of Artificial Sequence: Synthetic oligonucleotide

<400>  99
cagccuacag uuauguucag ucaca                                          25


<210>  100
<211>  25
<212>  RNA
<213>  Artificial Sequence

<220>
<223>  Description of Artificial Sequence: Synthetic oligonucleotide

<400>  100
guuauguuca gucacacaca cauac                                          25


<210>  101
<211>  25
<212>  RNA
<213>  Artificial Sequence

<220>
<223>  Description of Artificial Sequence: Synthetic oligonucleotide

<400>  101
cacauacaaa auguuccuuu ugcuu                                          25


<210>  102
<211>  25

<212>  RNA
<213>  Artificial Sequence

<220>
<223>  Description of Artificial Sequence: Synthetic oligonucleotide

<400>  102
uccuuuugcu uuuaaaguaa uuuuu                                          25


<210>  103
<211>  25
<212>  RNA
<213>  Artificial Sequence

<220>
<223>  Description of Artificial Sequence: Synthetic oligonucleotide

<400>  103
ugaccuguga agcaacaguc aaugg                                          25


<210>  104
<211>  25
<212>  RNA
<213>  Artificial Sequence

<220>
<223>  Description of Artificial Sequence: Synthetic oligonucleotide

<400>  104
cuaucucaca caucgacaaa ccaau                                          25


<210>  105
<211>  25
<212>  RNA
<213>  Artificial Sequence

<220>
<223>  Description of Artificial Sequence: Synthetic oligonucleotide

<400>  105
uguccucaau uguacugcua ccacu                                          25


<210>  106
<211>  25
<212>  RNA
<213>  Artificial Sequence

<220>
<223>  Description of Artificial Sequence: Synthetic oligonucleotide

<400>  106
aaaccguagc uggcaagcgg ucuua                                          25


<210>  107
<211>  25
<212>  RNA

<213>  Artificial Sequence

<220>
<223>  Description of Artificial Sequence: Synthetic oligonucleotide

<400>  107
uagcuggcaa gcggucuuac cggcu                                          25


<210>  108
<211>  25
<212>  RNA
<213>  Artificial Sequence

<220>
<223>  Description of Artificial Sequence: Synthetic oligonucleotide

<400>  108
   uuguaugguu aaaagauggg uuacc                                           25


<210>  109
<211>  25
<212>  RNA
<213>  Artificial Sequence

<220>
<223>  Description of Artificial Sequence: Synthetic oligonucleotide

<400>  109
ugguuaaaag auggguuacc ugcga                                          25


<210>  110
<211>  25
<212>  RNA
<213>  Artificial Sequence

<220>
<223>  Description of Artificial Sequence: Synthetic oligonucleotide

<400>  110
cagggaauua uacaaucuug cugag                                          25


<210>  111
<211>  25
<212>  RNA
<213>  Artificial Sequence

<220>
<223>  Description of Artificial Sequence: Synthetic oligonucleotide

<400>  111
acaaucuugc ugagcauaaa acagu                                          25


<210>  112
<211>  25
<212>  RNA
<213>  Artificial Sequence

<220>
<223>  Description of Artificial Sequence: Synthetic oligonucleotide

<400>  112
ccaauaauga agaguccuuu auccu                                          25


<210>  113
<211>  25
<212>  RNA
<213>  Artificial Sequence

<220>
<223>  Description of Artificial Sequence: Synthetic oligonucleotide

<400>  113
acuuuggaug uuccaacgca aguug                                          25


<210>  114
<211>  25
<212>  RNA
<213>  Artificial Sequence

<220>
<223>  Description of Artificial Sequence: Synthetic oligonucleotide

<400>  114
aaugcuucca cuaaacugaa accau                                          25


<210>  115
<211>  25
<212>  RNA
<213>  Artificial Sequence

<220>
<223>  Description of Artificial Sequence: Synthetic oligonucleotide

<400>  115
gagaaaguuu gacuuuguua aauau                                          25


<210>  116
<211>  25
<212>  RNA
<213>  Artificial Sequence

<220>
<223>  Description of Artificial Sequence: Synthetic oligonucleotide

<400>  116
aaagaacuac uguauauuaa aaguu                                          25


<210>  117
<211>  25
<212>  RNA
<213>  Artificial Sequence

<220>
<223>  Description of Artificial Sequence: Synthetic oligonucleotide

<400>  117
uuagaaauac ggguuuugac uuaac                                          25


<210>  118
<211>  25
<212>  RNA
<213>  Artificial Sequence

<220>
<223>  Description of Artificial Sequence: Synthetic oligonucleotide

<400>  118
aacaugggua cagcaaacuc agcac                                          25


<210>  119
<211>  25
<212>  RNA
<213>  Artificial Sequence

<220>
<223>  Description of Artificial Sequence: Synthetic oligonucleotide

<400>  119
aaagacacag aagaugcuga ccuca                                          25


<210>  120
<211>  25
<212>  RNA
<213>  Artificial Sequence

<220>
<223>  Description of Artificial Sequence: Synthetic oligonucleotide

<400>  120
uaguagggag guuuauucag aucgc                                          25


<210>  121
<211>  25
<212>  RNA
<213>  Artificial Sequence

<220>
<223>  Description of Artificial Sequence: Synthetic oligonucleotide

<400>  121
gccuucugca gcaggguucu gggau                                          25


<210>  122
<211>  25
<212>  RNA
<213>  Artificial Sequence

<220>

<223>  Description of Artificial Sequence: Synthetic oligonucleotide

<400>  122
ggucugguac auauuggaaa uuaug                                          25


<210>  123
<211>  25
<212>  RNA
<213>  Artificial Sequence

<220>
<223>  Description of Artificial Sequence: Synthetic oligonucleotide

<400>  123
cuaguccuuc cgauggaagc acuag                                          25


<210>  124
<211>  25
<212>  RNA
<213>  Artificial Sequence

<220>
<223>  Description of Artificial Sequence: Synthetic oligonucleotide

<400>  124
ccagugaaua uuguuuuuau gugga                                          25


<210>  125
<211>  25
<212>  RNA
<213>  Artificial Sequence

<220>
<223>  Description of Artificial Sequence: Synthetic oligonucleotide

<400>  125
augaauucaa guuggaauug guaga                                          25


<210>  126
<211>  25
<212>  RNA
<213>  Artificial Sequence

<220>
<223>  Description of Artificial Sequence: Synthetic oligonucleotide

<400>  126
caggacacag auuuagacuu ggaga                                          25


<210>  127
<211>  25
<212>  RNA
<213>  Artificial Sequence

<220>
<223>  Description of Artificial Sequence: Synthetic oligonucleotide

<400>  127
cucaaagcac aguuacagua uucca                                          25


<210>  128
<211>  25
<212>  RNA
<213>  Artificial Sequence

<220>
<223>  Description of Artificial Sequence: Synthetic oligonucleotide

<400>  128
accacugcca ccacugauga auuaa                                          25


<210>  129
<211>  25
<212>  RNA
<213>  Artificial Sequence

<220>
<223>  Description of Artificial Sequence: Synthetic oligonucleotide

<400>  129
gaaacuacua gugccacauc aucac                                          25


<210>  130
<211>  25
<212>  RNA
<213>  Artificial Sequence

<220>
<223>  Description of Artificial Sequence: Synthetic oligonucleotide

<400>  130
aagucggaca gccucaccaa acaga                                          25


<210>  131
<211>  25
<212>  RNA
<213>  Artificial Sequence

<220>
<223>  Description of Artificial Sequence: Synthetic oligonucleotide

<400>  131
gaaagcgaaa aauggaacau gaugg                                          25


<210>  132
<211>  25
<212>  RNA
<213>  Artificial Sequence

<220>
<223>  Description of Artificial Sequence: Synthetic oligonucleotide

<400>  132
cccucugauu uagcauguag acugc                                           25


<210>  133
<211>  25
<212>  RNA
<213>  Artificial Sequence

<220>
<223>  Description of Artificial Sequence: Synthetic oligonucleotide

<400>  133
ugagcuauuu aaggaucuau uuaug                                           25


<210>  134
<211>  25
<212>  RNA
<213>  Artificial Sequence

<220>
<223>  Description of Artificial Sequence: Synthetic oligonucleotide

<400>  134
aaaaggugaa aaagcacuau uauca                                           25


<210>  135
<211>  25
<212>  RNA
<213>  Artificial Sequence

<220>
<223>  Description of Artificial Sequence: Synthetic oligonucleotide

<400>  135
gaaaaagcac uauuaucagu ucugc                                           25


<210>  136
<211>  25
<212>  RNA
<213>  Artificial Sequence

<220>
<223>  Description of Artificial Sequence: Synthetic oligonucleotide

<400>  136
ggcugaaaag aaagauuaaa ccuac                                           25


<210>  137
<211>  25
<212>  RNA
<213>  Artificial Sequence

<220>
<223>  Description of Artificial Sequence: Synthetic oligonucleotide

<400>  137

uaaacccuua uaauaaaauc cuucu                                          25

<210>  138
<211>  25
<212>  RNA
<213>  Artificial Sequence

<220>
<223>  Description of Artificial Sequence: Synthetic oligonucleotide

<400>  138
cauacuauua gccaaugcug uagac                                          25

<210>  139
<211>  25
<212>  RNA
<213>  Artificial Sequence

<220>
<223>  Description of Artificial Sequence: Synthetic oligonucleotide

<400>  139
gacagaagca uuuugauagg aauag                                          25

<210>  140
<211>  25
<212>  RNA
<213>  Artificial Sequence

<220>
<223>  Description of Artificial Sequence: Synthetic oligonucleotide

<400>  140
agagcaaaua agauaauggc ccuga                                          25

<210>  141
<211>  25
<212>  RNA
<213>  Artificial Sequence

<220>
<223>  Description of Artificial Sequence: Synthetic oligonucleotide

<400>  141
ccaacauuuu ucucuuccuc aagca                                          25

<210>  142
<211>  25
<212>  RNA
<213>  Artificial Sequence

<220>
<223>  Description of Artificial Sequence: Synthetic oligonucleotide

<400>  142
uuaaguauga gaaaaguuca gccca                                          25

<210> 143
<211> 25
<212> RNA
<213> Artificial Sequence

<220>
<223> Description of Artificial Sequence: Synthetic oligonucleotide

<400> 143
caggaauaaa gauggcugcu gaacc 25


<210> 144
<211> 25
<212> RNA
<213> Artificial Sequence

<220>
<223> Description of Artificial Sequence: Synthetic oligonucleotide

<400> 144
aauuugaaug accaaguucu cuuca 25


<210> 145
<211> 25
<212> RNA
<213> Artificial Sequence

<220>
<223> Description of Artificial Sequence: Synthetic oligonucleotide

<400> 145
auguauaaag auagccagcc uagag 25


<210> 146
<211> 25
<212> RNA
<213> Artificial Sequence

<220>
<223> Description of Artificial Sequence: Synthetic oligonucleotide

<400> 146
ggcuguaacu aucucuguga agugu 25


<210> 147
<211> 25
<212> RNA
<213> Artificial Sequence

<220>
<223> Description of Artificial Sequence: Synthetic oligonucleotide

<400> 147
ucugugaagu gugagaaaau uucaa 25

<210>  148
<211>  25
<212>  RNA
<213>  Artificial Sequence

<220>
<223>  Description of Artificial Sequence: Synthetic oligonucleotide

<400>  148
ccuuuaagga aaugaauccu ccuga                                          25


<210>  149
<211>  25
<212>  RNA
<213>  Artificial Sequence

<220>
<223>  Description of Artificial Sequence: Synthetic oligonucleotide

<400>  149
aaggauacaa aaagugacau cauau                                          25


<210>  150
<211>  25
<212>  RNA
<213>  Artificial Sequence

<220>
<223>  Description of Artificial Sequence: Synthetic oligonucleotide

<400>  150
agaugcaauu ugaaucuuca ucaua                                          25


<210>  151
<211>  25
<212>  RNA
<213>  Artificial Sequence

<220>
<223>  Description of Artificial Sequence: Synthetic oligonucleotide

<400>  151
guucaaaacg aagacuagcu auuaa                                          25


<210>  152
<211>  25
<212>  RNA
<213>  Artificial Sequence

<220>
<223>  Description of Artificial Sequence: Synthetic oligonucleotide

<400>  152
gugaaaccuc aucucuacua aaaau                                          25

<210>  153
<211>  25
<212>  RNA
<213>  Artificial Sequence

<220>
<223>  Description of Artificial Sequence: Synthetic oligonucleotide

<400>  153
acgaaagaga agcucuaucu cgccu                                          25


<210>  154
<211>  25
<212>  RNA
<213>  Artificial Sequence

<220>
<223>  Description of Artificial Sequence: Synthetic oligonucleotide

<400>  154
cuccacaagc gccuucgguc caguu                                          25


<210>  155
<211>  25
<212>  RNA
<213>  Artificial Sequence

<220>
<223>  Description of Artificial Sequence: Synthetic oligonucleotide

<400>  155
gagaagauuc caaagaugua gccgc                                          25


<210>  156
<211>  25
<212>  RNA
<213>  Artificial Sequence

<220>
<223>  Description of Artificial Sequence: Synthetic oligonucleotide

<400>  156
aaucuggauu caaugaggag acuug                                          25


<210>  157
<211>  25
<212>  RNA
<213>  Artificial Sequence

<220>
<223>  Description of Artificial Sequence: Synthetic oligonucleotide

<400>  157
agaacagauu ugagaguagu gagga                                          25


<210>  158

<211>  25
<212>  RNA
<213>  Artificial Sequence

<220>
<223>  Description of Artificial Sequence: Synthetic oligonucleotide

<400>  158
ccagagcugu gcagaugagu acaaa                                          25


<210>  159
<211>  25
<212>  RNA
<213>  Artificial Sequence

<220>
<223>  Description of Artificial Sequence: Synthetic oligonucleotide

<400>  159
ccucagauug uuguuguuaa ugggc                                          25


<210>  160
<211>  25
<212>  RNA
<213>  Artificial Sequence

<220>
<223>  Description of Artificial Sequence: Synthetic oligonucleotide

<400>  160
cuauuuuaau uauuuuuaau uuauu                                          25


<210>  161
<211>  25
<212>  RNA
<213>  Artificial Sequence

<220>
<223>  Description of Artificial Sequence: Synthetic oligonucleotide

<400>  161
uuuaauuuau uaauauuuaa auaug                                          25


<210>  162
<211>  25
<212>  RNA
<213>  Artificial Sequence

<220>
<223>  Description of Artificial Sequence: Synthetic oligonucleotide

<400>  162
augcaguuug aauauccuuu guuuc                                          25


<210>  163
<211>  25

<212>  RNA
<213>  Artificial Sequence

<220>
<223>  Description of Artificial Sequence: Synthetic oligonucleotide

<400>  163
caugcugcug gcgucuaagu guuug                                          25


<210>  164
<211>  25
<212>  RNA
<213>  Artificial Sequence

<220>
<223>  Description of Artificial Sequence: Synthetic oligonucleotide

<400>  164
agaugugcau uucaccugug acaaa                                          25


<210>  165
<211>  25
<212>  RNA
<213>  Artificial Sequence

<220>
<223>  Description of Artificial Sequence: Synthetic oligonucleotide

<400>  165
ucaaaaccug ugccaggcug aauua                                          25


<210>  166
<211>  25
<212>  RNA
<213>  Artificial Sequence

<220>
<223>  Description of Artificial Sequence: Synthetic oligonucleotide

<400>  166
gaaugugggu agucauucuu acaau                                          25


<210>  167
<211>  25
<212>  RNA
<213>  Artificial Sequence

<220>
<223>  Description of Artificial Sequence: Synthetic oligonucleotide

<400>  167
augugguag ucauucuuac aauug                                           25


<210>  168
<211>  25
<212>  RNA

<213>  Artificial Sequence

<220>
<223>  Description of Artificial Sequence: Synthetic oligonucleotide

<400>  168
ugaaaaugag caucagagag uguac                                          25


<210>  169
<211>  25
<212>  RNA
<213>  Artificial Sequence

<220>
<223>  Description of Artificial Sequence: Synthetic oligonucleotide

<400>  169
uugcuuuuca uguagaacuc agcag                                          25


<210>  170
<211>  25
<212>  RNA
<213>  Artificial Sequence

<220>
<223>  Description of Artificial Sequence: Synthetic oligonucleotide

<400>  170
uguauuucua uauuuauuuu cagua                                          25


<210>  171
<211>  25
<212>  RNA
<213>  Artificial Sequence

<220>
<223>  Description of Artificial Sequence: Synthetic oligonucleotide

<400>  171
uuugauuaau guuucuuaaa uggaa                                          25


<210>  172
<211>  25
<212>  RNA
<213>  Artificial Sequence

<220>
<223>  Description of Artificial Sequence: Synthetic oligonucleotide

<400>  172
caacguguau agugccuaaa auugu                                          25


<210>  173
<211>  25
<212>  RNA
<213>  Artificial Sequence

<220>
<223>  Description of Artificial Sequence: Synthetic oligonucleotide

<400>  173
cauauccuug gcuacuaaca ucugg                                          25


<210>  174
<211>  25
<212>  RNA
<213>  Artificial Sequence

<220>
<223>  Description of Artificial Sequence: Synthetic oligonucleotide

<400>  174
uacuaacauc uggagacugu gagcu                                          25


<210>  175
<211>  25
<212>  RNA
<213>  Artificial Sequence

<220>
<223>  Description of Artificial Sequence: Synthetic oligonucleotide

<400>  175
cauaaguugu gugcuuuuua uuaau                                          25


<210>  176
<211>  25
<212>  RNA
<213>  Artificial Sequence

<220>
<223>  Description of Artificial Sequence: Synthetic oligonucleotide

<400>  176
gcaucauuuu ggcucuucuu acauu                                          25


<210>  177
<211>  25
<212>  RNA
<213>  Artificial Sequence

<220>
<223>  Description of Artificial Sequence: Synthetic oligonucleotide

<400>  177
gcucuucuua cauuuguaaa aaugu                                          25


<210>  178
<211>  25
<212>  RNA
<213>  Artificial Sequence

<220>
<223>  Description of Artificial Sequence: Synthetic oligonucleotide

<400>  178
agauuagguc aucuuaauuc auauu                                           25


<210>  179
<211>  25
<212>  RNA
<213>  Artificial Sequence

<220>
<223>  Description of Artificial Sequence: Synthetic oligonucleotide

<400>  179
auggaauuga aagaacuaau cauga                                           25


<210>  180
<211>  25
<212>  RNA
<213>  Artificial Sequence

<220>
<223>  Description of Artificial Sequence: Synthetic oligonucleotide

<400>  180
cacacucauu ccuucugcuc uuggg                                           25


<210>  181
<211>  25
<212>  RNA
<213>  Artificial Sequence

<220>
<223>  Description of Artificial Sequence: Synthetic oligonucleotide

<400>  181
uguagaggua accaguagcu uugag                                           25


<210>  182
<211>  25
<212>  RNA
<213>  Artificial Sequence

<220>
<223>  Description of Artificial Sequence: Synthetic oligonucleotide

<400>  182
caaccacaug ccacguaaua uuuca                                           25


<210>  183
<211>  25
<212>  RNA
<213>  Artificial Sequence

<220>

<223>  Description of Artificial Sequence: Synthetic oligonucleotide

<400>  183
ucggaaacaa guuauucucu ucacu                                          25


<210>  184
<211>  25
<212>  RNA
<213>  Artificial Sequence

<220>
<223>  Description of Artificial Sequence: Synthetic oligonucleotide

<400>  184
acucccaaua acuaaugcua agaaa                                          25


<210>  185
<211>  25
<212>  RNA
<213>  Artificial Sequence

<220>
<223>  Description of Artificial Sequence: Synthetic oligonucleotide

<400>  185
aaugcuaaga aaugcugaaa aucaa                                          25


<210>  186
<211>  25
<212>  RNA
<213>  Artificial Sequence

<220>
<223>  Description of Artificial Sequence: Synthetic oligonucleotide

<400>  186
gucuuucucu aaauaugauu acuuu                                          25


<210>  187
<211>  25
<212>  RNA
<213>  Artificial Sequence

<220>
<223>  Description of Artificial Sequence: Synthetic oligonucleotide

<400>  187
ugaauuucag gcauuuuguu cuaca                                          25


<210>  188
<211>  25
<212>  RNA
<213>  Artificial Sequence

<220>
<223>  Description of Artificial Sequence: Synthetic oligonucleotide

<400>  188
cgauucccuc ucacccggga cucuc                                          25


<210>  189
<211>  25
<212>  RNA
<213>  Artificial Sequence

<220>
<223>  Description of Artificial Sequence: Synthetic oligonucleotide

<400>  189
aggaaaguga accuuuaaag uaaag                                          25


<210>  190
<211>  25
<212>  RNA
<213>  Artificial Sequence

<220>
<223>  Description of Artificial Sequence: Synthetic oligonucleotide

<400>  190
gaggcugcau gcucuggaag ccugg                                          25


<210>  191
<211>  25
<212>  RNA
<213>  Artificial Sequence

<220>
<223>  Description of Artificial Sequence: Synthetic oligonucleotide

<400>  191
ucucugaaca gaaaacaaaa gagag                                          25


<210>  192
<211>  25
<212>  RNA
<213>  Artificial Sequence

<220>
<223>  Description of Artificial Sequence: Synthetic oligonucleotide

<400>  192
aacuuggcug uaaucaguua ugccg                                          25


<210>  193
<211>  25
<212>  RNA
<213>  Artificial Sequence

<220>
<223>  Description of Artificial Sequence: Synthetic oligonucleotide

<400>  193
agaagccaaa auuaaaagaa gucca                                          25


<210>  194
<211>  25
<212>  RNA
<213>  Artificial Sequence

<220>
<223>  Description of Artificial Sequence: Synthetic oligonucleotide

<400>  194
auuaaaagaa guccagguga gguua                                          25


<210>  195
<211>  25
<212>  RNA
<213>  Artificial Sequence

<220>
<223>  Description of Artificial Sequence: Synthetic oligonucleotide

<400>  195
gaauccggau uaucgggaag aggac                                          25


<210>  196
<211>  25
<212>  RNA
<213>  Artificial Sequence

<220>
<223>  Description of Artificial Sequence: Synthetic oligonucleotide

<400>  196
aaugugacau caaagcaagu auugu                                          25


<210>  197
<211>  25
<212>  RNA
<213>  Artificial Sequence

<220>
<223>  Description of Artificial Sequence: Synthetic oligonucleotide

<400>  197
caucaaagca aguauuguag cacuc                                          25


<210>  198
<211>  25
<212>  RNA
<213>  Artificial Sequence

<220>
<223>  Description of Artificial Sequence: Synthetic oligonucleotide

<400>  198

agagagagaa aacaaaacca caaau                                          25

<210>  199
<211>  25
<212>  RNA
<213>  Artificial Sequence

<220>
<223>  Description of Artificial Sequence: Synthetic oligonucleotide

<400>  199
ucgcuguagu auuuaagccc auaca                                          25

<210>  200
<211>  25
<212>  RNA
<213>  Artificial Sequence

<220>
<223>  Description of Artificial Sequence: Synthetic oligonucleotide

<400>  200
cgcuguagua uuuaagccca uacag                                          25

<210>  201
<211>  25
<212>  RNA
<213>  Artificial Sequence

<220>
<223>  Description of Artificial Sequence: Synthetic oligonucleotide

<400>  201
auuuaagccc auacagaaac cuucc                                          25

<210>  202
<211>  25
<212>  RNA
<213>  Artificial Sequence

<220>
<223>  Description of Artificial Sequence: Synthetic oligonucleotide

<400>  202
auuaaaauaa acaugguaua ccuac                                          25

<210>  203
<211>  25
<212>  RNA
<213>  Artificial Sequence

<220>
<223>  Description of Artificial Sequence: Synthetic oligonucleotide

<400>  203
cuguucugau cggccaguuu ucgga                                          25

<210>  204
<211>  25
<212>  RNA
<213>  Artificial Sequence

<220>
<223>  Description of Artificial Sequence: Synthetic oligonucleotide

<400>  204
aaauaauuug aacuuuggaa caggg                                          25


<210>  205
<211>  25
<212>  RNA
<213>  Artificial Sequence

<220>
<223>  Description of Artificial Sequence: Synthetic oligonucleotide

<400>  205
ugcgaccuua auuuaacuuu ccagu                                          25


<210>  206
<211>  25
<212>  RNA
<213>  Artificial Sequence

<220>
<223>  Description of Artificial Sequence: Synthetic oligonucleotide

<400>  206
cugagaaagc uaaaguuugg uuuug                                          25


<210>  207
<211>  25
<212>  RNA
<213>  Artificial Sequence

<220>
<223>  Description of Artificial Sequence: Synthetic oligonucleotide

<400>  207
aguaaagaug cuacuuccca cugua                                          25


<210>  208
<211>  25
<212>  RNA
<213>  Artificial Sequence

<220>
<223>  Description of Artificial Sequence: Synthetic oligonucleotide

<400>  208
cugcuuaauu gcugauacca uauga                                          25

<210>  209
<211>  25
<212>  RNA
<213>  Artificial Sequence

<220>
<223>  Description of Artificial Sequence: Synthetic oligonucleotide

<400>  209
uaccauauga augaaacaug ggcug                                          25


<210>  210
<211>  25
<212>  RNA
<213>  Artificial Sequence

<220>
<223>  Description of Artificial Sequence: Synthetic oligonucleotide

<400>  210
aacuuucuua uccaacuuuu ucaua                                          25


<210>  211
<211>  25
<212>  RNA
<213>  Artificial Sequence

<220>
<223>  Description of Artificial Sequence: Synthetic oligonucleotide

<400>  211
ccuugcauga caucaugagg ccgga                                          25


<210>  212
<211>  25
<212>  RNA
<213>  Artificial Sequence

<220>
<223>  Description of Artificial Sequence: Synthetic oligonucleotide

<400>  212
ugaauuugua uaugacugca uuugu                                          25


<210>  213
<211>  25
<212>  RNA
<213>  Artificial Sequence

<220>
<223>  Description of Artificial Sequence: Synthetic oligonucleotide

<400>  213
gaauccuagu agaauguuua cuacc                                          25

<210>  214
<211>  25
<212>  RNA
<213>  Artificial Sequence

<220>
<223>  Description of Artificial Sequence: Synthetic oligonucleotide

<400>  214
gaaagggaag aauuuuuuga ugaaa                                          25


<210>  215
<211>  25
<212>  RNA
<213>  Artificial Sequence

<220>
<223>  Description of Artificial Sequence: Synthetic oligonucleotide

<400>  215
uaucggcaug ccagugugug aauuu                                          25


<210>  216
<211>  25
<212>  RNA
<213>  Artificial Sequence

<220>
<223>  Description of Artificial Sequence: Synthetic oligonucleotide

<400>  216
caccucauag uagagcaaug uaugu                                          25


<210>  217
<211>  25
<212>  RNA
<213>  Artificial Sequence

<220>
<223>  Description of Artificial Sequence: Synthetic oligonucleotide

<400>  217
ccagaauugc caaagcacau auaua                                          25


<210>  218
<211>  25
<212>  RNA
<213>  Artificial Sequence

<220>
<223>  Description of Artificial Sequence: Synthetic oligonucleotide

<400>  218
uggugaucug gguaauaguu ucucc                                          25


<210>  219

<211>  25
<212>  RNA
<213>  Artificial Sequence

<220>
<223>  Description of Artificial Sequence: Synthetic oligonucleotide

<400>  219
gaucugggua auaguuucuc caaau                                          25


<210>  220
<211>  25
<212>  RNA
<213>  Artificial Sequence

<220>
<223>  Description of Artificial Sequence: Synthetic oligonucleotide

<400>  220
ggaugugaug aauacuuccu agaaa                                          25


<210>  221
<211>  25
<212>  RNA
<213>  Artificial Sequence

<220>
<223>  Description of Artificial Sequence: Synthetic oligonucleotide

<400>  221
cauuuccaca gcuacaccau auaug                                          25


<210>  222
<211>  25
<212>  RNA
<213>  Artificial Sequence

<220>
<223>  Description of Artificial Sequence: Synthetic oligonucleotide

<400>  222
acagcuacac cauauaugaa uggag                                          25


<210>  223
<211>  25
<212>  RNA
<213>  Artificial Sequence

<220>
<223>  Description of Artificial Sequence: Synthetic oligonucleotide

<400>  223
ugcaguucuu acacgagaag aagau                                          25


<210>  224
<211>  25

<212>  RNA
<213>  Artificial Sequence

<220>
<223>  Description of Artificial Sequence: Synthetic oligonucleotide

<400>  224
acgagaagaa gaucauuuac aggga                                           25


<210>  225
<211>  25
<212>  RNA
<213>  Artificial Sequence

<220>
<223>  Description of Artificial Sequence: Synthetic oligonucleotide

<400>  225
cgagaagaag aucauuuaca gggac                                           25


<210>  226
<211>  25
<212>  RNA
<213>  Artificial Sequence

<220>
<223>  Description of Artificial Sequence: Synthetic oligonucleotide

<400>  226
aagaagauca uuuacaggga ccuga                                           25


<210>  227
<211>  25
<212>  RNA
<213>  Artificial Sequence

<220>
<223>  Description of Artificial Sequence: Synthetic oligonucleotide

<400>  227
agaggaagag guguuugacu gcauc                                           25


<210>  228
<211>  25
<212>  RNA
<213>  Artificial Sequence

<220>
<223>  Description of Artificial Sequence: Synthetic oligonucleotide

<400>  228
gaggaagagg uguuugacug caucg                                           25


<210>  229
<211>  25
<212>  RNA

<213>  Artificial Sequence

<220>
<223>  Description of Artificial Sequence: Synthetic oligonucleotide

<400>  229
cuacuuugag ggcgaguuca caggg                                          25


<210>  230
<211>  25
<212>  RNA
<213>  Artificial Sequence

<220>
<223>  Description of Artificial Sequence: Synthetic oligonucleotide

<400>  230
agggcaucuc cuggcaccuc ugucc                                          25


<210>  231
<211>  25
<212>  RNA
<213>  Artificial Sequence

<220>
<223>  Description of Artificial Sequence: Synthetic oligonucleotide

<400>  231
ggagugauau gguuugucuu uuuaa                                          25


<210>  232
<211>  25
<212>  RNA
<213>  Artificial Sequence

<220>
<223>  Description of Artificial Sequence: Synthetic oligonucleotide

<400>  232
gagugauaug guuugucuuu uuaag                                          25


<210>  233
<211>  25
<212>  RNA
<213>  Artificial Sequence

<220>
<223>  Description of Artificial Sequence: Synthetic oligonucleotide

<400>  233
ugcaguaaag auccuaaagg uuguc                                          25


<210>  234
<211>  25
<212>  RNA
<213>  Artificial Sequence

<220>
<223>  Description of Artificial Sequence: Synthetic oligonucleotide

<400>  234
aguaaagauc cuaaagguug ucgac                                           25


<210>  235
<211>  25
<212>  RNA
<213>  Artificial Sequence

<220>
<223>  Description of Artificial Sequence: Synthetic oligonucleotide

<400>  235
ugacaaagga caaccuggca auugu                                           25


<210>  236
<211>  25
<212>  RNA
<213>  Artificial Sequence

<220>
<223>  Description of Artificial Sequence: Synthetic oligonucleotide

<400>  236
gcaauuguga cccaguggug cgagg                                           25


<210>  237
<211>  25
<212>  RNA
<213>  Artificial Sequence

<220>
<223>  Description of Artificial Sequence: Synthetic oligonucleotide

<400>  237
aacaucaucc auagagacau gaaau                                           25


<210>  238
<211>  25
<212>  RNA
<213>  Artificial Sequence

<220>
<223>  Description of Artificial Sequence: Synthetic oligonucleotide

<400>  238
ugaaauccaa caauauauuu cucca                                           25


<210>  239
<211>  25
<212>  RNA
<213>  Artificial Sequence

<220>
<223>  Description of Artificial Sequence: Synthetic oligonucleotide

<400>  239
aacaauauau uucuccauga aggcu                                          25


<210>  240
<211>  25
<212>  RNA
<213>  Artificial Sequence

<220>
<223>  Description of Artificial Sequence: Synthetic oligonucleotide

<400>  240
aacaguaaag ucacgcugga guggu                                          25


<210>  241
<211>  25
<212>  RNA
<213>  Artificial Sequence

<220>
<223>  Description of Artificial Sequence: Synthetic oligonucleotide

<400>  241
ugugaagaaa guaaaggaag agagg                                          25


<210>  242
<211>  25
<212>  RNA
<213>  Artificial Sequence

<220>
<223>  Description of Artificial Sequence: Synthetic oligonucleotide

<400>  242
cuuccgagcc auccuugcau cgggc                                          25


<210>  243
<211>  25
<212>  RNA
<213>  Artificial Sequence

<220>
<223>  Description of Artificial Sequence: Synthetic oligonucleotide

<400>  243
aauggagguu gaauauccua cugug                                          25


<210>  244
<211>  25
<212>  RNA
<213>  Artificial Sequence

<220>

<223>  Description of Artificial Sequence: Synthetic oligonucleotide

<400>  244
ggagguugaa uauccuacug uguaa                                          25


<210>  245
<211>  25
<212>  RNA
<213>  Artificial Sequence

<220>
<223>  Description of Artificial Sequence: Synthetic oligonucleotide

<400>  245
auuuugaguu uucccuugua gugua                                          25


<210>  246
<211>  25
<212>  RNA
<213>  Artificial Sequence

<220>
<223>  Description of Artificial Sequence: Synthetic oligonucleotide

<400>  246
uauccuguuu guucuuuguu gauug                                          25


<210>  247
<211>  25
<212>  RNA
<213>  Artificial Sequence

<220>
<223>  Description of Artificial Sequence: Synthetic oligonucleotide

<400>  247
ccuguuuguu cuuuguugau ugaaa                                          25


<210>  248
<211>  25
<212>  RNA
<213>  Artificial Sequence

<220>
<223>  Description of Artificial Sequence: Synthetic oligonucleotide

<400>  248
   cucuacagcc uucuuuuucu uccau                                           25


<210>  249
<211>  25
<212>  RNA
<213>  Artificial Sequence

<220>
<223>  Description of Artificial Sequence: Synthetic oligonucleotide

<400>  249
ucuuccauag cuaaucuucc uucua                                          25


<210>  250
<211>  25
<212>  RNA
<213>  Artificial Sequence

<220>
<223>  Description of Artificial Sequence: Synthetic oligonucleotide

<400>  250
auaaucuucc uguugaaugc uucau                                          25


<210>  251
<211>  25
<212>  RNA
<213>  Artificial Sequence

<220>
<223>  Description of Artificial Sequence: Synthetic oligonucleotide

<400>  251
uaaucuuccu guugaaugcu ucaug                                          25


<210>  252
<211>  25
<212>  RNA
<213>  Artificial Sequence

<220>
<223>  Description of Artificial Sequence: Synthetic oligonucleotide

<400>  252
cuucaugacu ugaauucuac uuuga                                          25


<210>  253
<211>  25
<212>  RNA
<213>  Artificial Sequence

<220>
<223>  Description of Artificial Sequence: Synthetic oligonucleotide

<400>  253
aagagggaga gaagcaacua cagac                                          25


<210>  254
<211>  25
<212>  RNA
<213>  Artificial Sequence

<220>
<223>  Description of Artificial Sequence: Synthetic oligonucleotide

<400>  254
cgucuccuac cagaccaagg ucaac                                          25


<210>  255
<211>  25
<212>  RNA
<213>  Artificial Sequence

<220>
<223>  Description of Artificial Sequence: Synthetic oligonucleotide

<400>  255
gaucaaucgg cccgacuauc ucgac                                          25


<210>  256
<211>  25
<212>  RNA
<213>  Artificial Sequence

<220>
<223>  Description of Artificial Sequence: Synthetic oligonucleotide

<400>  256
ggacgaacau ccaaccuucc caaac                                          25


<210>  257
<211>  25
<212>  RNA
<213>  Artificial Sequence

<220>
<223>  Description of Artificial Sequence: Synthetic oligonucleotide

<400>  257
agggucggaa cccaagcuua gaacu                                          25


<210>  258
<211>  25
<212>  RNA
<213>  Artificial Sequence

<220>
<223>  Description of Artificial Sequence: Synthetic oligonucleotide

<400>  258
ucggaaccca agcuuagaac uuuaa                                          25


<210>  259
<211>  25
<212>  RNA
<213>  Artificial Sequence

<220>
<223>  Description of Artificial Sequence: Synthetic oligonucleotide

<400>  259

acccaagcuu agaacuuuaa gcaac 25

<210> 260
<211> 25
<212> RNA
<213> Artificial Sequence

<220>
<223> Description of Artificial Sequence: Synthetic oligonucleotide

<400> 260
gaacuuuaag caacaagacc accac 25

<210> 261
<211> 25
<212> RNA
<213> Artificial Sequence

<220>
<223> Description of Artificial Sequence: Synthetic oligonucleotide

<400> 261
acuauucagu ggcgagaaau aaagu 25

<210> 262
<211> 25
<212> RNA
<213> Artificial Sequence

<220>
<223> Description of Artificial Sequence: Synthetic oligonucleotide

<400> 262
cuauucagug gcgagaaaua aaguu 25

<210> 263
<211> 25
<212> RNA
<213> Artificial Sequence

<220>
<223> Description of Artificial Sequence: Synthetic oligonucleotide

<400> 263
aaacacagau aacaggaaau gaucc 25

<210> 264
<211> 25
<212> RNA
<213> Artificial Sequence

<220>
<223> Description of Artificial Sequence: Synthetic oligonucleotide

<400> 264
cuuaagaaaa gagaagaaau gaaac 25

<210>  265
<211>  25
<212>  RNA
<213>  Artificial Sequence

<220>
<223>  Description of Artificial Sequence: Synthetic oligonucleotide

<400>  265
cugaaggagu guguuuccau ccucc                                          25


<210>  266
<211>  25
<212>  RNA
<213>  Artificial Sequence

<220>
<223>  Description of Artificial Sequence: Synthetic oligonucleotide

<400>  266
ucaccgcggg acugaaaauc uuuga                                          25


<210>  267
<211>  25
<212>  RNA
<213>  Artificial Sequence

<220>
<223>  Description of Artificial Sequence: Synthetic oligonucleotide

<400>  267
agcagaaaua agcgugccgu ucagg                                          25


<210>  268
<211>  25
<212>  RNA
<213>  Artificial Sequence

<220>
<223>  Description of Artificial Sequence: Synthetic oligonucleotide

<400>  268
aagcgugccg uucaggucc agaag                                          25


<210>  269
<211>  25
<212>  RNA
<213>  Artificial Sequence

<220>
<223>  Description of Artificial Sequence: Synthetic oligonucleotide

<400>  269
agaaacaguc acucaagacu gcuug                                          25

<210>  270
<211>  25
<212>  RNA
<213>  Artificial Sequence

<220>
<223>  Description of Artificial Sequence: Synthetic oligonucleotide

<400>  270
agaggaagaa gguccauguc uuugg                                          25


<210>  271
<211>  25
<212>  RNA
<213>  Artificial Sequence

<220>
<223>  Description of Artificial Sequence: Synthetic oligonucleotide

<400>  271
uguauucaaa auaugccuga aacac                                          25


<210>  272
<211>  25
<212>  RNA
<213>  Artificial Sequence

<220>
<223>  Description of Artificial Sequence: Synthetic oligonucleotide

<400>  272
auuuuccucc cuuucucugu accuc                                          25


<210>  273
<211>  25
<212>  RNA
<213>  Artificial Sequence

<220>
<223>  Description of Artificial Sequence: Synthetic oligonucleotide

<400>  273
caaagaaaga uagagcaaga caaga                                          25


<210>  274
<211>  25
<212>  RNA
<213>  Artificial Sequence

<220>
<223>  Description of Artificial Sequence: Synthetic oligonucleotide

<400>  274
aagaaagaua gagcaagaca agaaa                                          25

<210>  275
<211>  25
<212>  RNA
<213>  Artificial Sequence

<220>
<223>  Description of Artificial Sequence: Synthetic oligonucleotide

<400>  275
gaaagcauuu guuuguacaa gaucc                                          25


<210>  276
<211>  25
<212>  RNA
<213>  Artificial Sequence

<220>
<223>  Description of Artificial Sequence: Synthetic oligonucleotide

<400>  276
ugaguuaaac gaacguacuu gcaga                                          25


<210>  277
<211>  25
<212>  RNA
<213>  Artificial Sequence

<220>
<223>  Description of Artificial Sequence: Synthetic oligonucleotide

<400>  277
acugauacag aacgaucgau acaga                                          25


<210>  278
<211>  25
<212>  RNA
<213>  Artificial Sequence

<220>
<223>  Description of Artificial Sequence: Synthetic oligonucleotide

<400>  278
auauuauaua uauauaaaaa uaaau                                          25


<210>  279
<211>  25
<212>  RNA
<213>  Artificial Sequence

<220>
<223>  Description of Artificial Sequence: Synthetic oligonucleotide

<400>  279
auuauauaua uauaaaaaua aauau                                          25


<210>  280

<211>  25
<212>  RNA
<213>  Artificial Sequence

<220>
<223>  Description of Artificial Sequence: Synthetic oligonucleotide

<400>  280
ucacuggaug uauuugacug cugug                                          25


<210>  281
<211>  25
<212>  RNA
<213>  Artificial Sequence

<220>
<223>  Description of Artificial Sequence: Synthetic oligonucleotide

<400>  281
cagggaagag gaggagauga gagac                                          25


<210>  282
<211>  25
<212>  RNA
<213>  Artificial Sequence

<220>
<223>  Description of Artificial Sequence: Synthetic oligonucleotide

<400>  282
   augaucuuuu uuuuguccca cuugg                                           25


<210>  283
<211>  27
<212>  RNA
<213>  Artificial Sequence

<220>
<223>  Description of Artificial Sequence: Synthetic oligonucleotide

<400>  283
ggccgugaac uccucaucaa aauaccu                                        27


<210>  284
<211>  27
<212>  RNA
<213>  Artificial Sequence

<220>
<223>  Description of Artificial Sequence: Synthetic oligonucleotide

<400>  284
uggugugaug gugaucaucu gggccgu                                        27


<210>  285
<211>  27

<212>  RNA
<213>  Artificial Sequence

<220>
<223>  Description of Artificial Sequence: Synthetic oligonucleotide

<400>  285
aaacccgcag gauaguuuuc uucccua                                          27


<210>  286
<211>  27
<212>  RNA
<213>  Artificial Sequence

<220>
<223>  Description of Artificial Sequence: Synthetic oligonucleotide

<400>  286
caaacuggau gaaauaaauu aaaaccc                                          27


<210>  287
<211>  27
<212>  RNA
<213>  Artificial Sequence

<220>
<223>  Description of Artificial Sequence: Synthetic oligonucleotide

<400>  287
agaaguccuu aacauuuccc uacguga                                          27


<210>  288
<211>  27
<212>  RNA
<213>  Artificial Sequence

<220>
<223>  Description of Artificial Sequence: Synthetic oligonucleotide

<400>  288
   cucugiccca cuggguaaac ccuggcc                                          27


<210>  289
<211>  27
<212>  RNA
<213>  Artificial Sequence

<220>
<223>  Description of Artificial Sequence: Synthetic oligonucleotide

<400>  289
cacaauaaca aauuuaaacc uugcucc                                          27


<210>  290
<211>  27
<212>  RNA

<213>  Artificial Sequence

<220>
<223>  Description of Artificial Sequence: Synthetic oligonucleotide

<400>  290
aaaugcauuu gaacaacaua auacaca                                          27


<210>  291
<211>  27
<212>  RNA
<213>  Artificial Sequence

<220>
<223>  Description of Artificial Sequence: Synthetic oligonucleotide

<400>  291
ggcuuuccug ucacaaagau uaaaaac                                          27


<210>  292
<211>  27
<212>  RNA
<213>  Artificial Sequence

<220>
<223>  Description of Artificial Sequence: Synthetic oligonucleotide

<400>  292
agggcuuucc ugucacaaag auuaaaa                                          27


<210>  293
<211>  27
<212>  RNA
<213>  Artificial Sequence

<220>
<223>  Description of Artificial Sequence: Synthetic oligonucleotide

<400>  293
ggccaugcug ggagacauaa gcagcag                                          27


<210>  294
<211>  27
<212>  RNA
<213>  Artificial Sequence

<220>
<223>  Description of Artificial Sequence: Synthetic oligonucleotide

<400>  294
ucagggagaa gcuucugaaa cacuucu                                          27


<210>  295
<211>  27
<212>  RNA
<213>  Artificial Sequence

<220>
<223>  Description of Artificial Sequence: Synthetic oligonucleotide

<400>  295
aagggcuucu uccuuauuga uggucag                                          27


<210>  296
<211>  27
<212>  RNA
<213>  Artificial Sequence

<220>
<223>  Description of Artificial Sequence: Synthetic oligonucleotide

<400>  296
ugaagggcuu cuuccuuauu gaugguc                                          27


<210>  297
<211>  27
<212>  RNA
<213>  Artificial Sequence

<220>
<223>  Description of Artificial Sequence: Synthetic oligonucleotide

<400>  297
cgcugaaggg cuucuuccuu auugaug                                          27


<210>  298
<211>  27
<212>  RNA
<213>  Artificial Sequence

<220>
<223>  Description of Artificial Sequence: Synthetic oligonucleotide

<400>  298
gccgcugaag ggcuucuucc uuauuga                                          27


<210>  299
<211>  27
<212>  RNA
<213>  Artificial Sequence

<220>
<223>  Description of Artificial Sequence: Synthetic oligonucleotide

<400>  299
acuggccgcu gaagggcuuc uuccuua                                          27


<210>  300
<211>  27
<212>  RNA
<213>  Artificial Sequence

<220>
<223>  Description of Artificial Sequence: Synthetic oligonucleotide

<400>  300
cggagcuuuu caccuuuagu uaugcuu                                          27


<210>  301
<211>  27
<212>  RNA
<213>  Artificial Sequence

<220>
<223>  Description of Artificial Sequence: Synthetic oligonucleotide

<400>  301
ccggagcuuu ucaccuuuag uuaugcu                                          27


<210>  302
<211>  27
<212>  RNA
<213>  Artificial Sequence

<220>
<223>  Description of Artificial Sequence: Synthetic oligonucleotide

<400>  302
cagacuguug acuggcguga uguaguu                                          27


<210>  303
<211>  27
<212>  RNA
<213>  Artificial Sequence

<220>
<223>  Description of Artificial Sequence: Synthetic oligonucleotide

<400>  303
cuuuugaacu cugcuuaaau ccagugg                                          27


<210>  304
<211>  27
<212>  RNA
<213>  Artificial Sequence

<220>
<223>  Description of Artificial Sequence: Synthetic oligonucleotide

<400>  304
gcuuuugaac ucugcuuaaa uccagug                                          27


<210>  305
<211>  27
<212>  RNA
<213>  Artificial Sequence

<220>

<223>  Description of Artificial Sequence: Synthetic oligonucleotide

<400>  305
agggcuuuug aacucugcuu aaaucca                                          27


<210>  306
<211>  27
<212>  RNA
<213>  Artificial Sequence

<220>
<223>  Description of Artificial Sequence: Synthetic oligonucleotide

<400>  306
aagggcuuuu gaacucugcu uaaaucc                                          27


<210>  307
<211>  27
<212>  RNA
<213>  Artificial Sequence

<220>
<223>  Description of Artificial Sequence: Synthetic oligonucleotide

<400>  307
ugaagggcuu uugaacucug cuuaaau                                          27


<210>  308
<211>  27
<212>  RNA
<213>  Artificial Sequence

<220>
<223>  Description of Artificial Sequence: Synthetic oligonucleotide

<400>  308
cugaagggcu uuugaacucu gcuuaaa                                          27


<210>  309
<211>  27
<212>  RNA
<213>  Artificial Sequence

<220>
<223>  Description of Artificial Sequence: Synthetic oligonucleotide

<400>  309
cgcugaaggg cuuuugaacu cugcuua                                          27


<210>  310
<211>  27
<212>  RNA
<213>  Artificial Sequence

<220>
<223>  Description of Artificial Sequence: Synthetic oligonucleotide

<400>  310
cgagcggcuu cacucagacc cugaggc                                          27


<210>  311
<211>  27
<212>  RNA
<213>  Artificial Sequence

<220>
<223>  Description of Artificial Sequence: Synthetic oligonucleotide

<400>  311
uugacuggcg ugauguaguu gcuuggg                                          27


<210>  312
<211>  27
<212>  RNA
<213>  Artificial Sequence

<220>
<223>  Description of Artificial Sequence: Synthetic oligonucleotide

<400>  312
cacgcaccaa gaagcugcca uugaucc                                          27


<210>  313
<211>  27
<212>  RNA
<213>  Artificial Sequence

<220>
<223>  Description of Artificial Sequence: Synthetic oligonucleotide

<400>  313
cugaugaauc caaugugggc uggaauc                                          27


<210>  314
<211>  27
<212>  RNA
<213>  Artificial Sequence

<220>
<223>  Description of Artificial Sequence: Synthetic oligonucleotide

<400>  314
cuuagguauu ccacauucuc agcugug                                          27


<210>  315
<211>  27
<212>  RNA
<213>  Artificial Sequence

<220>
<223>  Description of Artificial Sequence: Synthetic oligonucleotide

<400>  315
ugagccucaa auuaggagau acguuuu                                          27


<210>  316
<211>  27
<212>  RNA
<213>  Artificial Sequence

<220>
<223>  Description of Artificial Sequence: Synthetic oligonucleotide

<400>  316
ccaauuguag agaaauuauu uuaggaa                                          27


<210>  317
<211>  27
<212>  RNA
<213>  Artificial Sequence

<220>
<223>  Description of Artificial Sequence: Synthetic oligonucleotide

<400>  317
ggcuccuaac uagcugaauc uuccaau                                          27


<210>  318
<211>  27
<212>  RNA
<213>  Artificial Sequence

<220>
<223>  Description of Artificial Sequence: Synthetic oligonucleotide

<400>  318
guggauauug acuaggagag uuuaaaa                                          27


<210>  319
<211>  27
<212>  RNA
<213>  Artificial Sequence

<220>
<223>  Description of Artificial Sequence: Synthetic oligonucleotide

<400>  319
ugugacugaa cauaacugua ggcugaa                                          27


<210>  320
<211>  27
<212>  RNA
<213>  Artificial Sequence

<220>
<223>  Description of Artificial Sequence: Synthetic oligonucleotide

<400>  320

guaugugugu gugacugaac auaacug                                          27

<210>  321
<211>  27
<212>  RNA
<213>  Artificial Sequence

<220>
<223>  Description of Artificial Sequence: Synthetic oligonucleotide

<400>  321
aagcaaaagg aacauuuugu augugug                                          27

<210>  322
<211>  27
<212>  RNA
<213>  Artificial Sequence

<220>
<223>  Description of Artificial Sequence: Synthetic oligonucleotide

<400>  322
aaaaauuacu uuaaaagcaa aaggaac                                          27

<210>  323
<211>  27
<212>  RNA
<213>  Artificial Sequence

<220>
<223>  Description of Artificial Sequence: Synthetic oligonucleotide

<400>  323
ccauugacug uugcuucaca ggucaga                                          27

<210>  324
<211>  27
<212>  RNA
<213>  Artificial Sequence

<220>
<223>  Description of Artificial Sequence: Synthetic oligonucleotide

<400>  324
auugguuugu cgauguguga gauaguu                                          27

<210>  325
<211>  27
<212>  RNA
<213>  Artificial Sequence

<220>
<223>  Description of Artificial Sequence: Synthetic oligonucleotide

<400>  325
agugguagca guacaauuga ggacaag                                          27

<210>  326
<211>  27
<212>  RNA
<213>  Artificial Sequence

<220>
<223>  Description of Artificial Sequence: Synthetic oligonucleotide

<400>  326
uaagaccgcu ugccagcuac gguuuca                                          27


<210>  327
<211>  27
<212>  RNA
<213>  Artificial Sequence

<220>
<223>  Description of Artificial Sequence: Synthetic oligonucleotide

<400>  327
agccgguaag accgcuugcc agcuacg                                          27


<210>  328
<211>  27
<212>  RNA
<213>  Artificial Sequence

<220>
<223>  Description of Artificial Sequence: Synthetic oligonucleotide

<400>  328
gguaacccau cuuuuaacca uacaacu                                          27


<210>  329
<211>  27
<212>  RNA
<213>  Artificial Sequence

<220>
<223>  Description of Artificial Sequence: Synthetic oligonucleotide

<400>  329
ucgcagguaa cccaucuuuu aaccaua                                          27


<210>  330
<211>  27
<212>  RNA
<213>  Artificial Sequence

<220>
<223>  Description of Artificial Sequence: Synthetic oligonucleotide

<400>  330
cucagcaaga uuguauaauu cccugca                                          27

<210>  331
<211>  27
<212>  RNA
<213>  Artificial Sequence

<220>
<223>  Description of Artificial Sequence: Synthetic oligonucleotide

<400>  331
acuguuuuau gcucagcaag auuguau                                          27


<210>  332
<211>  27
<212>  RNA
<213>  Artificial Sequence

<220>
<223>  Description of Artificial Sequence: Synthetic oligonucleotide

<400>  332
aggauaaagg acucuucauu auuggaa                                          27


<210>  333
<211>  27
<212>  RNA
<213>  Artificial Sequence

<220>
<223>  Description of Artificial Sequence: Synthetic oligonucleotide

<400>  333
caacuugcgu uggaacaucc aaagugu                                          27


<210>  334
<211>  27
<212>  RNA
<213>  Artificial Sequence

<220>
<223>  Description of Artificial Sequence: Synthetic oligonucleotide

<400>  334
augguuucag uuuaguggaa gcauuua                                          27


<210>  335
<211>  27
<212>  RNA
<213>  Artificial Sequence

<220>
<223>  Description of Artificial Sequence: Synthetic oligonucleotide

<400>  335
auauuuaaca aagucaaacu uucucac                                          27

<210>  336
<211>  27
<212>  RNA
<213>  Artificial Sequence

<220>
<223>  Description of Artificial Sequence: Synthetic oligonucleotide

<400>  336
aacuuuuaau auacaguagu ucuuuuc                                          27


<210>  337
<211>  27
<212>  RNA
<213>  Artificial Sequence

<220>
<223>  Description of Artificial Sequence: Synthetic oligonucleotide

<400>  337
guuaagucaa aacccguauu ucuaaag                                          27


<210>  338
<211>  27
<212>  RNA
<213>  Artificial Sequence

<220>
<223>  Description of Artificial Sequence: Synthetic oligonucleotide

<400>  338
gugcugaguu ugcuguaccc auguuga                                          27


<210>  339
<211>  27
<212>  RNA
<213>  Artificial Sequence

<220>
<223>  Description of Artificial Sequence: Synthetic oligonucleotide

<400>  339
ugaggucagc aucuucugug ucuuuac                                          27


<210>  340
<211>  27
<212>  RNA
<213>  Artificial Sequence

<220>
<223>  Description of Artificial Sequence: Synthetic oligonucleotide

<400>  340
gcgaucugaa uaaaccuccc uacuagc                                          27


<210>  341

<211>  27
<212>  RNA
<213>  Artificial Sequence

<220>
<223>  Description of Artificial Sequence: Synthetic oligonucleotide

<400>  341
aucccagaac ccugcugcag aaggcca                                          27


<210>  342
<211>  27
<212>  RNA
<213>  Artificial Sequence

<220>
<223>  Description of Artificial Sequence: Synthetic oligonucleotide

<400>  342
cauaauuucc aauauguacc agaccuu                                          27


<210>  343
<211>  27
<212>  RNA
<213>  Artificial Sequence

<220>
<223>  Description of Artificial Sequence: Synthetic oligonucleotide

<400>  343
cuagugcuuc caucggaagg acuaggu                                          27


<210>  344
<211>  27
<212>  RNA
<213>  Artificial Sequence

<220>
<223>  Description of Artificial Sequence: Synthetic oligonucleotide

<400>  344
uccacauaaa aacaauauuc acuggga                                          27


<210>  345
<211>  27
<212>  RNA
<213>  Artificial Sequence

<220>
<223>  Description of Artificial Sequence: Synthetic oligonucleotide

<400>  345
ucuaccaauu ccaacuugaa uucauug                                          27


<210>  346
<211>  27

<212>  RNA
<213>  Artificial Sequence

<220>
<223>  Description of Artificial Sequence: Synthetic oligonucleotide

<400>  346
ucuccaaguc uaaaucugug uccugag                                          27


<210>  347
<211>  27
<212>  RNA
<213>  Artificial Sequence

<220>
<223>  Description of Artificial Sequence: Synthetic oligonucleotide

<400>  347
uggaauacug uaacugugcu uugagga                                          27


<210>  348
<211>  27
<212>  RNA
<213>  Artificial Sequence

<220>
<223>  Description of Artificial Sequence: Synthetic oligonucleotide

<400>  348
uuaauucauc aguggugca gugguag                                           27


<210>  349
<211>  27
<212>  RNA
<213>  Artificial Sequence

<220>
<223>  Description of Artificial Sequence: Synthetic oligonucleotide

<400>  349
gugaugaugu ggcacuagua guuucuu                                          27


<210>  350
<211>  27
<212>  RNA
<213>  Artificial Sequence

<220>
<223>  Description of Artificial Sequence: Synthetic oligonucleotide

<400>  350
ucuguuuggu gaggcugucc gacuuug                                          27


<210>  351
<211>  27
<212>  RNA

<213>  Artificial Sequence

<220>
<223>  Description of Artificial Sequence: Synthetic oligonucleotide

<400>  351
ccaucauguu ccauuuuucg cuuucuc                                          27


<210>  352
<211>  27
<212>  RNA
<213>  Artificial Sequence

<220>
<223>  Description of Artificial Sequence: Synthetic oligonucleotide

<400>  352
gcagucuaca ugcuaaauca gagggua                                          27


<210>  353
<211>  27
<212>  RNA
<213>  Artificial Sequence

<220>
<223>  Description of Artificial Sequence: Synthetic oligonucleotide

<400>  353
cauaaauaga uccuuaaaua gcucaaa                                          27


<210>  354
<211>  27
<212>  RNA
<213>  Artificial Sequence

<220>
<223>  Description of Artificial Sequence: Synthetic oligonucleotide

<400>  354
ugauaauagu gcuuuuucac cuuuuuc                                          27


<210>  355
<211>  27
<212>  RNA
<213>  Artificial Sequence

<220>
<223>  Description of Artificial Sequence: Synthetic oligonucleotide

<400>  355
gcagaacuga uaauagugcu uuuucac                                          27


<210>  356
<211>  27
<212>  RNA
<213>  Artificial Sequence

<220>
<223>  Description of Artificial Sequence: Synthetic oligonucleotide

<400>  356
guagguuuaa ucuuucuuuu cagccau                                          27


<210>  357
<211>  27
<212>  RNA
<213>  Artificial Sequence

<220>
<223>  Description of Artificial Sequence: Synthetic oligonucleotide

<400>  357
agaaggauuu uauuauaagg guuuaau                                          27


<210>  358
<211>  27
<212>  RNA
<213>  Artificial Sequence

<220>
<223>  Description of Artificial Sequence: Synthetic oligonucleotide

<400>  358
gucuacagca uuggcuaaua guaugaa                                          27


<210>  359
<211>  27
<212>  RNA
<213>  Artificial Sequence

<220>
<223>  Description of Artificial Sequence: Synthetic oligonucleotide

<400>  359
cuauuccuau caaaaugcuu cugucua                                          27


<210>  360
<211>  27
<212>  RNA
<213>  Artificial Sequence

<220>
<223>  Description of Artificial Sequence: Synthetic oligonucleotide

<400>  360
ucagggccau uaucuuauuu gcucuau                                          27


<210>  361
<211>  27
<212>  RNA
<213>  Artificial Sequence

<220>
<223>  Description of Artificial Sequence: Synthetic oligonucleotide

<400>  361
ugcuugagga agagaaaaau guugguc                                          27


<210>  362
<211>  27
<212>  RNA
<213>  Artificial Sequence

<220>
<223>  Description of Artificial Sequence: Synthetic oligonucleotide

<400>  362
ugggcugaac uuuucucaua cuuaaag                                          27


<210>  363
<211>  27
<212>  RNA
<213>  Artificial Sequence

<220>
<223>  Description of Artificial Sequence: Synthetic oligonucleotide

<400>  363
gguucagcag ccaucuuuau uccugcg                                          27


<210>  364
<211>  27
<212>  RNA
<213>  Artificial Sequence

<220>
<223>  Description of Artificial Sequence: Synthetic oligonucleotide

<400>  364
ugaagagaac uuggucauuc aaauuuc                                          27


<210>  365
<211>  27
<212>  RNA
<213>  Artificial Sequence

<220>
<223>  Description of Artificial Sequence: Synthetic oligonucleotide

<400>  365
cucuaggcug gcuaucuuua uacauac                                          27


<210>  366
<211>  27
<212>  RNA
<213>  Artificial Sequence

<220>

<223>  Description of Artificial Sequence: Synthetic oligonucleotide

<400>  366
acacuucaca gagauaguua cagccau                                          27


<210>  367
<211>  27
<212>  RNA
<213>  Artificial Sequence

<220>
<223>  Description of Artificial Sequence: Synthetic oligonucleotide

<400>  367
uugaaauuuu cucacacuuc acagaga                                          27


<210>  368
<211>  27
<212>  RNA
<213>  Artificial Sequence

<220>
<223>  Description of Artificial Sequence: Synthetic oligonucleotide

<400>  368
ucaggaggau ucauuuccuu aaaggaa                                          27


<210>  369
<211>  27
<212>  RNA
<213>  Artificial Sequence

<220>
<223>  Description of Artificial Sequence: Synthetic oligonucleotide

<400>  369
auaugauguc acuuuuugua uccuuga                                          27


<210>  370
<211>  27
<212>  RNA
<213>  Artificial Sequence

<220>
<223>  Description of Artificial Sequence: Synthetic oligonucleotide

<400>  370
uaugaugaag auucaaauug caucuua                                          27


<210>  371
<211>  27
<212>  RNA
<213>  Artificial Sequence

<220>
<223>  Description of Artificial Sequence: Synthetic oligonucleotide

<400>  371
uuaauagcua gucuucguuu ugaacag                                          27


<210>  372
<211>  27
<212>  RNA
<213>  Artificial Sequence

<220>
<223>  Description of Artificial Sequence: Synthetic oligonucleotide

<400>  372
auuuuuagua gagaugaggu uucacca                                          27


<210>  373
<211>  27
<212>  RNA
<213>  Artificial Sequence

<220>
<223>  Description of Artificial Sequence: Synthetic oligonucleotide

<400>  373
aggcgagaua gagcuucucu uucguuc                                          27


<210>  374
<211>  27
<212>  RNA
<213>  Artificial Sequence

<220>
<223>  Description of Artificial Sequence: Synthetic oligonucleotide

<400>  374
aacuggaccg aaggcgcuug uggagaa                                          27


<210>  375
<211>  27
<212>  RNA
<213>  Artificial Sequence

<220>
<223>  Description of Artificial Sequence: Synthetic oligonucleotide

<400>  375
gcggcuacau cuuuggaauc uucuccu                                          27


<210>  376
<211>  27
<212>  RNA
<213>  Artificial Sequence

<220>
<223>  Description of Artificial Sequence: Synthetic oligonucleotide

<400>  376
caagucuccu cauugaaucc agauugg                                          27


<210>  377
<211>  27
<212>  RNA
<213>  Artificial Sequence

<220>
<223>  Description of Artificial Sequence: Synthetic oligonucleotide

<400>  377
uccucacuac ucucaaaucu guucugg                                          27


<210>  378
<211>  27
<212>  RNA
<213>  Artificial Sequence

<220>
<223>  Description of Artificial Sequence: Synthetic oligonucleotide

<400>  378
uuuguacuca ucugcacagc ucuggcu                                          27


<210>  379
<211>  27
<212>  RNA
<213>  Artificial Sequence

<220>
<223>  Description of Artificial Sequence: Synthetic oligonucleotide

<400>  379
gcccauuaac aacaacaauc ugaggug                                          27


<210>  380
<211>  27
<212>  RNA
<213>  Artificial Sequence

<220>
<223>  Description of Artificial Sequence: Synthetic oligonucleotide

<400>  380
aauaaauuaa aaauaauuaa aauagug                                          27


<210>  381
<211>  27
<212>  RNA
<213>  Artificial Sequence

<220>
<223>  Description of Artificial Sequence: Synthetic oligonucleotide

<400>  381

cauauuuaaa uauuaauaaa uuaaaaa                                          27

<210>  382
<211>  27
<212>  RNA
<213>  Artificial Sequence

<220>
<223>  Description of Artificial Sequence: Synthetic oligonucleotide

<400>  382
gaaacaaagg auauucaaac ugcauag                                          27

<210>  383
<211>  27
<212>  RNA
<213>  Artificial Sequence

<220>
<223>  Description of Artificial Sequence: Synthetic oligonucleotide

<400>  383
caaacacuua gacgccagca gcauggg                                          27

<210>  384
<211>  27
<212>  RNA
<213>  Artificial Sequence

<220>
<223>  Description of Artificial Sequence: Synthetic oligonucleotide

<400>  384
uuugucacag gugaaaugca caucuga                                          27

<210>  385
<211>  27
<212>  RNA
<213>  Artificial Sequence

<220>
<223>  Description of Artificial Sequence: Synthetic oligonucleotide

<400>  385
uaauucagcc uggcacaggu uuugauc                                          27

<210>  386
<211>  27
<212>  RNA
<213>  Artificial Sequence

<220>
<223>  Description of Artificial Sequence: Synthetic oligonucleotide

<400>  386
auuguaagaa ugacuaccca cauucac                                          27

<210> 387
<211> 27
<212> RNA
<213> Artificial Sequence

<220>
<223> Description of Artificial Sequence: Synthetic oligonucleotide

<400> 387
caauuguaag aaugacuacc cacauuc 27


<210> 388
<211> 27
<212> RNA
<213> Artificial Sequence

<220>
<223> Description of Artificial Sequence: Synthetic oligonucleotide

<400> 388
guacacucuc ugaugcucau uuucaua 27


<210> 389
<211> 27
<212> RNA
<213> Artificial Sequence

<220>
<223> Description of Artificial Sequence: Synthetic oligonucleotide

<400> 389
cugcugaguu cuacaugaaa agcaaau 27


<210> 390
<211> 27
<212> RNA
<213> Artificial Sequence

<220>
<223> Description of Artificial Sequence: Synthetic oligonucleotide

<400> 390
uacugaaaau aaauauagaa auacaac 27


<210> 391
<211> 27
<212> RNA
<213> Artificial Sequence

<220>
<223> Description of Artificial Sequence: Synthetic oligonucleotide

<400> 391
uuccauuuaa gaaacauuaa ucaaaac 27

<210>  392
<211>  27
<212>  RNA
<213>  Artificial Sequence

<220>
<223>  Description of Artificial Sequence: Synthetic oligonucleotide

<400>  392
acaauuuuag gcacuauaca cguuguu                                          27


<210>  393
<211>  27
<212>  RNA
<213>  Artificial Sequence

<220>
<223>  Description of Artificial Sequence: Synthetic oligonucleotide

<400>  393
ccagauguua guagccaagg auauggu                                          27


<210>  394
<211>  27
<212>  RNA
<213>  Artificial Sequence

<220>
<223>  Description of Artificial Sequence: Synthetic oligonucleotide

<400>  394
agcucacagu cuccagaugu uaguagc                                          27


<210>  395
<211>  27
<212>  RNA
<213>  Artificial Sequence

<220>
<223>  Description of Artificial Sequence: Synthetic oligonucleotide

<400>  395
auuaauaaaa agcacacaac uuauggc                                          27


<210>  396
<211>  27
<212>  RNA
<213>  Artificial Sequence

<220>
<223>  Description of Artificial Sequence: Synthetic oligonucleotide

<400>  396
aauguaagaa gagccaaaau gaugcau                                          27

<210>  397
<211>  27
<212>  RNA
<213>  Artificial Sequence

<220>
<223>  Description of Artificial Sequence: Synthetic oligonucleotide

<400>  397
acauuuuuac aaauguaaga agagcca                                          27


<210>  398
<211>  27
<212>  RNA
<213>  Artificial Sequence

<220>
<223>  Description of Artificial Sequence: Synthetic oligonucleotide

<400>  398
aauaugaauu aagaugaccu aaucugu                                          27


<210>  399
<211>  27
<212>  RNA
<213>  Artificial Sequence

<220>
<223>  Description of Artificial Sequence: Synthetic oligonucleotide

<400>  399
ucaugauuag uucuuucaau uccaucc                                          27


<210>  400
<211>  27
<212>  RNA
<213>  Artificial Sequence

<220>
<223>  Description of Artificial Sequence: Synthetic oligonucleotide

<400>  400
cccaagagca gaaggaauga gugugca                                          27


<210>  401
<211>  27
<212>  RNA
<213>  Artificial Sequence

<220>
<223>  Description of Artificial Sequence: Synthetic oligonucleotide

<400>  401
cucaaagcua cugguuaccu cuacacc                                          27


<210>  402

<211>  27
<212>  RNA
<213>  Artificial Sequence

<220>
<223>  Description of Artificial Sequence: Synthetic oligonucleotide

<400>  402
ugaaauauua cguggcaugu gguuggg                                          27


<210>  403
<211>  27
<212>  RNA
<213>  Artificial Sequence

<220>
<223>  Description of Artificial Sequence: Synthetic oligonucleotide

<400>  403
agugaagaga auaacuuguu uccgaag                                          27


<210>  404
<211>  27
<212>  RNA
<213>  Artificial Sequence

<220>
<223>  Description of Artificial Sequence: Synthetic oligonucleotide

<400>  404
uuucuuagca uuaguuauug ggaguga                                          27


<210>  405
<211>  27
<212>  RNA
<213>  Artificial Sequence

<220>
<223>  Description of Artificial Sequence: Synthetic oligonucleotide

<400>  405
uugauuuuca gcauuucuua gcauuag                                          27


<210>  406
<211>  27
<212>  RNA
<213>  Artificial Sequence

<220>
<223>  Description of Artificial Sequence: Synthetic oligonucleotide

<400>  406
aaaguaauca uauuuagaga aagacag                                          27


<210>  407
<211>  27

<212>  RNA
<213>  Artificial Sequence

<220>
<223>  Description of Artificial Sequence: Synthetic oligonucleotide

<400>  407
uguagaacaa aaugccugaa auucagc                                          27


<210>  408
<211>  27
<212>  RNA
<213>  Artificial Sequence

<220>
<223>  Description of Artificial Sequence: Synthetic oligonucleotide

<400>  408
gagagucccg ggugagaggg aaucgcc                                          27


<210>  409
<211>  27
<212>  RNA
<213>  Artificial Sequence

<220>
<223>  Description of Artificial Sequence: Synthetic oligonucleotide

<400>  409
cuuuacuuua aagguucacu uuccuug                                          27


<210>  410
<211>  27
<212>  RNA
<213>  Artificial Sequence

<220>
<223>  Description of Artificial Sequence: Synthetic oligonucleotide

<400>  410
ccaggcuucc agagcaugca gccuccu                                          27


<210>  411
<211>  27
<212>  RNA
<213>  Artificial Sequence

<220>
<223>  Description of Artificial Sequence: Synthetic oligonucleotide

<400>  411
cucucuuuug uuuucuguuc agagaaa                                          27


<210>  412
<211>  27
<212>  RNA

<213>  Artificial Sequence

<220>
<223>  Description of Artificial Sequence: Synthetic oligonucleotide

<400>  412
cggcauaacu gauuacagcc aaguuca                                          27


<210>  413
<211>  27
<212>  RNA
<213>  Artificial Sequence

<220>
<223>  Description of Artificial Sequence: Synthetic oligonucleotide

<400>  413
uggacuucuu uuaauuuugg cuucuuc                                          27


<210>  414
<211>  27
<212>  RNA
<213>  Artificial Sequence

<220>
<223>  Description of Artificial Sequence: Synthetic oligonucleotide

<400>  414
uaaccucacc uggacuucuu uuaauuu                                          27


<210>  415
<211>  27
<212>  RNA
<213>  Artificial Sequence

<220>
<223>  Description of Artificial Sequence: Synthetic oligonucleotide

<400>  415
guccucuucc cgauaauccg gauucag                                          27


<210>  416
<211>  27
<212>  RNA
<213>  Artificial Sequence

<220>
<223>  Description of Artificial Sequence: Synthetic oligonucleotide

<400>  416
acaauacuug cuuugauguc acauuaa                                          27


<210>  417
<211>  27
<212>  RNA
<213>  Artificial Sequence

<220>
<223>  Description of Artificial Sequence: Synthetic oligonucleotide

<400>  417
gagugcuaca auacuugcuu ugauguc                                          27


<210>  418
<211>  27
<212>  RNA
<213>  Artificial Sequence

<220>
<223>  Description of Artificial Sequence: Synthetic oligonucleotide

<400>  418
auuugugguu uuguuuucuc ucucucu                                          27


<210>  419
<211>  27
<212>  RNA
<213>  Artificial Sequence

<220>
<223>  Description of Artificial Sequence: Synthetic oligonucleotide

<400>  419
uguaugggcu uaaauacuac agcgagg                                          27


<210>  420
<211>  27
<212>  RNA
<213>  Artificial Sequence

<220>
<223>  Description of Artificial Sequence: Synthetic oligonucleotide

<400>  420
cuguaugggc uuaaauacua cagcgag                                          27


<210>  421
<211>  27
<212>  RNA
<213>  Artificial Sequence

<220>
<223>  Description of Artificial Sequence: Synthetic oligonucleotide

<400>  421
ggaagguuuc uguaugggcu uaaauac                                          27


<210>  422
<211>  27
<212>  RNA
<213>  Artificial Sequence

<220>
<223>  Description of Artificial Sequence: Synthetic oligonucleotide

<400>  422
guagguauac cauguuuauu uuaauac                                          27


<210>  423
<211>  27
<212>  RNA
<213>  Artificial Sequence

<220>
<223>  Description of Artificial Sequence: Synthetic oligonucleotide

<400>  423
uccgaaaacu ggccgaucag aacagcc                                          27


<210>  424
<211>  27
<212>  RNA
<213>  Artificial Sequence

<220>
<223>  Description of Artificial Sequence: Synthetic oligonucleotide

<400>  424
cccuguucca aaguucaaau uauuugu                                          27


<210>  425
<211>  27
<212>  RNA
<213>  Artificial Sequence

<220>
<223>  Description of Artificial Sequence: Synthetic oligonucleotide

<400>  425
acuggaaagu uaaauuaagg ucgcaau                                          27


<210>  426
<211>  27
<212>  RNA
<213>  Artificial Sequence

<220>
<223>  Description of Artificial Sequence: Synthetic oligonucleotide

<400>  426
caaaaccaaa cuuuagcuuu cucagcc                                          27


<210>  427
<211>  27
<212>  RNA
<213>  Artificial Sequence

<220>

<223>  Description of Artificial Sequence: Synthetic oligonucleotide

<400>  427
uacaguggga aguagcaucu uuacuuu                                          27


<210>  428
<211>  27
<212>  RNA
<213>  Artificial Sequence

<220>
<223>  Description of Artificial Sequence: Synthetic oligonucleotide

<400>  428
ucauauggua ucagcaauua agcagua                                          27


<210>  429
<211>  27
<212>  RNA
<213>  Artificial Sequence

<220>
<223>  Description of Artificial Sequence: Synthetic oligonucleotide

<400>  429
cagcccaugu uucauucaua ugguauc                                          27


<210>  430
<211>  27
<212>  RNA
<213>  Artificial Sequence

<220>
<223>  Description of Artificial Sequence: Synthetic oligonucleotide

<400>  430
uaugaaaaag uuggauaaga aaguugg                                          27


<210>  431
<211>  27
<212>  RNA
<213>  Artificial Sequence

<220>
<223>  Description of Artificial Sequence: Synthetic oligonucleotide

<400>  431
uccggccuca ugaugucaug caaggcu                                          27


<210>  432
<211>  27
<212>  RNA
<213>  Artificial Sequence

<220>
<223>  Description of Artificial Sequence: Synthetic oligonucleotide

<400>  432
acaaaugcag ucauauacaa auucagg                                          27


<210>  433
<211>  27
<212>  RNA
<213>  Artificial Sequence

<220>
<223>  Description of Artificial Sequence: Synthetic oligonucleotide

<400>  433
gguaguaaac auucuacuag gauucuu                                          27


<210>  434
<211>  27
<212>  RNA
<213>  Artificial Sequence

<220>
<223>  Description of Artificial Sequence: Synthetic oligonucleotide

<400>  434
uuucaucaaa aaauucuucc cuuucug                                          27


<210>  435
<211>  27
<212>  RNA
<213>  Artificial Sequence

<220>
<223>  Description of Artificial Sequence: Synthetic oligonucleotide

<400>  435
aaauucacac acuggcaugc cgauagc                                          27


<210>  436
<211>  27
<212>  RNA
<213>  Artificial Sequence

<220>
<223>  Description of Artificial Sequence: Synthetic oligonucleotide

<400>  436
acauacauug cucuacuaug aggugaa                                          27


<210>  437
<211>  27
<212>  RNA
<213>  Artificial Sequence

<220>
<223>  Description of Artificial Sequence: Synthetic oligonucleotide

<400>  437
uauauaugug cuuuggcaau ucuggug                                          27


<210>  438
<211>  27
<212>  RNA
<213>  Artificial Sequence

<220>
<223>  Description of Artificial Sequence: Synthetic oligonucleotide

<400>  438
ggagaaacua uuacccagau caccacu                                          27


<210>  439
<211>  27
<212>  RNA
<213>  Artificial Sequence

<220>
<223>  Description of Artificial Sequence: Synthetic oligonucleotide

<400>  439
auuuggagaa acuauuaccc agaucac                                          27


<210>  440
<211>  27
<212>  RNA
<213>  Artificial Sequence

<220>
<223>  Description of Artificial Sequence: Synthetic oligonucleotide

<400>  440
uuucuaggaa guauucauca cauccac                                          27


<210>  441
<211>  27
<212>  RNA
<213>  Artificial Sequence

<220>
<223>  Description of Artificial Sequence: Synthetic oligonucleotide

<400>  441
cauauauggu guagcugugg aaaugcg                                          27


<210>  442
<211>  27
<212>  RNA
<213>  Artificial Sequence

<220>
<223>  Description of Artificial Sequence: Synthetic oligonucleotide

<400>  442

cuccauucau auauggugua gcugugg                                          27

<210>  443
<211>  27
<212>  RNA
<213>  Artificial Sequence

<220>
<223>  Description of Artificial Sequence: Synthetic oligonucleotide

<400>  443
aucuucuucu cguguaagaa cugcagc                                          27

<210>  444
<211>  27
<212>  RNA
<213>  Artificial Sequence

<220>
<223>  Description of Artificial Sequence: Synthetic oligonucleotide

<400>  444
ucccuguaaa ugaucuucuu cucgugu                                          27

<210>  445
<211>  27
<212>  RNA
<213>  Artificial Sequence

<220>
<223>  Description of Artificial Sequence: Synthetic oligonucleotide

<400>  445
gucccuguaa augaucuucu ucucgug                                          27

<210>  446
<211>  27
<212>  RNA
<213>  Artificial Sequence

<220>
<223>  Description of Artificial Sequence: Synthetic oligonucleotide

<400>  446
ucaggucccu guaaaugauc uucuucu                                          27

<210>  447
<211>  27
<212>  RNA
<213>  Artificial Sequence

<220>
<223>  Description of Artificial Sequence: Synthetic oligonucleotide

<400>  447
gaugcaguca aacaccucuu ccucugu                                          27

<210> 448
<211> 27
<212> RNA
<213> Artificial Sequence

<220>
<223> Description of Artificial Sequence: Synthetic oligonucleotide

<400> 448
cgaugcaguc aaacaccucu uccucug 27


<210> 449
<211> 27
<212> RNA
<213> Artificial Sequence

<220>
<223> Description of Artificial Sequence: Synthetic oligonucleotide

<400> 449
cccugugaac ucgcccucaa aguagcg 27


<210> 450
<211> 27
<212> RNA
<213> Artificial Sequence

<220>
<223> Description of Artificial Sequence: Synthetic oligonucleotide

<400> 450
ggacagaggu gccaggagau gcccuca 27


<210> 451
<211> 27
<212> RNA
<213> Artificial Sequence

<220>
<223> Description of Artificial Sequence: Synthetic oligonucleotide

<400> 451
uuaaaaagac aaaccauauc acuccuu 27


<210> 452
<211> 27
<212> RNA
<213> Artificial Sequence

<220>
<223> Description of Artificial Sequence: Synthetic oligonucleotide

<400> 452
cuuaaaaaga caaaccauau cacuccu 27

<210>  453
<211>  27
<212>  RNA
<213>  Artificial Sequence

<220>
<223>  Description of Artificial Sequence: Synthetic oligonucleotide

<400>  453
gacaaccuuu aggaucuuua cugcaac                                          27


<210>  454
<211>  27
<212>  RNA
<213>  Artificial Sequence

<220>
<223>  Description of Artificial Sequence: Synthetic oligonucleotide

<400>  454
gucgacaacc uuuaggaucu uuacugc                                          27


<210>  455
<211>  27
<212>  RNA
<213>  Artificial Sequence

<220>
<223>  Description of Artificial Sequence: Synthetic oligonucleotide

<400>  455
acaauugcca gguuguccuu ugucaug                                          27


<210>  456
<211>  27
<212>  RNA
<213>  Artificial Sequence

<220>
<223>  Description of Artificial Sequence: Synthetic oligonucleotide

<400>  456
ccucgcacca cugggucaca auugcca                                          27


<210>  457
<211>  27
<212>  RNA
<213>  Artificial Sequence

<220>
<223>  Description of Artificial Sequence: Synthetic oligonucleotide

<400>  457
auuucauguc ucuauggaug auguucu                                          27

<210>  458
<211>  27
<212>  RNA
<213>  Artificial Sequence

<220>
<223>  Description of Artificial Sequence: Synthetic oligonucleotide

<400>  458
uggagaaaua uauuguugga uuucaug                                          27


<210>  459
<211>  27
<212>  RNA
<213>  Artificial Sequence

<220>
<223>  Description of Artificial Sequence: Synthetic oligonucleotide

<400>  459
agccuucaug gagaaauaua uuguugg                                          27


<210>  460
<211>  27
<212>  RNA
<213>  Artificial Sequence

<220>
<223>  Description of Artificial Sequence: Synthetic oligonucleotide

<400>  460
accacuccag cgugacuuua cuguugc                                          27


<210>  461
<211>  27
<212>  RNA
<213>  Artificial Sequence

<220>
<223>  Description of Artificial Sequence: Synthetic oligonucleotide

<400>  461
ccucucuucc uuuacuuucu ucacaca                                          27


<210>  462
<211>  27
<212>  RNA
<213>  Artificial Sequence

<220>
<223>  Description of Artificial Sequence: Synthetic oligonucleotide

<400>  462
gcccgaugca aggauggcuc ggaagcg                                          27


<210>  463

<211>  27
<212>  RNA
<213>  Artificial Sequence

<220>
<223>  Description of Artificial Sequence: Synthetic oligonucleotide

<400>  463
cacaguagga uauucaaccu ccauuuc                                          27


<210>  464
<211>  27
<212>  RNA
<213>  Artificial Sequence

<220>
<223>  Description of Artificial Sequence: Synthetic oligonucleotide

<400>  464
uuacacagua ggauauucaa ccuccau                                          27


<210>  465
<211>  27
<212>  RNA
<213>  Artificial Sequence

<220>
<223>  Description of Artificial Sequence: Synthetic oligonucleotide

<400>  465
uacacuacaa gggaaaacuc aaaaucu                                          27


<210>  466
<211>  27
<212>  RNA
<213>  Artificial Sequence

<220>
<223>  Description of Artificial Sequence: Synthetic oligonucleotide

<400>  466
caaucaacaa agaacaaaca ggauaaa                                          27


<210>  467
<211>  27
<212>  RNA
<213>  Artificial Sequence

<220>
<223>  Description of Artificial Sequence: Synthetic oligonucleotide

<400>  467
uuucaaucaa caaagaacaa acaggau                                          27


<210>  468
<211>  27

<212>  RNA
<213>  Artificial Sequence

<220>
<223>  Description of Artificial Sequence: Synthetic oligonucleotide

<400>  468
auggaagaaa aagaaggcug uagagaa                                          27


<210>  469
<211>  27
<212>  RNA
<213>  Artificial Sequence

<220>
<223>  Description of Artificial Sequence: Synthetic oligonucleotide

<400>  469
uagaaggaag auuagcuaug gaagaaa                                          27


<210>  470
<211>  27
<212>  RNA
<213>  Artificial Sequence

<220>
<223>  Description of Artificial Sequence: Synthetic oligonucleotide

<400>  470
augaagcauu caacaggaag auuauuu                                          27


<210>  471
<211>  27
<212>  RNA
<213>  Artificial Sequence

<220>
<223>  Description of Artificial Sequence: Synthetic oligonucleotide

<400>  471
caugaagcau ucaacaggaa gauuauu                                          27


<210>  472
<211>  27
<212>  RNA
<213>  Artificial Sequence

<220>
<223>  Description of Artificial Sequence: Synthetic oligonucleotide

<400>  472
ucaaaguaga auucaaguca ugaagca                                          27


<210>  473
<211>  27
<212>  RNA

<213>  Artificial Sequence

<220>
<223>  Description of Artificial Sequence: Synthetic oligonucleotide

<400>  473
gucuguaguu gcuucucucc cucuuag                                          27


<210>  474
<211>  27
<212>  RNA
<213>  Artificial Sequence

<220>
<223>  Description of Artificial Sequence: Synthetic oligonucleotide

<400>  474
guugaccuug gucugguagg agacggc                                          27


<210>  475
<211>  27
<212>  RNA
<213>  Artificial Sequence

<220>
<223>  Description of Artificial Sequence: Synthetic oligonucleotide

<400>  475
gucgagauag ucgggccgau ugaucuc                                          27


<210>  476
<211>  27
<212>  RNA
<213>  Artificial Sequence

<220>
<223>  Description of Artificial Sequence: Synthetic oligonucleotide

<400>  476
guuugggaag guuggauguu cguccuc                                          27


<210>  477
<211>  27
<212>  RNA
<213>  Artificial Sequence

<220>
<223>  Description of Artificial Sequence: Synthetic oligonucleotide

<400>  477
aguucuaagc uuggguuccg acccuaa                                          27


<210>  478
<211>  27
<212>  RNA
<213>  Artificial Sequence

<220>
<223>  Description of Artificial Sequence: Synthetic oligonucleotide

<400>  478
uuaaaguucu aagcuugggu uccgacc                                          27


<210>  479
<211>  27
<212>  RNA
<213>  Artificial Sequence

<220>
<223>  Description of Artificial Sequence: Synthetic oligonucleotide

<400>  479
guugcuuaaa guucuaagcu uggguuc                                          27


<210>  480
<211>  27
<212>  RNA
<213>  Artificial Sequence

<220>
<223>  Description of Artificial Sequence: Synthetic oligonucleotide

<400>  480
gugguggucu uguugcuuaa aguucua                                          27


<210>  481
<211>  27
<212>  RNA
<213>  Artificial Sequence

<220>
<223>  Description of Artificial Sequence: Synthetic oligonucleotide

<400>  481
acuuuauuuc ucgccacuga auaguag                                          27


<210>  482
<211>  27
<212>  RNA
<213>  Artificial Sequence

<220>
<223>  Description of Artificial Sequence: Synthetic oligonucleotide

<400>  482
aacuuuauuu cucgccacug aauagua                                          27


<210>  483
<211>  27
<212>  RNA
<213>  Artificial Sequence

<220>
<223>  Description of Artificial Sequence: Synthetic oligonucleotide

<400>  483
ggaucauuuc cuguuaucug uguuugu                                          27


<210>  484
<211>  27
<212>  RNA
<213>  Artificial Sequence

<220>
<223>  Description of Artificial Sequence: Synthetic oligonucleotide

<400>  484
guuucauuuc uucucuuuuc uuaaggc                                          27


<210>  485
<211>  27
<212>  RNA
<213>  Artificial Sequence

<220>
<223>  Description of Artificial Sequence: Synthetic oligonucleotide

<400>  485
ggaggaugga aacacacucc uucaguu                                          27


<210>  486
<211>  27
<212>  RNA
<213>  Artificial Sequence

<220>
<223>  Description of Artificial Sequence: Synthetic oligonucleotide

<400>  486
ucaaagauuu ucagucccgc ggugaca                                          27


<210>  487
<211>  27
<212>  RNA
<213>  Artificial Sequence

<220>
<223>  Description of Artificial Sequence: Synthetic oligonucleotide

<400>  487
ccugaacggc acgcuuauuu cugcugu                                          27


<210>  488
<211>  27
<212>  RNA
<213>  Artificial Sequence

<220>

<223>  Description of Artificial Sequence: Synthetic oligonucleotide

<400>  488
cuucuggacc cugaacggca cgcuuau                                          27


<210>  489
<211>  27
<212>  RNA
<213>  Artificial Sequence

<220>
<223>  Description of Artificial Sequence: Synthetic oligonucleotide

<400>  489
caagcagucu ugagugacug uuucuuc                                          27


<210>  490
<211>  27
<212>  RNA
<213>  Artificial Sequence

<220>
<223>  Description of Artificial Sequence: Synthetic oligonucleotide

<400>  490
ccaaagacau ggaccuucuu ccucuga                                          27


<210>  491
<211>  27
<212>  RNA
<213>  Artificial Sequence

<220>
<223>  Description of Artificial Sequence: Synthetic oligonucleotide

<400>  491
guguuucagg cauauuuuga auacauc                                          27


<210>  492
<211>  27
<212>  RNA
<213>  Artificial Sequence

<220>
<223>  Description of Artificial Sequence: Synthetic oligonucleotide

<400>  492
gagguacaga gaaagggagg aaaauag                                          27


<210>  493
<211>  27
<212>  RNA
<213>  Artificial Sequence

<220>
<223>  Description of Artificial Sequence: Synthetic oligonucleotide

<400>  493
ucuugucuug cucuaucuuu cuuuggu                                          27


<210>  494
<211>  27
<212>  RNA
<213>  Artificial Sequence

<220>
<223>  Description of Artificial Sequence: Synthetic oligonucleotide

<400>  494
uuucuugucu ugcucuaucu uucuuug                                          27


<210>  495
<211>  27
<212>  RNA
<213>  Artificial Sequence

<220>
<223>  Description of Artificial Sequence: Synthetic oligonucleotide

<400>  495
ggaucuugua caaacaaaug cuuucuc                                          27


<210>  496
<211>  27
<212>  RNA
<213>  Artificial Sequence

<220>
<223>  Description of Artificial Sequence: Synthetic oligonucleotide

<400>  496
ucugcaagua cguucguuua acucaag                                          27


<210>  497
<211>  27
<212>  RNA
<213>  Artificial Sequence

<220>
<223>  Description of Artificial Sequence: Synthetic oligonucleotide

<400>  497
ucuguaucga ucguucugua ucagucu                                          27


<210>  498
<211>  27
<212>  RNA
<213>  Artificial Sequence

<220>
<223>  Description of Artificial Sequence: Synthetic oligonucleotide

<400>  498
auuuauuuuu auauauauau aauauau                                          27


<210>  499
<211>  27
<212>  RNA
<213>  Artificial Sequence

<220>
<223>  Description of Artificial Sequence: Synthetic oligonucleotide

<400>  499
auauuuauuu uuauauauau auaauau                                          27


<210>  500
<211>  27
<212>  RNA
<213>  Artificial Sequence

<220>
<223>  Description of Artificial Sequence: Synthetic oligonucleotide

<400>  500
cacagcaguc aaauacaucc agugaag                                          27


<210>  501
<211>  27
<212>  RNA
<213>  Artificial Sequence

<220>
<223>  Description of Artificial Sequence: Synthetic oligonucleotide

<400>  501
gucucucauc uccuccucuu cccuguc                                          27


<210>  502
<211>  27
<212>  RNA
<213>  Artificial Sequence

<220>
<223>  Description of Artificial Sequence: Synthetic oligonucleotide

<400>  502
ccaaguggga caaaaaaaag aucaugc                                          27


<210>  503
<211>  14
<212>  RNA
<213>  Artificial Sequence

<220>
<223>  Description of Artificial Sequence: Synthetic oligonucleotide

<400>  503

guauuuugau gagg                                                          14

<210>  504
<211>  12
<212>  RNA
<213>  Artificial Sequence

<220>
<223>  Description of Artificial Sequence: Synthetic oligonucleotide

<400>  504
ggcccagaug au                                                            12

<210>  505
<211>  14
<212>  RNA
<213>  Artificial Sequence

<220>
<223>  Description of Artificial Sequence: Synthetic oligonucleotide

<400>  505
gggaagaaaa cuau                                                          14

<210>  506
<211>  15
<212>  RNA
<213>  Artificial Sequence

<220>
<223>  Description of Artificial Sequence: Synthetic oligonucleotide

<400>  506
guuuuaauuu auuuc                                                         15

<210>  507
<211>  12
<212>  RNA
<213>  Artificial Sequence

<220>
<223>  Description of Artificial Sequence: Synthetic oligonucleotide

<400>  507
acguagggaa au                                                            12

<210>  508
<211>  12
<212>  RNA
<213>  Artificial Sequence

<220>
<223>  Description of Artificial Sequence: Synthetic oligonucleotide

<400>  508
ccaggguuua cc                                                            12

<210>  509
<211>  11
<212>  RNA
<213>  Artificial Sequence

<220>
<223>  Description of Artificial Sequence: Synthetic oligonucleotide

<400>  509
agcaagguuu a                                                            11


<210>  510
<211>  12
<212>  RNA
<213>  Artificial Sequence

<220>
<223>  Description of Artificial Sequence: Synthetic oligonucleotide

<400>  510
uguauuaugu ug                                                           12


<210>  511
<211>  15
<212>  RNA
<213>  Artificial Sequence

<220>
<223>  Description of Artificial Sequence: Synthetic oligonucleotide

<400>  511
uuuuaaucuu uguga                                                        15


<210>  512
<211>  14
<212>  RNA
<213>  Artificial Sequence

<220>
<223>  Description of Artificial Sequence: Synthetic oligonucleotide

<400>  512
uuaaucuuug ugac                                                         14


<210>  513
<211>  13
<212>  RNA
<213>  Artificial Sequence

<220>
<223>  Description of Artificial Sequence: Synthetic oligonucleotide

<400>  513
gcugcuuaug ucu                                                          13

<210>  514
<211>  14
<212>  RNA
<213>  Artificial Sequence

<220>
<223>  Description of Artificial Sequence: Synthetic oligonucleotide

<400>  514
aaguguuuca gaag                                                          14


<210>  515
<211>  13
<212>  RNA
<213>  Artificial Sequence

<220>
<223>  Description of Artificial Sequence: Synthetic oligonucleotide

<400>  515
gaccaucaau aag                                                           13


<210>  516
<211>  13
<212>  RNA
<213>  Artificial Sequence

<220>
<223>  Description of Artificial Sequence: Synthetic oligonucleotide

<400>  516
ccaucaauaa gga                                                           13


<210>  517
<211>  14
<212>  RNA
<213>  Artificial Sequence

<220>
<223>  Description of Artificial Sequence: Synthetic oligonucleotide

<400>  517
ucaauaagga agaa                                                          14


<210>  518
<211>  14
<212>  RNA
<213>  Artificial Sequence

<220>
<223>  Description of Artificial Sequence: Synthetic oligonucleotide

<400>  518
aauaaggaag aagc                                                          14

<210>  519
<211>  13
<212>  RNA
<213>  Artificial Sequence

<220>
<223>  Description of Artificial Sequence: Synthetic oligonucleotide

<400>  519
aggaagaagc ccu                                                          13


<210>  520
<211>  13
<212>  RNA
<213>  Artificial Sequence

<220>
<223>  Description of Artificial Sequence: Synthetic oligonucleotide

<400>  520
gcauaacuaa agg                                                          13


<210>  521
<211>  14
<212>  RNA
<213>  Artificial Sequence

<220>
<223>  Description of Artificial Sequence: Synthetic oligonucleotide

<400>  521
cauaacuaaa ggug                                                         14


<210>  522
<211>  12
<212>  RNA
<213>  Artificial Sequence

<220>
<223>  Description of Artificial Sequence: Synthetic oligonucleotide

<400>  522
cuacaucacg cc                                                           12


<210>  523
<211>  12
<212>  RNA
<213>  Artificial Sequence

<220>
<223>  Description of Artificial Sequence: Synthetic oligonucleotide

<400>  523
acuggauuua ag                                                           12


<210>  524

<211>  12
<212>  RNA
<213>  Artificial Sequence

<220>
<223>  Description of Artificial Sequence: Synthetic oligonucleotide

<400>  524
cuggauuuaa gc                                                          12


<210>  525
<211>  13
<212>  RNA
<213>  Artificial Sequence

<220>
<223>  Description of Artificial Sequence: Synthetic oligonucleotide

<400>  525
gauuuaagca gag                                                         13


<210>  526
<211>  13
<212>  RNA
<213>  Artificial Sequence

<220>
<223>  Description of Artificial Sequence: Synthetic oligonucleotide

<400>  526
auuuaagcag agu                                                         13


<210>  527
<211>  13
<212>  RNA
<213>  Artificial Sequence

<220>
<223>  Description of Artificial Sequence: Synthetic oligonucleotide

<400>  527
uuaagcagag uuc                                                         13


<210>  528
<211>  13
<212>  RNA
<213>  Artificial Sequence

<220>
<223>  Description of Artificial Sequence: Synthetic oligonucleotide

<400>  528
uaagcagagu uca                                                         13


<210>  529
<211>  14

<212>  RNA
<213>  Artificial Sequence

<220>
<223>  Description of Artificial Sequence: Synthetic oligonucleotide

<400>  529
agcagaguuc aaaa                                                        14


<210>  530
<211>  12
<212>  RNA
<213>  Artificial Sequence

<220>
<223>  Description of Artificial Sequence: Synthetic oligonucleotide

<400>  530
cucagggucu ga                                                          12


<210>  531
<211>  13
<212>  RNA
<213>  Artificial Sequence

<220>
<223>  Description of Artificial Sequence: Synthetic oligonucleotide

<400>  531
caagcaacua cau                                                         13


<210>  532
<211>  12
<212>  RNA
<213>  Artificial Sequence

<220>
<223>  Description of Artificial Sequence: Synthetic oligonucleotide

<400>  532
aucaauggca gc                                                          12


<210>  533
<211>  11
<212>  RNA
<213>  Artificial Sequence

<220>
<223>  Description of Artificial Sequence: Synthetic oligonucleotide

<400>  533
uuccagccca c                                                           11


<210>  534
<211>  12
<212>  RNA

<213>  Artificial Sequence

<220>
<223>  Description of Artificial Sequence: Synthetic oligonucleotide

<400>  534
cagcugagaa ug                                                          12


<210>  535
<211>  13
<212>  RNA
<213>  Artificial Sequence

<220>
<223>  Description of Artificial Sequence: Synthetic oligonucleotide

<400>  535
aacguaucuc cua                                                         13


<210>  536
<211>  14
<212>  RNA
<213>  Artificial Sequence

<220>
<223>  Description of Artificial Sequence: Synthetic oligonucleotide

<400>  536
ccuaaaauaa uuuc                                                        14


<210>  537
<211>  13
<212>  RNA
<213>  Artificial Sequence

<220>
<223>  Description of Artificial Sequence: Synthetic oligonucleotide

<400>  537
uggaagauuc agc                                                         13


<210>  538
<211>  12
<212>  RNA
<213>  Artificial Sequence

<220>
<223>  Description of Artificial Sequence: Synthetic oligonucleotide

<400>  538
uuaaacucuc cu                                                          12


<210>  539
<211>  12
<212>  RNA
<213>  Artificial Sequence

<220>
<223>  Description of Artificial Sequence: Synthetic oligonucleotide

<400>  539
cagccuacag uu                                                          12


<210>  540
<211>  13
<212>  RNA
<213>  Artificial Sequence

<220>
<223>  Description of Artificial Sequence: Synthetic oligonucleotide

<400>  540
guuauguuca guc                                                         13


<210>  541
<211>  13
<212>  RNA
<213>  Artificial Sequence

<220>
<223>  Description of Artificial Sequence: Synthetic oligonucleotide

<400>  541
cacauacaaa aug                                                         13


<210>  542
<211>  9
<212>  RNA
<213>  Artificial Sequence

<220>
<223>  Description of Artificial Sequence: Synthetic oligonucleotide

<400>  542
uccuuuugc                                                               9


<210>  543
<211>  12
<212>  RNA
<213>  Artificial Sequence

<220>
<223>  Description of Artificial Sequence: Synthetic oligonucleotide

<400>  543
ugaccuguga ag                                                          12


<210>  544
<211>  12
<212>  RNA
<213>  Artificial Sequence

<220>
<223>  Description of Artificial Sequence: Synthetic oligonucleotide

<400>  544
cuaucucaca ca                                                          12


<210>  545
<211>  13
<212>  RNA
<213>  Artificial Sequence

<220>
<223>  Description of Artificial Sequence: Synthetic oligonucleotide

<400>  545
uguccucaau ugu                                                         13


<210>  546
<211>  12
<212>  RNA
<213>  Artificial Sequence

<220>
<223>  Description of Artificial Sequence: Synthetic oligonucleotide

<400>  546
aaaccguagc ug                                                          12


<210>  547
<211>  12
<212>  RNA
<213>  Artificial Sequence

<220>
<223>  Description of Artificial Sequence: Synthetic oligonucleotide

<400>  547
uagcuggcaa gc                                                          12


<210>  548
<211>  14
<212>  RNA
<213>  Artificial Sequence

<220>
<223>  Description of Artificial Sequence: Synthetic oligonucleotide

<400>  548
uuguaugguu aaaa                                                        14


<210>  549
<211>  14
<212>  RNA
<213>  Artificial Sequence

<220>

<223>  Description of Artificial Sequence: Synthetic oligonucleotide

<400>  549
ugguuaaaag augg                                                        14


<210>  550
<211>  13
<212>  RNA
<213>  Artificial Sequence

<220>
<223>  Description of Artificial Sequence: Synthetic oligonucleotide

<400>  550
cagggaauua uac                                                         13


<210>  551
<211>  12
<212>  RNA
<213>  Artificial Sequence

<220>
<223>  Description of Artificial Sequence: Synthetic oligonucleotide

<400>  551
acaaucuugc ug                                                          12


<210>  552
<211>  13
<212>  RNA
<213>  Artificial Sequence

<220>
<223>  Description of Artificial Sequence: Synthetic oligonucleotide

<400>  552
ccaauaauga aga                                                         13


<210>  553
<211>  13
<212>  RNA
<213>  Artificial Sequence

<220>
<223>  Description of Artificial Sequence: Synthetic oligonucleotide

<400>  553
acuuuggaug uuc                                                         13


<210>  554
<211>  12
<212>  RNA
<213>  Artificial Sequence

<220>
<223>  Description of Artificial Sequence: Synthetic oligonucleotide

<400>  554
aaugcuucca cu                                                          12


<210>  555
<211>  11
<212>  RNA
<213>  Artificial Sequence

<220>
<223>  Description of Artificial Sequence: Synthetic oligonucleotide

<400>  555
gagaaaguuu g                                                           11


<210>  556
<211>  11
<212>  RNA
<213>  Artificial Sequence

<220>
<223>  Description of Artificial Sequence: Synthetic oligonucleotide

<400>  556
aaagaacuac u                                                           11


<210>  557
<211>  12
<212>  RNA
<213>  Artificial Sequence

<220>
<223>  Description of Artificial Sequence: Synthetic oligonucleotide

<400>  557
uuagaaauac gg                                                          12


<210>  558
<211>  12
<212>  RNA
<213>  Artificial Sequence

<220>
<223>  Description of Artificial Sequence: Synthetic oligonucleotide

<400>  558
aacaugggua ca                                                          12


<210>  559
<211>  14
<212>  RNA
<213>  Artificial Sequence

<220>
<223>  Description of Artificial Sequence: Synthetic oligonucleotide

<400>  559
aaagacacag aaga                                                         14


<210>  560
<211>  11
<212>  RNA
<213>  Artificial Sequence

<220>
<223>  Description of Artificial Sequence: Synthetic oligonucleotide

<400>  560
uaguagggag g                                                            11


<210>  561
<211>  12
<212>  RNA
<213>  Artificial Sequence

<220>
<223>  Description of Artificial Sequence: Synthetic oligonucleotide

<400>  561
gccuucugca gc                                                           12


<210>  562
<211>  10
<212>  RNA
<213>  Artificial Sequence

<220>
<223>  Description of Artificial Sequence: Synthetic oligonucleotide

<400>  562
ggucugguac                                                              10


<210>  563
<211>  12
<212>  RNA
<213>  Artificial Sequence

<220>
<223>  Description of Artificial Sequence: Synthetic oligonucleotide

<400>  563
cuaguccuuc cg                                                           12


<210>  564
<211>  12
<212>  RNA
<213>  Artificial Sequence

<220>
<223>  Description of Artificial Sequence: Synthetic oligonucleotide

<400>  564

ccagugaaua uu                                                          12

<210>  565
<211>  13
<212>  RNA
<213>  Artificial Sequence

<220>
<223>  Description of Artificial Sequence: Synthetic oligonucleotide

<400>  565
augaauucaa guu                                                         13

<210>  566
<211>  12
<212>  RNA
<213>  Artificial Sequence

<220>
<223>  Description of Artificial Sequence: Synthetic oligonucleotide

<400>  566
caggacacag au                                                          12

<210>  567
<211>  12
<212>  RNA
<213>  Artificial Sequence

<220>
<223>  Description of Artificial Sequence: Synthetic oligonucleotide

<400>  567
cucaaagcac ag                                                          12

<210>  568
<211>  10
<212>  RNA
<213>  Artificial Sequence

<220>
<223>  Description of Artificial Sequence: Synthetic oligonucleotide

<400>  568
accacugcca                                                             10

<210>  569
<211>  13
<212>  RNA
<213>  Artificial Sequence

<220>
<223>  Description of Artificial Sequence: Synthetic oligonucleotide

<400>  569
gaaacuacua gug                                                         13

<210>  570
<211>  12
<212>  RNA
<213>  Artificial Sequence

<220>
<223>  Description of Artificial Sequence: Synthetic oligonucleotide

<400>  570
aagucggaca gc                                                          12


<210>  571
<211>  13
<212>  RNA
<213>  Artificial Sequence

<220>
<223>  Description of Artificial Sequence: Synthetic oligonucleotide

<400>  571
gaaagcgaaa aau                                                         13


<210>  572
<211>  13
<212>  RNA
<213>  Artificial Sequence

<220>
<223>  Description of Artificial Sequence: Synthetic oligonucleotide

<400>  572
cccucugauu uag                                                         13


<210>  573
<211>  12
<212>  RNA
<213>  Artificial Sequence

<220>
<223>  Description of Artificial Sequence: Synthetic oligonucleotide

<400>  573
ugagcuauuu aa                                                          12


<210>  574
<211>  13
<212>  RNA
<213>  Artificial Sequence

<220>
<223>  Description of Artificial Sequence: Synthetic oligonucleotide

<400>  574
aaaaggugaa aaa                                                         13

<210>  575
<211>  14
<212>  RNA
<213>  Artificial Sequence

<220>
<223>  Description of Artificial Sequence: Synthetic oligonucleotide

<400>  575
gaaaaagcac uauu                                                          14


<210>  576
<211>  12
<212>  RNA
<213>  Artificial Sequence

<220>
<223>  Description of Artificial Sequence: Synthetic oligonucleotide

<400>  576
ggcugaaaag aa                                                            12


<210>  577
<211>  12
<212>  RNA
<213>  Artificial Sequence

<220>
<223>  Description of Artificial Sequence: Synthetic oligonucleotide

<400>  577
uaaacccuua ua                                                            12


<210>  578
<211>  13
<212>  RNA
<213>  Artificial Sequence

<220>
<223>  Description of Artificial Sequence: Synthetic oligonucleotide

<400>  578
cauacuauua gcc                                                           13


<210>  579
<211>  12
<212>  RNA
<213>  Artificial Sequence

<220>
<223>  Description of Artificial Sequence: Synthetic oligonucleotide

<400>  579
gacagaagca uu                                                            12

<210>  580
<211>  14
<212>  RNA
<213>  Artificial Sequence

<220>
<223>  Description of Artificial Sequence: Synthetic oligonucleotide

<400>  580
agagcaaaua agau                                                    14


<210>  581
<211>  14
<212>  RNA
<213>  Artificial Sequence

<220>
<223>  Description of Artificial Sequence: Synthetic oligonucleotide

<400>  581
ccaacauuuu ucuc                                                    14


<210>  582
<211>  15
<212>  RNA
<213>  Artificial Sequence

<220>
<223>  Description of Artificial Sequence: Synthetic oligonucleotide

<400>  582
uuaaguauga gaaaa                                                   15


<210>  583
<211>  14
<212>  RNA
<213>  Artificial Sequence

<220>
<223>  Description of Artificial Sequence: Synthetic oligonucleotide

<400>  583
caggaauaaa gaug                                                    14


<210>  584
<211>  13
<212>  RNA
<213>  Artificial Sequence

<220>
<223>  Description of Artificial Sequence: Synthetic oligonucleotide

<400>  584
aauuugaaug acc                                                     13


<210>  585

<211>  14
<212>  RNA
<213>  Artificial Sequence

<220>
<223>  Description of Artificial Sequence: Synthetic oligonucleotide

<400>  585
auguauaaag auag                                                    14


<210>  586
<211>  12
<212>  RNA
<213>  Artificial Sequence

<220>
<223>  Description of Artificial Sequence: Synthetic oligonucleotide

<400>  586
ggcuguaacu au                                                      12


<210>  587
<211>  11
<212>  RNA
<213>  Artificial Sequence

<220>
<223>  Description of Artificial Sequence: Synthetic oligonucleotide

<400>  587
ucugugaagu g                                                       11


<210>  588
<211>  14
<212>  RNA
<213>  Artificial Sequence

<220>
<223>  Description of Artificial Sequence: Synthetic oligonucleotide

<400>  588
ccuuuaagga aaug                                                    14


<210>  589
<211>  13
<212>  RNA
<213>  Artificial Sequence

<220>
<223>  Description of Artificial Sequence: Synthetic oligonucleotide

<400>  589
aaggauacaa aaa                                                     13


<210>  590
<211>  12

<212>  RNA
<213>  Artificial Sequence

<220>
<223>  Description of Artificial Sequence: Synthetic oligonucleotide

<400>  590
agaugcaauu ug                                                          12


<210>  591
<211>  12
<212>  RNA
<213>  Artificial Sequence

<220>
<223>  Description of Artificial Sequence: Synthetic oligonucleotide

<400>  591
guucaaaacg aa                                                          12


<210>  592
<211>  11
<212>  RNA
<213>  Artificial Sequence

<220>
<223>  Description of Artificial Sequence: Synthetic oligonucleotide

<400>  592
gugaaaccuc a                                                           11


<210>  593
<211>  13
<212>  RNA
<213>  Artificial Sequence

<220>
<223>  Description of Artificial Sequence: Synthetic oligonucleotide

<400>  593
acgaaagaga agc                                                         13


<210>  594
<211>  12
<212>  RNA
<213>  Artificial Sequence

<220>
<223>  Description of Artificial Sequence: Synthetic oligonucleotide

<400>  594
cuccacaagc gc                                                          12


<210>  595
<211>  14
<212>  RNA

<213>  Artificial Sequence

<220>
<223>  Description of Artificial Sequence: Synthetic oligonucleotide

<400>  595
gagaagauuc caaa                                                          14


<210>  596
<211>  13
<212>  RNA
<213>  Artificial Sequence

<220>
<223>  Description of Artificial Sequence: Synthetic oligonucleotide

<400>  596
aaucuggauu caa                                                           13


<210>  597
<211>  13
<212>  RNA
<213>  Artificial Sequence

<220>
<223>  Description of Artificial Sequence: Synthetic oligonucleotide

<400>  597
agaacagauu uga                                                           13


<210>  598
<211>  11
<212>  RNA
<213>  Artificial Sequence

<220>
<223>  Description of Artificial Sequence: Synthetic oligonucleotide

<400>  598
ccagagcugu g                                                             11


<210>  599
<211>  12
<212>  RNA
<213>  Artificial Sequence

<220>
<223>  Description of Artificial Sequence: Synthetic oligonucleotide

<400>  599
ccucagauug uu                                                            12


<210>  600
<211>  12
<212>  RNA
<213>  Artificial Sequence

<220>
<223>  Description of Artificial Sequence: Synthetic oligonucleotide

<400>  600
cuauuuuaau ua                                                          12


<210>  601
<211>  13
<212>  RNA
<213>  Artificial Sequence

<220>
<223>  Description of Artificial Sequence: Synthetic oligonucleotide

<400>  601
uuuaauuuau uaa                                                         13


<210>  602
<211>  12
<212>  RNA
<213>  Artificial Sequence

<220>
<223>  Description of Artificial Sequence: Synthetic oligonucleotide

<400>  602
augcaguuug aa                                                          12


<210>  603
<211>  11
<212>  RNA
<213>  Artificial Sequence

<220>
<223>  Description of Artificial Sequence: Synthetic oligonucleotide

<400>  603
caugcugcug g                                                           11


<210>  604
<211>  13
<212>  RNA
<213>  Artificial Sequence

<220>
<223>  Description of Artificial Sequence: Synthetic oligonucleotide

<400>  604
agaugugcau uuc                                                         13


<210>  605
<211>  12
<212>  RNA
<213>  Artificial Sequence

<220>
<223>  Description of Artificial Sequence: Synthetic oligonucleotide

<400>  605
ucaaaaccug ug                                                          12


<210>  606
<211>  11
<212>  RNA
<213>  Artificial Sequence

<220>
<223>  Description of Artificial Sequence: Synthetic oligonucleotide

<400>  606
gaaugugggu a                                                           11


<210>  607
<211>  10
<212>  RNA
<213>  Artificial Sequence

<220>
<223>  Description of Artificial Sequence: Synthetic oligonucleotide

<400>  607
   auguggguag                                                              10


<210>  608
<211>  13
<212>  RNA
<213>  Artificial Sequence

<220>
<223>  Description of Artificial Sequence: Synthetic oligonucleotide

<400>  608
ugaaaaugag cau                                                         13


<210>  609
<211>  13
<212>  RNA
<213>  Artificial Sequence

<220>
<223>  Description of Artificial Sequence: Synthetic oligonucleotide

<400>  609
uugcuuuuca ugu                                                         13


<210>  610
<211>  12
<212>  RNA
<213>  Artificial Sequence

<220>

<223>  Description of Artificial Sequence: Synthetic oligonucleotide

<400>  610
uguauuucua ua                                                          12


<210>  611
<211>  13
<212>  RNA
<213>  Artificial Sequence

<220>
<223>  Description of Artificial Sequence: Synthetic oligonucleotide

<400>  611
uuugauuaau guu                                                         13


<210>  612
<211>  12
<212>  RNA
<213>  Artificial Sequence

<220>
<223>  Description of Artificial Sequence: Synthetic oligonucleotide

<400>  612
caacguguau ag                                                          12


<210>  613
<211>  12
<212>  RNA
<213>  Artificial Sequence

<220>
<223>  Description of Artificial Sequence: Synthetic oligonucleotide

<400>  613
cauauccuug gc                                                          12


<210>  614
<211>  13
<212>  RNA
<213>  Artificial Sequence

<220>
<223>  Description of Artificial Sequence: Synthetic oligonucleotide

<400>  614
uacuaacauc ugg                                                         13


<210>  615
<211>  11
<212>  RNA
<213>  Artificial Sequence

<220>
<223>  Description of Artificial Sequence: Synthetic oligonucleotide

<400>  615
cauaaguugu g                                                            11


<210>  616
<211>  12
<212>  RNA
<213>  Artificial Sequence

<220>
<223>  Description of Artificial Sequence: Synthetic oligonucleotide

<400>  616
gcaucauuuu gg                                                           12


<210>  617
<211>  10
<212>  RNA
<213>  Artificial Sequence

<220>
<223>  Description of Artificial Sequence: Synthetic oligonucleotide

<400>  617
gcucuucuua                                                              10


<210>  618
<211>  10
<212>  RNA
<213>  Artificial Sequence

<220>
<223>  Description of Artificial Sequence: Synthetic oligonucleotide

<400>  618
agauuagguc                                                              10


<210>  619
<211>  12
<212>  RNA
<213>  Artificial Sequence

<220>
<223>  Description of Artificial Sequence: Synthetic oligonucleotide

<400>  619
auggaauuga aa                                                           12


<210>  620
<211>  13
<212>  RNA
<213>  Artificial Sequence

<220>
<223>  Description of Artificial Sequence: Synthetic oligonucleotide

<400>  620
cacacucauu ccu                                                          13


<210>  621
<211>  12
<212>  RNA
<213>  Artificial Sequence

<220>
<223>  Description of Artificial Sequence: Synthetic oligonucleotide

<400>  621
uguagaggua ac                                                           12


<210>  622
<211>  11
<212>  RNA
<213>  Artificial Sequence

<220>
<223>  Description of Artificial Sequence: Synthetic oligonucleotide

<400>  622
caaccacaug c                                                            11


<210>  623
<211>  12
<212>  RNA
<213>  Artificial Sequence

<220>
<223>  Description of Artificial Sequence: Synthetic oligonucleotide

<400>  623
ucggaaacaa gu                                                           12


<210>  624
<211>  11
<212>  RNA
<213>  Artificial Sequence

<220>
<223>  Description of Artificial Sequence: Synthetic oligonucleotide

<400>  624
acucccaaua a                                                            11


<210>  625
<211>  13
<212>  RNA
<213>  Artificial Sequence

<220>
<223>  Description of Artificial Sequence: Synthetic oligonucleotide

<400>  625

aaugcuaaga aau                                                          13

<210>  626
<211>  11
<212>  RNA
<213>  Artificial Sequence

<220>
<223>  Description of Artificial Sequence: Synthetic oligonucleotide

<400>  626
gucuuucucu a                                                            11

<210>  627
<211>  11
<212>  RNA
<213>  Artificial Sequence

<220>
<223>  Description of Artificial Sequence: Synthetic oligonucleotide

<400>  627
ugaauuucag g                                                            11

<210>  628
<211>  13
<212>  RNA
<213>  Artificial Sequence

<220>
<223>  Description of Artificial Sequence: Synthetic oligonucleotide

<400>  628
cgauucccuc uca                                                          13

<210>  629
<211>  11
<212>  RNA
<213>  Artificial Sequence

<220>
<223>  Description of Artificial Sequence: Synthetic oligonucleotide

<400>  629
aggaaaguga a                                                            11

<210>  630
<211>  12
<212>  RNA
<213>  Artificial Sequence

<220>
<223>  Description of Artificial Sequence: Synthetic oligonucleotide

<400>  630
gaggcugcau gc                                                           12

<210>  631
<211>  11
<212>  RNA
<213>  Artificial Sequence

<220>
<223>  Description of Artificial Sequence: Synthetic oligonucleotide

<400>  631
ucucugaaca g                                                            11


<210>  632
<211>  12
<212>  RNA
<213>  Artificial Sequence

<220>
<223>  Description of Artificial Sequence: Synthetic oligonucleotide

<400>  632
aacuuggcug ua                                                           12


<210>  633
<211>  12
<212>  RNA
<213>  Artificial Sequence

<220>
<223>  Description of Artificial Sequence: Synthetic oligonucleotide

<400>  633
agaagccaaa au                                                           12


<210>  634
<211>  14
<212>  RNA
<213>  Artificial Sequence

<220>
<223>  Description of Artificial Sequence: Synthetic oligonucleotide

<400>  634
auuaaaagaa gucc                                                         14


<210>  635
<211>  14
<212>  RNA
<213>  Artificial Sequence

<220>
<223>  Description of Artificial Sequence: Synthetic oligonucleotide

<400>  635
gaauccggau uauc                                                         14

<210>  636
<211>  12
<212>  RNA
<213>  Artificial Sequence

<220>
<223>  Description of Artificial Sequence: Synthetic oligonucleotide

<400>  636
aaugugacau ca                                                          12


<210>  637
<211>  13
<212>  RNA
<213>  Artificial Sequence

<220>
<223>  Description of Artificial Sequence: Synthetic oligonucleotide

<400>  637
caucaaagca agu                                                         13


<210>  638
<211>  11
<212>  RNA
<213>  Artificial Sequence

<220>
<223>  Description of Artificial Sequence: Synthetic oligonucleotide

<400>  638
agagagagaa a                                                           11


<210>  639
<211>  13
<212>  RNA
<213>  Artificial Sequence

<220>
<223>  Description of Artificial Sequence: Synthetic oligonucleotide

<400>  639
ucgcuguagu auu                                                         13


<210>  640
<211>  14
<212>  RNA
<213>  Artificial Sequence

<220>
<223>  Description of Artificial Sequence: Synthetic oligonucleotide

<400>  640
cgcuguagua uuua                                                        14

<210>  641
<211>  13
<212>  RNA
<213>  Artificial Sequence

<220>
<223>  Description of Artificial Sequence: Synthetic oligonucleotide

<400>  641
auuuaagccc aua                                                          13


<210>  642
<211>  15
<212>  RNA
<213>  Artificial Sequence

<220>
<223>  Description of Artificial Sequence: Synthetic oligonucleotide

<400>  642
auuaaaauaa acaug                                                        15


<210>  643
<211>  12
<212>  RNA
<213>  Artificial Sequence

<220>
<223>  Description of Artificial Sequence: Synthetic oligonucleotide

<400>  643
cuguucugau cg                                                           12


<210>  644
<211>  16
<212>  RNA
<213>  Artificial Sequence

<220>
<223>  Description of Artificial Sequence: Synthetic oligonucleotide

<400>  644
aaauaauuug aacuuu                                                       16


<210>  645
<211>  12
<212>  RNA
<213>  Artificial Sequence

<220>
<223>  Description of Artificial Sequence: Synthetic oligonucleotide

<400>  645
ugcgaccuua au                                                           12


<210>  646

<211>  11
<212>  RNA
<213>  Artificial Sequence

<220>
<223>  Description of Artificial Sequence: Synthetic oligonucleotide

<400>  646
cugagaaagc u                                                          11


<210>  647
<211>  13
<212>  RNA
<213>  Artificial Sequence

<220>
<223>  Description of Artificial Sequence: Synthetic oligonucleotide

<400>  647
aguaaagaug cua                                                        13


<210>  648
<211>  12
<212>  RNA
<213>  Artificial Sequence

<220>
<223>  Description of Artificial Sequence: Synthetic oligonucleotide

<400>  648
cugcuuaauu gc                                                         12


<210>  649
<211>  14
<212>  RNA
<213>  Artificial Sequence

<220>
<223>  Description of Artificial Sequence: Synthetic oligonucleotide

<400>  649
uaccauauga auga                                                       14


<210>  650
<211>  12
<212>  RNA
<213>  Artificial Sequence

<220>
<223>  Description of Artificial Sequence: Synthetic oligonucleotide

<400>  650
aacuuucuua uc                                                         12


<210>  651
<211>  13

<212>  RNA
<213>  Artificial Sequence

<220>
<223>  Description of Artificial Sequence: Synthetic oligonucleotide

<400>  651
ccuugcauga cau                                                          13


<210>  652
<211>  14
<212>  RNA
<213>  Artificial Sequence

<220>
<223>  Description of Artificial Sequence: Synthetic oligonucleotide

<400>  652
ugaauuugua uaug                                                         14


<210>  653
<211>  12
<212>  RNA
<213>  Artificial Sequence

<220>
<223>  Description of Artificial Sequence: Synthetic oligonucleotide

<400>  653
gaauccuagu ag                                                           12


<210>  654
<211>  10
<212>  RNA
<213>  Artificial Sequence

<220>
<223>  Description of Artificial Sequence: Synthetic oligonucleotide

<400>  654
gaaagggaag                                                              10


<210>  655
<211>  11
<212>  RNA
<213>  Artificial Sequence

<220>
<223>  Description of Artificial Sequence: Synthetic oligonucleotide

<400>  655
uaucggcaug c                                                            11


<210>  656
<211>  12
<212>  RNA

<213>  Artificial Sequence

<220>
<223>  Description of Artificial Sequence: Synthetic oligonucleotide

<400>  656
caccucauag ua                                                          12


<210>  657
<211>  11
<212>  RNA
<213>  Artificial Sequence

<220>
<223>  Description of Artificial Sequence: Synthetic oligonucleotide

<400>  657
ccagaauugc c                                                           11


<210>  658
<211>  11
<212>  RNA
<213>  Artificial Sequence

<220>
<223>  Description of Artificial Sequence: Synthetic oligonucleotide

<400>  658
uggugaucug g                                                           11


<210>  659
<211>  12
<212>  RNA
<213>  Artificial Sequence

<220>
<223>  Description of Artificial Sequence: Synthetic oligonucleotide

<400>  659
gaucugggua au                                                          12


<210>  660
<211>  12
<212>  RNA
<213>  Artificial Sequence

<220>
<223>  Description of Artificial Sequence: Synthetic oligonucleotide

<400>  660
ggaugugaug aa                                                          12


<210>  661
<211>  12
<212>  RNA
<213>  Artificial Sequence

<220>
<223>  Description of Artificial Sequence: Synthetic oligonucleotide

<400>  661
cauuuccaca gc                                                          12


<210>  662
<211>  11
<212>  RNA
<213>  Artificial Sequence

<220>
<223>  Description of Artificial Sequence: Synthetic oligonucleotide

<400>  662
acagcuacac c                                                           11


<210>  663
<211>  12
<212>  RNA
<213>  Artificial Sequence

<220>
<223>  Description of Artificial Sequence: Synthetic oligonucleotide

<400>  663
ugcaguucuu ac                                                          12


<210>  664
<211>  12
<212>  RNA
<213>  Artificial Sequence

<220>
<223>  Description of Artificial Sequence: Synthetic oligonucleotide

<400>  664
acgagaagaa ga                                                          12


<210>  665
<211>  12
<212>  RNA
<213>  Artificial Sequence

<220>
<223>  Description of Artificial Sequence: Synthetic oligonucleotide

<400>  665
cgagaagaag au                                                          12


<210>  666
<211>  15
<212>  RNA
<213>  Artificial Sequence

<220>
<223>  Description of Artificial Sequence: Synthetic oligonucleotide

<400>  666
aagaagauca uuuac                                                    15


<210>  667
<211>  11
<212>  RNA
<213>  Artificial Sequence

<220>
<223>  Description of Artificial Sequence: Synthetic oligonucleotide

<400>  667
agaggaagag g                                                        11


<210>  668
<211>  12
<212>  RNA
<213>  Artificial Sequence

<220>
<223>  Description of Artificial Sequence: Synthetic oligonucleotide

<400>  668
gaggaagagg ug                                                       12


<210>  669
<211>  13
<212>  RNA
<213>  Artificial Sequence

<220>
<223>  Description of Artificial Sequence: Synthetic oligonucleotide

<400>  669
cuacuuugag ggc                                                      13


<210>  670
<211>  12
<212>  RNA
<213>  Artificial Sequence

<220>
<223>  Description of Artificial Sequence: Synthetic oligonucleotide

<400>  670
agggcaucuc cu                                                       12


<210>  671
<211>  10
<212>  RNA
<213>  Artificial Sequence

<220>

<223>  Description of Artificial Sequence: Synthetic oligonucleotide

<400>  671
ggagugauau                                                              10


<210>  672
<211>  11
<212>  RNA
<213>  Artificial Sequence

<220>
<223>  Description of Artificial Sequence: Synthetic oligonucleotide

<400>  672
gagugauaug g                                                            11


<210>  673
<211>  12
<212>  RNA
<213>  Artificial Sequence

<220>
<223>  Description of Artificial Sequence: Synthetic oligonucleotide

<400>  673
ugcaguaaag au                                                           12


<210>  674
<211>  13
<212>  RNA
<213>  Artificial Sequence

<220>
<223>  Description of Artificial Sequence: Synthetic oligonucleotide

<400>  674
aguaaagauc cua                                                          13


<210>  675
<211>  12
<212>  RNA
<213>  Artificial Sequence

<220>
<223>  Description of Artificial Sequence: Synthetic oligonucleotide

<400>  675
ugacaaagga ca                                                           12


<210>  676
<211>  13
<212>  RNA
<213>  Artificial Sequence

<220>
<223>  Description of Artificial Sequence: Synthetic oligonucleotide

<400>  676
gcaauuguga ccc                                                          13


<210>  677
<211>  12
<212>  RNA
<213>  Artificial Sequence

<220>
<223>  Description of Artificial Sequence: Synthetic oligonucleotide

<400>  677
aacaucaucc au                                                           12


<210>  678
<211>  12
<212>  RNA
<213>  Artificial Sequence

<220>
<223>  Description of Artificial Sequence: Synthetic oligonucleotide

<400>  678
ugaaauccaa ca                                                           12


<210>  679
<211>  15
<212>  RNA
<213>  Artificial Sequence

<220>
<223>  Description of Artificial Sequence: Synthetic oligonucleotide

<400>  679
aacaauauau uucuc                                                        15


<210>  680
<211>  14
<212>  RNA
<213>  Artificial Sequence

<220>
<223>  Description of Artificial Sequence: Synthetic oligonucleotide

<400>  680
aacaguaaag ucac                                                         14


<210>  681
<211>  14
<212>  RNA
<213>  Artificial Sequence

<220>
<223>  Description of Artificial Sequence: Synthetic oligonucleotide

<400>  681
ugugaagaaa guaa                                                         14

<210>  682
<211>  13
<212>  RNA
<213>  Artificial Sequence

<220>
<223>  Description of Artificial Sequence: Synthetic oligonucleotide

<400>  682
cuuccgagcc auc                                                          13

<210>  683
<211>  12
<212>  RNA
<213>  Artificial Sequence

<220>
<223>  Description of Artificial Sequence: Synthetic oligonucleotide

<400>  683
aauggagguu ga                                                           12

<210>  684
<211>  12
<212>  RNA
<213>  Artificial Sequence

<220>
<223>  Description of Artificial Sequence: Synthetic oligonucleotide

<400>  684
ggagguugaa ua                                                           12

<210>  685
<211>  13
<212>  RNA
<213>  Artificial Sequence

<220>
<223>  Description of Artificial Sequence: Synthetic oligonucleotide

<400>  685
auuuugaguu uuc                                                          13

<210>  686
<211>  12
<212>  RNA
<213>  Artificial Sequence

<220>
<223>  Description of Artificial Sequence: Synthetic oligonucleotide

<400>  686

uauccuguuu gu                                                          12

<210>  687
<211>  11
<212>  RNA
<213>  Artificial Sequence

<220>
<223>  Description of Artificial Sequence: Synthetic oligonucleotide

<400>  687
ccuguuuguu c                                                           11

<210>  688
<211>  10
<212>  RNA
<213>  Artificial Sequence

<220>
<223>  Description of Artificial Sequence: Synthetic oligonucleotide

<400>  688
cucuacagcc                                                             10

<210>  689
<211>  12
<212>  RNA
<213>  Artificial Sequence

<220>
<223>  Description of Artificial Sequence: Synthetic oligonucleotide

<400>  689
ucuuccauag cu                                                          12

<210>  690
<211>  12
<212>  RNA
<213>  Artificial Sequence

<220>
<223>  Description of Artificial Sequence: Synthetic oligonucleotide

<400>  690
auaaucuucc ug                                                          12

<210>  691
<211>  12
<212>  RNA
<213>  Artificial Sequence

<220>
<223>  Description of Artificial Sequence: Synthetic oligonucleotide

<400>  691
uaaucuuccu gu                                                          12

<210>  692
<211>  12
<212>  RNA
<213>  Artificial Sequence

<220>
<223>  Description of Artificial Sequence: Synthetic oligonucleotide

<400>  692
cuucaugacu ug                                                          12


<210>  693
<211>  12
<212>  RNA
<213>  Artificial Sequence

<220>
<223>  Description of Artificial Sequence: Synthetic oligonucleotide

<400>  693
aagagggaga ga                                                          12


<210>  694
<211>  12
<212>  RNA
<213>  Artificial Sequence

<220>
<223>  Description of Artificial Sequence: Synthetic oligonucleotide

<400>  694
cgucuccuac ca                                                          12


<210>  695
<211>  12
<212>  RNA
<213>  Artificial Sequence

<220>
<223>  Description of Artificial Sequence: Synthetic oligonucleotide

<400>  695
gaucaaucgg cc                                                          12


<210>  696
<211>  12
<212>  RNA
<213>  Artificial Sequence

<220>
<223>  Description of Artificial Sequence: Synthetic oligonucleotide

<400>  696
ggacgaacau cc                                                          12

<210>  697
<211>  11
<212>  RNA
<213>  Artificial Sequence

<220>
<223>  Description of Artificial Sequence: Synthetic oligonucleotide

<400>  697
agggucggaa c                                                            11


<210>  698
<211>  10
<212>  RNA
<213>  Artificial Sequence

<220>
<223>  Description of Artificial Sequence: Synthetic oligonucleotide

<400>  698
ucggaaccca                                                              10


<210>  699
<211>  11
<212>  RNA
<213>  Artificial Sequence

<220>
<223>  Description of Artificial Sequence: Synthetic oligonucleotide

<400>  699
acccaagcuu a                                                            11


<210>  700
<211>  14
<212>  RNA
<213>  Artificial Sequence

<220>
<223>  Description of Artificial Sequence: Synthetic oligonucleotide

<400>  700
gaacuuuaag caac                                                         14


<210>  701
<211>  11
<212>  RNA
<213>  Artificial Sequence

<220>
<223>  Description of Artificial Sequence: Synthetic oligonucleotide

<400>  701
acuauucagu g                                                            11

<210>  702
<211>  11
<212>  RNA
<213>  Artificial Sequence

<220>
<223>  Description of Artificial Sequence: Synthetic oligonucleotide

<400>  702
cuauucagug g                                                            11


<210>  703
<211>  13
<212>  RNA
<213>  Artificial Sequence

<220>
<223>  Description of Artificial Sequence: Synthetic oligonucleotide

<400>  703
aaacacagau aac                                                          13


<210>  704
<211>  12
<212>  RNA
<213>  Artificial Sequence

<220>
<223>  Description of Artificial Sequence: Synthetic oligonucleotide

<400>  704
cuuaagaaaa ga                                                           12


<210>  705
<211>  12
<212>  RNA
<213>  Artificial Sequence

<220>
<223>  Description of Artificial Sequence: Synthetic oligonucleotide

<400>  705
cugaaggagu gu                                                           12


<210>  706
<211>  10
<212>  RNA
<213>  Artificial Sequence

<220>
<223>  Description of Artificial Sequence: Synthetic oligonucleotide

<400>  706
ucaccgcggg                                                              10


<210>  707

<211>  14
<212>  RNA
<213>  Artificial Sequence

<220>
<223>  Description of Artificial Sequence: Synthetic oligonucleotide

<400>  707
agcagaaaua agcg                                                          14


<210>  708
<211>  12
<212>  RNA
<213>  Artificial Sequence

<220>
<223>  Description of Artificial Sequence: Synthetic oligonucleotide

<400>  708
aagcgugccg uu                                                            12


<210>  709
<211>  12
<212>  RNA
<213>  Artificial Sequence

<220>
<223>  Description of Artificial Sequence: Synthetic oligonucleotide

<400>  709
agaaacaguc ac                                                            12


<210>  710
<211>  12
<212>  RNA
<213>  Artificial Sequence

<220>
<223>  Description of Artificial Sequence: Synthetic oligonucleotide

<400>  710
agaggaagaa gg                                                            12


<210>  711
<211>  14
<212>  RNA
<213>  Artificial Sequence

<220>
<223>  Description of Artificial Sequence: Synthetic oligonucleotide

<400>  711
uguauucaaa auau                                                          14


<210>  712
<211>  13

<212>  RNA
<213>  Artificial Sequence

<220>
<223>  Description of Artificial Sequence: Synthetic oligonucleotide

<400>  712
auuuuccucc cuu                                                          13


<210>  713
<211>  13
<212>  RNA
<213>  Artificial Sequence

<220>
<223>  Description of Artificial Sequence: Synthetic oligonucleotide

<400>  713
caaagaaaga uag                                                          13


<210>  714
<211>  12
<212>  RNA
<213>  Artificial Sequence

<220>
<223>  Description of Artificial Sequence: Synthetic oligonucleotide

<400>  714
aagaaagaua ga                                                           12


<210>  715
<211>  13
<212>  RNA
<213>  Artificial Sequence

<220>
<223>  Description of Artificial Sequence: Synthetic oligonucleotide

<400>  715
gaaagcauuu guu                                                          13


<210>  716
<211>  13
<212>  RNA
<213>  Artificial Sequence

<220>
<223>  Description of Artificial Sequence: Synthetic oligonucleotide

<400>  716
ugaguuaaac gaa                                                          13


<210>  717
<211>  12
<212>  RNA

<213>  Artificial Sequence

<220>
<223>  Description of Artificial Sequence: Synthetic oligonucleotide

<400>  717
acugauacag aa                                                          12


<210>  718
<211>  12
<212>  RNA
<213>  Artificial Sequence

<220>
<223>  Description of Artificial Sequence: Synthetic oligonucleotide

<400>  718
auauuauaua ua                                                          12


<210>  719
<211>  12
<212>  RNA
<213>  Artificial Sequence

<220>
<223>  Description of Artificial Sequence: Synthetic oligonucleotide

<400>  719
auuauauaua ua                                                          12


<210>  720
<211>  12
<212>  RNA
<213>  Artificial Sequence

<220>
<223>  Description of Artificial Sequence: Synthetic oligonucleotide

<400>  720
ucacuggaug ua                                                          12


<210>  721
<211>  12
<212>  RNA
<213>  Artificial Sequence

<220>
<223>  Description of Artificial Sequence: Synthetic oligonucleotide

<400>  721
cagggaagag ga                                                          12


<210>  722
<211>  15
<212>  RNA
<213>  Artificial Sequence

<220>
<223>  Description of Artificial Sequence: Synthetic oligonucleotide

<400>  722
augaucuuuu uuuug                                                        15


<210>  723
<211>  11
<212>  RNA
<213>  Artificial Sequence

<220>
<223>  Description of Artificial Sequence: Synthetic oligonucleotide

<400>  723
aguucacggc c                                                            11


<210>  724
<211>  13
<212>  RNA
<213>  Artificial Sequence

<220>
<223>  Description of Artificial Sequence: Synthetic oligonucleotide

<400>  724
caccaucaca cca                                                          13


<210>  725
<211>  11
<212>  RNA
<213>  Artificial Sequence

<220>
<223>  Description of Artificial Sequence: Synthetic oligonucleotide

<400>  725
ccugcggguu u                                                            11


<210>  726
<211>  10
<212>  RNA
<213>  Artificial Sequence

<220>
<223>  Description of Artificial Sequence: Synthetic oligonucleotide

<400>  726
auccaguuug                                                              10


<210>  727
<211>  13
<212>  RNA
<213>  Artificial Sequence

<220>
<223>  Description of Artificial Sequence: Synthetic oligonucleotide

<400>  727
guuaaggacu ucu                                                          13


<210>  728
<211>  13
<212>  RNA
<213>  Artificial Sequence

<220>
<223>  Description of Artificial Sequence: Synthetic oligonucleotide

<400>  728
cagugggaca gag                                                          13


<210>  729
<211>  14
<212>  RNA
<213>  Artificial Sequence

<220>
<223>  Description of Artificial Sequence: Synthetic oligonucleotide

<400>  729
aauuuguuau ugug                                                         14


<210>  730
<211>  13
<212>  RNA
<213>  Artificial Sequence

<220>
<223>  Description of Artificial Sequence: Synthetic oligonucleotide

<400>  730
uucaaaugca uuu                                                          13


<210>  731
<211>  10
<212>  RNA
<213>  Artificial Sequence

<220>
<223>  Description of Artificial Sequence: Synthetic oligonucleotide

<400>  731
caggaaagcc                                                              10


<210>  732
<211>  11
<212>  RNA
<213>  Artificial Sequence

<220>

<223>  Description of Artificial Sequence: Synthetic oligonucleotide

<400>  732
aggaaagccc u                                                            11


<210>  733
<211>  12
<212>  RNA
<213>  Artificial Sequence

<220>
<223>  Description of Artificial Sequence: Synthetic oligonucleotide

<400>  733
cccagcaugg cc                                                           12


<210>  734
<211>  11
<212>  RNA
<213>  Artificial Sequence

<220>
<223>  Description of Artificial Sequence: Synthetic oligonucleotide

<400>  734
cuucucccug a                                                            11


<210>  735
<211>  12
<212>  RNA
<213>  Artificial Sequence

<220>
<223>  Description of Artificial Sequence: Synthetic oligonucleotide

<400>  735
gaagaagccc uu                                                           12


<210>  736
<211>  12
<212>  RNA
<213>  Artificial Sequence

<220>
<223>  Description of Artificial Sequence: Synthetic oligonucleotide

<400>  736
agaagcccuu ca                                                           12


<210>  737
<211>  11
<212>  RNA
<213>  Artificial Sequence

<220>
<223>  Description of Artificial Sequence: Synthetic oligonucleotide

<400>  737
gcccuucagc g                                                            11


<210>  738
<211>  11
<212>  RNA
<213>  Artificial Sequence

<220>
<223>  Description of Artificial Sequence: Synthetic oligonucleotide

<400>  738
ccuucagcgg c                                                            11


<210>  739
<211>  12
<212>  RNA
<213>  Artificial Sequence

<220>
<223>  Description of Artificial Sequence: Synthetic oligonucleotide

<400>  739
ucagcggcca gu                                                           12


<210>  740
<211>  12
<212>  RNA
<213>  Artificial Sequence

<220>
<223>  Description of Artificial Sequence: Synthetic oligonucleotide

<400>  740
ugaaaagcuc cg                                                           12


<210>  741
<211>  11
<212>  RNA
<213>  Artificial Sequence

<220>
<223>  Description of Artificial Sequence: Synthetic oligonucleotide

<400>  741
aaaagcuccg g                                                            11


<210>  742
<211>  13
<212>  RNA
<213>  Artificial Sequence

<220>
<223>  Description of Artificial Sequence: Synthetic oligonucleotide

<400>  742
agucaacagu cug                                                          13


<210>  743
<211>  13
<212>  RNA
<213>  Artificial Sequence

<220>
<223>  Description of Artificial Sequence: Synthetic oligonucleotide

<400>  743
cagaguucaa aag                                                          13


<210>  744
<211>  13
<212>  RNA
<213>  Artificial Sequence

<220>
<223>  Description of Artificial Sequence: Synthetic oligonucleotide

<400>  744
agaguucaaa agc                                                          13


<210>  745
<211>  12
<212>  RNA
<213>  Artificial Sequence

<220>
<223>  Description of Artificial Sequence: Synthetic oligonucleotide

<400>  745
uucaaaagcc cu                                                           12


<210>  746
<211>  12
<212>  RNA
<213>  Artificial Sequence

<220>
<223>  Description of Artificial Sequence: Synthetic oligonucleotide

<400>  746
ucaaaagccc uu                                                           12


<210>  747
<211>  12
<212>  RNA
<213>  Artificial Sequence

<220>
<223>  Description of Artificial Sequence: Synthetic oligonucleotide

<400>  747

aaaagcccuu ca                                                          12

<210>  748
<211>  12
<212>  RNA
<213>  Artificial Sequence

<220>
<223>  Description of Artificial Sequence: Synthetic oligonucleotide

<400>  748
aaagcccuuc ag                                                          12

<210>  749
<211>  13
<212>  RNA
<213>  Artificial Sequence

<220>
<223>  Description of Artificial Sequence: Synthetic oligonucleotide

<400>  749
gugaagccgc ucg                                                         13

<210>  750
<211>  12
<212>  RNA
<213>  Artificial Sequence

<220>
<223>  Description of Artificial Sequence: Synthetic oligonucleotide

<400>  750
cacgccaguc aa                                                          12

<210>  751
<211>  13
<212>  RNA
<213>  Artificial Sequence

<220>
<223>  Description of Artificial Sequence: Synthetic oligonucleotide

<400>  751
uucuuggugc gug                                                         13

<210>  752
<211>  14
<212>  RNA
<213>  Artificial Sequence

<220>
<223>  Description of Artificial Sequence: Synthetic oligonucleotide

<400>  752
auuggauuca ucag                                                        14

<210>  753
<211>  13
<212>  RNA
<213>  Artificial Sequence

<220>
<223>  Description of Artificial Sequence: Synthetic oligonucleotide

<400>  753
uggaauaccu aag                                                          13


<210>  754
<211>  12
<212>  RNA
<213>  Artificial Sequence

<220>
<223>  Description of Artificial Sequence: Synthetic oligonucleotide

<400>  754
auuugaggcu ca                                                           12


<210>  755
<211>  11
<212>  RNA
<213>  Artificial Sequence

<220>
<223>  Description of Artificial Sequence: Synthetic oligonucleotide

<400>  755
ucuacaauug g                                                            11


<210>  756
<211>  12
<212>  RNA
<213>  Artificial Sequence

<220>
<223>  Description of Artificial Sequence: Synthetic oligonucleotide

<400>  756
uaguuaggag cc                                                           12


<210>  757
<211>  13
<212>  RNA
<213>  Artificial Sequence

<220>
<223>  Description of Artificial Sequence: Synthetic oligonucleotide

<400>  757
agucaauauc cac                                                          13

<210>  758
<211>  13
<212>  RNA
<213>  Artificial Sequence

<220>
<223>  Description of Artificial Sequence: Synthetic oligonucleotide

<400>  758
auguucaguc aca                                                          13


<210>  759
<211>  12
<212>  RNA
<213>  Artificial Sequence

<220>
<223>  Description of Artificial Sequence: Synthetic oligonucleotide

<400>  759
acacacacau ac                                                           12


<210>  760
<211>  12
<212>  RNA
<213>  Artificial Sequence

<220>
<223>  Description of Artificial Sequence: Synthetic oligonucleotide

<400>  760
uuccuuuugc uu                                                           12


<210>  761
<211>  16
<212>  RNA
<213>  Artificial Sequence

<220>
<223>  Description of Artificial Sequence: Synthetic oligonucleotide

<400>  761
uuuuaaagua auuuuu                                                       16


<210>  762
<211>  13
<212>  RNA
<213>  Artificial Sequence

<220>
<223>  Description of Artificial Sequence: Synthetic oligonucleotide

<400>  762
caacagucaa ugg                                                          13

<210>  763
<211>  13
<212>  RNA
<213>  Artificial Sequence

<220>
<223>  Description of Artificial Sequence: Synthetic oligonucleotide

<400>  763
ucgacaaacc aau                                                          13


<210>  764
<211>  12
<212>  RNA
<213>  Artificial Sequence

<220>
<223>  Description of Artificial Sequence: Synthetic oligonucleotide

<400>  764
acugcuacca cu                                                           12


<210>  765
<211>  13
<212>  RNA
<213>  Artificial Sequence

<220>
<223>  Description of Artificial Sequence: Synthetic oligonucleotide

<400>  765
gcaagcgguc uua                                                          13


<210>  766
<211>  13
<212>  RNA
<213>  Artificial Sequence

<220>
<223>  Description of Artificial Sequence: Synthetic oligonucleotide

<400>  766
ggucuuaccg gcu                                                          13


<210>  767
<211>  11
<212>  RNA
<213>  Artificial Sequence

<220>
<223>  Description of Artificial Sequence: Synthetic oligonucleotide

<400>  767
gaugguuac c                                                             11


<210>  768

<211>  11
<212>  RNA
<213>  Artificial Sequence

<220>
<223>  Description of Artificial Sequence: Synthetic oligonucleotide

<400>  768
guuaccugcg a                                                            11


<210>  769
<211>  12
<212>  RNA
<213>  Artificial Sequence

<220>
<223>  Description of Artificial Sequence: Synthetic oligonucleotide

<400>  769
aaucuugcug ag                                                           12


<210>  770
<211>  13
<212>  RNA
<213>  Artificial Sequence

<220>
<223>  Description of Artificial Sequence: Synthetic oligonucleotide

<400>  770
agcauaaaac agu                                                          13


<210>  771
<211>  12
<212>  RNA
<213>  Artificial Sequence

<220>
<223>  Description of Artificial Sequence: Synthetic oligonucleotide

<400>  771
guccuuuauc cu                                                           12


<210>  772
<211>  12
<212>  RNA
<213>  Artificial Sequence

<220>
<223>  Description of Artificial Sequence: Synthetic oligonucleotide

<400>  772
caacgcaagu ug                                                           12


<210>  773
<211>  13

<212>  RNA
<213>  Artificial Sequence

<220>
<223>  Description of Artificial Sequence: Synthetic oligonucleotide

<400>  773
aaacugaaac cau                                                          13


<210>  774
<211>  14
<212>  RNA
<213>  Artificial Sequence

<220>
<223>  Description of Artificial Sequence: Synthetic oligonucleotide

<400>  774
acuuuguuaa auau                                                         14


<210>  775
<211>  14
<212>  RNA
<213>  Artificial Sequence

<220>
<223>  Description of Artificial Sequence: Synthetic oligonucleotide

<400>  775
guauauuaaa aguu                                                         14


<210>  776
<211>  13
<212>  RNA
<213>  Artificial Sequence

<220>
<223>  Description of Artificial Sequence: Synthetic oligonucleotide

<400>  776
guuuugacuu aac                                                          13


<210>  777
<211>  13
<212>  RNA
<213>  Artificial Sequence

<220>
<223>  Description of Artificial Sequence: Synthetic oligonucleotide

<400>  777
gcaaacucag cac                                                          13


<210>  778
<211>  11
<212>  RNA

<213>  Artificial Sequence

<220>
<223>  Description of Artificial Sequence: Synthetic oligonucleotide

<400>  778
ugcugaccuc a                                                            11


<210>  779
<211>  14
<212>  RNA
<213>  Artificial Sequence

<220>
<223>  Description of Artificial Sequence: Synthetic oligonucleotide

<400>  779
uuuauucaga ucgc                                                         14


<210>  780
<211>  13
<212>  RNA
<213>  Artificial Sequence

<220>
<223>  Description of Artificial Sequence: Synthetic oligonucleotide

<400>  780
aggguucugg gau                                                          13


<210>  781
<211>  15
<212>  RNA
<213>  Artificial Sequence

<220>
<223>  Description of Artificial Sequence: Synthetic oligonucleotide

<400>  781
auauuggaaa uuaug                                                        15


<210>  782
<211>  13
<212>  RNA
<213>  Artificial Sequence

<220>
<223>  Description of Artificial Sequence: Synthetic oligonucleotide

<400>  782
auggaagcac uag                                                          13


<210>  783
<211>  13
<212>  RNA
<213>  Artificial Sequence

<220>
<223>  Description of Artificial Sequence: Synthetic oligonucleotide

<400>  783
guuuuuaugu gga                                                          13


<210>  784
<211>  12
<212>  RNA
<213>  Artificial Sequence

<220>
<223>  Description of Artificial Sequence: Synthetic oligonucleotide

<400>  784
ggaauuggua ga                                                           12


<210>  785
<211>  13
<212>  RNA
<213>  Artificial Sequence

<220>
<223>  Description of Artificial Sequence: Synthetic oligonucleotide

<400>  785
uuagacuugg aga                                                          13


<210>  786
<211>  13
<212>  RNA
<213>  Artificial Sequence

<220>
<223>  Description of Artificial Sequence: Synthetic oligonucleotide

<400>  786
uuacaguauu cca                                                          13


<210>  787
<211>  15
<212>  RNA
<213>  Artificial Sequence

<220>
<223>  Description of Artificial Sequence: Synthetic oligonucleotide

<400>  787
ccacugauga auuaa                                                        15


<210>  788
<211>  12
<212>  RNA
<213>  Artificial Sequence

<220>
<223>  Description of Artificial Sequence: Synthetic oligonucleotide

<400>  788
ccacaucauc ac                                                          12


<210>  789
<211>  13
<212>  RNA
<213>  Artificial Sequence

<220>
<223>  Description of Artificial Sequence: Synthetic oligonucleotide

<400>  789
cucaccaaac aga                                                         13


<210>  790
<211>  12
<212>  RNA
<213>  Artificial Sequence

<220>
<223>  Description of Artificial Sequence: Synthetic oligonucleotide

<400>  790
ggaacaugau gg                                                          12


<210>  791
<211>  12
<212>  RNA
<213>  Artificial Sequence

<220>
<223>  Description of Artificial Sequence: Synthetic oligonucleotide

<400>  791
cauguagacu gc                                                          12


<210>  792
<211>  13
<212>  RNA
<213>  Artificial Sequence

<220>
<223>  Description of Artificial Sequence: Synthetic oligonucleotide

<400>  792
ggaucuauuu aug                                                         13


<210>  793
<211>  12
<212>  RNA
<213>  Artificial Sequence

<220>

<223>  Description of Artificial Sequence: Synthetic oligonucleotide

<400>  793
gcacuauuau ca                                                          12


<210>  794
<211>  11
<212>  RNA
<213>  Artificial Sequence

<220>
<223>  Description of Artificial Sequence: Synthetic oligonucleotide

<400>  794
aucaguucug c                                                           11


<210>  795
<211>  13
<212>  RNA
<213>  Artificial Sequence

<220>
<223>  Description of Artificial Sequence: Synthetic oligonucleotide

<400>  795
agauuaaacc uac                                                         13


<210>  796
<211>  13
<212>  RNA
<213>  Artificial Sequence

<220>
<223>  Description of Artificial Sequence: Synthetic oligonucleotide

<400>  796
auaaaauccu ucu                                                         13


<210>  797
<211>  12
<212>  RNA
<213>  Artificial Sequence

<220>
<223>  Description of Artificial Sequence: Synthetic oligonucleotide

<400>  797
aaugcuguag ac                                                          12


<210>  798
<211>  13
<212>  RNA
<213>  Artificial Sequence

<220>
<223>  Description of Artificial Sequence: Synthetic oligonucleotide

<400>  798
uugauaggaa uag                                                          13


<210>  799
<211>  11
<212>  RNA
<213>  Artificial Sequence

<220>
<223>  Description of Artificial Sequence: Synthetic oligonucleotide

<400>  799
aauggcccug a                                                            11


<210>  800
<211>  11
<212>  RNA
<213>  Artificial Sequence

<220>
<223>  Description of Artificial Sequence: Synthetic oligonucleotide

<400>  800
uuccucaagc a                                                            11


<210>  801
<211>  10
<212>  RNA
<213>  Artificial Sequence

<220>
<223>  Description of Artificial Sequence: Synthetic oligonucleotide

<400>  801
guucagccca                                                              10


<210>  802
<211>  11
<212>  RNA
<213>  Artificial Sequence

<220>
<223>  Description of Artificial Sequence: Synthetic oligonucleotide

<400>  802
gcugcugaac c                                                            11


<210>  803
<211>  12
<212>  RNA
<213>  Artificial Sequence

<220>
<223>  Description of Artificial Sequence: Synthetic oligonucleotide

<400>  803
aaguucucuu ca                                                          12


<210>  804
<211>  11
<212>  RNA
<213>  Artificial Sequence

<220>
<223>  Description of Artificial Sequence: Synthetic oligonucleotide

<400>  804
ccagccuaga g                                                           11


<210>  805
<211>  13
<212>  RNA
<213>  Artificial Sequence

<220>
<223>  Description of Artificial Sequence: Synthetic oligonucleotide

<400>  805
cucugugaag ugu                                                         13


<210>  806
<211>  14
<212>  RNA
<213>  Artificial Sequence

<220>
<223>  Description of Artificial Sequence: Synthetic oligonucleotide

<400>  806
ugagaaaauu ucaa                                                        14


<210>  807
<211>  11
<212>  RNA
<213>  Artificial Sequence

<220>
<223>  Description of Artificial Sequence: Synthetic oligonucleotide

<400>  807
aauccuccug a                                                           11


<210>  808
<211>  12
<212>  RNA
<213>  Artificial Sequence

<220>
<223>  Description of Artificial Sequence: Synthetic oligonucleotide

<400>  808

gugacaucau au                                                          12

<210>  809
<211>  13
<212>  RNA
<213>  Artificial Sequence

<220>
<223>  Description of Artificial Sequence: Synthetic oligonucleotide

<400>  809
aaucuucauc aua                                                         13

<210>  810
<211>  13
<212>  RNA
<213>  Artificial Sequence

<220>
<223>  Description of Artificial Sequence: Synthetic oligonucleotide

<400>  810
gacuagcuau uaa                                                         13

<210>  811
<211>  14
<212>  RNA
<213>  Artificial Sequence

<220>
<223>  Description of Artificial Sequence: Synthetic oligonucleotide

<400>  811
ucucuacuaa aaau                                                        14

<210>  812
<211>  12
<212>  RNA
<213>  Artificial Sequence

<220>
<223>  Description of Artificial Sequence: Synthetic oligonucleotide

<400>  812
ucuaucucgc cu                                                          12

<210>  813
<211>  13
<212>  RNA
<213>  Artificial Sequence

<220>
<223>  Description of Artificial Sequence: Synthetic oligonucleotide

<400>  813
cuucggucca guu                                                         13

<210>  814
<211>  11
<212>  RNA
<213>  Artificial Sequence

<220>
<223>  Description of Artificial Sequence: Synthetic oligonucleotide

<400>  814
gauguagccg c                                                            11


<210>  815
<211>  12
<212>  RNA
<213>  Artificial Sequence

<220>
<223>  Description of Artificial Sequence: Synthetic oligonucleotide

<400>  815
ugaggagacu ug                                                           12


<210>  816
<211>  12
<212>  RNA
<213>  Artificial Sequence

<220>
<223>  Description of Artificial Sequence: Synthetic oligonucleotide

<400>  816
gaguagugag ga                                                           12


<210>  817
<211>  14
<212>  RNA
<213>  Artificial Sequence

<220>
<223>  Description of Artificial Sequence: Synthetic oligonucleotide

<400>  817
cagaugagua caaa                                                         14


<210>  818
<211>  13
<212>  RNA
<213>  Artificial Sequence

<220>
<223>  Description of Artificial Sequence: Synthetic oligonucleotide

<400>  818
guuguuaaug ggc                                                          13

<210>  819
<211>  13
<212>  RNA
<213>  Artificial Sequence

<220>
<223>  Description of Artificial Sequence: Synthetic oligonucleotide

<400>  819
uuuuuaauuu auu                                                          13


<210>  820
<211>  12
<212>  RNA
<213>  Artificial Sequence

<220>
<223>  Description of Artificial Sequence: Synthetic oligonucleotide

<400>  820
uauuuaaaua ug                                                           12


<210>  821
<211>  13
<212>  RNA
<213>  Artificial Sequence

<220>
<223>  Description of Artificial Sequence: Synthetic oligonucleotide

<400>  821
uauccuuugu uuc                                                          13


<210>  822
<211>  14
<212>  RNA
<213>  Artificial Sequence

<220>
<223>  Description of Artificial Sequence: Synthetic oligonucleotide

<400>  822
cgucuaagug uuug                                                         14


<210>  823
<211>  12
<212>  RNA
<213>  Artificial Sequence

<220>
<223>  Description of Artificial Sequence: Synthetic oligonucleotide

<400>  823
accugugaca aa                                                           12

<210>  824
<211>  13
<212>  RNA
<213>  Artificial Sequence

<220>
<223>  Description of Artificial Sequence: Synthetic oligonucleotide

<400>  824
ccaggcugaa uua                                                          13


<210>  825
<211>  14
<212>  RNA
<213>  Artificial Sequence

<220>
<223>  Description of Artificial Sequence: Synthetic oligonucleotide

<400>  825
gucauucuua caau                                                         14


<210>  826
<211>  15
<212>  RNA
<213>  Artificial Sequence

<220>
<223>  Description of Artificial Sequence: Synthetic oligonucleotide

<400>  826
ucauucuuac aauug                                                        15


<210>  827
<211>  12
<212>  RNA
<213>  Artificial Sequence

<220>
<223>  Description of Artificial Sequence: Synthetic oligonucleotide

<400>  827
cagagagugu ac                                                           12


<210>  828
<211>  12
<212>  RNA
<213>  Artificial Sequence

<220>
<223>  Description of Artificial Sequence: Synthetic oligonucleotide

<400>  828
agaacucagc ag                                                           12


<210>  829

<211>  13
<212>  RNA
<213>  Artificial Sequence

<220>
<223>  Description of Artificial Sequence: Synthetic oligonucleotide

<400>  829
uuuauuuuca gua                                                          13


<210>  830
<211>  12
<212>  RNA
<213>  Artificial Sequence

<220>
<223>  Description of Artificial Sequence: Synthetic oligonucleotide

<400>  830
ucuuaaaugg aa                                                           12


<210>  831
<211>  13
<212>  RNA
<213>  Artificial Sequence

<220>
<223>  Description of Artificial Sequence: Synthetic oligonucleotide

<400>  831
ugccuaaaau ugu                                                          13


<210>  832
<211>  12
<212>  RNA
<213>  Artificial Sequence

<220>
<223>  Description of Artificial Sequence: Synthetic oligonucleotide

<400>  832
agacugugag cu                                                           12


<210>  833
<211>  14
<212>  RNA
<213>  Artificial Sequence

<220>
<223>  Description of Artificial Sequence: Synthetic oligonucleotide

<400>  833
ugcuuuuuau uaau                                                         14


<210>  834
<211>  13

<212>  RNA
<213>  Artificial Sequence

<220>
<223>  Description of Artificial Sequence: Synthetic oligonucleotide

<400>  834
cucuucuuac auu                                                          13


<210>  835
<211>  15
<212>  RNA
<213>  Artificial Sequence

<220>
<223>  Description of Artificial Sequence: Synthetic oligonucleotide

<400>  835
cauuuguaaa aaugu                                                        15


<210>  836
<211>  15
<212>  RNA
<213>  Artificial Sequence

<220>
<223>  Description of Artificial Sequence: Synthetic oligonucleotide

<400>  836
aucuuaauuc auauu                                                        15


<210>  837
<211>  13
<212>  RNA
<213>  Artificial Sequence

<220>
<223>  Description of Artificial Sequence: Synthetic oligonucleotide

<400>  837
gaacuaauca uga                                                          13


<210>  838
<211>  12
<212>  RNA
<213>  Artificial Sequence

<220>
<223>  Description of Artificial Sequence: Synthetic oligonucleotide

<400>  838
ucugcucuug gg                                                           12


<210>  839
<211>  13
<212>  RNA

<213>  Artificial Sequence

<220>
<223>  Description of Artificial Sequence: Synthetic oligonucleotide

<400>  839
caguagcuuu gag                                                          13


<210>  840
<211>  14
<212>  RNA
<213>  Artificial Sequence

<220>
<223>  Description of Artificial Sequence: Synthetic oligonucleotide

<400>  840
cacguaauau uuca                                                         14


<210>  841
<211>  13
<212>  RNA
<213>  Artificial Sequence

<220>
<223>  Description of Artificial Sequence: Synthetic oligonucleotide

<400>  841
uauucucuuc acu                                                          13


<210>  842
<211>  14
<212>  RNA
<213>  Artificial Sequence

<220>
<223>  Description of Artificial Sequence: Synthetic oligonucleotide

<400>  842
cuaaugcuaa gaaa                                                         14


<210>  843
<211>  12
<212>  RNA
<213>  Artificial Sequence

<220>
<223>  Description of Artificial Sequence: Synthetic oligonucleotide

<400>  843
gcugaaaauc aa                                                           12


<210>  844
<211>  14
<212>  RNA
<213>  Artificial Sequence

<220>
<223>  Description of Artificial Sequence: Synthetic oligonucleotide

<400>  844
aauaugauua cuuu                                                    14


<210>  845
<211>  14
<212>  RNA
<213>  Artificial Sequence

<220>
<223>  Description of Artificial Sequence: Synthetic oligonucleotide

<400>  845
cauuuuguuc uaca                                                    14


<210>  846
<211>  12
<212>  RNA
<213>  Artificial Sequence

<220>
<223>  Description of Artificial Sequence: Synthetic oligonucleotide

<400>  846
cccgggacuc uc                                                      12


<210>  847
<211>  14
<212>  RNA
<213>  Artificial Sequence

<220>
<223>  Description of Artificial Sequence: Synthetic oligonucleotide

<400>  847
ccuuuaaagu aaag                                                    14


<210>  848
<211>  13
<212>  RNA
<213>  Artificial Sequence

<220>
<223>  Description of Artificial Sequence: Synthetic oligonucleotide

<400>  848
ucuggaagcc ugg                                                     13


<210>  849
<211>  14
<212>  RNA
<213>  Artificial Sequence

<220>
<223>  Description of Artificial Sequence: Synthetic oligonucleotide

<400>  849
aaaacaaaag agag                                                    14


<210>  850
<211>  13
<212>  RNA
<213>  Artificial Sequence

<220>
<223>  Description of Artificial Sequence: Synthetic oligonucleotide

<400>  850
aucaguuaug ccg                                                     13


<210>  851
<211>  13
<212>  RNA
<213>  Artificial Sequence

<220>
<223>  Description of Artificial Sequence: Synthetic oligonucleotide

<400>  851
uaaaagaagu cca                                                     13


<210>  852
<211>  11
<212>  RNA
<213>  Artificial Sequence

<220>
<223>  Description of Artificial Sequence: Synthetic oligonucleotide

<400>  852
aggugagguu a                                                       11


<210>  853
<211>  11
<212>  RNA
<213>  Artificial Sequence

<220>
<223>  Description of Artificial Sequence: Synthetic oligonucleotide

<400>  853
gggaagagga c                                                       11


<210>  854
<211>  13
<212>  RNA
<213>  Artificial Sequence

<220>

<223>  Description of Artificial Sequence: Synthetic oligonucleotide

<400>  854
aagcaaguau ugu                                                          13


<210>  855
<211>  12
<212>  RNA
<213>  Artificial Sequence

<220>
<223>  Description of Artificial Sequence: Synthetic oligonucleotide

<400>  855
auuguagcac uc                                                           12


<210>  856
<211>  14
<212>  RNA
<213>  Artificial Sequence

<220>
<223>  Description of Artificial Sequence: Synthetic oligonucleotide

<400>  856
acaaaaccac aaau                                                         14


<210>  857
<211>  12
<212>  RNA
<213>  Artificial Sequence

<220>
<223>  Description of Artificial Sequence: Synthetic oligonucleotide

<400>  857
uaagcccaua ca                                                           12


<210>  858
<211>  11
<212>  RNA
<213>  Artificial Sequence

<220>
<223>  Description of Artificial Sequence: Synthetic oligonucleotide

<400>  858
agcccauaca g                                                            11


<210>  859
<211>  12
<212>  RNA
<213>  Artificial Sequence

<220>
<223>  Description of Artificial Sequence: Synthetic oligonucleotide

<400>  859
cagaaaccuu cc                                                          12


<210>  860
<211>  10
<212>  RNA
<213>  Artificial Sequence

<220>
<223>  Description of Artificial Sequence: Synthetic oligonucleotide

<400>  860
guauaccuac                                                             10


<210>  861
<211>  13
<212>  RNA
<213>  Artificial Sequence

<220>
<223>  Description of Artificial Sequence: Synthetic oligonucleotide

<400>  861
gccaguuuuc gga                                                         13


<210>  862
<211>  9
<212>  RNA
<213>  Artificial Sequence

<220>
<223>  Description of Artificial Sequence: Synthetic oligonucleotide

<400>  862
ggaacaggg                                                               9


<210>  863
<211>  13
<212>  RNA
<213>  Artificial Sequence

<220>
<223>  Description of Artificial Sequence: Synthetic oligonucleotide

<400>  863
uuaacuuucc agu                                                         13


<210>  864
<211>  14
<212>  RNA
<213>  Artificial Sequence

<220>
<223>  Description of Artificial Sequence: Synthetic oligonucleotide

<400>  864
aaaguuuggu uuug                                                         14


<210>  865
<211>  12
<212>  RNA
<213>  Artificial Sequence

<220>
<223>  Description of Artificial Sequence: Synthetic oligonucleotide

<400>  865
cuucccacug ua                                                           12


<210>  866
<211>  13
<212>  RNA
<213>  Artificial Sequence

<220>
<223>  Description of Artificial Sequence: Synthetic oligonucleotide

<400>  866
ugauaccaua uga                                                          13


<210>  867
<211>  11
<212>  RNA
<213>  Artificial Sequence

<220>
<223>  Description of Artificial Sequence: Synthetic oligonucleotide

<400>  867
aacaugggcu g                                                            11


<210>  868
<211>  13
<212>  RNA
<213>  Artificial Sequence

<220>
<223>  Description of Artificial Sequence: Synthetic oligonucleotide

<400>  868
caacuuuuuc aua                                                          13


<210>  869
<211>  12
<212>  RNA
<213>  Artificial Sequence

<220>
<223>  Description of Artificial Sequence: Synthetic oligonucleotide

<400>  869

caugaggccg ga                                                          12

<210>  870
<211>  11
<212>  RNA
<213>  Artificial Sequence

<220>
<223>  Description of Artificial Sequence: Synthetic oligonucleotide

<400>  870
acugcauuug u                                                           11

<210>  871
<211>  13
<212>  RNA
<213>  Artificial Sequence

<220>
<223>  Description of Artificial Sequence: Synthetic oligonucleotide

<400>  871
aauguuuacu acc                                                         13

<210>  872
<211>  15
<212>  RNA
<213>  Artificial Sequence

<220>
<223>  Description of Artificial Sequence: Synthetic oligonucleotide

<400>  872
aauuuuuuga ugaaa                                                       15

<210>  873
<211>  14
<212>  RNA
<213>  Artificial Sequence

<220>
<223>  Description of Artificial Sequence: Synthetic oligonucleotide

<400>  873
caguguguga auuu                                                        14

<210>  874
<211>  13
<212>  RNA
<213>  Artificial Sequence

<220>
<223>  Description of Artificial Sequence: Synthetic oligonucleotide

<400>  874
gagcaaugua ugu                                                         13

<210>  875
<211>  14
<212>  RNA
<213>  Artificial Sequence

<220>
<223>  Description of Artificial Sequence: Synthetic oligonucleotide

<400>  875
aaagcacaua uaua                                                    14


<210>  876
<211>  14
<212>  RNA
<213>  Artificial Sequence

<220>
<223>  Description of Artificial Sequence: Synthetic oligonucleotide

<400>  876
guaauaguuu cucc                                                    14


<210>  877
<211>  13
<212>  RNA
<213>  Artificial Sequence

<220>
<223>  Description of Artificial Sequence: Synthetic oligonucleotide

<400>  877
aguuucucca aau                                                     13


<210>  878
<211>  13
<212>  RNA
<213>  Artificial Sequence

<220>
<223>  Description of Artificial Sequence: Synthetic oligonucleotide

<400>  878
uacuuccuag aaa                                                     13


<210>  879
<211>  13
<212>  RNA
<213>  Artificial Sequence

<220>
<223>  Description of Artificial Sequence: Synthetic oligonucleotide

<400>  879
uacaccauau aug                                                     13

<210>  880
<211>  14
<212>  RNA
<213>  Artificial Sequence

<220>
<223>  Description of Artificial Sequence: Synthetic oligonucleotide

<400>  880
auauaugaau ggag                                                          14


<210>  881
<211>  13
<212>  RNA
<213>  Artificial Sequence

<220>
<223>  Description of Artificial Sequence: Synthetic oligonucleotide

<400>  881
acgagaagaa gau                                                           13


<210>  882
<211>  13
<212>  RNA
<213>  Artificial Sequence

<220>
<223>  Description of Artificial Sequence: Synthetic oligonucleotide

<400>  882
ucauuuacag gga                                                           13


<210>  883
<211>  13
<212>  RNA
<213>  Artificial Sequence

<220>
<223>  Description of Artificial Sequence: Synthetic oligonucleotide

<400>  883
cauuuacagg gac                                                           13


<210>  884
<211>  10
<212>  RNA
<213>  Artificial Sequence

<220>
<223>  Description of Artificial Sequence: Synthetic oligonucleotide

<400>  884
agggaccuga                                                               10

<210>  885
<211>  14
<212>  RNA
<213>  Artificial Sequence

<220>
<223>  Description of Artificial Sequence: Synthetic oligonucleotide

<400>  885
uguuugacug cauc                                                    14


<210>  886
<211>  13
<212>  RNA
<213>  Artificial Sequence

<220>
<223>  Description of Artificial Sequence: Synthetic oligonucleotide

<400>  886
uuugacugca ucg                                                     13


<210>  887
<211>  12
<212>  RNA
<213>  Artificial Sequence

<220>
<223>  Description of Artificial Sequence: Synthetic oligonucleotide

<400>  887
gaguucacag gg                                                      12


<210>  888
<211>  13
<212>  RNA
<213>  Artificial Sequence

<220>
<223>  Description of Artificial Sequence: Synthetic oligonucleotide

<400>  888
ggcaccucug ucc                                                     13


<210>  889
<211>  15
<212>  RNA
<213>  Artificial Sequence

<220>
<223>  Description of Artificial Sequence: Synthetic oligonucleotide

<400>  889
gguuugucuu uuuaa                                                   15


<210>  890

<211>  14
<212>  RNA
<213>  Artificial Sequence

<220>
<223>  Description of Artificial Sequence: Synthetic oligonucleotide

<400>  890
uuugucuuuu uaag                                                         14


<210>  891
<211>  13
<212>  RNA
<213>  Artificial Sequence

<220>
<223>  Description of Artificial Sequence: Synthetic oligonucleotide

<400>  891
ccuaaagguu guc                                                          13


<210>  892
<211>  12
<212>  RNA
<213>  Artificial Sequence

<220>
<223>  Description of Artificial Sequence: Synthetic oligonucleotide

<400>  892
aagguugucg ac                                                           12


<210>  893
<211>  13
<212>  RNA
<213>  Artificial Sequence

<220>
<223>  Description of Artificial Sequence: Synthetic oligonucleotide

<400>  893
accuggcaau ugu                                                          13


<210>  894
<211>  12
<212>  RNA
<213>  Artificial Sequence

<220>
<223>  Description of Artificial Sequence: Synthetic oligonucleotide

<400>  894
aguggugcga gg                                                           12


<210>  895
<211>  13

<212>  RNA
<213>  Artificial Sequence

<220>
<223>  Description of Artificial Sequence: Synthetic oligonucleotide

<400>  895
agagacauga aau                                                          13


<210>  896
<211>  13
<212>  RNA
<213>  Artificial Sequence

<220>
<223>  Description of Artificial Sequence: Synthetic oligonucleotide

<400>  896
auauauuucu cca                                                          13


<210>  897
<211>  10
<212>  RNA
<213>  Artificial Sequence

<220>
<223>  Description of Artificial Sequence: Synthetic oligonucleotide

<400>  897
caugaaggcu                                                              10


<210>  898
<211>  11
<212>  RNA
<213>  Artificial Sequence

<220>
<223>  Description of Artificial Sequence: Synthetic oligonucleotide

<400>  898
gcuggagugg u                                                            11


<210>  899
<211>  11
<212>  RNA
<213>  Artificial Sequence

<220>
<223>  Description of Artificial Sequence: Synthetic oligonucleotide

<400>  899
aggaagagag g                                                            11


<210>  900
<211>  12
<212>  RNA

<213>  Artificial Sequence

<220>
<223>  Description of Artificial Sequence: Synthetic oligonucleotide

<400>  900
cuugcaucgg gc                                                          12


<210>  901
<211>  13
<212>  RNA
<213>  Artificial Sequence

<220>
<223>  Description of Artificial Sequence: Synthetic oligonucleotide

<400>  901
auauccuacu gug                                                         13


<210>  902
<211>  13
<212>  RNA
<213>  Artificial Sequence

<220>
<223>  Description of Artificial Sequence: Synthetic oligonucleotide

<400>  902
uccuacugug uaa                                                         13


<210>  903
<211>  12
<212>  RNA
<213>  Artificial Sequence

<220>
<223>  Description of Artificial Sequence: Synthetic oligonucleotide

<400>  903
ccuuguagug ua                                                          12


<210>  904
<211>  13
<212>  RNA
<213>  Artificial Sequence

<220>
<223>  Description of Artificial Sequence: Synthetic oligonucleotide

<400>  904
ucuuuguuga uug                                                         13


<210>  905
<211>  14
<212>  RNA
<213>  Artificial Sequence

<220>
<223>  Description of Artificial Sequence: Synthetic oligonucleotide

<400>  905
uuuguugauu gaaa                                                    14


<210>  906
<211>  15
<212>  RNA
<213>  Artificial Sequence

<220>
<223>  Description of Artificial Sequence: Synthetic oligonucleotide

<400>  906
uucuuuuucu uccau                                                   15


<210>  907
<211>  13
<212>  RNA
<213>  Artificial Sequence

<220>
<223>  Description of Artificial Sequence: Synthetic oligonucleotide

<400>  907
aaucuuccuu cua                                                     13


<210>  908
<211>  13
<212>  RNA
<213>  Artificial Sequence

<220>
<223>  Description of Artificial Sequence: Synthetic oligonucleotide

<400>  908
uugaaugcuu cau                                                     13


<210>  909
<211>  13
<212>  RNA
<213>  Artificial Sequence

<220>
<223>  Description of Artificial Sequence: Synthetic oligonucleotide

<400>  909
ugaaugcuuc aug                                                     13


<210>  910
<211>  13
<212>  RNA
<213>  Artificial Sequence

<220>
<223>  Description of Artificial Sequence: Synthetic oligonucleotide

<400>  910
aauucuacuu uga                                                          13


<210>  911
<211>  13
<212>  RNA
<213>  Artificial Sequence

<220>
<223>  Description of Artificial Sequence: Synthetic oligonucleotide

<400>  911
agcaacuaca gac                                                          13


<210>  912
<211>  13
<212>  RNA
<213>  Artificial Sequence

<220>
<223>  Description of Artificial Sequence: Synthetic oligonucleotide

<400>  912
gaccaagguc aac                                                          13


<210>  913
<211>  13
<212>  RNA
<213>  Artificial Sequence

<220>
<223>  Description of Artificial Sequence: Synthetic oligonucleotide

<400>  913
cgacuaucuc gac                                                          13


<210>  914
<211>  13
<212>  RNA
<213>  Artificial Sequence

<220>
<223>  Description of Artificial Sequence: Synthetic oligonucleotide

<400>  914
aaccuuccca aac                                                          13


<210>  915
<211>  14
<212>  RNA
<213>  Artificial Sequence

<220>

<223>  Description of Artificial Sequence: Synthetic oligonucleotide

<400>  915
ccaagcuuag aacu                                                    14


<210>  916
<211>  15
<212>  RNA
<213>  Artificial Sequence

<220>
<223>  Description of Artificial Sequence: Synthetic oligonucleotide

<400>  916
agcuuagaac uuuaa                                                   15


<210>  917
<211>  11
<212>  RNA
<213>  Artificial Sequence

<220>
<223>  Description of Artificial Sequence: Synthetic oligonucleotide

<400>  917
aagaccacca c                                                       11


<210>  918
<211>  14
<212>  RNA
<213>  Artificial Sequence

<220>
<223>  Description of Artificial Sequence: Synthetic oligonucleotide

<400>  918
gcgagaaaua aagu                                                    14


<210>  919
<211>  14
<212>  RNA
<213>  Artificial Sequence

<220>
<223>  Description of Artificial Sequence: Synthetic oligonucleotide

<400>  919
cgagaaauaa aguu                                                    14


<210>  920
<211>  12
<212>  RNA
<213>  Artificial Sequence

<220>
<223>  Description of Artificial Sequence: Synthetic oligonucleotide

<400>  920
aggaaaugau cc                                                          12


<210>  921
<211>  13
<212>  RNA
<213>  Artificial Sequence

<220>
<223>  Description of Artificial Sequence: Synthetic oligonucleotide

<400>  921
gaagaaauga aac                                                         13


<210>  922
<211>  13
<212>  RNA
<213>  Artificial Sequence

<220>
<223>  Description of Artificial Sequence: Synthetic oligonucleotide

<400>  922
guuuccaucc ucc                                                         13


<210>  923
<211>  15
<212>  RNA
<213>  Artificial Sequence

<220>
<223>  Description of Artificial Sequence: Synthetic oligonucleotide

<400>  923
acugaaaauc uuuga                                                       15


<210>  924
<211>  11
<212>  RNA
<213>  Artificial Sequence

<220>
<223>  Description of Artificial Sequence: Synthetic oligonucleotide

<400>  924
ugccguucag g                                                           11


<210>  925
<211>  13
<212>  RNA
<213>  Artificial Sequence

<220>
<223>  Description of Artificial Sequence: Synthetic oligonucleotide

<400>  925
   caggguccag aag                                                          13

<210>  926
<211>  13
<212>  RNA
<213>  Artificial Sequence

<220>
<223>  Description of Artificial Sequence: Synthetic oligonucleotide

<400>  926
ucaagacugc uug                                                         13

<210>  927
<211>  13
<212>  RNA
<213>  Artificial Sequence

<220>
<223>  Description of Artificial Sequence: Synthetic oligonucleotide

<400>  927
uccaugucuu ugg                                                         13

<210>  928
<211>  11
<212>  RNA
<213>  Artificial Sequence

<220>
<223>  Description of Artificial Sequence: Synthetic oligonucleotide

<400>  928
gccugaaaca c                                                           11

<210>  929
<211>  12
<212>  RNA
<213>  Artificial Sequence

<220>
<223>  Description of Artificial Sequence: Synthetic oligonucleotide

<400>  929
ucucuguacc uc                                                          12

<210>  930
<211>  12
<212>  RNA
<213>  Artificial Sequence

<220>
<223>  Description of Artificial Sequence: Synthetic oligonucleotide

<400>  930

agcaagacaa ga                                                          12

<210>  931
<211>  13
<212>  RNA
<213>  Artificial Sequence

<220>
<223>  Description of Artificial Sequence: Synthetic oligonucleotide

<400>  931
gcaagacaag aaa                                                         13

<210>  932
<211>  12
<212>  RNA
<213>  Artificial Sequence

<220>
<223>  Description of Artificial Sequence: Synthetic oligonucleotide

<400>  932
uguacaagau cc                                                          12

<210>  933
<211>  12
<212>  RNA
<213>  Artificial Sequence

<220>
<223>  Description of Artificial Sequence: Synthetic oligonucleotide

<400>  933
cguacuugca ga                                                          12

<210>  934
<211>  13
<212>  RNA
<213>  Artificial Sequence

<220>
<223>  Description of Artificial Sequence: Synthetic oligonucleotide

<400>  934
cgaucgauac aga                                                         13

<210>  935
<211>  13
<212>  RNA
<213>  Artificial Sequence

<220>
<223>  Description of Artificial Sequence: Synthetic oligonucleotide

<400>  935
uauaaaaaua aau                                                         13

<210>  936
<211>  13
<212>  RNA
<213>  Artificial Sequence

<220>
<223>  Description of Artificial Sequence: Synthetic oligonucleotide

<400>  936
uaaaaauaaa uau                                                          13


<210>  937
<211>  13
<212>  RNA
<213>  Artificial Sequence

<220>
<223>  Description of Artificial Sequence: Synthetic oligonucleotide

<400>  937
uuugacugcu gug                                                          13


<210>  938
<211>  13
<212>  RNA
<213>  Artificial Sequence

<220>
<223>  Description of Artificial Sequence: Synthetic oligonucleotide

<400>  938
ggagaugaga gac                                                          13


<210>  939
<211>  10
<212>  RNA
<213>  Artificial Sequence

<220>
<223>  Description of Artificial Sequence: Synthetic oligonucleotide

<400>  939
ucccacuugg                                                              10


<210>  940
<211>  10
<212>  RNA
<213>  Artificial Sequence

<220>
<223>  Description of Artificial Sequence: Synthetic oligonucleotide

<400>  940
guucacggcc                                                              10

<210>  941
<211>  12
<212>  RNA
<213>  Artificial Sequence

<220>
<223>  Description of Artificial Sequence: Synthetic oligonucleotide

<400>  941
accaucacac ca                                                          12


<210>  942
<211>  10
<212>  RNA
<213>  Artificial Sequence

<220>
<223>  Description of Artificial Sequence: Synthetic oligonucleotide

<400>  942
cugcggguuu                                                             10


<210>  943
<211>  9
<212>  RNA
<213>  Artificial Sequence

<220>
<223>  Description of Artificial Sequence: Synthetic oligonucleotide

<400>  943
uccaguuug                                                               9


<210>  944
<211>  12
<212>  RNA
<213>  Artificial Sequence

<220>
<223>  Description of Artificial Sequence: Synthetic oligonucleotide

<400>  944
uuaaggacuu cu                                                          12


<210>  945
<211>  12
<212>  RNA
<213>  Artificial Sequence

<220>
<223>  Description of Artificial Sequence: Synthetic oligonucleotide

<400>  945
agugggacag ag                                                          12

<210>  946
<211>  13
<212>  RNA
<213>  Artificial Sequence

<220>
<223>  Description of Artificial Sequence: Synthetic oligonucleotide

<400>  946
auuuguuauu gug                                                          13


<210>  947
<211>  12
<212>  RNA
<213>  Artificial Sequence

<220>
<223>  Description of Artificial Sequence: Synthetic oligonucleotide

<400>  947
ucaaaugcau uu                                                           12


<210>  948
<211>  9
<212>  RNA
<213>  Artificial Sequence

<220>
<223>  Description of Artificial Sequence: Synthetic oligonucleotide

<400>  948
aggaaagcc                                                                9


<210>  949
<211>  10
<212>  RNA
<213>  Artificial Sequence

<220>
<223>  Description of Artificial Sequence: Synthetic oligonucleotide

<400>  949
ggaaagcccu                                                              10


<210>  950
<211>  11
<212>  RNA
<213>  Artificial Sequence

<220>
<223>  Description of Artificial Sequence: Synthetic oligonucleotide

<400>  950
ccagcauggc c                                                            11


<210>  951

<211>  10
<212>  RNA
<213>  Artificial Sequence

<220>
<223>  Description of Artificial Sequence: Synthetic oligonucleotide

<400>  951
uucucccuga                                                              10


<210>  952
<211>  11
<212>  RNA
<213>  Artificial Sequence

<220>
<223>  Description of Artificial Sequence: Synthetic oligonucleotide

<400>  952
aagaagcccu u                                                            11


<210>  953
<211>  11
<212>  RNA
<213>  Artificial Sequence

<220>
<223>  Description of Artificial Sequence: Synthetic oligonucleotide

<400>  953
gaagcccuuc a                                                            11


<210>  954
<211>  10
<212>  RNA
<213>  Artificial Sequence

<220>
<223>  Description of Artificial Sequence: Synthetic oligonucleotide

<400>  954
cccuucagcg                                                              10


<210>  955
<211>  10
<212>  RNA
<213>  Artificial Sequence

<220>
<223>  Description of Artificial Sequence: Synthetic oligonucleotide

<400>  955
cuucagcggc                                                              10


<210>  956
<211>  11

<212>  RNA
<213>  Artificial Sequence

<220>
<223>  Description of Artificial Sequence: Synthetic oligonucleotide

<400>  956
cagcggccag u                                                            11


<210>  957
<211>  11
<212>  RNA
<213>  Artificial Sequence

<220>
<223>  Description of Artificial Sequence: Synthetic oligonucleotide

<400>  957
gaaaagcucc g                                                            11


<210>  958
<211>  10
<212>  RNA
<213>  Artificial Sequence

<220>
<223>  Description of Artificial Sequence: Synthetic oligonucleotide

<400>  958
aaagcuccgg                                                              10


<210>  959
<211>  12
<212>  RNA
<213>  Artificial Sequence

<220>
<223>  Description of Artificial Sequence: Synthetic oligonucleotide

<400>  959
gucaacaguc ug                                                           12


<210>  960
<211>  12
<212>  RNA
<213>  Artificial Sequence

<220>
<223>  Description of Artificial Sequence: Synthetic oligonucleotide

<400>  960
agaguucaaa ag                                                           12


<210>  961
<211>  12
<212>  RNA

<213>  Artificial Sequence

<220>
<223>  Description of Artificial Sequence: Synthetic oligonucleotide

<400>  961
gaguucaaaa gc                                                          12


<210>  962
<211>  11
<212>  RNA
<213>  Artificial Sequence

<220>
<223>  Description of Artificial Sequence: Synthetic oligonucleotide

<400>  962
ucaaaagccc u                                                           11


<210>  963
<211>  11
<212>  RNA
<213>  Artificial Sequence

<220>
<223>  Description of Artificial Sequence: Synthetic oligonucleotide

<400>  963
caaaagcccu u                                                           11


<210>  964
<211>  11
<212>  RNA
<213>  Artificial Sequence

<220>
<223>  Description of Artificial Sequence: Synthetic oligonucleotide

<400>  964
aaagcccuuc a                                                           11


<210>  965
<211>  11
<212>  RNA
<213>  Artificial Sequence

<220>
<223>  Description of Artificial Sequence: Synthetic oligonucleotide

<400>  965
aagcccuuca g                                                           11


<210>  966
<211>  12
<212>  RNA
<213>  Artificial Sequence

<220>
<223>  Description of Artificial Sequence: Synthetic oligonucleotide

<400>  966
ugaagccgcu cg                                                          12


<210>  967
<211>  11
<212>  RNA
<213>  Artificial Sequence

<220>
<223>  Description of Artificial Sequence: Synthetic oligonucleotide

<400>  967
acgccaguca a                                                           11


<210>  968
<211>  12
<212>  RNA
<213>  Artificial Sequence

<220>
<223>  Description of Artificial Sequence: Synthetic oligonucleotide

<400>  968
ucuuggugcg ug                                                          12


<210>  969
<211>  13
<212>  RNA
<213>  Artificial Sequence

<220>
<223>  Description of Artificial Sequence: Synthetic oligonucleotide

<400>  969
uuggauucau cag                                                         13


<210>  970
<211>  12
<212>  RNA
<213>  Artificial Sequence

<220>
<223>  Description of Artificial Sequence: Synthetic oligonucleotide

<400>  970
ggaauaccua ag                                                          12


<210>  971
<211>  11
<212>  RNA
<213>  Artificial Sequence

<220>
<223>  Description of Artificial Sequence: Synthetic oligonucleotide

<400>  971
uuugaggcuc a                                                            11


<210>  972
<211>  10
<212>  RNA
<213>  Artificial Sequence

<220>
<223>  Description of Artificial Sequence: Synthetic oligonucleotide

<400>  972
cuacaauugg                                                              10


<210>  973
<211>  11
<212>  RNA
<213>  Artificial Sequence

<220>
<223>  Description of Artificial Sequence: Synthetic oligonucleotide

<400>  973
aguuaggagc c                                                            11


<210>  974
<211>  12
<212>  RNA
<213>  Artificial Sequence

<220>
<223>  Description of Artificial Sequence: Synthetic oligonucleotide

<400>  974
gucaauaucc ac                                                           12


<210>  975
<211>  12
<212>  RNA
<213>  Artificial Sequence

<220>
<223>  Description of Artificial Sequence: Synthetic oligonucleotide

<400>  975
uguucaguca ca                                                           12


<210>  976
<211>  11
<212>  RNA
<213>  Artificial Sequence

<220>

<223>  Description of Artificial Sequence: Synthetic oligonucleotide

<400>  976
cacacacaua c                                                            11


<210>  977
<211>  11
<212>  RNA
<213>  Artificial Sequence

<220>
<223>  Description of Artificial Sequence: Synthetic oligonucleotide

<400>  977
uccuuuugcu u                                                            11


<210>  978
<211>  15
<212>  RNA
<213>  Artificial Sequence

<220>
<223>  Description of Artificial Sequence: Synthetic oligonucleotide

<400>  978
uuuaaaguaa uuuuu                                                        15


<210>  979
<211>  12
<212>  RNA
<213>  Artificial Sequence

<220>
<223>  Description of Artificial Sequence: Synthetic oligonucleotide

<400>  979
aacagucaau gg                                                           12


<210>  980
<211>  12
<212>  RNA
<213>  Artificial Sequence

<220>
<223>  Description of Artificial Sequence: Synthetic oligonucleotide

<400>  980
cgacaaacca au                                                           12


<210>  981
<211>  11
<212>  RNA
<213>  Artificial Sequence

<220>
<223>  Description of Artificial Sequence: Synthetic oligonucleotide

<400>  981
cugcuaccac u                                                            11


<210>  982
<211>  12
<212>  RNA
<213>  Artificial Sequence

<220>
<223>  Description of Artificial Sequence: Synthetic oligonucleotide

<400>  982
caagcggucu ua                                                           12


<210>  983
<211>  12
<212>  RNA
<213>  Artificial Sequence

<220>
<223>  Description of Artificial Sequence: Synthetic oligonucleotide

<400>  983
gucuuaccgg cu                                                           12


<210>  984
<211>  10
<212>  RNA
<213>  Artificial Sequence

<220>
<223>  Description of Artificial Sequence: Synthetic oligonucleotide

<400>  984
auggguuacc                                                              10


<210>  985
<211>  10
<212>  RNA
<213>  Artificial Sequence

<220>
<223>  Description of Artificial Sequence: Synthetic oligonucleotide

<400>  985
uuaccugcga                                                              10


<210>  986
<211>  11
<212>  RNA
<213>  Artificial Sequence

<220>
<223>  Description of Artificial Sequence: Synthetic oligonucleotide

<400>  986
aucuugcuga g                                                            11


<210>  987
<211>  12
<212>  RNA
<213>  Artificial Sequence

<220>
<223>  Description of Artificial Sequence: Synthetic oligonucleotide

<400>  987
gcauaaaaca gu                                                           12


<210>  988
<211>  11
<212>  RNA
<213>  Artificial Sequence

<220>
<223>  Description of Artificial Sequence: Synthetic oligonucleotide

<400>  988
uccuuuaucc u                                                            11


<210>  989
<211>  11
<212>  RNA
<213>  Artificial Sequence

<220>
<223>  Description of Artificial Sequence: Synthetic oligonucleotide

<400>  989
aacgcaaguu g                                                            11


<210>  990
<211>  12
<212>  RNA
<213>  Artificial Sequence

<220>
<223>  Description of Artificial Sequence: Synthetic oligonucleotide

<400>  990
aacugaaacc au                                                           12


<210>  991
<211>  13
<212>  RNA
<213>  Artificial Sequence

<220>
<223>  Description of Artificial Sequence: Synthetic oligonucleotide

<400>  991

cuuuguuaaa uau                                                          13

<210>  992
<211>  13
<212>  RNA
<213>  Artificial Sequence

<220>
<223>  Description of Artificial Sequence: Synthetic oligonucleotide

<400>  992
uauauuaaaa guu                                                          13

<210>  993
<211>  12
<212>  RNA
<213>  Artificial Sequence

<220>
<223>  Description of Artificial Sequence: Synthetic oligonucleotide

<400>  993
uuuugacuua ac                                                           12

<210>  994
<211>  12
<212>  RNA
<213>  Artificial Sequence

<220>
<223>  Description of Artificial Sequence: Synthetic oligonucleotide

<400>  994
caaacucagc ac                                                           12

<210>  995
<211>  10
<212>  RNA
<213>  Artificial Sequence

<220>
<223>  Description of Artificial Sequence: Synthetic oligonucleotide

<400>  995
gcugaccuca                                                              10

<210>  996
<211>  13
<212>  RNA
<213>  Artificial Sequence

<220>
<223>  Description of Artificial Sequence: Synthetic oligonucleotide

<400>  996
uuauucagau cgc                                                          13

<210>  997
<211>  12
<212>  RNA
<213>  Artificial Sequence

<220>
<223>  Description of Artificial Sequence: Synthetic oligonucleotide

<400>  997
ggguucuggg au                                                          12


<210>  998
<211>  14
<212>  RNA
<213>  Artificial Sequence

<220>
<223>  Description of Artificial Sequence: Synthetic oligonucleotide

<400>  998
uauuggaaau uaug                                                        14


<210>  999
<211>  12
<212>  RNA
<213>  Artificial Sequence

<220>
<223>  Description of Artificial Sequence: Synthetic oligonucleotide

<400>  999
uggaagcacu ag                                                          12


<210>  1000
<211>  12
<212>  RNA
<213>  Artificial Sequence

<220>
<223>  Description of Artificial Sequence: Synthetic oligonucleotide

<400>  1000
uuuuuaugug ga                                                          12


<210>  1001
<211>  11
<212>  RNA
<213>  Artificial Sequence

<220>
<223>  Description of Artificial Sequence: Synthetic oligonucleotide

<400>  1001
gaauugguag a                                                           11

<210>  1002
<211>  12
<212>  RNA
<213>  Artificial Sequence

<220>
<223>  Description of Artificial Sequence: Synthetic oligonucleotide

<400>  1002
uagacuugga ga                                                          12


<210>  1003
<211>  12
<212>  RNA
<213>  Artificial Sequence

<220>
<223>  Description of Artificial Sequence: Synthetic oligonucleotide

<400>  1003
uacaguauuc ca                                                          12


<210>  1004
<211>  14
<212>  RNA
<213>  Artificial Sequence

<220>
<223>  Description of Artificial Sequence: Synthetic oligonucleotide

<400>  1004
cacugaugaa uuaa                                                        14


<210>  1005
<211>  11
<212>  RNA
<213>  Artificial Sequence

<220>
<223>  Description of Artificial Sequence: Synthetic oligonucleotide

<400>  1005
cacaucauca c                                                           11


<210>  1006
<211>  12
<212>  RNA
<213>  Artificial Sequence

<220>
<223>  Description of Artificial Sequence: Synthetic oligonucleotide

<400>  1006
ucaccaaaca ga                                                          12

<210>  1007
<211>  11
<212>  RNA
<213>  Artificial Sequence

<220>
<223>  Description of Artificial Sequence: Synthetic oligonucleotide

<400>  1007
gaacaugaug g                                                            11


<210>  1008
<211>  11
<212>  RNA
<213>  Artificial Sequence

<220>
<223>  Description of Artificial Sequence: Synthetic oligonucleotide

<400>  1008
auguagacug c                                                            11


<210>  1009
<211>  12
<212>  RNA
<213>  Artificial Sequence

<220>
<223>  Description of Artificial Sequence: Synthetic oligonucleotide

<400>  1009
gaucuauuua ug                                                           12


<210>  1010
<211>  11
<212>  RNA
<213>  Artificial Sequence

<220>
<223>  Description of Artificial Sequence: Synthetic oligonucleotide

<400>  1010
cacuauuauc a                                                            11


<210>  1011
<211>  10
<212>  RNA
<213>  Artificial Sequence

<220>
<223>  Description of Artificial Sequence: Synthetic oligonucleotide

<400>  1011
ucaguucugc                                                              10


<210>  1012

<211>  12
<212>  RNA
<213>  Artificial Sequence

<220>
<223>  Description of Artificial Sequence: Synthetic oligonucleotide

<400>  1012
gauuaaaccu ac                                                          12


<210>  1013
<211>  12
<212>  RNA
<213>  Artificial Sequence

<220>
<223>  Description of Artificial Sequence: Synthetic oligonucleotide

<400>  1013
uaaaauccuu cu                                                          12


<210>  1014
<211>  11
<212>  RNA
<213>  Artificial Sequence

<220>
<223>  Description of Artificial Sequence: Synthetic oligonucleotide

<400>  1014
augcuguaga c                                                           11


<210>  1015
<211>  12
<212>  RNA
<213>  Artificial Sequence

<220>
<223>  Description of Artificial Sequence: Synthetic oligonucleotide

<400>  1015
ugauaggaau ag                                                          12


<210>  1016
<211>  10
<212>  RNA
<213>  Artificial Sequence

<220>
<223>  Description of Artificial Sequence: Synthetic oligonucleotide

<400>  1016
auggcccuga                                                             10


<210>  1017
<211>  10

<212>  RNA
<213>  Artificial Sequence

<220>
<223>  Description of Artificial Sequence: Synthetic oligonucleotide

<400>  1017
uccucaagca                                                              10


<210>  1018
<211>  9
<212>  RNA
<213>  Artificial Sequence

<220>
<223>  Description of Artificial Sequence: Synthetic oligonucleotide

<400>  1018
uucagccca                                                                9


<210>  1019
<211>  10
<212>  RNA
<213>  Artificial Sequence

<220>
<223>  Description of Artificial Sequence: Synthetic oligonucleotide

<400>  1019
cugcugaacc                                                              10


<210>  1020
<211>  11
<212>  RNA
<213>  Artificial Sequence

<220>
<223>  Description of Artificial Sequence: Synthetic oligonucleotide

<400>  1020
aguucucuuc a                                                            11


<210>  1021
<211>  10
<212>  RNA
<213>  Artificial Sequence

<220>
<223>  Description of Artificial Sequence: Synthetic oligonucleotide

<400>  1021
cagccuagag                                                              10


<210>  1022
<211>  12
<212>  RNA

<213>  Artificial Sequence

<220>
<223>  Description of Artificial Sequence: Synthetic oligonucleotide

<400>  1022
ucugugaagu gu                                                          12


<210>  1023
<211>  13
<212>  RNA
<213>  Artificial Sequence

<220>
<223>  Description of Artificial Sequence: Synthetic oligonucleotide

<400>  1023
gagaaaauuu caa                                                         13


<210>  1024
<211>  10
<212>  RNA
<213>  Artificial Sequence

<220>
<223>  Description of Artificial Sequence: Synthetic oligonucleotide

<400>  1024
auccuccuga                                                             10


<210>  1025
<211>  11
<212>  RNA
<213>  Artificial Sequence

<220>
<223>  Description of Artificial Sequence: Synthetic oligonucleotide

<400>  1025
ugacaucaua u                                                           11


<210>  1026
<211>  12
<212>  RNA
<213>  Artificial Sequence

<220>
<223>  Description of Artificial Sequence: Synthetic oligonucleotide

<400>  1026
aucuucauca ua                                                          12


<210>  1027
<211>  12
<212>  RNA
<213>  Artificial Sequence

<220>
<223>  Description of Artificial Sequence: Synthetic oligonucleotide

<400>  1027
acuagcuauu aa                                                          12


<210>  1028
<211>  13
<212>  RNA
<213>  Artificial Sequence

<220>
<223>  Description of Artificial Sequence: Synthetic oligonucleotide

<400>  1028
cucuacuaaa aau                                                         13


<210>  1029
<211>  11
<212>  RNA
<213>  Artificial Sequence

<220>
<223>  Description of Artificial Sequence: Synthetic oligonucleotide

<400>  1029
cuaucucgcc u                                                           11


<210>  1030
<211>  12
<212>  RNA
<213>  Artificial Sequence

<220>
<223>  Description of Artificial Sequence: Synthetic oligonucleotide

<400>  1030
uucgguccag uu                                                          12


<210>  1031
<211>  10
<212>  RNA
<213>  Artificial Sequence

<220>
<223>  Description of Artificial Sequence: Synthetic oligonucleotide

<400>  1031
auguagccgc                                                             10


<210>  1032
<211>  11
<212>  RNA
<213>  Artificial Sequence

<220>
<223>  Description of Artificial Sequence: Synthetic oligonucleotide

<400>  1032
gaggagacuu g                                                            11


<210>  1033
<211>  11
<212>  RNA
<213>  Artificial Sequence

<220>
<223>  Description of Artificial Sequence: Synthetic oligonucleotide

<400>  1033
aguagugagg a                                                            11


<210>  1034
<211>  13
<212>  RNA
<213>  Artificial Sequence

<220>
<223>  Description of Artificial Sequence: Synthetic oligonucleotide

<400>  1034
agaugaguac aaa                                                          13


<210>  1035
<211>  12
<212>  RNA
<213>  Artificial Sequence

<220>
<223>  Description of Artificial Sequence: Synthetic oligonucleotide

<400>  1035
uuguuaaugg gc                                                           12


<210>  1036
<211>  12
<212>  RNA
<213>  Artificial Sequence

<220>
<223>  Description of Artificial Sequence: Synthetic oligonucleotide

<400>  1036
uuuuaauuua uu                                                           12


<210>  1037
<211>  11
<212>  RNA
<213>  Artificial Sequence

<220>

<223>  Description of Artificial Sequence: Synthetic oligonucleotide

<400>  1037
auuuaaauau g                                                            11


<210>  1038
<211>  12
<212>  RNA
<213>  Artificial Sequence

<220>
<223>  Description of Artificial Sequence: Synthetic oligonucleotide

<400>  1038
auccuuuguu uc                                                           12


<210>  1039
<211>  13
<212>  RNA
<213>  Artificial Sequence

<220>
<223>  Description of Artificial Sequence: Synthetic oligonucleotide

<400>  1039
gucuaagugu uug                                                          13


<210>  1040
<211>  11
<212>  RNA
<213>  Artificial Sequence

<220>
<223>  Description of Artificial Sequence: Synthetic oligonucleotide

<400>  1040
ccugugacaa a                                                            11


<210>  1041
<211>  12
<212>  RNA
<213>  Artificial Sequence

<220>
<223>  Description of Artificial Sequence: Synthetic oligonucleotide

<400>  1041
caggcugaau ua                                                           12


<210>  1042
<211>  13
<212>  RNA
<213>  Artificial Sequence

<220>
<223>  Description of Artificial Sequence: Synthetic oligonucleotide

<400>  1042
ucauucuuac aau                                                          13


<210>  1043
<211>  14
<212>  RNA
<213>  Artificial Sequence

<220>
<223>  Description of Artificial Sequence: Synthetic oligonucleotide

<400>  1043
cauucuuaca auug                                                         14


<210>  1044
<211>  11
<212>  RNA
<213>  Artificial Sequence

<220>
<223>  Description of Artificial Sequence: Synthetic oligonucleotide

<400>  1044
agagagugua c                                                            11


<210>  1045
<211>  11
<212>  RNA
<213>  Artificial Sequence

<220>
<223>  Description of Artificial Sequence: Synthetic oligonucleotide

<400>  1045
gaacucagca g                                                            11


<210>  1046
<211>  12
<212>  RNA
<213>  Artificial Sequence

<220>
<223>  Description of Artificial Sequence: Synthetic oligonucleotide

<400>  1046
uuauuuucag ua                                                           12


<210>  1047
<211>  11
<212>  RNA
<213>  Artificial Sequence

<220>
<223>  Description of Artificial Sequence: Synthetic oligonucleotide

<400>  1047
cuuaaaugga a                                                            11


<210>  1048
<211>  12
<212>  RNA
<213>  Artificial Sequence

<220>
<223>  Description of Artificial Sequence: Synthetic oligonucleotide

<400>  1048
gccuaaaauu gu                                                           12


<210>  1049
<211>  12
<212>  RNA
<213>  Artificial Sequence

<220>
<223>  Description of Artificial Sequence: Synthetic oligonucleotide

<400>  1049
acuaacaucu gg                                                           12


<210>  1050
<211>  11
<212>  RNA
<213>  Artificial Sequence

<220>
<223>  Description of Artificial Sequence: Synthetic oligonucleotide

<400>  1050
gacugugagc u                                                            11


<210>  1051
<211>  13
<212>  RNA
<213>  Artificial Sequence

<220>
<223>  Description of Artificial Sequence: Synthetic oligonucleotide

<400>  1051
gcuuuuuauu aau                                                          13


<210>  1052
<211>  12
<212>  RNA
<213>  Artificial Sequence

<220>
<223>  Description of Artificial Sequence: Synthetic oligonucleotide

<400>  1052

ucuucuuaca uu                                                          12

<210>  1053
<211>  14
<212>  RNA
<213>  Artificial Sequence

<220>
<223>  Description of Artificial Sequence: Synthetic oligonucleotide

<400>  1053
auuuguaaaa augu                                                        14

<210>  1054
<211>  14
<212>  RNA
<213>  Artificial Sequence

<220>
<223>  Description of Artificial Sequence: Synthetic oligonucleotide

<400>  1054
ucuuaauuca uauu                                                        14

<210>  1055
<211>  12
<212>  RNA
<213>  Artificial Sequence

<220>
<223>  Description of Artificial Sequence: Synthetic oligonucleotide

<400>  1055
aacuaaucau ga                                                          12

<210>  1056
<211>  11
<212>  RNA
<213>  Artificial Sequence

<220>
<223>  Description of Artificial Sequence: Synthetic oligonucleotide

<400>  1056
cugcucuugg g                                                           11

<210>  1057
<211>  12
<212>  RNA
<213>  Artificial Sequence

<220>
<223>  Description of Artificial Sequence: Synthetic oligonucleotide

<400>  1057
aguagcuuug ag                                                          12

<210>  1058
<211>  13
<212>  RNA
<213>  Artificial Sequence

<220>
<223>  Description of Artificial Sequence: Synthetic oligonucleotide

<400>  1058
acguaauauu uca                                                          13


<210>  1059
<211>  12
<212>  RNA
<213>  Artificial Sequence

<220>
<223>  Description of Artificial Sequence: Synthetic oligonucleotide

<400>  1059
auucucuuca cu                                                           12


<210>  1060
<211>  13
<212>  RNA
<213>  Artificial Sequence

<220>
<223>  Description of Artificial Sequence: Synthetic oligonucleotide

<400>  1060
uaaugcuaag aaa                                                          13


<210>  1061
<211>  11
<212>  RNA
<213>  Artificial Sequence

<220>
<223>  Description of Artificial Sequence: Synthetic oligonucleotide

<400>  1061
cugaaaauca a                                                            11


<210>  1062
<211>  13
<212>  RNA
<213>  Artificial Sequence

<220>
<223>  Description of Artificial Sequence: Synthetic oligonucleotide

<400>  1062
auaugauuac uuu                                                          13

<210>  1063
<211>  13
<212>  RNA
<213>  Artificial Sequence

<220>
<223>  Description of Artificial Sequence: Synthetic oligonucleotide

<400>  1063
auuuuguucu aca                                                          13


<210>  1064
<211>  11
<212>  RNA
<213>  Artificial Sequence

<220>
<223>  Description of Artificial Sequence: Synthetic oligonucleotide

<400>  1064
ccgggacucu c                                                            11


<210>  1065
<211>  13
<212>  RNA
<213>  Artificial Sequence

<220>
<223>  Description of Artificial Sequence: Synthetic oligonucleotide

<400>  1065
cuuuaaagua aag                                                          13


<210>  1066
<211>  12
<212>  RNA
<213>  Artificial Sequence

<220>
<223>  Description of Artificial Sequence: Synthetic oligonucleotide

<400>  1066
cuggaagccu gg                                                           12


<210>  1067
<211>  13
<212>  RNA
<213>  Artificial Sequence

<220>
<223>  Description of Artificial Sequence: Synthetic oligonucleotide

<400>  1067
aaacaaaaga gag                                                          13

<210>  1068
<211>  12
<212>  RNA
<213>  Artificial Sequence

<220>
<223>  Description of Artificial Sequence: Synthetic oligonucleotide

<400>  1068
ucaguuaugc cg                                                          12


<210>  1069
<211>  12
<212>  RNA
<213>  Artificial Sequence

<220>
<223>  Description of Artificial Sequence: Synthetic oligonucleotide

<400>  1069
aaaagaaguc ca                                                          12


<210>  1070
<211>  10
<212>  RNA
<213>  Artificial Sequence

<220>
<223>  Description of Artificial Sequence: Synthetic oligonucleotide

<400>  1070
ggugagguua                                                             10


<210>  1071
<211>  10
<212>  RNA
<213>  Artificial Sequence

<220>
<223>  Description of Artificial Sequence: Synthetic oligonucleotide

<400>  1071
ggaagaggac                                                             10


<210>  1072
<211>  12
<212>  RNA
<213>  Artificial Sequence

<220>
<223>  Description of Artificial Sequence: Synthetic oligonucleotide

<400>  1072
agcaaguauu gu                                                          12


<210>  1073

<211>  11
<212>  RNA
<213>  Artificial Sequence

<220>
<223>  Description of Artificial Sequence: Synthetic oligonucleotide

<400>  1073
uuguagcacu c                                                          11


<210>  1074
<211>  13
<212>  RNA
<213>  Artificial Sequence

<220>
<223>  Description of Artificial Sequence: Synthetic oligonucleotide

<400>  1074
caaaaccaca aau                                                        13


<210>  1075
<211>  11
<212>  RNA
<213>  Artificial Sequence

<220>
<223>  Description of Artificial Sequence: Synthetic oligonucleotide

<400>  1075
aagcccauac a                                                          11


<210>  1076
<211>  10
<212>  RNA
<213>  Artificial Sequence

<220>
<223>  Description of Artificial Sequence: Synthetic oligonucleotide

<400>  1076
gcccauacag                                                            10


<210>  1077
<211>  11
<212>  RNA
<213>  Artificial Sequence

<220>
<223>  Description of Artificial Sequence: Synthetic oligonucleotide

<400>  1077
agaaaccuuc c                                                          11


<210>  1078
<211>  9

<212>  RNA
<213>  Artificial Sequence

<220>
<223>  Description of Artificial Sequence: Synthetic oligonucleotide

<400>  1078
uauaccuac                                                              9


<210>  1079
<211>  12
<212>  RNA
<213>  Artificial Sequence

<220>
<223>  Description of Artificial Sequence: Synthetic oligonucleotide

<400>  1079
   ccaguuuucg ga                                                          12


<210>  1080
<211>  8
<212>  RNA
<213>  Artificial Sequence

<220>
<223>  Description of Artificial Sequence: Synthetic oligonucleotide

<400>  1080
gaacaggg                                                               8


<210>  1081
<211>  12
<212>  RNA
<213>  Artificial Sequence

<220>
<223>  Description of Artificial Sequence: Synthetic oligonucleotide

<400>  1081
uaacuuucca gu                                                         12


<210>  1082
<211>  13
<212>  RNA
<213>  Artificial Sequence

<220>
<223>  Description of Artificial Sequence: Synthetic oligonucleotide

<400>  1082
aaguuugguu uug                                                        13


<210>  1083
<211>  11
<212>  RNA

<213>  Artificial Sequence

<220>
<223>  Description of Artificial Sequence: Synthetic oligonucleotide

<400>  1083
uucccacugu a                                                          11


<210>  1084
<211>  12
<212>  RNA
<213>  Artificial Sequence

<220>
<223>  Description of Artificial Sequence: Synthetic oligonucleotide

<400>  1084
gauaccauau ga                                                         12


<210>  1085
<211>  10
<212>  RNA
<213>  Artificial Sequence

<220>
<223>  Description of Artificial Sequence: Synthetic oligonucleotide

<400>  1085
acaugggcug                                                            10


<210>  1086
<211>  12
<212>  RNA
<213>  Artificial Sequence

<220>
<223>  Description of Artificial Sequence: Synthetic oligonucleotide

<400>  1086
aacuuuuuca ua                                                         12


<210>  1087
<211>  11
<212>  RNA
<213>  Artificial Sequence

<220>
<223>  Description of Artificial Sequence: Synthetic oligonucleotide

<400>  1087
augaggccgg a                                                          11


<210>  1088
<211>  10
<212>  RNA
<213>  Artificial Sequence

<220>
<223>  Description of Artificial Sequence: Synthetic oligonucleotide

<400>  1088
cugcauuugu                                                              10


<210>  1089
<211>  12
<212>  RNA
<213>  Artificial Sequence

<220>
<223>  Description of Artificial Sequence: Synthetic oligonucleotide

<400>  1089
auguuuacua cc                                                           12


<210>  1090
<211>  14
<212>  RNA
<213>  Artificial Sequence

<220>
<223>  Description of Artificial Sequence: Synthetic oligonucleotide

<400>  1090
auuuuuugau gaaa                                                         14


<210>  1091
<211>  13
<212>  RNA
<213>  Artificial Sequence

<220>
<223>  Description of Artificial Sequence: Synthetic oligonucleotide

<400>  1091
agugugugaa uuu                                                          13


<210>  1092
<211>  12
<212>  RNA
<213>  Artificial Sequence

<220>
<223>  Description of Artificial Sequence: Synthetic oligonucleotide

<400>  1092
agcaauguau gu                                                           12


<210>  1093
<211>  13
<212>  RNA
<213>  Artificial Sequence

<220>
<223>  Description of Artificial Sequence: Synthetic oligonucleotide

<400>  1093
aagcacauau aua                                                          13


<210>  1094
<211>  13
<212>  RNA
<213>  Artificial Sequence

<220>
<223>  Description of Artificial Sequence: Synthetic oligonucleotide

<400>  1094
uaauaguuuc ucc                                                          13


<210>  1095
<211>  12
<212>  RNA
<213>  Artificial Sequence

<220>
<223>  Description of Artificial Sequence: Synthetic oligonucleotide

<400>  1095
guuucuccaa au                                                           12


<210>  1096
<211>  12
<212>  RNA
<213>  Artificial Sequence

<220>
<223>  Description of Artificial Sequence: Synthetic oligonucleotide

<400>  1096
acuuccuaga aa                                                           12


<210>  1097
<211>  12
<212>  RNA
<213>  Artificial Sequence

<220>
<223>  Description of Artificial Sequence: Synthetic oligonucleotide

<400>  1097
acaccauaua ug                                                           12


<210>  1098
<211>  13
<212>  RNA
<213>  Artificial Sequence

<220>

<223>  Description of Artificial Sequence: Synthetic oligonucleotide

<400>  1098
uauaugaaug gag                                                          13


<210>  1099
<211>  12
<212>  RNA
<213>  Artificial Sequence

<220>
<223>  Description of Artificial Sequence: Synthetic oligonucleotide

<400>  1099
cauuuacagg ga                                                           12


<210>  1100
<211>  12
<212>  RNA
<213>  Artificial Sequence

<220>
<223>  Description of Artificial Sequence: Synthetic oligonucleotide

<400>  1100
auuuacaggg ac                                                           12


<210>  1101
<211>  9
<212>  RNA
<213>  Artificial Sequence

<220>
<223>  Description of Artificial Sequence: Synthetic oligonucleotide

<400>  1101
gggaccuga                                                                9


<210>  1102
<211>  13
<212>  RNA
<213>  Artificial Sequence

<220>
<223>  Description of Artificial Sequence: Synthetic oligonucleotide

<400>  1102
guuugacugc auc                                                          13


<210>  1103
<211>  12
<212>  RNA
<213>  Artificial Sequence

<220>
<223>  Description of Artificial Sequence: Synthetic oligonucleotide

<400>  1103
uugacugcau cg                                                          12


<210>  1104
<211>  11
<212>  RNA
<213>  Artificial Sequence

<220>
<223>  Description of Artificial Sequence: Synthetic oligonucleotide

<400>  1104
aguucacagg g                                                           11


<210>  1105
<211>  12
<212>  RNA
<213>  Artificial Sequence

<220>
<223>  Description of Artificial Sequence: Synthetic oligonucleotide

<400>  1105
gcaccucugu cc                                                          12


<210>  1106
<211>  14
<212>  RNA
<213>  Artificial Sequence

<220>
<223>  Description of Artificial Sequence: Synthetic oligonucleotide

<400>  1106
guuugucuuu uuaa                                                        14


<210>  1107
<211>  13
<212>  RNA
<213>  Artificial Sequence

<220>
<223>  Description of Artificial Sequence: Synthetic oligonucleotide

<400>  1107
uugucuuuuu aag                                                         13


<210>  1108
<211>  12
<212>  RNA
<213>  Artificial Sequence

<220>
<223>  Description of Artificial Sequence: Synthetic oligonucleotide

<400>  1108
cuaaagguug uc                                                          12


<210>  1109
<211>  11
<212>  RNA
<213>  Artificial Sequence

<220>
<223>  Description of Artificial Sequence: Synthetic oligonucleotide

<400>  1109
agguugucga c                                                           11


<210>  1110
<211>  12
<212>  RNA
<213>  Artificial Sequence

<220>
<223>  Description of Artificial Sequence: Synthetic oligonucleotide

<400>  1110
ccuggcaauu gu                                                          12


<210>  1111
<211>  11
<212>  RNA
<213>  Artificial Sequence

<220>
<223>  Description of Artificial Sequence: Synthetic oligonucleotide

<400>  1111
guggugcgag g                                                           11


<210>  1112
<211>  12
<212>  RNA
<213>  Artificial Sequence

<220>
<223>  Description of Artificial Sequence: Synthetic oligonucleotide

<400>  1112
gagacaugaa au                                                          12


<210>  1113
<211>  12
<212>  RNA
<213>  Artificial Sequence

<220>
<223>  Description of Artificial Sequence: Synthetic oligonucleotide

<400>  1113

uauauuucuc ca                                                          12

<210>  1114
<211>  9
<212>  RNA
<213>  Artificial Sequence

<220>
<223>  Description of Artificial Sequence: Synthetic oligonucleotide

<400>  1114
augaaggcu                                                              9

<210>  1115
<211>  10
<212>  RNA
<213>  Artificial Sequence

<220>
<223>  Description of Artificial Sequence: Synthetic oligonucleotide

<400>  1115
cuggaguggu                                                             10

<210>  1116
<211>  10
<212>  RNA
<213>  Artificial Sequence

<220>
<223>  Description of Artificial Sequence: Synthetic oligonucleotide

<400>  1116
ggaagagagg                                                             10

<210>  1117
<211>  11
<212>  RNA
<213>  Artificial Sequence

<220>
<223>  Description of Artificial Sequence: Synthetic oligonucleotide

<400>  1117
uugcaucggg c                                                           11

<210>  1118
<211>  12
<212>  RNA
<213>  Artificial Sequence

<220>
<223>  Description of Artificial Sequence: Synthetic oligonucleotide

<400>  1118
uauccuacug ug                                                          12

<210>  1119
<211>  12
<212>  RNA
<213>  Artificial Sequence

<220>
<223>  Description of Artificial Sequence: Synthetic oligonucleotide

<400>  1119
ccuacugugu aa                                                          12


<210>  1120
<211>  11
<212>  RNA
<213>  Artificial Sequence

<220>
<223>  Description of Artificial Sequence: Synthetic oligonucleotide

<400>  1120
cuuguagugu a                                                           11


<210>  1121
<211>  12
<212>  RNA
<213>  Artificial Sequence

<220>
<223>  Description of Artificial Sequence: Synthetic oligonucleotide

<400>  1121
cuuuguugau ug                                                          12


<210>  1122
<211>  13
<212>  RNA
<213>  Artificial Sequence

<220>
<223>  Description of Artificial Sequence: Synthetic oligonucleotide

<400>  1122
uuguugauug aaa                                                         13


<210>  1123
<211>  14
<212>  RNA
<213>  Artificial Sequence

<220>
<223>  Description of Artificial Sequence: Synthetic oligonucleotide

<400>  1123
ucuuuuucuu ccau                                                        14

<210>  1124
<211>  12
<212>  RNA
<213>  Artificial Sequence

<220>
<223>  Description of Artificial Sequence: Synthetic oligonucleotide

<400>  1124
aucuuccuuc ua                                                          12


<210>  1125
<211>  12
<212>  RNA
<213>  Artificial Sequence

<220>
<223>  Description of Artificial Sequence: Synthetic oligonucleotide

<400>  1125
ugaaugcuuc au                                                          12


<210>  1126
<211>  12
<212>  RNA
<213>  Artificial Sequence

<220>
<223>  Description of Artificial Sequence: Synthetic oligonucleotide

<400>  1126
gaaugcuuca ug                                                          12


<210>  1127
<211>  12
<212>  RNA
<213>  Artificial Sequence

<220>
<223>  Description of Artificial Sequence: Synthetic oligonucleotide

<400>  1127
auucuacuuu ga                                                          12


<210>  1128
<211>  12
<212>  RNA
<213>  Artificial Sequence

<220>
<223>  Description of Artificial Sequence: Synthetic oligonucleotide

<400>  1128
gcaacuacag ac                                                          12

<210>  1129
<211>  12
<212>  RNA
<213>  Artificial Sequence

<220>
<223>  Description of Artificial Sequence: Synthetic oligonucleotide

<400>  1129
accaagguca ac                                                          12


<210>  1130
<211>  12
<212>  RNA
<213>  Artificial Sequence

<220>
<223>  Description of Artificial Sequence: Synthetic oligonucleotide

<400>  1130
gacuaucucg ac                                                          12


<210>  1131
<211>  12
<212>  RNA
<213>  Artificial Sequence

<220>
<223>  Description of Artificial Sequence: Synthetic oligonucleotide

<400>  1131
accuucccaa ac                                                          12


<210>  1132
<211>  13
<212>  RNA
<213>  Artificial Sequence

<220>
<223>  Description of Artificial Sequence: Synthetic oligonucleotide

<400>  1132
caagcuuaga acu                                                         13


<210>  1133
<211>  14
<212>  RNA
<213>  Artificial Sequence

<220>
<223>  Description of Artificial Sequence: Synthetic oligonucleotide

<400>  1133
gcuuagaacu uuaa                                                        14


<210>  1134

<211>  13
<212>  RNA
<213>  Artificial Sequence

<220>
<223>  Description of Artificial Sequence: Synthetic oligonucleotide

<400>  1134
aacuuuaagc aac                                                          13


<210>  1135
<211>  10
<212>  RNA
<213>  Artificial Sequence

<220>
<223>  Description of Artificial Sequence: Synthetic oligonucleotide

<400>  1135
agaccaccac                                                              10


<210>  1136
<211>  13
<212>  RNA
<213>  Artificial Sequence

<220>
<223>  Description of Artificial Sequence: Synthetic oligonucleotide

<400>  1136
cgagaaauaa agu                                                          13


<210>  1137
<211>  13
<212>  RNA
<213>  Artificial Sequence

<220>
<223>  Description of Artificial Sequence: Synthetic oligonucleotide

<400>  1137
gagaaauaaa guu                                                          13


<210>  1138
<211>  11
<212>  RNA
<213>  Artificial Sequence

<220>
<223>  Description of Artificial Sequence: Synthetic oligonucleotide

<400>  1138
ggaaaugauc c                                                            11


<210>  1139
<211>  12

<212>  RNA
<213>  Artificial Sequence

<220>
<223>  Description of Artificial Sequence: Synthetic oligonucleotide

<400>  1139
aagaaaugaa ac                                                          12


<210>  1140
<211>  12
<212>  RNA
<213>  Artificial Sequence

<220>
<223>  Description of Artificial Sequence: Synthetic oligonucleotide

<400>  1140
uuuccauccu cc                                                          12


<210>  1141
<211>  14
<212>  RNA
<213>  Artificial Sequence

<220>
<223>  Description of Artificial Sequence: Synthetic oligonucleotide

<400>  1141
cugaaaaucu uuga                                                        14


<210>  1142
<211>  10
<212>  RNA
<213>  Artificial Sequence

<220>
<223>  Description of Artificial Sequence: Synthetic oligonucleotide

<400>  1142
gccguucagg                                                             10


<210>  1143
<211>  12
<212>  RNA
<213>  Artificial Sequence

<220>
<223>  Description of Artificial Sequence: Synthetic oligonucleotide

<400>  1143
aggguccaga ag                                                          12


<210>  1144
<211>  12
<212>  RNA

<213>  Artificial Sequence

<220>
<223>  Description of Artificial Sequence: Synthetic oligonucleotide

<400>  1144
caagacugcu ug                                                          12


<210>  1145
<211>  12
<212>  RNA
<213>  Artificial Sequence

<220>
<223>  Description of Artificial Sequence: Synthetic oligonucleotide

<400>  1145
ccaugucuuu gg                                                          12


<210>  1146
<211>  10
<212>  RNA
<213>  Artificial Sequence

<220>
<223>  Description of Artificial Sequence: Synthetic oligonucleotide

<400>  1146
ccugaaacac                                                             10


<210>  1147
<211>  11
<212>  RNA
<213>  Artificial Sequence

<220>
<223>  Description of Artificial Sequence: Synthetic oligonucleotide

<400>  1147
cucuguaccu c                                                           11


<210>  1148
<211>  11
<212>  RNA
<213>  Artificial Sequence

<220>
<223>  Description of Artificial Sequence: Synthetic oligonucleotide

<400>  1148
gcaagacaag a                                                           11


<210>  1149
<211>  12
<212>  RNA
<213>  Artificial Sequence

<220>
<223>  Description of Artificial Sequence: Synthetic oligonucleotide

<400>  1149
caagacaaga aa                                                          12


<210>  1150
<211>  11
<212>  RNA
<213>  Artificial Sequence

<220>
<223>  Description of Artificial Sequence: Synthetic oligonucleotide

<400>  1150
guacaagauc c                                                           11


<210>  1151
<211>  11
<212>  RNA
<213>  Artificial Sequence

<220>
<223>  Description of Artificial Sequence: Synthetic oligonucleotide

<400>  1151
guacuugcag a                                                           11


<210>  1152
<211>  12
<212>  RNA
<213>  Artificial Sequence

<220>
<223>  Description of Artificial Sequence: Synthetic oligonucleotide

<400>  1152
gaucgauaca ga                                                          12


<210>  1153
<211>  12
<212>  RNA
<213>  Artificial Sequence

<220>
<223>  Description of Artificial Sequence: Synthetic oligonucleotide

<400>  1153
auaaaaauaa au                                                          12


<210>  1154
<211>  12
<212>  RNA
<213>  Artificial Sequence

<220>
<223>  Description of Artificial Sequence: Synthetic oligonucleotide

<400>  1154
aaaaauaaau au                                                          12


<210>  1155
<211>  12
<212>  RNA
<213>  Artificial Sequence

<220>
<223>  Description of Artificial Sequence: Synthetic oligonucleotide

<400>  1155
uugacugcug ug                                                          12


<210>  1156
<211>  12
<212>  RNA
<213>  Artificial Sequence

<220>
<223>  Description of Artificial Sequence: Synthetic oligonucleotide

<400>  1156
gagaugagag ac                                                          12


<210>  1157
<211>  9
<212>  RNA
<213>  Artificial Sequence

<220>
<223>  Description of Artificial Sequence: Synthetic oligonucleotide

<400>  1157
cccacuugg                                                               9


<210>  1158
<211>  2368
<212>  RNA
<213>  Homo sapiens

<400>  1158
gcggugccgc ccgccguggc cgccucagcc caccagccgg gaccgcgagc caugcugucc      60

gccgcccgcc cccaggguug uuaaagccag acugcgaacu cucgccacug ccgccaccgc     120

cgcgucccgu cccaccgucg cgggcaacaa ccaaagucgc cgcaacugca gcacagagcg     180

ggcaaagcca ggcaggccau ggggcucugg gcgcuguugc cuggcugggu uucugcuacg     240

cugcugcugg cgcuggccgc ucugcccgca gcccuggcug ccaacagcag uggccgaugg     300

ugggguauug ugaacguagc cuccuccacg aaccugcuua cagacuccaa gagucugcaa     360

cugguacucg agcccagucu gcagcuguug agccgcaaac agcggcgucu gauacgccaa 420

aauccgggga uccugcacag cgugaguggg gggcugcaga gugccgugcg cgagugcaag 480

uggcaguucc ggaaucgccg cuggaacugu cccacugcuc cagggcccca ccucuucggc 540

aagaucguca accgaggcug ucgagaaacg gcguuuaucu ucgcuaucac cuccgccggg 600

gucacccauu cgguggcgcg cuccugcuca gaagguucca ucgaauccug cacgugugac 660

uaccggcggc gcggccccgg gggccccgac uggcacuggg ggggcugcag cgacaacauu 720

gacuucggcc gccucuucgg ccgggaguuc guggacuccg ggagaagggg gcgggaccug 780

cgcuuccuca ugaaccuuca caacaacgag gcaggccgua cgaccguauu cuccgagaug 840

cgccaggagu gcaagugcca cgggaugucc ggcucaugca cggugcgcac gugcuggaug 900

cggcugccca cgcugcgcgc cgugggcgau gugcugcgcg accgcuucga cggcgccucg 960

cgcguccugu acggcaaccg cggcagcaac cgcgcuucgc gagcggagcu gcugcgccug 1020

gagccggaag acccggccca caaaccgccc uccccccacg accucgucua cuucgagaaa 1080

ucgcccaacu ucugcacgua cagcggacgc cugggcacag caggcacggc agggcgcgcc 1140

uguaacagcu cgucgcccgc gcuggacggc ugcgagcugc ucugcugcgg caggggccac 1200

cgcacgcgca cgcagcgcgu caccgagcgc ugcaacugca ccuuccacug gugcugccac 1260

gucagcugcc gcaacugcac gcacacgcgc guacugcacg agugucugug aggcgcugcg 1320

cggacucgcc cccaggaaac gcucuccucg agcccucccc caaacagacu cgcuagcacu 1380

caagacccgg uuauucgccc acccgaguac cuccagucac acuccccgcg guucauacgc 1440

aucccaucuc ucccacuucc uccuaccugg ggacuccuca aaccacuugc cuggggcggc 1500

augaacccuc uugccauccu gauggaccug ccccggaccu accucccucc cucuccgcgg 1560

gagaccccuu guugcacugc ccccugcuug gccaggaggu gagagaagga uggguccccu 1620

ccgccauggg gucggcuccu gaugguguca uucugccugc uccaucgcgc cagcgaccuc 1680

ucugccucuc uucuuccccu uuguccugcg uuuucuccgg guccuccuaa gucccuuccu 1740

auucuccugc caugggugca gacccugaac ccacaccugg gcaucagggc cuuucuccuc 1800

cccaccugua gcugaagcag gagguuacag ggcaaaaggg cagcugugau gauguggaaa 1860

ugagguuggg ggaaccagca gaaaugcccc cauucuccca gucucugucg uggagccauu 1920

gaacagcugu gagccaugcc ucccugggcc accuccuacc ccuuccuguc cugccuccuc 1980

aucagugugu aaauaauuug cacugaaacg uggauacaga gccacgaguu uggauguugu 2040

aaauaaaacu auuuauugug cuggguccca gccugguuug caaagaccac cuccaaccca 2100

acccaauccc ucuccacucu ucucuccuuu cucccugcag ccuuuucugg ucccucuucu 2160

cuccucaguu ucucaaagau gcguuugccu ccuggaauca guauuuccuu ccacuguagc 2220

uauuagcggc uccucgcccc caccagugua gcaucuuccu cugcagaaua aaaucucuau 2280

uuuuaucgau gacuuggugg cuuuuccuug aauccagaac acaaccuugu uugugguguc 2340

cccuauccuc cccuuuuacc acucccag 2368

<210> 1159
<211> 19
<212> RNA
<213> Artificial Sequence

<220>
<223> Description of Artificial Sequence: Synthetic oligonucleotide

<400> 1159
gcggugccgc ccgccgugg 19

<210> 1160
<211> 19
<212> RNA
<213> Artificial Sequence

<220>
<223> Description of Artificial Sequence: Synthetic oligonucleotide

<400> 1160
gccgccucag cccaccagc 19

<210> 1161
<211> 19
<212> RNA
<213> Artificial Sequence

<220>
<223> Description of Artificial Sequence: Synthetic oligonucleotide

<400> 1161
ccgggaccgc gagccaugc 19

<210> 1162
<211> 19
<212> RNA
<213> Artificial Sequence

<220>
<223> Description of Artificial Sequence: Synthetic oligonucleotide

<400> 1162
cuguccgccg cccgccccc 19

<210> 1163
<211> 19
<212> RNA
<213> Artificial Sequence

<220>
<223>  Description of Artificial Sequence: Synthetic oligonucleotide

<400>  1163
cagggguugu aaagccaga                                                    19


<210>  1164
<211>  19
<212>  RNA
<213>  Artificial Sequence

<220>
<223>  Description of Artificial Sequence: Synthetic oligonucleotide

<400>  1164
acugcgaacu cucgccacu                                                    19


<210>  1165
<211>  19
<212>  RNA
<213>  Artificial Sequence

<220>
<223>  Description of Artificial Sequence: Synthetic oligonucleotide

<400>  1165
ugccgccacc gccgcgucc                                                    19


<210>  1166
<211>  19
<212>  RNA
<213>  Artificial Sequence

<220>
<223>  Description of Artificial Sequence: Synthetic oligonucleotide

<400>  1166
ccgucccacc gucgcgggc                                                    19


<210>  1167
<211>  19
<212>  RNA
<213>  Artificial Sequence

<220>
<223>  Description of Artificial Sequence: Synthetic oligonucleotide

<400>  1167
caacaaccaa agucgccgc                                                    19


<210>  1168
<211>  19
<212>  RNA
<213>  Artificial Sequence

<220>

<223>  Description of Artificial Sequence: Synthetic oligonucleotide

<400>  1168
caacugcagc acagagcgg                                                    19


<210>  1169
<211>  19
<212>  RNA
<213>  Artificial Sequence

<220>
<223>  Description of Artificial Sequence: Synthetic oligonucleotide

<400>  1169
ggcaaagcca ggcaggcca                                                    19


<210>  1170
<211>  19
<212>  RNA
<213>  Artificial Sequence

<220>
<223>  Description of Artificial Sequence: Synthetic oligonucleotide

<400>  1170
auggggcucu gggcgcugu                                                    19


<210>  1171
<211>  19
<212>  RNA
<213>  Artificial Sequence

<220>
<223>  Description of Artificial Sequence: Synthetic oligonucleotide

<400>  1171
uugccuggcu ggguuucug                                                    19


<210>  1172
<211>  19
<212>  RNA
<213>  Artificial Sequence

<220>
<223>  Description of Artificial Sequence: Synthetic oligonucleotide

<400>  1172
gcuacgcugc ugcuggcgc                                                    19


<210>  1173
<211>  19
<212>  RNA
<213>  Artificial Sequence

<220>
<223>  Description of Artificial Sequence: Synthetic oligonucleotide

<400>  1173
cuggccgcuc ugcccgcag                                                    19


<210>  1174
<211>  19
<212>  RNA
<213>  Artificial Sequence

<220>
<223>  Description of Artificial Sequence: Synthetic oligonucleotide

<400>  1174
gcccuggcug ccaacagca                                                    19


<210>  1175
<211>  19
<212>  RNA
<213>  Artificial Sequence

<220>
<223>  Description of Artificial Sequence: Synthetic oligonucleotide

<400>  1175
aguggccgau gguggggua                                                    19


<210>  1176
<211>  19
<212>  RNA
<213>  Artificial Sequence

<220>
<223>  Description of Artificial Sequence: Synthetic oligonucleotide

<400>  1176
auugugaacg uagccuccu                                                    19


<210>  1177
<211>  19
<212>  RNA
<213>  Artificial Sequence

<220>
<223>  Description of Artificial Sequence: Synthetic oligonucleotide

<400>  1177
uccacgaacc ugcuuacag                                                    19


<210>  1178
<211>  19
<212>  RNA
<213>  Artificial Sequence

<220>
<223>  Description of Artificial Sequence: Synthetic oligonucleotide

<400>  1178
gacuccaaga gucugcaac                                                    19


<210>  1179
<211>  19
<212>  RNA
<213>  Artificial Sequence

<220>
<223>  Description of Artificial Sequence: Synthetic oligonucleotide

<400>  1179
cugguacucg agcccaguc                                                    19


<210>  1180
<211>  19
<212>  RNA
<213>  Artificial Sequence

<220>
<223>  Description of Artificial Sequence: Synthetic oligonucleotide

<400>  1180
cugcagcugu ugagccgca                                                    19


<210>  1181
<211>  19
<212>  RNA
<213>  Artificial Sequence

<220>
<223>  Description of Artificial Sequence: Synthetic oligonucleotide

<400>  1181
aaacagcggc gucugauac                                                    19


<210>  1182
<211>  19
<212>  RNA
<213>  Artificial Sequence

<220>
<223>  Description of Artificial Sequence: Synthetic oligonucleotide

<400>  1182
cgccaaaauc cggggaucc                                                    19


<210>  1183
<211>  19
<212>  RNA
<213>  Artificial Sequence

<220>
<223>  Description of Artificial Sequence: Synthetic oligonucleotide

<400>  1183

cugcacagcg ugaguggggg 19

<210> 1184
<211> 19
<212> RNA
<213> Artificial Sequence

<220>
<223> Description of Artificial Sequence: Synthetic oligonucleotide

<400> 1184
gggcugcaga gugccgugc 19

<210> 1185
<211> 19
<212> RNA
<213> Artificial Sequence

<220>
<223> Description of Artificial Sequence: Synthetic oligonucleotide

<400> 1185
cgcgagugca aguggcagu 19

<210> 1186
<211> 19
<212> RNA
<213> Artificial Sequence

<220>
<223> Description of Artificial Sequence: Synthetic oligonucleotide

<400> 1186
uuccggaauc gccgcugga 19

<210> 1187
<211> 19
<212> RNA
<213> Artificial Sequence

<220>
<223> Description of Artificial Sequence: Synthetic oligonucleotide

<400> 1187
aacuguccca cugcuccag 19

<210> 1188
<211> 19
<212> RNA
<213> Artificial Sequence

<220>
<223> Description of Artificial Sequence: Synthetic oligonucleotide

<400> 1188
gggccccacc ucuucggca 19

<210> 1189
<211> 19
<212> RNA
<213> Artificial Sequence

<220>
<223> Description of Artificial Sequence: Synthetic oligonucleotide

<400> 1189
aagaucguca accgaggcu 19


<210> 1190
<211> 19
<212> RNA
<213> Artificial Sequence

<220>
<223> Description of Artificial Sequence: Synthetic oligonucleotide

<400> 1190
ugucgagaaa cggcguuua 19


<210> 1191
<211> 19
<212> RNA
<213> Artificial Sequence

<220>
<223> Description of Artificial Sequence: Synthetic oligonucleotide

<400> 1191
aucuucgcua ucaccuccg 19


<210> 1192
<211> 19
<212> RNA
<213> Artificial Sequence

<220>
<223> Description of Artificial Sequence: Synthetic oligonucleotide

<400> 1192
gccgggguca cccauucgg 19


<210> 1193
<211> 19
<212> RNA
<213> Artificial Sequence

<220>
<223> Description of Artificial Sequence: Synthetic oligonucleotide

<400> 1193
guggcgcgcu ccugcucag 19

<210>  1194
<211>  19
<212>  RNA
<213>  Artificial Sequence

<220>
<223>  Description of Artificial Sequence: Synthetic oligonucleotide

<400>  1194
gaagguucca ucgaauccu                                                    19


<210>  1195
<211>  19
<212>  RNA
<213>  Artificial Sequence

<220>
<223>  Description of Artificial Sequence: Synthetic oligonucleotide

<400>  1195
ugcacgugug acuaccggc                                                    19


<210>  1196
<211>  19
<212>  RNA
<213>  Artificial Sequence

<220>
<223>  Description of Artificial Sequence: Synthetic oligonucleotide

<400>  1196
cggcgcggcc ccgggggcc                                                    19


<210>  1197
<211>  19
<212>  RNA
<213>  Artificial Sequence

<220>
<223>  Description of Artificial Sequence: Synthetic oligonucleotide

<400>  1197
cccgacuggc acugggggg                                                    19


<210>  1198
<211>  19
<212>  RNA
<213>  Artificial Sequence

<220>
<223>  Description of Artificial Sequence: Synthetic oligonucleotide

<400>  1198
ggcugcagcg acaacauug                                                    19

<210> 1199
<211> 19
<212> RNA
<213> Artificial Sequence

<220>
<223> Description of Artificial Sequence: Synthetic oligonucleotide

<400> 1199
gacuucggcc gccucuucg 19


<210> 1200
<211> 19
<212> RNA
<213> Artificial Sequence

<220>
<223> Description of Artificial Sequence: Synthetic oligonucleotide

<400> 1200
ggccgggagu ucguggacu 19


<210> 1201
<211> 19
<212> RNA
<213> Artificial Sequence

<220>
<223> Description of Artificial Sequence: Synthetic oligonucleotide

<400> 1201
uccggggaga aggggcggg 19


<210> 1202
<211> 19
<212> RNA
<213> Artificial Sequence

<220>
<223> Description of Artificial Sequence: Synthetic oligonucleotide

<400> 1202
gaccugcgcu uccucauga 19


<210> 1203
<211> 19
<212> RNA
<213> Artificial Sequence

<220>
<223> Description of Artificial Sequence: Synthetic oligonucleotide

<400> 1203
aaccuucaca acaacgagg 19


<210> 1204

<211>  19
<212>  RNA
<213>  Artificial Sequence

<220>
<223>  Description of Artificial Sequence: Synthetic oligonucleotide

<400>  1204
gcaggccgua cgaccguau                                                    19


<210>  1205
<211>  19
<212>  RNA
<213>  Artificial Sequence

<220>
<223>  Description of Artificial Sequence: Synthetic oligonucleotide

<400>  1205
uucuccgaga ugcgccagg                                                    19


<210>  1206
<211>  19
<212>  RNA
<213>  Artificial Sequence

<220>
<223>  Description of Artificial Sequence: Synthetic oligonucleotide

<400>  1206
gagugcaagu gccacggga                                                    19


<210>  1207
<211>  19
<212>  RNA
<213>  Artificial Sequence

<220>
<223>  Description of Artificial Sequence: Synthetic oligonucleotide

<400>  1207
auguccggcu caugcacgg                                                    19


<210>  1208
<211>  19
<212>  RNA
<213>  Artificial Sequence

<220>
<223>  Description of Artificial Sequence: Synthetic oligonucleotide

<400>  1208
gugcgcacgu gcuggaugc                                                    19


<210>  1209
<211>  19

<212>  RNA
<213>  Artificial Sequence

<220>
<223>  Description of Artificial Sequence: Synthetic oligonucleotide

<400>  1209
cggcugccca cgcugcgcg                                                    19


<210>  1210
<211>  19
<212>  RNA
<213>  Artificial Sequence

<220>
<223>  Description of Artificial Sequence: Synthetic oligonucleotide

<400>  1210
gccgugggcg augugcugc                                                    19


<210>  1211
<211>  19
<212>  RNA
<213>  Artificial Sequence

<220>
<223>  Description of Artificial Sequence: Synthetic oligonucleotide

<400>  1211
cgcgaccgcu ucgacggcg                                                    19


<210>  1212
<211>  19
<212>  RNA
<213>  Artificial Sequence

<220>
<223>  Description of Artificial Sequence: Synthetic oligonucleotide

<400>  1212
gccucgcgcg uccuguacg                                                    19


<210>  1213
<211>  19
<212>  RNA
<213>  Artificial Sequence

<220>
<223>  Description of Artificial Sequence: Synthetic oligonucleotide

<400>  1213
ggcaaccgcg gcagcaacc                                                    19


<210>  1214
<211>  19
<212>  RNA

<213>  Artificial Sequence

<220>
<223>  Description of Artificial Sequence: Synthetic oligonucleotide

<400>  1214
cgcgcuucgc gagcggagc                                                    19


<210>  1215
<211>  19
<212>  RNA
<213>  Artificial Sequence

<220>
<223>  Description of Artificial Sequence: Synthetic oligonucleotide

<400>  1215
cugcugcgcc uggagccgg                                                    19


<210>  1216
<211>  19
<212>  RNA
<213>  Artificial Sequence

<220>
<223>  Description of Artificial Sequence: Synthetic oligonucleotide

<400>  1216
gaagacccgg cccacaaac                                                    19


<210>  1217
<211>  19
<212>  RNA
<213>  Artificial Sequence

<220>
<223>  Description of Artificial Sequence: Synthetic oligonucleotide

<400>  1217
ccgcccuccc cccacgacc                                                    19


<210>  1218
<211>  19
<212>  RNA
<213>  Artificial Sequence

<220>
<223>  Description of Artificial Sequence: Synthetic oligonucleotide

<400>  1218
cucgucuacu ucgagaaau                                                    19


<210>  1219
<211>  19
<212>  RNA
<213>  Artificial Sequence

<220>
<223>  Description of Artificial Sequence: Synthetic oligonucleotide

<400>  1219
ucgcccaacu ucugcacgu                                                    19


<210>  1220
<211>  19
<212>  RNA
<213>  Artificial Sequence

<220>
<223>  Description of Artificial Sequence: Synthetic oligonucleotide

<400>  1220
uacagcggac gccugggca                                                    19


<210>  1221
<211>  19
<212>  RNA
<213>  Artificial Sequence

<220>
<223>  Description of Artificial Sequence: Synthetic oligonucleotide

<400>  1221
acagcaggca cggcagggc                                                    19


<210>  1222
<211>  19
<212>  RNA
<213>  Artificial Sequence

<220>
<223>  Description of Artificial Sequence: Synthetic oligonucleotide

<400>  1222
cgcgccugua acagcucgu                                                    19


<210>  1223
<211>  19
<212>  RNA
<213>  Artificial Sequence

<220>
<223>  Description of Artificial Sequence: Synthetic oligonucleotide

<400>  1223
ucgcccgcgc uggacggcu                                                    19


<210>  1224
<211>  19
<212>  RNA
<213>  Artificial Sequence

<220>
<223>  Description of Artificial Sequence: Synthetic oligonucleotide

<400>  1224
ugcgagcugc ucugcugcg                                                    19


<210>  1225
<211>  19
<212>  RNA
<213>  Artificial Sequence

<220>
<223>  Description of Artificial Sequence: Synthetic oligonucleotide

<400>  1225
ggcaggggcc accgcacgc                                                    19


<210>  1226
<211>  19
<212>  RNA
<213>  Artificial Sequence

<220>
<223>  Description of Artificial Sequence: Synthetic oligonucleotide

<400>  1226
cgcacgcagc gcgucaccg                                                    19


<210>  1227
<211>  19
<212>  RNA
<213>  Artificial Sequence

<220>
<223>  Description of Artificial Sequence: Synthetic oligonucleotide

<400>  1227
gagcgcugca acugcaccu                                                    19


<210>  1228
<211>  19
<212>  RNA
<213>  Artificial Sequence

<220>
<223>  Description of Artificial Sequence: Synthetic oligonucleotide

<400>  1228
uuccacuggu gcugccacg                                                    19


<210>  1229
<211>  19
<212>  RNA
<213>  Artificial Sequence

<220>

<223>  Description of Artificial Sequence: Synthetic oligonucleotide

<400>  1229
gucagcugcc gcaacugca                                                    19


<210>  1230
<211>  19
<212>  RNA
<213>  Artificial Sequence

<220>
<223>  Description of Artificial Sequence: Synthetic oligonucleotide

<400>  1230
acgcacacgc gcguacugc                                                    19


<210>  1231
<211>  19
<212>  RNA
<213>  Artificial Sequence

<220>
<223>  Description of Artificial Sequence: Synthetic oligonucleotide

<400>  1231
cacgaguguc ugugaggcg                                                    19


<210>  1232
<211>  19
<212>  RNA
<213>  Artificial Sequence

<220>
<223>  Description of Artificial Sequence: Synthetic oligonucleotide

<400>  1232
gcugcgcgga cucgccccc                                                    19


<210>  1233
<211>  19
<212>  RNA
<213>  Artificial Sequence

<220>
<223>  Description of Artificial Sequence: Synthetic oligonucleotide

<400>  1233
caggaaacgc ucuccucga                                                    19


<210>  1234
<211>  19
<212>  RNA
<213>  Artificial Sequence

<220>
<223>  Description of Artificial Sequence: Synthetic oligonucleotide

<400>  1234
agcccucccc caaacagac                                                    19


<210>  1235
<211>  19
<212>  RNA
<213>  Artificial Sequence

<220>
<223>  Description of Artificial Sequence: Synthetic oligonucleotide

<400>  1235
cucgcuagca cucaagacc                                                    19


<210>  1236
<211>  19
<212>  RNA
<213>  Artificial Sequence

<220>
<223>  Description of Artificial Sequence: Synthetic oligonucleotide

<400>  1236
ccgguuauuc gcccacccg                                                    19


<210>  1237
<211>  19
<212>  RNA
<213>  Artificial Sequence

<220>
<223>  Description of Artificial Sequence: Synthetic oligonucleotide

<400>  1237
gaguaccucc agucacacu                                                    19


<210>  1238
<211>  19
<212>  RNA
<213>  Artificial Sequence

<220>
<223>  Description of Artificial Sequence: Synthetic oligonucleotide

<400>  1238
uccccgcggu ucauacgca                                                    19


<210>  1239
<211>  19
<212>  RNA
<213>  Artificial Sequence

<220>
<223>  Description of Artificial Sequence: Synthetic oligonucleotide

<400>  1239
aucccaucuc ucccacuuc                                                    19


<210>  1240
<211>  19
<212>  RNA
<213>  Artificial Sequence

<220>
<223>  Description of Artificial Sequence: Synthetic oligonucleotide

<400>  1240
ccuccuaccu ggggacucc                                                    19


<210>  1241
<211>  19
<212>  RNA
<213>  Artificial Sequence

<220>
<223>  Description of Artificial Sequence: Synthetic oligonucleotide

<400>  1241
cucaaaccac uugccuggg                                                    19


<210>  1242
<211>  19
<212>  RNA
<213>  Artificial Sequence

<220>
<223>  Description of Artificial Sequence: Synthetic oligonucleotide

<400>  1242
ggcggcauga acccucuug                                                    19


<210>  1243
<211>  19
<212>  RNA
<213>  Artificial Sequence

<220>
<223>  Description of Artificial Sequence: Synthetic oligonucleotide

<400>  1243
gccauccuga uggaccugc                                                    19


<210>  1244
<211>  19
<212>  RNA
<213>  Artificial Sequence

<220>
<223>  Description of Artificial Sequence: Synthetic oligonucleotide

<400>  1244

ccccggaccu accucccuc                                                    19

<210>  1245
<211>  19
<212>  RNA
<213>  Artificial Sequence

<220>
<223>  Description of Artificial Sequence: Synthetic oligonucleotide

<400>  1245
cccucuccgc gggagaccc                                                    19

<210>  1246
<211>  19
<212>  RNA
<213>  Artificial Sequence

<220>
<223>  Description of Artificial Sequence: Synthetic oligonucleotide

<400>  1246
ccuuguugca cugcccccu                                                    19

<210>  1247
<211>  19
<212>  RNA
<213>  Artificial Sequence

<220>
<223>  Description of Artificial Sequence: Synthetic oligonucleotide

<400>  1247
ugcuuggcca ggaggugag                                                    19

<210>  1248
<211>  19
<212>  RNA
<213>  Artificial Sequence

<220>
<223>  Description of Artificial Sequence: Synthetic oligonucleotide

<400>  1248
gagaaggaug gguccccuc                                                    19

<210>  1249
<211>  19
<212>  RNA
<213>  Artificial Sequence

<220>
<223>  Description of Artificial Sequence: Synthetic oligonucleotide

<400>  1249
ccgccauggg gucggcucc                                                    19

<210> 1250
<211> 19
<212> RNA
<213> Artificial Sequence

<220>
<223> Description of Artificial Sequence: Synthetic oligonucleotide

<400> 1250
cugauggugu cauucugcc 19


<210> 1251
<211> 19
<212> RNA
<213> Artificial Sequence

<220>
<223> Description of Artificial Sequence: Synthetic oligonucleotide

<400> 1251
cugcuccauc gcgccagcg 19


<210> 1252
<211> 19
<212> RNA
<213> Artificial Sequence

<220>
<223> Description of Artificial Sequence: Synthetic oligonucleotide

<400> 1252
gaccucucug ccucucuuc 19


<210> 1253
<211> 19
<212> RNA
<213> Artificial Sequence

<220>
<223> Description of Artificial Sequence: Synthetic oligonucleotide

<400> 1253
cuuccccuuu guccugcgu 19


<210> 1254
<211> 19
<212> RNA
<213> Artificial Sequence

<220>
<223> Description of Artificial Sequence: Synthetic oligonucleotide

<400> 1254
uuuucuccgg guccuccua 19

<210>  1255
<211>  19
<212>  RNA
<213>  Artificial Sequence

<220>
<223>  Description of Artificial Sequence: Synthetic oligonucleotide

<400>  1255
aagucccuuc cuauucucc                                                    19


<210>  1256
<211>  19
<212>  RNA
<213>  Artificial Sequence

<220>
<223>  Description of Artificial Sequence: Synthetic oligonucleotide

<400>  1256
cugccauggg ugcagaccc                                                    19


<210>  1257
<211>  19
<212>  RNA
<213>  Artificial Sequence

<220>
<223>  Description of Artificial Sequence: Synthetic oligonucleotide

<400>  1257
cugaacccac accugggca                                                    19


<210>  1258
<211>  19
<212>  RNA
<213>  Artificial Sequence

<220>
<223>  Description of Artificial Sequence: Synthetic oligonucleotide

<400>  1258
aucagggccu uucuccucc                                                    19


<210>  1259
<211>  19
<212>  RNA
<213>  Artificial Sequence

<220>
<223>  Description of Artificial Sequence: Synthetic oligonucleotide

<400>  1259
cccaccugua gcugaagca                                                    19

<210>  1260
<211>  19
<212>  RNA
<213>  Artificial Sequence

<220>
<223>  Description of Artificial Sequence: Synthetic oligonucleotide

<400>  1260
aggagguuac agggcaaaa                                                    19


<210>  1261
<211>  19
<212>  RNA
<213>  Artificial Sequence

<220>
<223>  Description of Artificial Sequence: Synthetic oligonucleotide

<400>  1261
agggcagcug ugaugaugu                                                    19


<210>  1262
<211>  19
<212>  RNA
<213>  Artificial Sequence

<220>
<223>  Description of Artificial Sequence: Synthetic oligonucleotide

<400>  1262
uggaaaugag guuggggga                                                    19


<210>  1263
<211>  19
<212>  RNA
<213>  Artificial Sequence

<220>
<223>  Description of Artificial Sequence: Synthetic oligonucleotide

<400>  1263
aaccagcaga aaugccccc                                                    19


<210>  1264
<211>  19
<212>  RNA
<213>  Artificial Sequence

<220>
<223>  Description of Artificial Sequence: Synthetic oligonucleotide

<400>  1264
cauucuccca gucucuguc                                                    19


<210>  1265

<211>  19
<212>  RNA
<213>  Artificial Sequence

<220>
<223>  Description of Artificial Sequence: Synthetic oligonucleotide

<400>  1265
cguggagcca uugaacagc                                                    19


<210>  1266
<211>  19
<212>  RNA
<213>  Artificial Sequence

<220>
<223>  Description of Artificial Sequence: Synthetic oligonucleotide

<400>  1266
cugugagcca ugccucccu                                                    19


<210>  1267
<211>  19
<212>  RNA
<213>  Artificial Sequence

<220>
<223>  Description of Artificial Sequence: Synthetic oligonucleotide

<400>  1267
ugggccaccu ccuaccccu                                                    19


<210>  1268
<211>  19
<212>  RNA
<213>  Artificial Sequence

<220>
<223>  Description of Artificial Sequence: Synthetic oligonucleotide

<400>  1268
uuccuguccu gccuccuca                                                    19


<210>  1269
<211>  19
<212>  RNA
<213>  Artificial Sequence

<220>
<223>  Description of Artificial Sequence: Synthetic oligonucleotide

<400>  1269
aucagugugu aaauaauuu                                                    19


<210>  1270
<211>  19

<212>  RNA
<213>  Artificial Sequence

<220>
<223>  Description of Artificial Sequence: Synthetic oligonucleotide

<400>  1270
ugcacugaaa cguggauac                                                    19


<210>  1271
<211>  19
<212>  RNA
<213>  Artificial Sequence

<220>
<223>  Description of Artificial Sequence: Synthetic oligonucleotide

<400>  1271
cagagccacg aguuuggau                                                    19


<210>  1272
<211>  19
<212>  RNA
<213>  Artificial Sequence

<220>
<223>  Description of Artificial Sequence: Synthetic oligonucleotide

<400>  1272
uguuguaaau aaaacuauu                                                    19


<210>  1273
<211>  19
<212>  RNA
<213>  Artificial Sequence

<220>
<223>  Description of Artificial Sequence: Synthetic oligonucleotide

<400>  1273
   uuauugugcu gggucccag                                                     19


<210>  1274
<211>  19
<212>  RNA
<213>  Artificial Sequence

<220>
<223>  Description of Artificial Sequence: Synthetic oligonucleotide

<400>  1274
gccugguuug caaagacca                                                    19


<210>  1275
<211>  19
<212>  RNA

<213>  Artificial Sequence

<220>
<223>  Description of Artificial Sequence: Synthetic oligonucleotide

<400>  1275
accuccaacc caacccaau                                                    19


<210>  1276
<211>  19
<212>  RNA
<213>  Artificial Sequence

<220>
<223>  Description of Artificial Sequence: Synthetic oligonucleotide

<400>  1276
ucccucucca cucuucucu                                                    19


<210>  1277
<211>  19
<212>  RNA
<213>  Artificial Sequence

<220>
<223>  Description of Artificial Sequence: Synthetic oligonucleotide

<400>  1277
uccuuucucc cugcagccu                                                    19


<210>  1278
<211>  19
<212>  RNA
<213>  Artificial Sequence

<220>
<223>  Description of Artificial Sequence: Synthetic oligonucleotide

<400>  1278
uuuucugguc ccucuucuc                                                    19


<210>  1279
<211>  19
<212>  RNA
<213>  Artificial Sequence

<220>
<223>  Description of Artificial Sequence: Synthetic oligonucleotide

<400>  1279
cuccucaguu ucucaaaga                                                    19


<210>  1280
<211>  19
<212>  RNA
<213>  Artificial Sequence

<220>
<223>  Description of Artificial Sequence: Synthetic oligonucleotide

<400>  1280
augcguuugc cuccuggaa                                                    19


<210>  1281
<211>  19
<212>  RNA
<213>  Artificial Sequence

<220>
<223>  Description of Artificial Sequence: Synthetic oligonucleotide

<400>  1281
aucaguauuu ccuuccacu                                                    19


<210>  1282
<211>  19
<212>  RNA
<213>  Artificial Sequence

<220>
<223>  Description of Artificial Sequence: Synthetic oligonucleotide

<400>  1282
uguagcuauu agcggcucc                                                    19


<210>  1283
<211>  19
<212>  RNA
<213>  Artificial Sequence

<220>
<223>  Description of Artificial Sequence: Synthetic oligonucleotide

<400>  1283
cucgccccca ccaguguag                                                    19


<210>  1284
<211>  19
<212>  RNA
<213>  Artificial Sequence

<220>
<223>  Description of Artificial Sequence: Synthetic oligonucleotide

<400>  1284
gcaucuuccu cugcagaau                                                    19


<210>  1285
<211>  19
<212>  RNA
<213>  Artificial Sequence

<220>
<223>  Description of Artificial Sequence: Synthetic oligonucleotide

<400>  1285
uaaaaucucu auuuuuauc                                                    19


<210>  1286
<211>  19
<212>  RNA
<213>  Artificial Sequence

<220>
<223>  Description of Artificial Sequence: Synthetic oligonucleotide

<400>  1286
cgaugacuug guggcuuuu                                                    19


<210>  1287
<211>  19
<212>  RNA
<213>  Artificial Sequence

<220>
<223>  Description of Artificial Sequence: Synthetic oligonucleotide

<400>  1287
uccuugaauc cagaacaca                                                    19


<210>  1288
<211>  19
<212>  RNA
<213>  Artificial Sequence

<220>
<223>  Description of Artificial Sequence: Synthetic oligonucleotide

<400>  1288
aaccuuguuu guggugucc                                                    19


<210>  1289
<211>  19
<212>  RNA
<213>  Artificial Sequence

<220>
<223>  Description of Artificial Sequence: Synthetic oligonucleotide

<400>  1289
cccuauccuc cccuuuuac                                                    19


<210>  1290
<211>  19
<212>  RNA
<213>  Artificial Sequence

<220>

<223>  Description of Artificial Sequence: Synthetic oligonucleotide

<400>  1290
ccccuuuuac cacucccag                                                    19


<210>  1291
<211>  25
<212>  RNA
<213>  Artificial Sequence

<220>
<223>  Description of Artificial Sequence: Synthetic oligonucleotide

<400>  1291
gcggugccgc ccgccguggc cgccu                                             25


<210>  1292
<211>  25
<212>  RNA
<213>  Artificial Sequence

<220>
<223>  Description of Artificial Sequence: Synthetic oligonucleotide

<400>  1292
ucagcccacc agccgggacc gcgag                                             25


<210>  1293
<211>  25
<212>  RNA
<213>  Artificial Sequence

<220>
<223>  Description of Artificial Sequence: Synthetic oligonucleotide

<400>  1293
gccaugcugu ccgccgcccg ccccc                                             25


<210>  1294
<211>  25
<212>  RNA
<213>  Artificial Sequence

<220>
<223>  Description of Artificial Sequence: Synthetic oligonucleotide

<400>  1294
   caggguuguu aaagccagac ugcga                                            25


<210>  1295
<211>  25
<212>  RNA
<213>  Artificial Sequence

<220>
<223>  Description of Artificial Sequence: Synthetic oligonucleotide

<400>  1295
aacucucgcc acugccgcca ccgcc                                          25


<210>  1296
<211>  25
<212>  RNA
<213>  Artificial Sequence

<220>
<223>  Description of Artificial Sequence: Synthetic oligonucleotide

<400>  1296
cgcgucccgu cccaccgucg cgggc                                          25


<210>  1297
<211>  25
<212>  RNA
<213>  Artificial Sequence

<220>
<223>  Description of Artificial Sequence: Synthetic oligonucleotide

<400>  1297
caacaaccaa agucgccgca acugc                                          25


<210>  1298
<211>  25
<212>  RNA
<213>  Artificial Sequence

<220>
<223>  Description of Artificial Sequence: Synthetic oligonucleotide

<400>  1298
cagcacagag cgggcaaagc caggc                                          25


<210>  1299
<211>  25
<212>  RNA
<213>  Artificial Sequence

<220>
<223>  Description of Artificial Sequence: Synthetic oligonucleotide

<400>  1299
caggccaugg ggcucugggc gcugu                                          25


<210>  1300
<211>  25
<212>  RNA
<213>  Artificial Sequence

<220>
<223>  Description of Artificial Sequence: Synthetic oligonucleotide

<400>  1300
uugccuggcu ggguuucugc uacgc                                          25

<210>  1301
<211>  25
<212>  RNA
<213>  Artificial Sequence

<220>
<223>  Description of Artificial Sequence: Synthetic oligonucleotide

<400>  1301
cugcugcugg cgcuggccgc ucugc                                          25

<210>  1302
<211>  25
<212>  RNA
<213>  Artificial Sequence

<220>
<223>  Description of Artificial Sequence: Synthetic oligonucleotide

<400>  1302
cccgcagccc uggcugccaa cagca                                          25

<210>  1303
<211>  25
<212>  RNA
<213>  Artificial Sequence

<220>
<223>  Description of Artificial Sequence: Synthetic oligonucleotide

<400>  1303
aguggccgau ggugggguau uguga                                          25

<210>  1304
<211>  25
<212>  RNA
<213>  Artificial Sequence

<220>
<223>  Description of Artificial Sequence: Synthetic oligonucleotide

<400>  1304
aacguagccu ccuccacgaa ccugc                                          25

<210>  1305
<211>  25
<212>  RNA
<213>  Artificial Sequence

<220>
<223>  Description of Artificial Sequence: Synthetic oligonucleotide

<400>  1305

cuuacagacu ccaagagucu gcaac                                          25

<210>  1306
<211>  25
<212>  RNA
<213>  Artificial Sequence

<220>
<223>  Description of Artificial Sequence: Synthetic oligonucleotide

<400>  1306
cugguacucg agcccagucu gcagc                                          25

<210>  1307
<211>  25
<212>  RNA
<213>  Artificial Sequence

<220>
<223>  Description of Artificial Sequence: Synthetic oligonucleotide

<400>  1307
cuguugagcc gcaaacagcg gcguc                                          25

<210>  1308
<211>  25
<212>  RNA
<213>  Artificial Sequence

<220>
<223>  Description of Artificial Sequence: Synthetic oligonucleotide

<400>  1308
cugauacgcc aaaauccggg gaucc                                          25

<210>  1309
<211>  25
<212>  RNA
<213>  Artificial Sequence

<220>
<223>  Description of Artificial Sequence: Synthetic oligonucleotide

<400>  1309
cugcacagcg ugaguggggg gcugc                                          25

<210>  1310
<211>  25
<212>  RNA
<213>  Artificial Sequence

<220>
<223>  Description of Artificial Sequence: Synthetic oligonucleotide

<400>  1310
cagagugccg ugcgcgagug caagu                                          25

<210>  1311
<211>  25
<212>  RNA
<213>  Artificial Sequence

<220>
<223>  Description of Artificial Sequence: Synthetic oligonucleotide

<400>  1311
uggcaguucc ggaaucgccg cugga                                          25


<210>  1312
<211>  25
<212>  RNA
<213>  Artificial Sequence

<220>
<223>  Description of Artificial Sequence: Synthetic oligonucleotide

<400>  1312
aacuguccca cugcuccagg gcccc                                          25


<210>  1313
<211>  25
<212>  RNA
<213>  Artificial Sequence

<220>
<223>  Description of Artificial Sequence: Synthetic oligonucleotide

<400>  1313
caccucuucg gcaagaucgu caacc                                          25


<210>  1314
<211>  25
<212>  RNA
<213>  Artificial Sequence

<220>
<223>  Description of Artificial Sequence: Synthetic oligonucleotide

<400>  1314
cgaggcuguc gagaaacggc guuua                                          25


<210>  1315
<211>  25
<212>  RNA
<213>  Artificial Sequence

<220>
<223>  Description of Artificial Sequence: Synthetic oligonucleotide

<400>  1315
aucuucgcua ucaccuccgc cgggg                                          25

<210> 1316
<211> 25
<212> RNA
<213> Artificial Sequence

<220>
<223> Description of Artificial Sequence: Synthetic oligonucleotide

<400> 1316
gucacccauu cgguggcgcg cuccu 25


<210> 1317
<211> 25
<212> RNA
<213> Artificial Sequence

<220>
<223> Description of Artificial Sequence: Synthetic oligonucleotide

<400> 1317
ugcucagaag guuccaucga auccu 25


<210> 1318
<211> 25
<212> RNA
<213> Artificial Sequence

<220>
<223> Description of Artificial Sequence: Synthetic oligonucleotide

<400> 1318
ugcacgugug acuaccggcg gcgcg 25


<210> 1319
<211> 25
<212> RNA
<213> Artificial Sequence

<220>
<223> Description of Artificial Sequence: Synthetic oligonucleotide

<400> 1319
ggccccgggg gccccgacug gcacu 25


<210> 1320
<211> 25
<212> RNA
<213> Artificial Sequence

<220>
<223> Description of Artificial Sequence: Synthetic oligonucleotide

<400> 1320
ugggggggcu gcagcgacaa cauug 25

<210>  1321
<211>  25
<212>  RNA
<213>  Artificial Sequence

<220>
<223>  Description of Artificial Sequence: Synthetic oligonucleotide

<400>  1321
gacuucggcc gccucuucgg ccggg                                          25


<210>  1322
<211>  25
<212>  RNA
<213>  Artificial Sequence

<220>
<223>  Description of Artificial Sequence: Synthetic oligonucleotide

<400>  1322
gaguucgugg acuccgggga gaagg                                          25


<210>  1323
<211>  25
<212>  RNA
<213>  Artificial Sequence

<220>
<223>  Description of Artificial Sequence: Synthetic oligonucleotide

<400>  1323
gggcgggacc ugcgcuuccu cauga                                          25


<210>  1324
<211>  25
<212>  RNA
<213>  Artificial Sequence

<220>
<223>  Description of Artificial Sequence: Synthetic oligonucleotide

<400>  1324
aaccuucaca acaacgaggc aggcc                                          25


<210>  1325
<211>  25
<212>  RNA
<213>  Artificial Sequence

<220>
<223>  Description of Artificial Sequence: Synthetic oligonucleotide

<400>  1325
cguacgaccg uauucuccga gaugc                                          25


<210>  1326

<211>  25
<212>  RNA
<213>  Artificial Sequence

<220>
<223>  Description of Artificial Sequence: Synthetic oligonucleotide

<400>  1326
cgccaggagu gcaagugcca cggga                                          25


<210>  1327
<211>  25
<212>  RNA
<213>  Artificial Sequence

<220>
<223>  Description of Artificial Sequence: Synthetic oligonucleotide

<400>  1327
auguccggcu caugcacggu gcgca                                          25


<210>  1328
<211>  25
<212>  RNA
<213>  Artificial Sequence

<220>
<223>  Description of Artificial Sequence: Synthetic oligonucleotide

<400>  1328
acgugcugga ugcggcugcc cacgc                                          25


<210>  1329
<211>  25
<212>  RNA
<213>  Artificial Sequence

<220>
<223>  Description of Artificial Sequence: Synthetic oligonucleotide

<400>  1329
cugcgcgccg ugggcgaugu gcugc                                          25


<210>  1330
<211>  25
<212>  RNA
<213>  Artificial Sequence

<220>
<223>  Description of Artificial Sequence: Synthetic oligonucleotide

<400>  1330
cgcgaccgcu ucgacggcgc cucgc                                          25


<210>  1331
<211>  25

<212>  RNA
<213>  Artificial Sequence

<220>
<223>  Description of Artificial Sequence: Synthetic oligonucleotide

<400>  1331
cgcguccugu acggcaaccg cggca                                          25


<210>  1332
<211>  25
<212>  RNA
<213>  Artificial Sequence

<220>
<223>  Description of Artificial Sequence: Synthetic oligonucleotide

<400>  1332
agcaaccgcg cuucgcgagc ggagc                                          25


<210>  1333
<211>  25
<212>  RNA
<213>  Artificial Sequence

<220>
<223>  Description of Artificial Sequence: Synthetic oligonucleotide

<400>  1333
cugcugcgcc uggagccgga agacc                                          25


<210>  1334
<211>  25
<212>  RNA
<213>  Artificial Sequence

<220>
<223>  Description of Artificial Sequence: Synthetic oligonucleotide

<400>  1334
ccggcccaca aaccgcccuc ccccc                                          25


<210>  1335
<211>  25
<212>  RNA
<213>  Artificial Sequence

<220>
<223>  Description of Artificial Sequence: Synthetic oligonucleotide

<400>  1335
cacgaccucg ucuacuucga gaaau                                          25


<210>  1336
<211>  25
<212>  RNA

<213>  Artificial Sequence

<220>
<223>  Description of Artificial Sequence: Synthetic oligonucleotide

<400>  1336
ucgcccaacu ucugcacgua cagcg                                          25


<210>  1337
<211>  25
<212>  RNA
<213>  Artificial Sequence

<220>
<223>  Description of Artificial Sequence: Synthetic oligonucleotide

<400>  1337
ggacgccugg gcacagcagg cacgg                                          25


<210>  1338
<211>  25
<212>  RNA
<213>  Artificial Sequence

<220>
<223>  Description of Artificial Sequence: Synthetic oligonucleotide

<400>  1338
gcagggcgcg ccuguaacag cucgu                                          25


<210>  1339
<211>  25
<212>  RNA
<213>  Artificial Sequence

<220>
<223>  Description of Artificial Sequence: Synthetic oligonucleotide

<400>  1339
ucgcccgcgc uggacggcug cgagc                                          25


<210>  1340
<211>  25
<212>  RNA
<213>  Artificial Sequence

<220>
<223>  Description of Artificial Sequence: Synthetic oligonucleotide

<400>  1340
cugcucugcu gcggcagggg ccacc                                          25


<210>  1341
<211>  25
<212>  RNA
<213>  Artificial Sequence

<220>
<223>  Description of Artificial Sequence: Synthetic oligonucleotide

<400>  1341
cgcacgcgca cgcagcgcgu caccg                                          25


<210>  1342
<211>  25
<212>  RNA
<213>  Artificial Sequence

<220>
<223>  Description of Artificial Sequence: Synthetic oligonucleotide

<400>  1342
gagcgcugca acugcaccuu ccacu                                          25


<210>  1343
<211>  25
<212>  RNA
<213>  Artificial Sequence

<220>
<223>  Description of Artificial Sequence: Synthetic oligonucleotide

<400>  1343
uggugcugcc acgucagcug ccgca                                          25


<210>  1344
<211>  25
<212>  RNA
<213>  Artificial Sequence

<220>
<223>  Description of Artificial Sequence: Synthetic oligonucleotide

<400>  1344
aacugcacgc acacgcgcgu acugc                                          25


<210>  1345
<211>  25
<212>  RNA
<213>  Artificial Sequence

<220>
<223>  Description of Artificial Sequence: Synthetic oligonucleotide

<400>  1345
cacgaguguc ugugaggcgc ugcgc                                          25


<210>  1346
<211>  25
<212>  RNA
<213>  Artificial Sequence

<220>
<223>  Description of Artificial Sequence: Synthetic oligonucleotide

<400>  1346
cggacucgcc cccaggaaac gcucu                                          25


<210>  1347
<211>  25
<212>  RNA
<213>  Artificial Sequence

<220>
<223>  Description of Artificial Sequence: Synthetic oligonucleotide

<400>  1347
uccucgagcc cucccccaaa cagac                                          25


<210>  1348
<211>  25
<212>  RNA
<213>  Artificial Sequence

<220>
<223>  Description of Artificial Sequence: Synthetic oligonucleotide

<400>  1348
cucgcuagca cucaagaccc gguua                                          25


<210>  1349
<211>  25
<212>  RNA
<213>  Artificial Sequence

<220>
<223>  Description of Artificial Sequence: Synthetic oligonucleotide

<400>  1349
auucgcccac ccgaguaccu ccagu                                          25


<210>  1350
<211>  25
<212>  RNA
<213>  Artificial Sequence

<220>
<223>  Description of Artificial Sequence: Synthetic oligonucleotide

<400>  1350
ucacacuccc cgcgguucau acgca                                          25


<210>  1351
<211>  25
<212>  RNA
<213>  Artificial Sequence

<220>

<223>  Description of Artificial Sequence: Synthetic oligonucleotide

<400>  1351
aucccaucuc ucccacuucc uccua                                          25


<210>  1352
<211>  25
<212>  RNA
<213>  Artificial Sequence

<220>
<223>  Description of Artificial Sequence: Synthetic oligonucleotide

<400>  1352
accuggggac uccucaaacc acuug                                          25


<210>  1353
<211>  25
<212>  RNA
<213>  Artificial Sequence

<220>
<223>  Description of Artificial Sequence: Synthetic oligonucleotide

<400>  1353
gccuggggcg gcaugaaccc ucuug                                          25


<210>  1354
<211>  25
<212>  RNA
<213>  Artificial Sequence

<220>
<223>  Description of Artificial Sequence: Synthetic oligonucleotide

<400>  1354
gccauccuga uggaccugcc ccgga                                          25


<210>  1355
<211>  25
<212>  RNA
<213>  Artificial Sequence

<220>
<223>  Description of Artificial Sequence: Synthetic oligonucleotide

<400>  1355
accuaccucc cucccucucc gcggg                                          25


<210>  1356
<211>  25
<212>  RNA
<213>  Artificial Sequence

<220>
<223>  Description of Artificial Sequence: Synthetic oligonucleotide

<400>  1356
gagaccccuu guugcacugc ccccu                                              25


<210>  1357
<211>  25
<212>  RNA
<213>  Artificial Sequence

<220>
<223>  Description of Artificial Sequence: Synthetic oligonucleotide

<400>  1357
ugcuuggcca ggaggugaga gaagg                                              25


<210>  1358
<211>  25
<212>  RNA
<213>  Artificial Sequence

<220>
<223>  Description of Artificial Sequence: Synthetic oligonucleotide

<400>  1358
gaugggaccc cuccgccaug ggguc                                              25


<210>  1359
<211>  25
<212>  RNA
<213>  Artificial Sequence

<220>
<223>  Description of Artificial Sequence: Synthetic oligonucleotide

<400>  1359
cggcuccuga uggugucauu cugcc                                              25


<210>  1360
<211>  25
<212>  RNA
<213>  Artificial Sequence

<220>
<223>  Description of Artificial Sequence: Synthetic oligonucleotide

<400>  1360
cugcuccauc gcgccagcga ccucu                                              25


<210>  1361
<211>  25
<212>  RNA
<213>  Artificial Sequence

<220>
<223>  Description of Artificial Sequence: Synthetic oligonucleotide

<400>  1361
ucugccucuc uucuuccccu uuguc                                          25


<210>  1362
<211>  25
<212>  RNA
<213>  Artificial Sequence

<220>
<223>  Description of Artificial Sequence: Synthetic oligonucleotide

<400>  1362
ccugcguuuu cuccgggucc uccua                                          25


<210>  1363
<211>  25
<212>  RNA
<213>  Artificial Sequence

<220>
<223>  Description of Artificial Sequence: Synthetic oligonucleotide

<400>  1363
aagucccuuc cuauucuccu gccau                                          25


<210>  1364
<211>  25
<212>  RNA
<213>  Artificial Sequence

<220>
<223>  Description of Artificial Sequence: Synthetic oligonucleotide

<400>  1364
ugggugcaga cccugaaccc acacc                                          25


<210>  1365
<211>  25
<212>  RNA
<213>  Artificial Sequence

<220>
<223>  Description of Artificial Sequence: Synthetic oligonucleotide

<400>  1365
cugggcauca gggccuuucu ccucc                                          25


<210>  1366
<211>  25
<212>  RNA
<213>  Artificial Sequence

<220>
<223>  Description of Artificial Sequence: Synthetic oligonucleotide

<400>  1366

cccaccugua gcugaagcag gaggu                                          25

<210>  1367
<211>  25
<212>  RNA
<213>  Artificial Sequence

<220>
<223>  Description of Artificial Sequence: Synthetic oligonucleotide

<400>  1367
uuacagggca aaagggcagc uguga                                          25

<210>  1368
<211>  25
<212>  RNA
<213>  Artificial Sequence

<220>
<223>  Description of Artificial Sequence: Synthetic oligonucleotide

<400>  1368
augaugugga aaugagguug gggga                                          25

<210>  1369
<211>  25
<212>  RNA
<213>  Artificial Sequence

<220>
<223>  Description of Artificial Sequence: Synthetic oligonucleotide

<400>  1369
aaccagcaga aaugccccca uucuc                                          25

<210>  1370
<211>  25
<212>  RNA
<213>  Artificial Sequence

<220>
<223>  Description of Artificial Sequence: Synthetic oligonucleotide

<400>  1370
cccagucucu gucguggagc cauug                                          25

<210>  1371
<211>  25
<212>  RNA
<213>  Artificial Sequence

<220>
<223>  Description of Artificial Sequence: Synthetic oligonucleotide

<400>  1371
gaacagcugu gagccaugcc ucccu                                          25

<210>  1372
<211>  25
<212>  RNA
<213>  Artificial Sequence

<220>
<223>  Description of Artificial Sequence: Synthetic oligonucleotide

<400>  1372
ugggccaccu ccuaccccuu ccugu                                          25


<210>  1373
<211>  25
<212>  RNA
<213>  Artificial Sequence

<220>
<223>  Description of Artificial Sequence: Synthetic oligonucleotide

<400>  1373
uccugccucc ucaucagugu guaaa                                          25


<210>  1374
<211>  25
<212>  RNA
<213>  Artificial Sequence

<220>
<223>  Description of Artificial Sequence: Synthetic oligonucleotide

<400>  1374
auaauuugca cugaaacgug gauac                                          25


<210>  1375
<211>  25
<212>  RNA
<213>  Artificial Sequence

<220>
<223>  Description of Artificial Sequence: Synthetic oligonucleotide

<400>  1375
cagagccacg aguuuggaug uugua                                          25


<210>  1376
<211>  25
<212>  RNA
<213>  Artificial Sequence

<220>
<223>  Description of Artificial Sequence: Synthetic oligonucleotide

<400>  1376
aaauaaaacu auuuauugug cuggg                                          25

<210>  1377
<211>  25
<212>  RNA
<213>  Artificial Sequence

<220>
<223>  Description of Artificial Sequence: Synthetic oligonucleotide

<400>  1377
gucccagccu gguuugcaaa gacca                                          25


<210>  1378
<211>  25
<212>  RNA
<213>  Artificial Sequence

<220>
<223>  Description of Artificial Sequence: Synthetic oligonucleotide

<400>  1378
accuccaacc caacccaauc ccucu                                          25


<210>  1379
<211>  25
<212>  RNA
<213>  Artificial Sequence

<220>
<223>  Description of Artificial Sequence: Synthetic oligonucleotide

<400>  1379
uccacucuuc ucuccuuucu cccug                                          25


<210>  1380
<211>  25
<212>  RNA
<213>  Artificial Sequence

<220>
<223>  Description of Artificial Sequence: Synthetic oligonucleotide

<400>  1380
   gcagccuuuu cuggucccuc uucuc                                          25


<210>  1381
<211>  25
<212>  RNA
<213>  Artificial Sequence

<220>
<223>  Description of Artificial Sequence: Synthetic oligonucleotide

<400>  1381
cuccucaguu ucucaaagau gcguu                                          25

<210>  1382
<211>  25
<212>  RNA
<213>  Artificial Sequence

<220>
<223>  Description of Artificial Sequence: Synthetic oligonucleotide

<400>  1382
uugccuccug gaaucaguau uuccu                                          25


<210>  1383
<211>  25
<212>  RNA
<213>  Artificial Sequence

<220>
<223>  Description of Artificial Sequence: Synthetic oligonucleotide

<400>  1383
uuccacugua gcuauuagcg gcucc                                          25


<210>  1384
<211>  25
<212>  RNA
<213>  Artificial Sequence

<220>
<223>  Description of Artificial Sequence: Synthetic oligonucleotide

<400>  1384
cucgccccca ccaguguagc aucuu                                          25


<210>  1385
<211>  25
<212>  RNA
<213>  Artificial Sequence

<220>
<223>  Description of Artificial Sequence: Synthetic oligonucleotide

<400>  1385
uccucugcag aauaaaaucu cuauu                                          25


<210>  1386
<211>  25
<212>  RNA
<213>  Artificial Sequence

<220>
<223>  Description of Artificial Sequence: Synthetic oligonucleotide

<400>  1386
uuuuaucgau gacuuggugg cuuuu                                          25


<210>  1387

<211>  25
<212>  RNA
<213>  Artificial Sequence

<220>
<223>  Description of Artificial Sequence: Synthetic oligonucleotide

<400>  1387
uccuugaauc cagaacacaa ccuug                                          25


<210>  1388
<211>  25
<212>  RNA
<213>  Artificial Sequence

<220>
<223>  Description of Artificial Sequence: Synthetic oligonucleotide

<400>  1388
guuuguggug uccccuaucc ucccc                                          25


<210>  1389
<211>  25
<212>  RNA
<213>  Artificial Sequence

<220>
<223>  Description of Artificial Sequence: Synthetic oligonucleotide

<400>  1389
uauccucccc uuuuaccacu cccag                                          25


<210>  1390
<211>  2301
<212>  RNA
<213>  Homo sapiens

<400>  1390
agcagagcgg acgggcgcgc gggaggcgcg cagagcuuuc gggcugcagg cgcucgcugc      60

cgcuggggaa uugggcugug ggcgaggcgg uccgggcugg ccuuuaucgc ucgcugggcc     120

caucguuuga aacuuuauca gcgagucgcc acucgucgca ggaccgagcg gggggcgggg     180

gcgcggcgag gcggcggccg ugacgaggcg cucccggagc ugagcgcuuc ugcucugggc     240

acgcauggcg cccgcacacg gagucugacc ugaugcagac gcaagggggu uaauaugaac     300

gccccucucg guggaaucug gcucuggcuc ccucugcucu ugaccuggcu cacccccgag     360

gucaacucuu caugguggua caugagagcu acagguggcu ccuccagggu gaugugcgau     420

aaugugccag gccuggugag cagccagcgg cagcuguguc accgacaucc agaugugaug     480

cgugccauua gccagggcgu ggccgagugg acagcagaau gccagcacca guuccgccag     540

caccgcugga auugcaacac ccuggacagg gaucacagcc uuuuuggcag gguccuacuc     600

cgaaguaguc gggaaucugc cuuuguuuau gccaucuccu cagcuggagu uguauuugcc 660

aucaccaggg ccuguagcca aggagaagua aaauccuguu ccugugaucc aaagaagaug 720

ggaagcgcca aggacagcaa aggcauuuuu gauuggggug gcugcaguga uaacauugac 780

uaugggauca aauuugcccg cgcauuugug gaugcaaagg aaaggaaagg aaaggaugcc 840

agagcccuga ugaaucuuca caacaacaga gcuggcagga aggcuguaaa gcgguucuug 900

aaacaagagu gcaagugcca cggggugagc ggcucaugua cucucaggac augcuggcug 960

gccauggccg acuucaggaa aacgggcgau uaucucugga ggaaguacaa uggggccauc 1020

caggugguca ugaaccagga uggcacaggu uucacugugg cuaacgagag guuuaagaag 1080

ccaacgaaaa augaccucgu guauuuugag aauucuccag acuacuguau cagggaccga 1140

gaggcaggcu cccuggguac agcaggccgu gugugcaacc ugacuucccg gggcauggac 1200

agcugugaag ucaugugcug ugggagaggc uacgacaccu cccaugucac ccggaugacc 1260

aagugugggu guaaguucca cuggugcugc gccgugcgcu gucaggacug ccuggaagcu 1320

cuggaugugc acacaugcaa ggcccccaag aacgcugacu ggacaaccgc uacaugaccc 1380

cagcaggcgu caccauccac cuucccuucu acaaggacuc cauuggaucu gcaagaacac 1440

uggaccuuug gguucuuucu ggggggauau uuccuaaggc auguggccuu uaucucaacg 1500

gaagcccccu cuuccucccu gggggcccca ggaugggggg ccacacgcug caccuaaagc 1560

cuacccuauu cuauccaucu ccugguguuc ugcagucauc uccccuccug gcgaguucuc 1620

uuuggaaaua gcaugacagg cuguucagcc gggagggugg ugggcccaga ccacugucuc 1680

cacccaccuu gacguuucuu cuuucuagag caguuggcca agcagaaaaa aaagugucuc 1740

aaaggagcuu ucucaauguc uucccacaaa uggucccaau uaagaaauuc cauacuucuc 1800

ucagauggaa caguaaagaa agcagaauca acugccccug acuuaacuuu aacuuuugaa 1860

aagaccaaga cuuuugucug uacaaguggu uuuacagcua ccacccuuag gguaauuggu 1920

aauuaccugg agaagaaugg cuuucaauac ccuuuuaagu uuaaaaugug uauuuuucaa 1980

ggcauuuauu gccauauuaa aaucugaugu aacaaggugg ggacgugugu ccuuugguac 2040

uauggugugu uguaucuuug uaagagcaaa agccucagaa agggauugcu uugcauuacu 2100

guccccuuga uauaaaaaau cuuuagggaa ugagaguucc uucucacuua gaaucugaag 2160

ggaauuaaaa agaagaugaa uggucuggca auauucugua acuauugggu gaauauggug 2220

gaaaauaauu uaguggaugg aauaucagaa guauaucugu acagaucaag aaaaaaagga 2280

agaauaaaau uccuauauca u 2301

<210> 1391
<211> 19

<212>  RNA
<213>  Artificial Sequence

<220>
<223>  Description of Artificial Sequence: Synthetic oligonucleotide

<400>  1391
agcagagcgg acgggcgcg                                                    19


<210>  1392
<211>  19
<212>  RNA
<213>  Artificial Sequence

<220>
<223>  Description of Artificial Sequence: Synthetic oligonucleotide

<400>  1392
gcgggaggcg cgcagagcu                                                    19


<210>  1393
<211>  19
<212>  RNA
<213>  Artificial Sequence

<220>
<223>  Description of Artificial Sequence: Synthetic oligonucleotide

<400>  1393
uuucgggcug caggcgcuc                                                    19


<210>  1394
<211>  19
<212>  RNA
<213>  Artificial Sequence

<220>
<223>  Description of Artificial Sequence: Synthetic oligonucleotide

<400>  1394
cgcugccgcu ggggaauug                                                    19


<210>  1395
<211>  19
<212>  RNA
<213>  Artificial Sequence

<220>
<223>  Description of Artificial Sequence: Synthetic oligonucleotide

<400>  1395
gggcuguggg cgaggcggu                                                    19


<210>  1396
<211>  19
<212>  RNA

<213>  Artificial Sequence

<220>
<223>  Description of Artificial Sequence: Synthetic oligonucleotide

<400>  1396
uccgggcugg ccuuuaucg                                                    19


<210>  1397
<211>  19
<212>  RNA
<213>  Artificial Sequence

<220>
<223>  Description of Artificial Sequence: Synthetic oligonucleotide

<400>  1397
gcucgcuggg cccaucguu                                                    19


<210>  1398
<211>  19
<212>  RNA
<213>  Artificial Sequence

<220>
<223>  Description of Artificial Sequence: Synthetic oligonucleotide

<400>  1398
uugaaacuuu aucagcgag                                                    19


<210>  1399
<211>  19
<212>  RNA
<213>  Artificial Sequence

<220>
<223>  Description of Artificial Sequence: Synthetic oligonucleotide

<400>  1399
gucgccacuc gucgcagga                                                    19


<210>  1400
<211>  19
<212>  RNA
<213>  Artificial Sequence

<220>
<223>  Description of Artificial Sequence: Synthetic oligonucleotide

<400>  1400
accgagcggg gggcggggg                                                    19


<210>  1401
<211>  19
<212>  RNA
<213>  Artificial Sequence

<220>
<223>  Description of Artificial Sequence: Synthetic oligonucleotide

<400>  1401
gcgcggcgag gcggcggcc                                                    19


<210>  1402
<211>  19
<212>  RNA
<213>  Artificial Sequence

<220>
<223>  Description of Artificial Sequence: Synthetic oligonucleotide

<400>  1402
cgugacgagg cgcucccgg                                                    19


<210>  1403
<211>  19
<212>  RNA
<213>  Artificial Sequence

<220>
<223>  Description of Artificial Sequence: Synthetic oligonucleotide

<400>  1403
gagcugagcg cuucugcuc                                                    19


<210>  1404
<211>  19
<212>  RNA
<213>  Artificial Sequence

<220>
<223>  Description of Artificial Sequence: Synthetic oligonucleotide

<400>  1404
cugggcacgc auggcgccc                                                    19


<210>  1405
<211>  19
<212>  RNA
<213>  Artificial Sequence

<220>
<223>  Description of Artificial Sequence: Synthetic oligonucleotide

<400>  1405
cgcacacgga gucugaccu                                                    19


<210>  1406
<211>  19
<212>  RNA
<213>  Artificial Sequence

<220>
<223>  Description of Artificial Sequence: Synthetic oligonucleotide

<400>  1406
ugaugcagac gcaaggggg                                                    19


<210>  1407
<211>  19
<212>  RNA
<213>  Artificial Sequence

<220>
<223>  Description of Artificial Sequence: Synthetic oligonucleotide

<400>  1407
guuaauauga acgccccuc                                                    19


<210>  1408
<211>  19
<212>  RNA
<213>  Artificial Sequence

<220>
<223>  Description of Artificial Sequence: Synthetic oligonucleotide

<400>  1408
cucgguggaa ucuggcucu                                                    19


<210>  1409
<211>  19
<212>  RNA
<213>  Artificial Sequence

<220>
<223>  Description of Artificial Sequence: Synthetic oligonucleotide

<400>  1409
uggcucccuc ugcucuuga                                                    19


<210>  1410
<211>  19
<212>  RNA
<213>  Artificial Sequence

<220>
<223>  Description of Artificial Sequence: Synthetic oligonucleotide

<400>  1410
accuggcuca cccccgagg                                                    19


<210>  1411
<211>  19
<212>  RNA
<213>  Artificial Sequence

<220>

<223>  Description of Artificial Sequence: Synthetic oligonucleotide

<400>  1411
gucaacucuu cauggugu                                                    19


<210>  1412
<211>  19
<212>  RNA
<213>  Artificial Sequence

<220>
<223>  Description of Artificial Sequence: Synthetic oligonucleotide

<400>  1412
uacaugagag cuacaggug                                                   19


<210>  1413
<211>  19
<212>  RNA
<213>  Artificial Sequence

<220>
<223>  Description of Artificial Sequence: Synthetic oligonucleotide

<400>  1413
ggcuccucca gggugaugu                                                   19


<210>  1414
<211>  19
<212>  RNA
<213>  Artificial Sequence

<220>
<223>  Description of Artificial Sequence: Synthetic oligonucleotide

<400>  1414
ugcgauaaug ugccaggcc                                                   19


<210>  1415
<211>  19
<212>  RNA
<213>  Artificial Sequence

<220>
<223>  Description of Artificial Sequence: Synthetic oligonucleotide

<400>  1415
cuggugagca gccagcggc                                                   19


<210>  1416
<211>  19
<212>  RNA
<213>  Artificial Sequence

<220>
<223>  Description of Artificial Sequence: Synthetic oligonucleotide

<400>  1416
cagcuguguc accgacauc                                                    19


<210>  1417
<211>  19
<212>  RNA
<213>  Artificial Sequence

<220>
<223>  Description of Artificial Sequence: Synthetic oligonucleotide

<400>  1417
ccagauguga ugcgugcca                                                    19


<210>  1418
<211>  19
<212>  RNA
<213>  Artificial Sequence

<220>
<223>  Description of Artificial Sequence: Synthetic oligonucleotide

<400>  1418
auuagccagg gcguggccg                                                    19


<210>  1419
<211>  19
<212>  RNA
<213>  Artificial Sequence

<220>
<223>  Description of Artificial Sequence: Synthetic oligonucleotide

<400>  1419
gaguggacag cagaaugcc                                                    19


<210>  1420
<211>  19
<212>  RNA
<213>  Artificial Sequence

<220>
<223>  Description of Artificial Sequence: Synthetic oligonucleotide

<400>  1420
cagcaccagu uccgccagc                                                    19


<210>  1421
<211>  19
<212>  RNA
<213>  Artificial Sequence

<220>
<223>  Description of Artificial Sequence: Synthetic oligonucleotide

<400>  1421
caccgcugga auugcaaca                                                    19

<210>  1422
<211>  19
<212>  RNA
<213>  Artificial Sequence

<220>
<223>  Description of Artificial Sequence: Synthetic oligonucleotide

<400>  1422
acccuggaca gggaucaca                                                    19

<210>  1423
<211>  19
<212>  RNA
<213>  Artificial Sequence

<220>
<223>  Description of Artificial Sequence: Synthetic oligonucleotide

<400>  1423
   agccuuuuug gcaggcucc                                                    19

<210>  1424
<211>  19
<212>  RNA
<213>  Artificial Sequence

<220>
<223>  Description of Artificial Sequence: Synthetic oligonucleotide

<400>  1424
cuacuccgaa guagucggg                                                    19

<210>  1425
<211>  19
<212>  RNA
<213>  Artificial Sequence

<220>
<223>  Description of Artificial Sequence: Synthetic oligonucleotide

<400>  1425
gaaucugccu uuguuuaug                                                    19

<210>  1426
<211>  19
<212>  RNA
<213>  Artificial Sequence

<220>
<223>  Description of Artificial Sequence: Synthetic oligonucleotide

<400>  1426

gccaucuccu cagcuggag                                                    19

<210>  1427
<211>  19
<212>  RNA
<213>  Artificial Sequence

<220>
<223>  Description of Artificial Sequence: Synthetic oligonucleotide

<400>  1427
guuguauuug ccaucacca                                                    19

<210>  1428
<211>  19
<212>  RNA
<213>  Artificial Sequence

<220>
<223>  Description of Artificial Sequence: Synthetic oligonucleotide

<400>  1428
agggccugua gccaaggag                                                    19

<210>  1429
<211>  19
<212>  RNA
<213>  Artificial Sequence

<220>
<223>  Description of Artificial Sequence: Synthetic oligonucleotide

<400>  1429
gaaguaaaau ccuguuccu                                                    19

<210>  1430
<211>  19
<212>  RNA
<213>  Artificial Sequence

<220>
<223>  Description of Artificial Sequence: Synthetic oligonucleotide

<400>  1430
ugugauccaa agaagaugg                                                    19

<210>  1431
<211>  19
<212>  RNA
<213>  Artificial Sequence

<220>
<223>  Description of Artificial Sequence: Synthetic oligonucleotide

<400>  1431
ggaagcgcca aggacagca                                                    19

<210>  1432
<211>  19
<212>  RNA
<213>  Artificial Sequence

<220>
<223>  Description of Artificial Sequence: Synthetic oligonucleotide

<400>  1432
aaaggcauuu uugauuggg                                                    19


<210>  1433
<211>  19
<212>  RNA
<213>  Artificial Sequence

<220>
<223>  Description of Artificial Sequence: Synthetic oligonucleotide

<400>  1433
gguggcugca gugauaaca                                                    19


<210>  1434
<211>  19
<212>  RNA
<213>  Artificial Sequence

<220>
<223>  Description of Artificial Sequence: Synthetic oligonucleotide

<400>  1434
auugacuaug ggaucaaau                                                    19


<210>  1435
<211>  19
<212>  RNA
<213>  Artificial Sequence

<220>
<223>  Description of Artificial Sequence: Synthetic oligonucleotide

<400>  1435
uuugcccgcg cauuugugg                                                    19


<210>  1436
<211>  19
<212>  RNA
<213>  Artificial Sequence

<220>
<223>  Description of Artificial Sequence: Synthetic oligonucleotide

<400>  1436
gaugcaaagg aaaggaaag                                                    19

<210>  1437
<211>  19
<212>  RNA
<213>  Artificial Sequence

<220>
<223>  Description of Artificial Sequence: Synthetic oligonucleotide

<400>  1437
ggaaaggaug ccagagccc                                                    19


<210>  1438
<211>  19
<212>  RNA
<213>  Artificial Sequence

<220>
<223>  Description of Artificial Sequence: Synthetic oligonucleotide

<400>  1438
cugaugaauc uucacaaca                                                    19


<210>  1439
<211>  19
<212>  RNA
<213>  Artificial Sequence

<220>
<223>  Description of Artificial Sequence: Synthetic oligonucleotide

<400>  1439
aacagagcug gcaggaagg                                                    19


<210>  1440
<211>  19
<212>  RNA
<213>  Artificial Sequence

<220>
<223>  Description of Artificial Sequence: Synthetic oligonucleotide

<400>  1440
gcuguaaagc gguucuuga                                                    19


<210>  1441
<211>  19
<212>  RNA
<213>  Artificial Sequence

<220>
<223>  Description of Artificial Sequence: Synthetic oligonucleotide

<400>  1441
aaacaagagu gcaagugcc                                                    19

<210>  1442
<211>  19
<212>  RNA
<213>  Artificial Sequence

<220>
<223>  Description of Artificial Sequence: Synthetic oligonucleotide

<400>  1442
cacgggguga gcggcucau                                                    19


<210>  1443
<211>  19
<212>  RNA
<213>  Artificial Sequence

<220>
<223>  Description of Artificial Sequence: Synthetic oligonucleotide

<400>  1443
uguacucuca ggacaugcu                                                    19


<210>  1444
<211>  19
<212>  RNA
<213>  Artificial Sequence

<220>
<223>  Description of Artificial Sequence: Synthetic oligonucleotide

<400>  1444
uggcuggcca uggccgacu                                                    19


<210>  1445
<211>  19
<212>  RNA
<213>  Artificial Sequence

<220>
<223>  Description of Artificial Sequence: Synthetic oligonucleotide

<400>  1445
uucaggaaaa cgggcgauu                                                    19


<210>  1446
<211>  19
<212>  RNA
<213>  Artificial Sequence

<220>
<223>  Description of Artificial Sequence: Synthetic oligonucleotide

<400>  1446
uaucucugga ggaaguaca                                                    19


<210>  1447

<211>  19
<212>  RNA
<213>  Artificial Sequence

<220>
<223>  Description of Artificial Sequence: Synthetic oligonucleotide

<400>  1447
aauggggcca uccaggugg                                                    19


<210>  1448
<211>  19
<212>  RNA
<213>  Artificial Sequence

<220>
<223>  Description of Artificial Sequence: Synthetic oligonucleotide

<400>  1448
gucaugaacc aggauggca                                                    19


<210>  1449
<211>  19
<212>  RNA
<213>  Artificial Sequence

<220>
<223>  Description of Artificial Sequence: Synthetic oligonucleotide

<400>  1449
acagguuuca cuguggcua                                                    19


<210>  1450
<211>  19
<212>  RNA
<213>  Artificial Sequence

<220>
<223>  Description of Artificial Sequence: Synthetic oligonucleotide

<400>  1450
aacgagaggu uuaagaagc                                                    19


<210>  1451
<211>  19
<212>  RNA
<213>  Artificial Sequence

<220>
<223>  Description of Artificial Sequence: Synthetic oligonucleotide

<400>  1451
ccaacgaaaa augaccucg                                                    19


<210>  1452
<211>  19

<212>  RNA
<213>  Artificial Sequence

<220>
<223>  Description of Artificial Sequence: Synthetic oligonucleotide

<400>  1452
guguauuuug agaauucuc                                                    19


<210>  1453
<211>  19
<212>  RNA
<213>  Artificial Sequence

<220>
<223>  Description of Artificial Sequence: Synthetic oligonucleotide

<400>  1453
ccagacuacu guaucaggg                                                    19


<210>  1454
<211>  19
<212>  RNA
<213>  Artificial Sequence

<220>
<223>  Description of Artificial Sequence: Synthetic oligonucleotide

<400>  1454
gaccgagagg caggcuccc                                                    19


<210>  1455
<211>  19
<212>  RNA
<213>  Artificial Sequence

<220>
<223>  Description of Artificial Sequence: Synthetic oligonucleotide

<400>  1455
cuggguacag caggccgug                                                    19


<210>  1456
<211>  19
<212>  RNA
<213>  Artificial Sequence

<220>
<223>  Description of Artificial Sequence: Synthetic oligonucleotide

<400>  1456
gugugcaacc ugacuuccc                                                    19


<210>  1457
<211>  19
<212>  RNA

<213>  Artificial Sequence

<220>
<223>  Description of Artificial Sequence: Synthetic oligonucleotide

<400>  1457
cggggcaugg acagcugug                                                    19


<210>  1458
<211>  19
<212>  RNA
<213>  Artificial Sequence

<220>
<223>  Description of Artificial Sequence: Synthetic oligonucleotide

<400>  1458
gaagucaugu gcuguggga                                                    19


<210>  1459
<211>  19
<212>  RNA
<213>  Artificial Sequence

<220>
<223>  Description of Artificial Sequence: Synthetic oligonucleotide

<400>  1459
agaggcuacg acaccuccc                                                    19


<210>  1460
<211>  19
<212>  RNA
<213>  Artificial Sequence

<220>
<223>  Description of Artificial Sequence: Synthetic oligonucleotide

<400>  1460
caugucaccc ggaugacca                                                    19


<210>  1461
<211>  19
<212>  RNA
<213>  Artificial Sequence

<220>
<223>  Description of Artificial Sequence: Synthetic oligonucleotide

<400>  1461
aagugugggu guaaguucc                                                    19


<210>  1462
<211>  19
<212>  RNA
<213>  Artificial Sequence

<220>
<223>  Description of Artificial Sequence: Synthetic oligonucleotide

<400>  1462
cacuggugcu gcgccgugc                                                    19


<210>  1463
<211>  19
<212>  RNA
<213>  Artificial Sequence

<220>
<223>  Description of Artificial Sequence: Synthetic oligonucleotide

<400>  1463
cgcugucagg acugccugg                                                    19


<210>  1464
<211>  19
<212>  RNA
<213>  Artificial Sequence

<220>
<223>  Description of Artificial Sequence: Synthetic oligonucleotide

<400>  1464
gaagcucugg augugcaca                                                    19


<210>  1465
<211>  19
<212>  RNA
<213>  Artificial Sequence

<220>
<223>  Description of Artificial Sequence: Synthetic oligonucleotide

<400>  1465
acaugcaagg cccccaaga                                                    19


<210>  1466
<211>  19
<212>  RNA
<213>  Artificial Sequence

<220>
<223>  Description of Artificial Sequence: Synthetic oligonucleotide

<400>  1466
aacgcugacu ggacaaccg                                                    19


<210>  1467
<211>  19
<212>  RNA
<213>  Artificial Sequence

<220>
<223>  Description of Artificial Sequence: Synthetic oligonucleotide

<400>  1467
gcuacaugac cccagcagg                                                    19


<210>  1468
<211>  19
<212>  RNA
<213>  Artificial Sequence

<220>
<223>  Description of Artificial Sequence: Synthetic oligonucleotide

<400>  1468
gcgucaccau ccaccuucc                                                    19


<210>  1469
<211>  19
<212>  RNA
<213>  Artificial Sequence

<220>
<223>  Description of Artificial Sequence: Synthetic oligonucleotide

<400>  1469
ccuucuacaa ggacuccau                                                    19


<210>  1470
<211>  19
<212>  RNA
<213>  Artificial Sequence

<220>
<223>  Description of Artificial Sequence: Synthetic oligonucleotide

<400>  1470
uuggaucugc aagaacacu                                                    19


<210>  1471
<211>  19
<212>  RNA
<213>  Artificial Sequence

<220>
<223>  Description of Artificial Sequence: Synthetic oligonucleotide

<400>  1471
uggaccuuug gguucuuuc                                                    19


<210>  1472
<211>  19
<212>  RNA
<213>  Artificial Sequence

<220>

<223>  Description of Artificial Sequence: Synthetic oligonucleotide

<400>  1472
cugggggau auuuccuaa                                                    19


<210>  1473
<211>  19
<212>  RNA
<213>  Artificial Sequence

<220>
<223>  Description of Artificial Sequence: Synthetic oligonucleotide

<400>  1473
aggcaugugg ccuuuaucu                                                   19


<210>  1474
<211>  19
<212>  RNA
<213>  Artificial Sequence

<220>
<223>  Description of Artificial Sequence: Synthetic oligonucleotide

<400>  1474
ucaacggaag cccccucuu                                                   19


<210>  1475
<211>  19
<212>  RNA
<213>  Artificial Sequence

<220>
<223>  Description of Artificial Sequence: Synthetic oligonucleotide

<400>  1475
uccucccugg gggccccag                                                   19


<210>  1476
<211>  19
<212>  RNA
<213>  Artificial Sequence

<220>
<223>  Description of Artificial Sequence: Synthetic oligonucleotide

<400>  1476
ggaugggggg ccacacgcu                                                   19


<210>  1477
<211>  19
<212>  RNA
<213>  Artificial Sequence

<220>
<223>  Description of Artificial Sequence: Synthetic oligonucleotide

<400>  1477
ugcaccuaaa gccuacccu                                                    19

<210>  1478
<211>  19
<212>  RNA
<213>  Artificial Sequence

<220>
<223>  Description of Artificial Sequence: Synthetic oligonucleotide

<400>  1478
uauucuaucc aucuccugg                                                    19

<210>  1479
<211>  19
<212>  RNA
<213>  Artificial Sequence

<220>
<223>  Description of Artificial Sequence: Synthetic oligonucleotide

<400>  1479
guguucugca gucaucucc                                                    19

<210>  1480
<211>  19
<212>  RNA
<213>  Artificial Sequence

<220>
<223>  Description of Artificial Sequence: Synthetic oligonucleotide

<400>  1480
cccuccuggc gaguucucu                                                    19

<210>  1481
<211>  19
<212>  RNA
<213>  Artificial Sequence

<220>
<223>  Description of Artificial Sequence: Synthetic oligonucleotide

<400>  1481
uuuggaaaua gcaugacag                                                    19

<210>  1482
<211>  19
<212>  RNA
<213>  Artificial Sequence

<220>
<223>  Description of Artificial Sequence: Synthetic oligonucleotide

<400>  1482
ggcuguucag ccgggaggg                                                    19

<210>  1483
<211>  19
<212>  RNA
<213>  Artificial Sequence

<220>
<223>  Description of Artificial Sequence: Synthetic oligonucleotide

<400>  1483
guggugggcc cagaccacu                                                    19

<210>  1484
<211>  19
<212>  RNA
<213>  Artificial Sequence

<220>
<223>  Description of Artificial Sequence: Synthetic oligonucleotide

<400>  1484
ugucuccacc caccuugac                                                    19

<210>  1485
<211>  19
<212>  RNA
<213>  Artificial Sequence

<220>
<223>  Description of Artificial Sequence: Synthetic oligonucleotide

<400>  1485
cguuucuucu uucuagagc                                                    19

<210>  1486
<211>  19
<212>  RNA
<213>  Artificial Sequence

<220>
<223>  Description of Artificial Sequence: Synthetic oligonucleotide

<400>  1486
caguuggcca agcagaaaa                                                    19

<210>  1487
<211>  19
<212>  RNA
<213>  Artificial Sequence

<220>
<223>  Description of Artificial Sequence: Synthetic oligonucleotide

<400>  1487

aaaaaguguc ucaaaggag                                                    19

<210>  1488
<211>  19
<212>  RNA
<213>  Artificial Sequence

<220>
<223>  Description of Artificial Sequence: Synthetic oligonucleotide

<400>  1488
gcuuucucaa ugucuuccc                                                    19

<210>  1489
<211>  19
<212>  RNA
<213>  Artificial Sequence

<220>
<223>  Description of Artificial Sequence: Synthetic oligonucleotide

<400>  1489
cacaaauggu cccaauuaa                                                    19

<210>  1490
<211>  19
<212>  RNA
<213>  Artificial Sequence

<220>
<223>  Description of Artificial Sequence: Synthetic oligonucleotide

<400>  1490
agaaauucca uacuucucu                                                    19

<210>  1491
<211>  19
<212>  RNA
<213>  Artificial Sequence

<220>
<223>  Description of Artificial Sequence: Synthetic oligonucleotide

<400>  1491
ucagauggaa caguaaaga                                                    19

<210>  1492
<211>  19
<212>  RNA
<213>  Artificial Sequence

<220>
<223>  Description of Artificial Sequence: Synthetic oligonucleotide

<400>  1492
aaagcagaau caacugccc                                                    19

<210>  1493
<211>  19
<212>  RNA
<213>  Artificial Sequence

<220>
<223>  Description of Artificial Sequence: Synthetic oligonucleotide

<400>  1493
ccugacuuaa cuuuaacuu                                                    19


<210>  1494
<211>  19
<212>  RNA
<213>  Artificial Sequence

<220>
<223>  Description of Artificial Sequence: Synthetic oligonucleotide

<400>  1494
uuugaaaaga ccaagacuu                                                    19


<210>  1495
<211>  19
<212>  RNA
<213>  Artificial Sequence

<220>
<223>  Description of Artificial Sequence: Synthetic oligonucleotide

<400>  1495
uuugucugua caagugguu                                                    19


<210>  1496
<211>  19
<212>  RNA
<213>  Artificial Sequence

<220>
<223>  Description of Artificial Sequence: Synthetic oligonucleotide

<400>  1496
uuuacagcua ccacccuua                                                    19


<210>  1497
<211>  19
<212>  RNA
<213>  Artificial Sequence

<220>
<223>  Description of Artificial Sequence: Synthetic oligonucleotide

<400>  1497
aggguaauug guaauuacc                                                    19

<210>  1498
<211>  19
<212>  RNA
<213>  Artificial Sequence

<220>
<223>  Description of Artificial Sequence: Synthetic oligonucleotide

<400>  1498
cuggagaaga auggcuuuc                                                    19


<210>  1499
<211>  19
<212>  RNA
<213>  Artificial Sequence

<220>
<223>  Description of Artificial Sequence: Synthetic oligonucleotide

<400>  1499
caauacccuu uuaaguuua                                                    19


<210>  1500
<211>  19
<212>  RNA
<213>  Artificial Sequence

<220>
<223>  Description of Artificial Sequence: Synthetic oligonucleotide

<400>  1500
aaaaugugua uuuuucaag                                                    19


<210>  1501
<211>  19
<212>  RNA
<213>  Artificial Sequence

<220>
<223>  Description of Artificial Sequence: Synthetic oligonucleotide

<400>  1501
ggcauuuauu gccauauua                                                    19


<210>  1502
<211>  19
<212>  RNA
<213>  Artificial Sequence

<220>
<223>  Description of Artificial Sequence: Synthetic oligonucleotide

<400>  1502
aaaaucugau guaacaagg                                                    19

<210>  1503
<211>  19
<212>  RNA
<213>  Artificial Sequence

<220>
<223>  Description of Artificial Sequence: Synthetic oligonucleotide

<400>  1503
guggggacgu guguccuuu                                                    19


<210>  1504
<211>  19
<212>  RNA
<213>  Artificial Sequence

<220>
<223>  Description of Artificial Sequence: Synthetic oligonucleotide

<400>  1504
ugguacuaug guguguugu                                                    19


<210>  1505
<211>  19
<212>  RNA
<213>  Artificial Sequence

<220>
<223>  Description of Artificial Sequence: Synthetic oligonucleotide

<400>  1505
uaucuuugua agagcaaaa                                                    19


<210>  1506
<211>  19
<212>  RNA
<213>  Artificial Sequence

<220>
<223>  Description of Artificial Sequence: Synthetic oligonucleotide

<400>  1506
agccucagaa agggauugc                                                    19


<210>  1507
<211>  19
<212>  RNA
<213>  Artificial Sequence

<220>
<223>  Description of Artificial Sequence: Synthetic oligonucleotide

<400>  1507
cuuugcauua cuguccccu                                                    19


<210>  1508

<211>  19
<212>  RNA
<213>  Artificial Sequence

<220>
<223>  Description of Artificial Sequence: Synthetic oligonucleotide

<400>  1508
uugauauaaa aaaucuuua                                                    19


<210>  1509
<211>  19
<212>  RNA
<213>  Artificial Sequence

<220>
<223>  Description of Artificial Sequence: Synthetic oligonucleotide

<400>  1509
agggaaugag aguuccuuc                                                    19


<210>  1510
<211>  19
<212>  RNA
<213>  Artificial Sequence

<220>
<223>  Description of Artificial Sequence: Synthetic oligonucleotide

<400>  1510
cucacuuaga aucugaagg                                                    19


<210>  1511
<211>  19
<212>  RNA
<213>  Artificial Sequence

<220>
<223>  Description of Artificial Sequence: Synthetic oligonucleotide

<400>  1511
ggaauuaaaa agaagauga                                                    19


<210>  1512
<211>  19
<212>  RNA
<213>  Artificial Sequence

<220>
<223>  Description of Artificial Sequence: Synthetic oligonucleotide

<400>  1512
aauggucugg caauauucu                                                    19


<210>  1513
<211>  19

<212>  RNA
<213>  Artificial Sequence

<220>
<223>  Description of Artificial Sequence: Synthetic oligonucleotide

<400>  1513
uguaacuauu gggugaaua                                                    19


<210>  1514
<211>  19
<212>  RNA
<213>  Artificial Sequence

<220>
<223>  Description of Artificial Sequence: Synthetic oligonucleotide

<400>  1514
augguggaaa auaauuuag                                                    19


<210>  1515
<211>  19
<212>  RNA
<213>  Artificial Sequence

<220>
<223>  Description of Artificial Sequence: Synthetic oligonucleotide

<400>  1515
guggauggaa uaucagaag                                                    19


<210>  1516
<211>  19
<212>  RNA
<213>  Artificial Sequence

<220>
<223>  Description of Artificial Sequence: Synthetic oligonucleotide

<400>  1516
guauaucugu acagaucaa                                                    19


<210>  1517
<211>  19
<212>  RNA
<213>  Artificial Sequence

<220>
<223>  Description of Artificial Sequence: Synthetic oligonucleotide

<400>  1517
agaaaaaaag gaagaauaa                                                    19


<210>  1518
<211>  19
<212>  RNA

<213>  Artificial Sequence

<220>
<223>  Description of Artificial Sequence: Synthetic oligonucleotide

<400>  1518
aauaaaauuc cuauaucau                                                    19


<210>  1519
<211>  25
<212>  RNA
<213>  Artificial Sequence

<220>
<223>  Description of Artificial Sequence: Synthetic oligonucleotide

<400>  1519
agcagagcgg acgggcgcgc gggag                                             25


<210>  1520
<211>  25
<212>  RNA
<213>  Artificial Sequence

<220>
<223>  Description of Artificial Sequence: Synthetic oligonucleotide

<400>  1520
ggcgcgcaga gcuuucgggc ugcag                                             25


<210>  1521
<211>  25
<212>  RNA
<213>  Artificial Sequence

<220>
<223>  Description of Artificial Sequence: Synthetic oligonucleotide

<400>  1521
ggcgcucgcu gccgcugggg aauug                                             25


<210>  1522
<211>  25
<212>  RNA
<213>  Artificial Sequence

<220>
<223>  Description of Artificial Sequence: Synthetic oligonucleotide

<400>  1522
gggcuguggg cgaggcgguc cgggc                                             25


<210>  1523
<211>  25
<212>  RNA
<213>  Artificial Sequence

<220>
<223>  Description of Artificial Sequence: Synthetic oligonucleotide

<400>  1523
cuggccuuua ucgcucgcug ggccc                                          25


<210>  1524
<211>  25
<212>  RNA
<213>  Artificial Sequence

<220>
<223>  Description of Artificial Sequence: Synthetic oligonucleotide

<400>  1524
caucguuuga aacuuuauca gcgag                                          25


<210>  1525
<211>  25
<212>  RNA
<213>  Artificial Sequence

<220>
<223>  Description of Artificial Sequence: Synthetic oligonucleotide

<400>  1525
gucgccacuc gucgcaggac cgagc                                          25


<210>  1526
<211>  25
<212>  RNA
<213>  Artificial Sequence

<220>
<223>  Description of Artificial Sequence: Synthetic oligonucleotide

<400>  1526
cgggggcgg gggcgcggcg aggcg                                           25


<210>  1527
<211>  25
<212>  RNA
<213>  Artificial Sequence

<220>
<223>  Description of Artificial Sequence: Synthetic oligonucleotide

<400>  1527
ggcggccgug acgaggcgcu cccgg                                          25


<210>  1528
<211>  25
<212>  RNA
<213>  Artificial Sequence

<220>
<223>  Description of Artificial Sequence: Synthetic oligonucleotide

<400>  1528
gagcugagcg cuucugcucu gggca                                          25


<210>  1529
<211>  25
<212>  RNA
<213>  Artificial Sequence

<220>
<223>  Description of Artificial Sequence: Synthetic oligonucleotide

<400>  1529
acgcauggcg cccgcacacg gaguc                                          25


<210>  1530
<211>  25
<212>  RNA
<213>  Artificial Sequence

<220>
<223>  Description of Artificial Sequence: Synthetic oligonucleotide

<400>  1530
cugaccugau gcagacgcaa ggggg                                          25


<210>  1531
<211>  25
<212>  RNA
<213>  Artificial Sequence

<220>
<223>  Description of Artificial Sequence: Synthetic oligonucleotide

<400>  1531
guuaauauga acgccccucu cggug                                          25


<210>  1532
<211>  25
<212>  RNA
<213>  Artificial Sequence

<220>
<223>  Description of Artificial Sequence: Synthetic oligonucleotide

<400>  1532
ggaaucuggc ucuggcuccc ucugc                                          25


<210>  1533
<211>  25
<212>  RNA
<213>  Artificial Sequence

<220>

<223>  Description of Artificial Sequence: Synthetic oligonucleotide

<400>  1533
cucuugaccu ggcucacccc cgagg                                          25


<210>  1534
<211>  25
<212>  RNA
<213>  Artificial Sequence

<220>
<223>  Description of Artificial Sequence: Synthetic oligonucleotide

<400>  1534
   gucaacucuu caugguggua cauga                                          25


<210>  1535
<211>  25
<212>  RNA
<213>  Artificial Sequence

<220>
<223>  Description of Artificial Sequence: Synthetic oligonucleotide

<400>  1535
agagcuacag guggcuccuc caggg                                          25


<210>  1536
<211>  25
<212>  RNA
<213>  Artificial Sequence

<220>
<223>  Description of Artificial Sequence: Synthetic oligonucleotide

<400>  1536
gugaugugcg auaaugugcc aggcc                                          25


<210>  1537
<211>  25
<212>  RNA
<213>  Artificial Sequence

<220>
<223>  Description of Artificial Sequence: Synthetic oligonucleotide

<400>  1537
cuggugagca gccagcggca gcugu                                          25


<210>  1538
<211>  25
<212>  RNA
<213>  Artificial Sequence

<220>
<223>  Description of Artificial Sequence: Synthetic oligonucleotide

<400>  1538
ugucaccgac auccagaugu gaugc                                          25


<210>  1539
<211>  25
<212>  RNA
<213>  Artificial Sequence

<220>
<223>  Description of Artificial Sequence: Synthetic oligonucleotide

<400>  1539
cgugccauua gccagggcgu ggccg                                          25


<210>  1540
<211>  25
<212>  RNA
<213>  Artificial Sequence

<220>
<223>  Description of Artificial Sequence: Synthetic oligonucleotide

<400>  1540
gaguggacag cagaaugcca gcacc                                          25


<210>  1541
<211>  25
<212>  RNA
<213>  Artificial Sequence

<220>
<223>  Description of Artificial Sequence: Synthetic oligonucleotide

<400>  1541
caguuccgcc agcaccgcug gaauu                                          25


<210>  1542
<211>  25
<212>  RNA
<213>  Artificial Sequence

<220>
<223>  Description of Artificial Sequence: Synthetic oligonucleotide

<400>  1542
ugcaacaccc uggacaggga ucaca                                          25


<210>  1543
<211>  25
<212>  RNA
<213>  Artificial Sequence

<220>
<223>  Description of Artificial Sequence: Synthetic oligonucleotide

<400>  1543
agccuuuuug gcaggguccu acucc                                           25


<210>  1544
<211>  25
<212>  RNA
<213>  Artificial Sequence

<220>
<223>  Description of Artificial Sequence: Synthetic oligonucleotide

<400>  1544
cgaaguaguc gggaaucugc cuuug                                           25


<210>  1545
<211>  25
<212>  RNA
<213>  Artificial Sequence

<220>
<223>  Description of Artificial Sequence: Synthetic oligonucleotide

<400>  1545
guuuaugcca ucuccucagc uggag                                           25


<210>  1546
<211>  25
<212>  RNA
<213>  Artificial Sequence

<220>
<223>  Description of Artificial Sequence: Synthetic oligonucleotide

<400>  1546
guuguauuug ccaucaccag ggccu                                           25


<210>  1547
<211>  25
<212>  RNA
<213>  Artificial Sequence

<220>
<223>  Description of Artificial Sequence: Synthetic oligonucleotide

<400>  1547
uguagccaag gagaaguaaa auccu                                           25


<210>  1548
<211>  25
<212>  RNA
<213>  Artificial Sequence

<220>
<223>  Description of Artificial Sequence: Synthetic oligonucleotide

<400>  1548

uguuccugug auccaaagaa gaugg                                              25

<210>  1549
<211>  25
<212>  RNA
<213>  Artificial Sequence

<220>
<223>  Description of Artificial Sequence: Synthetic oligonucleotide

<400>  1549
ggaagcgcca aggacagcaa aggca                                              25

<210>  1550
<211>  25
<212>  RNA
<213>  Artificial Sequence

<220>
<223>  Description of Artificial Sequence: Synthetic oligonucleotide

<400>  1550
   auuuuugauu gggguggcug cagug                                              25

<210>  1551
<211>  25
<212>  RNA
<213>  Artificial Sequence

<220>
<223>  Description of Artificial Sequence: Synthetic oligonucleotide

<400>  1551
gauaacauug acuaugggau caaau                                              25

<210>  1552
<211>  25
<212>  RNA
<213>  Artificial Sequence

<220>
<223>  Description of Artificial Sequence: Synthetic oligonucleotide

<400>  1552
uuugcccgcg cauuugugga ugcaa                                              25

<210>  1553
<211>  25
<212>  RNA
<213>  Artificial Sequence

<220>
<223>  Description of Artificial Sequence: Synthetic oligonucleotide

<400>  1553
aaggaaagga aaggaaagga ugcca                                              25

<210>  1554
<211>  25
<212>  RNA
<213>  Artificial Sequence

<220>
<223>  Description of Artificial Sequence: Synthetic oligonucleotide

<400>  1554
agagcccuga ugaaucuuca caaca                                          25


<210>  1555
<211>  25
<212>  RNA
<213>  Artificial Sequence

<220>
<223>  Description of Artificial Sequence: Synthetic oligonucleotide

<400>  1555
aacagagcug gcaggaaggc uguaa                                          25


<210>  1556
<211>  25
<212>  RNA
<213>  Artificial Sequence

<220>
<223>  Description of Artificial Sequence: Synthetic oligonucleotide

<400>  1556
aagcgguucu ugaaacaaga gugca                                          25


<210>  1557
<211>  25
<212>  RNA
<213>  Artificial Sequence

<220>
<223>  Description of Artificial Sequence: Synthetic oligonucleotide

<400>  1557
aagugccacg ggugagcgg cucau                                           25


<210>  1558
<211>  25
<212>  RNA
<213>  Artificial Sequence

<220>
<223>  Description of Artificial Sequence: Synthetic oligonucleotide

<400>  1558
uguacucuca ggacaugcug gcugg                                          25

<210>  1559
<211>  25
<212>  RNA
<213>  Artificial Sequence

<220>
<223>  Description of Artificial Sequence: Synthetic oligonucleotide

<400>  1559
gccauggccg acuucaggaa aacgg                                          25


<210>  1560
<211>  25
<212>  RNA
<213>  Artificial Sequence

<220>
<223>  Description of Artificial Sequence: Synthetic oligonucleotide

<400>  1560
ggcgauuauc ucuggaggaa guaca                                          25


<210>  1561
<211>  25
<212>  RNA
<213>  Artificial Sequence

<220>
<223>  Description of Artificial Sequence: Synthetic oligonucleotide

<400>  1561
   aauggggcca uccagguggu cauga                                          25

<210>  1562
<211>  25
<212>  RNA
<213>  Artificial Sequence

<220>
<223>  Description of Artificial Sequence: Synthetic oligonucleotide

<400>  1562
aaccaggaug gcacagguuu cacug                                          25


<210>  1563
<211>  25
<212>  RNA
<213>  Artificial Sequence

<220>
<223>  Description of Artificial Sequence: Synthetic oligonucleotide

<400>  1563
guggcuaacg agagguuuaa gaagc                                          25

<210>  1564
<211>  25
<212>  RNA
<213>  Artificial Sequence

<220>
<223>  Description of Artificial Sequence: Synthetic oligonucleotide

<400>  1564
ccaacgaaaa augaccucgu guauu                                          25


<210>  1565
<211>  25
<212>  RNA
<213>  Artificial Sequence

<220>
<223>  Description of Artificial Sequence: Synthetic oligonucleotide

<400>  1565
uuugagaauu cuccagacua cugua                                          25


<210>  1566
<211>  25
<212>  RNA
<213>  Artificial Sequence

<220>
<223>  Description of Artificial Sequence: Synthetic oligonucleotide

<400>  1566
aucagggacc gagaggcagg cuccc                                          25


<210>  1567
<211>  25
<212>  RNA
<213>  Artificial Sequence

<220>
<223>  Description of Artificial Sequence: Synthetic oligonucleotide

<400>  1567
cuggguacag caggccgugu gugca                                          25


<210>  1568
<211>  25
<212>  RNA
<213>  Artificial Sequence

<220>
<223>  Description of Artificial Sequence: Synthetic oligonucleotide

<400>  1568
aaccugacuu cccggggcau ggaca                                          25


<210>  1569

<211>  25
<212>  RNA
<213>  Artificial Sequence

<220>
<223>  Description of Artificial Sequence: Synthetic oligonucleotide

<400>  1569
agcugugaag ucaugugcug uggga                                          25


<210>  1570
<211>  25
<212>  RNA
<213>  Artificial Sequence

<220>
<223>  Description of Artificial Sequence: Synthetic oligonucleotide

<400>  1570
agaggcuacg acaccuccca uguca                                          25


<210>  1571
<211>  25
<212>  RNA
<213>  Artificial Sequence

<220>
<223>  Description of Artificial Sequence: Synthetic oligonucleotide

<400>  1571
acccggauga ccaagugugg gugua                                          25


<210>  1572
<211>  25
<212>  RNA
<213>  Artificial Sequence

<220>
<223>  Description of Artificial Sequence: Synthetic oligonucleotide

<400>  1572
aaguuccacu ggugcugcgc cgugc                                          25


<210>  1573
<211>  25
<212>  RNA
<213>  Artificial Sequence

<220>
<223>  Description of Artificial Sequence: Synthetic oligonucleotide

<400>  1573
cgcugucagg acugccugga agcuc                                          25


<210>  1574
<211>  25

<212>  RNA
<213>  Artificial Sequence

<220>
<223>  Description of Artificial Sequence: Synthetic oligonucleotide

<400>  1574
cuggaugugc acacaugcaa ggccc                                          25


<210>  1575
<211>  25
<212>  RNA
<213>  Artificial Sequence

<220>
<223>  Description of Artificial Sequence: Synthetic oligonucleotide

<400>  1575
cccaagaacg cugacuggac aaccg                                          25


<210>  1576
<211>  25
<212>  RNA
<213>  Artificial Sequence

<220>
<223>  Description of Artificial Sequence: Synthetic oligonucleotide

<400>  1576
gcuacaugac cccagcaggc gucac                                          25


<210>  1577
<211>  25
<212>  RNA
<213>  Artificial Sequence

<220>
<223>  Description of Artificial Sequence: Synthetic oligonucleotide

<400>  1577
ccauccaccu ucccuucuac aagga                                          25


<210>  1578
<211>  25
<212>  RNA
<213>  Artificial Sequence

<220>
<223>  Description of Artificial Sequence: Synthetic oligonucleotide

<400>  1578
acuccauugg aucugcaaga acacu                                          25


<210>  1579
<211>  25
<212>  RNA

<213>  Artificial Sequence

<220>
<223>  Description of Artificial Sequence: Synthetic oligonucleotide

<400>  1579
uggaccuuug gguucuuucu ggggg                                          25


<210>  1580
<211>  25
<212>  RNA
<213>  Artificial Sequence

<220>
<223>  Description of Artificial Sequence: Synthetic oligonucleotide

<400>  1580
ggauauuucc uaaggcaugu ggccu                                          25


<210>  1581
<211>  25
<212>  RNA
<213>  Artificial Sequence

<220>
<223>  Description of Artificial Sequence: Synthetic oligonucleotide

<400>  1581
uuuaucucaa cggaagcccc cucuu                                          25


<210>  1582
<211>  25
<212>  RNA
<213>  Artificial Sequence

<220>
<223>  Description of Artificial Sequence: Synthetic oligonucleotide

<400>  1582
uccucccugg gggccccagg auggg                                          25


<210>  1583
<211>  25
<212>  RNA
<213>  Artificial Sequence

<220>
<223>  Description of Artificial Sequence: Synthetic oligonucleotide

<400>  1583
ggggccacac gcugcaccua aagcc                                          25


<210>  1584
<211>  25
<212>  RNA
<213>  Artificial Sequence

<220>
<223>  Description of Artificial Sequence: Synthetic oligonucleotide

<400>  1584
cuacccuauu cuauccaucu ccugg                                             25


<210>  1585
<211>  25
<212>  RNA
<213>  Artificial Sequence

<220>
<223>  Description of Artificial Sequence: Synthetic oligonucleotide

<400>  1585
guguucugca gucaucuccc cuccu                                             25


<210>  1586
<211>  25
<212>  RNA
<213>  Artificial Sequence

<220>
<223>  Description of Artificial Sequence: Synthetic oligonucleotide

<400>  1586
uggcgaguuc ucuuuggaaa uagca                                             25


<210>  1587
<211>  25
<212>  RNA
<213>  Artificial Sequence

<220>
<223>  Description of Artificial Sequence: Synthetic oligonucleotide

<400>  1587
augacaggcu guucagccgg gaggg                                             25


<210>  1588
<211>  25
<212>  RNA
<213>  Artificial Sequence

<220>
<223>  Description of Artificial Sequence: Synthetic oligonucleotide

<400>  1588
guggugggcc cagaccacug ucucc                                             25


<210>  1589
<211>  25
<212>  RNA
<213>  Artificial Sequence

<220>
<223>  Description of Artificial Sequence: Synthetic oligonucleotide

<400>  1589
cacccaccuu gacguuucuu cuuuc                                          25


<210>  1590
<211>  25
<212>  RNA
<213>  Artificial Sequence

<220>
<223>  Description of Artificial Sequence: Synthetic oligonucleotide

<400>  1590
cuagagcagu uggccaagca gaaaa                                          25


<210>  1591
<211>  25
<212>  RNA
<213>  Artificial Sequence

<220>
<223>  Description of Artificial Sequence: Synthetic oligonucleotide

<400>  1591
aaaaaguguc ucaaaggagc uuucu                                          25


<210>  1592
<211>  25
<212>  RNA
<213>  Artificial Sequence

<220>
<223>  Description of Artificial Sequence: Synthetic oligonucleotide

<400>  1592
ucaaugucuu cccacaaaug guccc                                          25


<210>  1593
<211>  25
<212>  RNA
<213>  Artificial Sequence

<220>
<223>  Description of Artificial Sequence: Synthetic oligonucleotide

<400>  1593
caauuaagaa auuccauacu ucucu                                          25


<210>  1594
<211>  25
<212>  RNA
<213>  Artificial Sequence

<220>

<223>  Description of Artificial Sequence: Synthetic oligonucleotide

<400>  1594
ucagauggaa caguaaagaa agcag                                          25


<210>  1595
<211>  25
<212>  RNA
<213>  Artificial Sequence

<220>
<223>  Description of Artificial Sequence: Synthetic oligonucleotide

<400>  1595
gaaucaacug ccccugacuu aacuu                                          25


<210>  1596
<211>  25
<212>  RNA
<213>  Artificial Sequence

<220>
<223>  Description of Artificial Sequence: Synthetic oligonucleotide

<400>  1596
uuaacuuuug aaaagaccaa gacuu                                          25


<210>  1597
<211>  25
<212>  RNA
<213>  Artificial Sequence

<220>
<223>  Description of Artificial Sequence: Synthetic oligonucleotide

<400>  1597
uuugucugua caagugguuu uacag                                          25


<210>  1598
<211>  25
<212>  RNA
<213>  Artificial Sequence

<220>
<223>  Description of Artificial Sequence: Synthetic oligonucleotide

<400>  1598
   gcuaccaccc uuagguaaau uggua                                          25


<210>  1599
<211>  25
<212>  RNA
<213>  Artificial Sequence

<220>
<223>  Description of Artificial Sequence: Synthetic oligonucleotide

<400> 1599
aauuaccugg agaagaaugg cuuuc 25

<210> 1600
<211> 25
<212> RNA
<213> Artificial Sequence

<220>
<223> Description of Artificial Sequence: Synthetic oligonucleotide

<400> 1600
caauacccuu uuaaguuuaa aaugu 25

<210> 1601
<211> 25
<212> RNA
<213> Artificial Sequence

<220>
<223> Description of Artificial Sequence: Synthetic oligonucleotide

<400> 1601
uguauuuuuc aaggcauuua uugcc 25

<210> 1602
<211> 25
<212> RNA
<213> Artificial Sequence

<220>
<223> Description of Artificial Sequence: Synthetic oligonucleotide

<400> 1602
cauauuaaaa ucugauguaa caagg 25

<210> 1603
<211> 25
<212> RNA
<213> Artificial Sequence

<220>
<223> Description of Artificial Sequence: Synthetic oligonucleotide

<400> 1603
guggggacgu guguccuuug guacu 25

<210> 1604
<211> 25
<212> RNA
<213> Artificial Sequence

<220>
<223> Description of Artificial Sequence: Synthetic oligonucleotide

<400>  1604
uauggugugu uguaucuuug uaaga                                            25


<210>  1605
<211>  25
<212>  RNA
<213>  Artificial Sequence

<220>
<223>  Description of Artificial Sequence: Synthetic oligonucleotide

<400>  1605
agcaaaagcc ucagaaaggg auugc                                            25


<210>  1606
<211>  25
<212>  RNA
<213>  Artificial Sequence

<220>
<223>  Description of Artificial Sequence: Synthetic oligonucleotide

<400>  1606
cuuugcauua cuguccccuu gauau                                            25


<210>  1607
<211>  25
<212>  RNA
<213>  Artificial Sequence

<220>
<223>  Description of Artificial Sequence: Synthetic oligonucleotide

<400>  1607
uaaaaaaucu uuagggaaug agagu                                            25


<210>  1608
<211>  25
<212>  RNA
<213>  Artificial Sequence

<220>
<223>  Description of Artificial Sequence: Synthetic oligonucleotide

<400>  1608
uuccuucuca cuuagaaucu gaagg                                            25


<210>  1609
<211>  25
<212>  RNA
<213>  Artificial Sequence

<220>
<223>  Description of Artificial Sequence: Synthetic oligonucleotide

<400>  1609

ggaauuaaaa agaagaugaa ugguc                                          25

<210>  1610
<211>  25
<212>  RNA
<213>  Artificial Sequence

<220>
<223>  Description of Artificial Sequence: Synthetic oligonucleotide

<400>  1610
cuggcaauau ucuguaacua uuggg                                          25

<210>  1611
<211>  25
<212>  RNA
<213>  Artificial Sequence

<220>
<223>  Description of Artificial Sequence: Synthetic oligonucleotide

<400>  1611
gugaauaugg uggaaaauaa uuuag                                          25

<210>  1612
<211>  25
<212>  RNA
<213>  Artificial Sequence

<220>
<223>  Description of Artificial Sequence: Synthetic oligonucleotide

<400>  1612
guggauggaa uaucagaagu auauc                                          25

<210>  1613
<211>  25
<212>  RNA
<213>  Artificial Sequence

<220>
<223>  Description of Artificial Sequence: Synthetic oligonucleotide

<400>  1613
cuguacagau caagaaaaaa aggaa                                          25

<210>  1614
<211>  25
<212>  RNA
<213>  Artificial Sequence

<220>
<223>  Description of Artificial Sequence: Synthetic oligonucleotide

<400>  1614
aggaagaaua aaauuccuau aucau                                          25

<210> 1615
<211> 2932
<212> RNA
<213> Homo sapiens

<400> 1615
agcucccagg gcccggcccc ccccggcgcu cacgcucucg gggcggacuc ccggcccucc 60

gcgcccucuc gcgcggcgau ggccccacuc ggauacuucu uacuccucug cagccugaag 120

caggcucugg gcagcuaccc gaucuggugg ucgcuggcug uugggccaca guauuccucc 180

cugggcucgc agcccauccu gugugccagc aucccgggcc ugguccccaa gcagcuccgc 240

uucugcagga acuacgugga gaucaugccc agcguggccg agggcaucaa gauuggcauc 300

caggagugcc agcaccaguu ccgcggccgc cgguggaacu gcaccaccgu ccacgacagc 360

cuggccaucu ucgggcccgu gcuggacaaa gcuaccaggg agucggccuu uguccacgcc 420

auugccucag ccgguguggc cuuugcagug acacgcucau gugcagaagg cacggccgcc 480

aucuguggcu gcagcagccg ccaccagggc ucaccaggca agggcuggaa gugggguggc 540

uguagcgagg acaucgaguu uggugggaug gugucucggg aguucgccga cgcccgggag 600

aaccggccag augcccgcuc agccaugaac cgccacaaca acgaggcugg gcgccaggcc 660

aucgccagcc acaugcaccu caagugcaag ugccacgggc ugucgggcag cugcgaggug 720

aagacaugcu gguggucgca acccgacuuc cgcgccaucg gugacuuccu caaggacaag 780

uacgacagcg ccucggagau ggugguggag aagcaccggg agucccgcgg cuggguggag 840

acccugcggc cgcgcuacac cuacuucaag gugcccacgg agcgcgaccu ggucuacuac 900

gaggccucgc ccaacuucug cgagcccaac ccugagacgg gcuccuucgg cacgcgcgac 960

cgcaccugca acgucagcuc gcacggcauc gacggcugcg accugcugug cugcggccgc 1020

ggccacaacg cgcgagcgga gcggcgccgg gagaagugcc gcugcguguu ccacuggugc 1080

ugcuacguca gcugccagga gugcacgcgc gucuacgacg ugcacaccug caaguaggca 1140

ccggccgcgg cucccccugg acggggcggg cccugccuga gggugggcuu uucccugggu 1200

ggagcaggac ucccaccuaa acggggcagu acuccucccu gggggcggga cuccucccug 1260

gggguggggc uccuaccugg gggcagaacu ccuaccugaa ggcagggcuc cucccuggag 1320

cuagugucuc cucucuggug gcugggcugc uccugaauga ggcggagcuc caggaugggg 1380

aggggcucug cguuggcuuc ucccugggga cggggcuccc cuggacagag gcggggcuac 1440

agauugggcg gggcuucucu ugggugggac agggcuucuc cugcgggggc gaggccccuc 1500

ccaguaaggg cguggcucug ggugggcggg gcacuaggua ggcuucuacc ugcaggcggg 1560

gcuccuccug aaggaggcgg ggcucuagga uggggcacgg cucuggggua ggcugcuccc 1620

ugagggcgga gcgccuccuu aggagugggg uuuuauggug gaugaggcuu cuuccuggau 1680

ggggcagagc uucuccugac cagggcaagg ccccuuccac gggggcugug gcucugggug 1740

ggcguggccu gcauaggcuc cuuccugugg guggggcuuc ucugggacca ggcuccaaug 1800

gggcggggcu ucucuccgcg ggugggacuc uucccuggga accgcccucc ugauuaaggc 1860

guggcuucug caggaauccc ggcuccagag caggaaauuc agcccaccag ccaccucauc 1920

cccaaccccc uguaagguuc cauccacccc ugcgucgagc ugggaagguu ccaugaagcg 1980

agucgggucc ccaacccgug ccccugggau ccgagggccc cucuccaagc gccuggcuuu 2040

ggaaugcucc aggcgcgccg acgccugugc caccccuucc ucagccuggg guuugaccac 2100

ccaccugacc aggggcccua ccuggggaaa gccugaaggg ccucccagcc cccaacccca 2160

agaccaagcu uaguccuggg agaggacagg gacuucgcag aggcaagcga ccgaggcccu 2220

cccaaagagg cccgcccugc ccgggcuccc acaccgucag guacuccugc cagggaacug 2280

gccugcugcg ccccaggccc cgcccgucuc ugcucugcuc agcugcgccc ccuucuuugc 2340

agcugcccag ccccuccucc cugcccucgg gucuccccac cugcacucca uccagcuaca 2400

ggagagauag aagccucucg ucccgucccu cccuuuccuc cgccugucca cagccccuua 2460

agggaaaggu aggaagagag guccagcccc ccaggcugcc cagagcugcu ggucucauuu 2520

gggggcguuc gggagguuug gggggcauca accccccgac ugugcugcuc gcgaaggucc 2580

cacagcccug agaugggccg gcccccuucc uggccccuca uggcgggacu ggagaaaugg 2640

uccgcuuucc uggagccaau ggcccggccc cuccugacuc auccgccugg cccgggaaug 2700

aauggggagg ccgcugaacc cacccggccc auaucccugg uugccucaug gccagcgccc 2760

cucagccucu gccacuguga accggcuccc acccucaagg ugcggggaga agaagcggcc 2820

aggcggggcg ccccaagagc ccaaaagagg gcacaccgcc auccucugcc ucaaauucug 2880

cguuuuuggu uuuaauguua uaucugaugc ugcuauaucc acuguccaac gg 2932

<210> 1616
<211> 19
<212> RNA
<213> Artificial Sequence

<220>
<223> Description of Artificial Sequence: Synthetic oligonucleotide

<400> 1616
agcucccagg gcccggccc 19

<210> 1617
<211> 19
<212> RNA

<213>  Artificial Sequence

<220>
<223>  Description of Artificial Sequence: Synthetic oligonucleotide

<400>  1617
ccccccggcg cucacgcuc                                                    19


<210>  1618
<211>  19
<212>  RNA
<213>  Artificial Sequence

<220>
<223>  Description of Artificial Sequence: Synthetic oligonucleotide

<400>  1618
cucggggcgg acucccggc                                                    19


<210>  1619
<211>  19
<212>  RNA
<213>  Artificial Sequence

<220>
<223>  Description of Artificial Sequence: Synthetic oligonucleotide

<400>  1619
cccuccgcgc ccucucgcg                                                    19


<210>  1620
<211>  19
<212>  RNA
<213>  Artificial Sequence

<220>
<223>  Description of Artificial Sequence: Synthetic oligonucleotide

<400>  1620
gcggcgaugg ccccacucg                                                    19


<210>  1621
<211>  19
<212>  RNA
<213>  Artificial Sequence

<220>
<223>  Description of Artificial Sequence: Synthetic oligonucleotide

<400>  1621
ggauacuucu uacuccucu                                                    19


<210>  1622
<211>  19
<212>  RNA
<213>  Artificial Sequence

<220>
<223>  Description of Artificial Sequence: Synthetic oligonucleotide

<400>  1622
ugcagccuga agcaggcuc                                                    19


<210>  1623
<211>  19
<212>  RNA
<213>  Artificial Sequence

<220>
<223>  Description of Artificial Sequence: Synthetic oligonucleotide

<400>  1623
cugggcagcu acccgaucu                                                    19


<210>  1624
<211>  19
<212>  RNA
<213>  Artificial Sequence

<220>
<223>  Description of Artificial Sequence: Synthetic oligonucleotide

<400>  1624
ugguggucgc uggcuguug                                                    19


<210>  1625
<211>  19
<212>  RNA
<213>  Artificial Sequence

<220>
<223>  Description of Artificial Sequence: Synthetic oligonucleotide

<400>  1625
gggccacagu auuccuccc                                                    19


<210>  1626
<211>  19
<212>  RNA
<213>  Artificial Sequence

<220>
<223>  Description of Artificial Sequence: Synthetic oligonucleotide

<400>  1626
cugggcucgc agcccaucc                                                    19


<210>  1627
<211>  19
<212>  RNA
<213>  Artificial Sequence

<220>
<223>  Description of Artificial Sequence: Synthetic oligonucleotide

<400>  1627
cugugugcca gcaucccgg                                                    19


<210>  1628
<211>  19
<212>  RNA
<213>  Artificial Sequence

<220>
<223>  Description of Artificial Sequence: Synthetic oligonucleotide

<400>  1628
ggccuggucc ccaagcagc                                                    19


<210>  1629
<211>  19
<212>  RNA
<213>  Artificial Sequence

<220>
<223>  Description of Artificial Sequence: Synthetic oligonucleotide

<400>  1629
cuccgcuucu gcaggaacu                                                    19


<210>  1630
<211>  19
<212>  RNA
<213>  Artificial Sequence

<220>
<223>  Description of Artificial Sequence: Synthetic oligonucleotide

<400>  1630
uacguggaga ucaugccca                                                    19


<210>  1631
<211>  19
<212>  RNA
<213>  Artificial Sequence

<220>
<223>  Description of Artificial Sequence: Synthetic oligonucleotide

<400>  1631
agcguggccg agggcauca                                                    19


<210>  1632
<211>  19
<212>  RNA
<213>  Artificial Sequence

<220>

<223>  Description of Artificial Sequence: Synthetic oligonucleotide

<400>  1632
aagauuggca uccaggagu                                                    19


<210>  1633
<211>  19
<212>  RNA
<213>  Artificial Sequence

<220>
<223>  Description of Artificial Sequence: Synthetic oligonucleotide

<400>  1633
ugccagcacc aguuccgcg                                                    19


<210>  1634
<211>  19
<212>  RNA
<213>  Artificial Sequence

<220>
<223>  Description of Artificial Sequence: Synthetic oligonucleotide

<400>  1634
ggccgccggu ggaacugca                                                    19


<210>  1635
<211>  19
<212>  RNA
<213>  Artificial Sequence

<220>
<223>  Description of Artificial Sequence: Synthetic oligonucleotide

<400>  1635
accaccgucc acgacagcc                                                    19


<210>  1636
<211>  19
<212>  RNA
<213>  Artificial Sequence

<220>
<223>  Description of Artificial Sequence: Synthetic oligonucleotide

<400>  1636
cuggccaucu ucgggcccg                                                    19


<210>  1637
<211>  19
<212>  RNA
<213>  Artificial Sequence

<220>
<223>  Description of Artificial Sequence: Synthetic oligonucleotide

<400>  1637
gugcuggaca aagcuacca                                                    19

<210>  1638
<211>  19
<212>  RNA
<213>  Artificial Sequence

<220>
<223>  Description of Artificial Sequence: Synthetic oligonucleotide

<400>  1638
agggagucgg ccuuugucc                                                    19

<210>  1639
<211>  19
<212>  RNA
<213>  Artificial Sequence

<220>
<223>  Description of Artificial Sequence: Synthetic oligonucleotide

<400>  1639
cacgccauug ccucagccg                                                    19

<210>  1640
<211>  19
<212>  RNA
<213>  Artificial Sequence

<220>
<223>  Description of Artificial Sequence: Synthetic oligonucleotide

<400>  1640
   gguguggccu uugcaguga                                                    19

<210>  1641
<211>  19
<212>  RNA
<213>  Artificial Sequence

<220>
<223>  Description of Artificial Sequence: Synthetic oligonucleotide

<400>  1641
acacgcucau gugcagaag                                                    19

<210>  1642
<211>  19
<212>  RNA
<213>  Artificial Sequence

<220>
<223>  Description of Artificial Sequence: Synthetic oligonucleotide

<400>  1642
ggcacggccg ccaucugug                                                    19

<210>  1643
<211>  19
<212>  RNA
<213>  Artificial Sequence

<220>
<223>  Description of Artificial Sequence: Synthetic oligonucleotide

<400>  1643
ggcugcagca gccgccacc                                                    19

<210>  1644
<211>  19
<212>  RNA
<213>  Artificial Sequence

<220>
<223>  Description of Artificial Sequence: Synthetic oligonucleotide

<400>  1644
cagggcucac caggcaagg                                                    19

<210>  1645
<211>  19
<212>  RNA
<213>  Artificial Sequence

<220>
<223>  Description of Artificial Sequence: Synthetic oligonucleotide

<400>  1645
   ggcuggaagu gggguggcu                                                   19

<210>  1646
<211>  19
<212>  RNA
<213>  Artificial Sequence

<220>
<223>  Description of Artificial Sequence: Synthetic oligonucleotide

<400>  1646
uguagcgagg acaucgagu                                                    19

<210>  1647
<211>  19
<212>  RNA
<213>  Artificial Sequence

<220>
<223>  Description of Artificial Sequence: Synthetic oligonucleotide

<400>  1647

uuuggugggga uggugucuc                                                    19

<210>  1648
<211>  19
<212>  RNA
<213>  Artificial Sequence

<220>
<223>  Description of Artificial Sequence: Synthetic oligonucleotide

<400>  1648
cgggaguucg ccgacgccc                                                    19

<210>  1649
<211>  19
<212>  RNA
<213>  Artificial Sequence

<220>
<223>  Description of Artificial Sequence: Synthetic oligonucleotide

<400>  1649
cgggagaacc ggccagaug                                                    19

<210>  1650
<211>  19
<212>  RNA
<213>  Artificial Sequence

<220>
<223>  Description of Artificial Sequence: Synthetic oligonucleotide

<400>  1650
gcccgcucag ccaugaacc                                                    19

<210>  1651
<211>  19
<212>  RNA
<213>  Artificial Sequence

<220>
<223>  Description of Artificial Sequence: Synthetic oligonucleotide

<400>  1651
cgccacaaca acgaggcug                                                    19

<210>  1652
<211>  19
<212>  RNA
<213>  Artificial Sequence

<220>
<223>  Description of Artificial Sequence: Synthetic oligonucleotide

<400>  1652
gggcgccagg ccaucgcca                                                    19

<210>  1653
<211>  19
<212>  RNA
<213>  Artificial Sequence

<220>
<223>  Description of Artificial Sequence: Synthetic oligonucleotide

<400>  1653
agccacaugc accucaagu                                                    19


<210>  1654
<211>  19
<212>  RNA
<213>  Artificial Sequence

<220>
<223>  Description of Artificial Sequence: Synthetic oligonucleotide

<400>  1654
ugcaagugcc acgggcugu                                                    19


<210>  1655
<211>  19
<212>  RNA
<213>  Artificial Sequence

<220>
<223>  Description of Artificial Sequence: Synthetic oligonucleotide

<400>  1655
ucgggcagcu gcgagguga                                                    19


<210>  1656
<211>  19
<212>  RNA
<213>  Artificial Sequence

<220>
<223>  Description of Artificial Sequence: Synthetic oligonucleotide

<400>  1656
aagacaugcu gguggucgc                                                    19


<210>  1657
<211>  19
<212>  RNA
<213>  Artificial Sequence

<220>
<223>  Description of Artificial Sequence: Synthetic oligonucleotide

<400>  1657
caacccgacu uccgcgcca                                                    19

<210>  1658
<211>  19
<212>  RNA
<213>  Artificial Sequence

<220>
<223>  Description of Artificial Sequence: Synthetic oligonucleotide

<400>  1658
aucggugacu uccucaagg                                                    19


<210>  1659
<211>  19
<212>  RNA
<213>  Artificial Sequence

<220>
<223>  Description of Artificial Sequence: Synthetic oligonucleotide

<400>  1659
gacaaguacg acagcgccu                                                    19


<210>  1660
<211>  19
<212>  RNA
<213>  Artificial Sequence

<220>
<223>  Description of Artificial Sequence: Synthetic oligonucleotide

<400>  1660
ucggagaugg ugguggaga                                                    19


<210>  1661
<211>  19
<212>  RNA
<213>  Artificial Sequence

<220>
<223>  Description of Artificial Sequence: Synthetic oligonucleotide

<400>  1661
aagcaccggg agucccgcg                                                    19


<210>  1662
<211>  19
<212>  RNA
<213>  Artificial Sequence

<220>
<223>  Description of Artificial Sequence: Synthetic oligonucleotide

<400>  1662
ggcugggugg agacccugc                                                    19

<210>  1663
<211>  19
<212>  RNA
<213>  Artificial Sequence

<220>
<223>  Description of Artificial Sequence: Synthetic oligonucleotide

<400>  1663
cggccgcgcu acaccuacu                                                    19


<210>  1664
<211>  19
<212>  RNA
<213>  Artificial Sequence

<220>
<223>  Description of Artificial Sequence: Synthetic oligonucleotide

<400>  1664
uucaaggugc ccacggagc                                                    19


<210>  1665
<211>  19
<212>  RNA
<213>  Artificial Sequence

<220>
<223>  Description of Artificial Sequence: Synthetic oligonucleotide

<400>  1665
cgcgaccugg ucuacuacg                                                    19


<210>  1666
<211>  19
<212>  RNA
<213>  Artificial Sequence

<220>
<223>  Description of Artificial Sequence: Synthetic oligonucleotide

<400>  1666
gaggccucgc ccaacuucu                                                    19


<210>  1667
<211>  19
<212>  RNA
<213>  Artificial Sequence

<220>
<223>  Description of Artificial Sequence: Synthetic oligonucleotide

<400>  1667
ugcgagccca acccugaga                                                    19


<210>  1668

<211>  19
<212>  RNA
<213>  Artificial Sequence

<220>
<223>  Description of Artificial Sequence: Synthetic oligonucleotide

<400>  1668
acgggcuccu ucggcacgc                                                    19


<210>  1669
<211>  19
<212>  RNA
<213>  Artificial Sequence

<220>
<223>  Description of Artificial Sequence: Synthetic oligonucleotide

<400>  1669
cgcgaccgca ccugcaacg                                                    19


<210>  1670
<211>  19
<212>  RNA
<213>  Artificial Sequence

<220>
<223>  Description of Artificial Sequence: Synthetic oligonucleotide

<400>  1670
gucagcucgc acggcaucg                                                    19


<210>  1671
<211>  19
<212>  RNA
<213>  Artificial Sequence

<220>
<223>  Description of Artificial Sequence: Synthetic oligonucleotide

<400>  1671
gacggcugcg accugcugu                                                    19


<210>  1672
<211>  19
<212>  RNA
<213>  Artificial Sequence

<220>
<223>  Description of Artificial Sequence: Synthetic oligonucleotide

<400>  1672
ugcugcggcc gcggccaca                                                    19


<210>  1673
<211>  19

<212>  RNA
<213>  Artificial Sequence

<220>
<223>  Description of Artificial Sequence: Synthetic oligonucleotide

<400>  1673
aacgcgcgag cggagcggc                                                    19


<210>  1674
<211>  19
<212>  RNA
<213>  Artificial Sequence

<220>
<223>  Description of Artificial Sequence: Synthetic oligonucleotide

<400>  1674
cgccgggaga agugccgcu                                                    19


<210>  1675
<211>  19
<212>  RNA
<213>  Artificial Sequence

<220>
<223>  Description of Artificial Sequence: Synthetic oligonucleotide

<400>  1675
ugcguguucc acuggugcu                                                    19


<210>  1676
<211>  19
<212>  RNA
<213>  Artificial Sequence

<220>
<223>  Description of Artificial Sequence: Synthetic oligonucleotide

<400>  1676
ugcuacguca gcugccagg                                                    19


<210>  1677
<211>  19
<212>  RNA
<213>  Artificial Sequence

<220>
<223>  Description of Artificial Sequence: Synthetic oligonucleotide

<400>  1677
gagugcacgc gcgucuacg                                                    19


<210>  1678
<211>  19
<212>  RNA

<213>  Artificial Sequence

<220>
<223>  Description of Artificial Sequence: Synthetic oligonucleotide

<400>  1678
gacgugcaca ccugcaagu                                                    19


<210>  1679
<211>  19
<212>  RNA
<213>  Artificial Sequence

<220>
<223>  Description of Artificial Sequence: Synthetic oligonucleotide

<400>  1679
uaggcaccgg ccgcggcuc                                                    19


<210>  1680
<211>  19
<212>  RNA
<213>  Artificial Sequence

<220>
<223>  Description of Artificial Sequence: Synthetic oligonucleotide

<400>  1680
cccccuggac ggggcgggc                                                    19


<210>  1681
<211>  19
<212>  RNA
<213>  Artificial Sequence

<220>
<223>  Description of Artificial Sequence: Synthetic oligonucleotide

<400>  1681
cccugccuga gggugggcu                                                    19


<210>  1682
<211>  19
<212>  RNA
<213>  Artificial Sequence

<220>
<223>  Description of Artificial Sequence: Synthetic oligonucleotide

<400>  1682
uuuucccugg guggagcag                                                    19


<210>  1683
<211>  19
<212>  RNA
<213>  Artificial Sequence

<220>
<223>  Description of Artificial Sequence: Synthetic oligonucleotide

<400>  1683
ggacucccac cuaaacggg                                                    19


<210>  1684
<211>  19
<212>  RNA
<213>  Artificial Sequence

<220>
<223>  Description of Artificial Sequence: Synthetic oligonucleotide

<400>  1684
ggcaguacuc cucccuggg                                                    19


<210>  1685
<211>  19
<212>  RNA
<213>  Artificial Sequence

<220>
<223>  Description of Artificial Sequence: Synthetic oligonucleotide

<400>  1685
gggcgggacu ccucccugg                                                    19


<210>  1686
<211>  19
<212>  RNA
<213>  Artificial Sequence

<220>
<223>  Description of Artificial Sequence: Synthetic oligonucleotide

<400>  1686
gggguggggc uccuaccug                                                    19


<210>  1687
<211>  19
<212>  RNA
<213>  Artificial Sequence

<220>
<223>  Description of Artificial Sequence: Synthetic oligonucleotide

<400>  1687
gggggcagaa cuccuaccu                                                    19


<210>  1688
<211>  19
<212>  RNA
<213>  Artificial Sequence

<220>
<223>  Description of Artificial Sequence: Synthetic oligonucleotide

<400>  1688
ugaaggcagg gcuccuccc                                                    19


<210>  1689
<211>  19
<212>  RNA
<213>  Artificial Sequence

<220>
<223>  Description of Artificial Sequence: Synthetic oligonucleotide

<400>  1689
cuggagcuag ugucuccuc                                                    19


<210>  1690
<211>  19
<212>  RNA
<213>  Artificial Sequence

<220>
<223>  Description of Artificial Sequence: Synthetic oligonucleotide

<400>  1690
cucugguggc ugggcugcu                                                    19


<210>  1691
<211>  19
<212>  RNA
<213>  Artificial Sequence

<220>
<223>  Description of Artificial Sequence: Synthetic oligonucleotide

<400>  1691
uccugaauga ggcggagcu                                                    19


<210>  1692
<211>  19
<212>  RNA
<213>  Artificial Sequence

<220>
<223>  Description of Artificial Sequence: Synthetic oligonucleotide

<400>  1692
uccaggaugg ggaggggcu                                                    19


<210>  1693
<211>  19
<212>  RNA
<213>  Artificial Sequence

<220>

<223>  Description of Artificial Sequence: Synthetic oligonucleotide

<400>  1693
ucugcguugg cuucucccu                                                    19


<210>  1694
<211>  19
<212>  RNA
<213>  Artificial Sequence

<220>
<223>  Description of Artificial Sequence: Synthetic oligonucleotide

<400>  1694
   uggggacggg gcuccccug                                                   19


<210>  1695
<211>  19
<212>  RNA
<213>  Artificial Sequence

<220>
<223>  Description of Artificial Sequence: Synthetic oligonucleotide

<400>  1695
ggacagaggc ggggcuaca                                                    19


<210>  1696
<211>  19
<212>  RNA
<213>  Artificial Sequence

<220>
<223>  Description of Artificial Sequence: Synthetic oligonucleotide

<400>  1696
agauugggcg gggcuucuc                                                    19


<210>  1697
<211>  19
<212>  RNA
<213>  Artificial Sequence

<220>
<223>  Description of Artificial Sequence: Synthetic oligonucleotide

<400>  1697
   cuuggguggg acagggcuu                                                   19


<210>  1698
<211>  19
<212>  RNA
<213>  Artificial Sequence

<220>
<223>  Description of Artificial Sequence: Synthetic oligonucleotide

<400>  1698
ucuccugcgg gggcgaggc                                                    19


<210>  1699
<211>  19
<212>  RNA
<213>  Artificial Sequence

<220>
<223>  Description of Artificial Sequence: Synthetic oligonucleotide

<400>  1699
ccccucccag uaagggcgu                                                    19


<210>  1700
<211>  19
<212>  RNA
<213>  Artificial Sequence

<220>
<223>  Description of Artificial Sequence: Synthetic oligonucleotide

<400>  1700
uggcucuggg ugggcgggg                                                    19


<210>  1701
<211>  19
<212>  RNA
<213>  Artificial Sequence

<220>
<223>  Description of Artificial Sequence: Synthetic oligonucleotide

<400>  1701
gcacuaggua ggcuucuac                                                    19


<210>  1702
<211>  19
<212>  RNA
<213>  Artificial Sequence

<220>
<223>  Description of Artificial Sequence: Synthetic oligonucleotide

<400>  1702
ccugcaggcg gggcuccuc                                                    19


<210>  1703
<211>  19
<212>  RNA
<213>  Artificial Sequence

<220>
<223>  Description of Artificial Sequence: Synthetic oligonucleotide

<400>  1703
ccugaaggag gcggggcuc                                                    19


<210>  1704
<211>  19
<212>  RNA
<213>  Artificial Sequence

<220>
<223>  Description of Artificial Sequence: Synthetic oligonucleotide

<400>  1704
cuaggauggg gcacggcuc                                                    19


<210>  1705
<211>  19
<212>  RNA
<213>  Artificial Sequence

<220>
<223>  Description of Artificial Sequence: Synthetic oligonucleotide

<400>  1705
cuggguagg cugcucccu                                                     19


<210>  1706
<211>  19
<212>  RNA
<213>  Artificial Sequence

<220>
<223>  Description of Artificial Sequence: Synthetic oligonucleotide

<400>  1706
ugagggcgga gcgccuccu                                                    19


<210>  1707
<211>  19
<212>  RNA
<213>  Artificial Sequence

<220>
<223>  Description of Artificial Sequence: Synthetic oligonucleotide

<400>  1707
uuaggagugg gguuuuaug                                                    19


<210>  1708
<211>  19
<212>  RNA
<213>  Artificial Sequence

<220>
<223>  Description of Artificial Sequence: Synthetic oligonucleotide

<400>  1708

gguggaugag gcuucuucc                                                    19

<210>  1709
<211>  19
<212>  RNA
<213>  Artificial Sequence

<220>
<223>  Description of Artificial Sequence: Synthetic oligonucleotide

<400>  1709
cuggaugggg cagagcuuc                                                    19

<210>  1710
<211>  19
<212>  RNA
<213>  Artificial Sequence

<220>
<223>  Description of Artificial Sequence: Synthetic oligonucleotide

<400>  1710
cuccugacca gggcaaggc                                                    19

<210>  1711
<211>  19
<212>  RNA
<213>  Artificial Sequence

<220>
<223>  Description of Artificial Sequence: Synthetic oligonucleotide

<400>  1711
ccccuuccac gggggcugu                                                    19

<210>  1712
<211>  19
<212>  RNA
<213>  Artificial Sequence

<220>
<223>  Description of Artificial Sequence: Synthetic oligonucleotide

<400>  1712
uggcucuggg ugggcgugg                                                    19

<210>  1713
<211>  19
<212>  RNA
<213>  Artificial Sequence

<220>
<223>  Description of Artificial Sequence: Synthetic oligonucleotide

<400>  1713
gccugcauag gcuccuucc                                                    19

<210>  1714
<211>  19
<212>  RNA
<213>  Artificial Sequence

<220>
<223>  Description of Artificial Sequence: Synthetic oligonucleotide

<400>  1714
cugugggugg ggcuucucu                                                    19


<210>  1715
<211>  19
<212>  RNA
<213>  Artificial Sequence

<220>
<223>  Description of Artificial Sequence: Synthetic oligonucleotide

<400>  1715
ugggaccagg cuccaaugg                                                    19


<210>  1716
<211>  19
<212>  RNA
<213>  Artificial Sequence

<220>
<223>  Description of Artificial Sequence: Synthetic oligonucleotide

<400>  1716
gggcggggcu ucucuccgc                                                    19


<210>  1717
<211>  19
<212>  RNA
<213>  Artificial Sequence

<220>
<223>  Description of Artificial Sequence: Synthetic oligonucleotide

<400>  1717
   cgggugggac ucuucccug                                                     19


<210>  1718
<211>  19
<212>  RNA
<213>  Artificial Sequence

<220>
<223>  Description of Artificial Sequence: Synthetic oligonucleotide

<400>  1718
gggaaccgcc cuccugauu                                                    19

<210> 1719
<211> 19
<212> RNA
<213> Artificial Sequence

<220>
<223> Description of Artificial Sequence: Synthetic oligonucleotide

<400> 1719
uaaggcgugg cuucugcag 19


<210> 1720
<211> 19
<212> RNA
<213> Artificial Sequence

<220>
<223> Description of Artificial Sequence: Synthetic oligonucleotide

<400> 1720
ggaaucccgg cuccagagc 19


<210> 1721
<211> 19
<212> RNA
<213> Artificial Sequence

<220>
<223> Description of Artificial Sequence: Synthetic oligonucleotide

<400> 1721
caggaaauuc agcccacca 19


<210> 1722
<211> 19
<212> RNA
<213> Artificial Sequence

<220>
<223> Description of Artificial Sequence: Synthetic oligonucleotide

<400> 1722
agccaccuca uccccaacc 19


<210> 1723
<211> 19
<212> RNA
<213> Artificial Sequence

<220>
<223> Description of Artificial Sequence: Synthetic oligonucleotide

<400> 1723
ccccuguaag guuccaucc 19

<210>  1724
<211>  19
<212>  RNA
<213>  Artificial Sequence

<220>
<223>  Description of Artificial Sequence: Synthetic oligonucleotide

<400>  1724
caccccugcg ucgagcugg                                                    19


<210>  1725
<211>  19
<212>  RNA
<213>  Artificial Sequence

<220>
<223>  Description of Artificial Sequence: Synthetic oligonucleotide

<400>  1725
ggaagguucc augaagcga                                                    19


<210>  1726
<211>  19
<212>  RNA
<213>  Artificial Sequence

<220>
<223>  Description of Artificial Sequence: Synthetic oligonucleotide

<400>  1726
agucgggucc ccaacccgu                                                    19


<210>  1727
<211>  19
<212>  RNA
<213>  Artificial Sequence

<220>
<223>  Description of Artificial Sequence: Synthetic oligonucleotide

<400>  1727
ugccccuggg auccgaggg                                                    19


<210>  1728
<211>  19
<212>  RNA
<213>  Artificial Sequence

<220>
<223>  Description of Artificial Sequence: Synthetic oligonucleotide

<400>  1728
gccccucucc aagcgccug                                                    19


<210>  1729

<211>  19
<212>  RNA
<213>  Artificial Sequence

<220>
<223>  Description of Artificial Sequence: Synthetic oligonucleotide

<400>  1729
ggcuuuggaa ugcuccagg                                                    19


<210>  1730
<211>  19
<212>  RNA
<213>  Artificial Sequence

<220>
<223>  Description of Artificial Sequence: Synthetic oligonucleotide

<400>  1730
gcgcgccgac gccugugcc                                                    19


<210>  1731
<211>  19
<212>  RNA
<213>  Artificial Sequence

<220>
<223>  Description of Artificial Sequence: Synthetic oligonucleotide

<400>  1731
caccccuucc ucagccugg                                                    19


<210>  1732
<211>  19
<212>  RNA
<213>  Artificial Sequence

<220>
<223>  Description of Artificial Sequence: Synthetic oligonucleotide

<400>  1732
ggguuugacc acccaccug                                                    19


<210>  1733
<211>  19
<212>  RNA
<213>  Artificial Sequence

<220>
<223>  Description of Artificial Sequence: Synthetic oligonucleotide

<400>  1733
gaccaggggc ccuaccugg                                                    19


<210>  1734
<211>  19

<212>  RNA
<213>  Artificial Sequence

<220>
<223>  Description of Artificial Sequence: Synthetic oligonucleotide

<400>  1734
gggaaagccu gaagggccu                                                    19


<210>  1735
<211>  19
<212>  RNA
<213>  Artificial Sequence

<220>
<223>  Description of Artificial Sequence: Synthetic oligonucleotide

<400>  1735
ucccagcccc caaccccaa                                                    19


<210>  1736
<211>  19
<212>  RNA
<213>  Artificial Sequence

<220>
<223>  Description of Artificial Sequence: Synthetic oligonucleotide

<400>  1736
agaccaagcu uaguccugg                                                    19


<210>  1737
<211>  19
<212>  RNA
<213>  Artificial Sequence

<220>
<223>  Description of Artificial Sequence: Synthetic oligonucleotide

<400>  1737
ggagaggaca gggacuucg                                                    19


<210>  1738
<211>  19
<212>  RNA
<213>  Artificial Sequence

<220>
<223>  Description of Artificial Sequence: Synthetic oligonucleotide

<400>  1738
gcagaggcaa gcgaccgag                                                    19


<210>  1739
<211>  19
<212>  RNA

<213>  Artificial Sequence

<220>
<223>  Description of Artificial Sequence: Synthetic oligonucleotide

<400>  1739
ggcccuccca aagaggccc                                                    19


<210>  1740
<211>  19
<212>  RNA
<213>  Artificial Sequence

<220>
<223>  Description of Artificial Sequence: Synthetic oligonucleotide

<400>  1740
cgcccugccc gggcuccca                                                    19


<210>  1741
<211>  19
<212>  RNA
<213>  Artificial Sequence

<220>
<223>  Description of Artificial Sequence: Synthetic oligonucleotide

<400>  1741
acaccgucag guacuccug                                                    19


<210>  1742
<211>  19
<212>  RNA
<213>  Artificial Sequence

<220>
<223>  Description of Artificial Sequence: Synthetic oligonucleotide

<400>  1742
gccagggaac uggccugcu                                                    19


<210>  1743
<211>  19
<212>  RNA
<213>  Artificial Sequence

<220>
<223>  Description of Artificial Sequence: Synthetic oligonucleotide

<400>  1743
ugcgccccag gccccgccc                                                    19


<210>  1744
<211>  19
<212>  RNA
<213>  Artificial Sequence

<220>
<223>  Description of Artificial Sequence: Synthetic oligonucleotide

<400>  1744
cgucucugcu cugcucagc                                                    19


<210>  1745
<211>  19
<212>  RNA
<213>  Artificial Sequence

<220>
<223>  Description of Artificial Sequence: Synthetic oligonucleotide

<400>  1745
cugcgccccc uucuuugca                                                    19


<210>  1746
<211>  19
<212>  RNA
<213>  Artificial Sequence

<220>
<223>  Description of Artificial Sequence: Synthetic oligonucleotide

<400>  1746
agcugcccag ccccuccuc                                                    19


<210>  1747
<211>  19
<212>  RNA
<213>  Artificial Sequence

<220>
<223>  Description of Artificial Sequence: Synthetic oligonucleotide

<400>  1747
cccugcccuc gggucuccc                                                    19


<210>  1748
<211>  19
<212>  RNA
<213>  Artificial Sequence

<220>
<223>  Description of Artificial Sequence: Synthetic oligonucleotide

<400>  1748
ccaccugcac uccauccag                                                    19


<210>  1749
<211>  19
<212>  RNA
<213>  Artificial Sequence

<220>
<223>  Description of Artificial Sequence: Synthetic oligonucleotide

<400>  1749
gcuacaggag agauagaag                                                    19


<210>  1750
<211>  19
<212>  RNA
<213>  Artificial Sequence

<220>
<223>  Description of Artificial Sequence: Synthetic oligonucleotide

<400>  1750
gccucucguc ccgucccuc                                                    19


<210>  1751
<211>  19
<212>  RNA
<213>  Artificial Sequence

<220>
<223>  Description of Artificial Sequence: Synthetic oligonucleotide

<400>  1751
cccuuuccuc cgccugucc                                                    19


<210>  1752
<211>  19
<212>  RNA
<213>  Artificial Sequence

<220>
<223>  Description of Artificial Sequence: Synthetic oligonucleotide

<400>  1752
cacagccccu uaagggaaa                                                    19


<210>  1753
<211>  19
<212>  RNA
<213>  Artificial Sequence

<220>
<223>  Description of Artificial Sequence: Synthetic oligonucleotide

<400>  1753
agguaggaag agaggucca                                                    19


<210>  1754
<211>  19
<212>  RNA
<213>  Artificial Sequence

<220>

<223>  Description of Artificial Sequence: Synthetic oligonucleotide

<400>  1754
agccccccag gcugcccag                                                    19


<210>  1755
<211>  19
<212>  RNA
<213>  Artificial Sequence

<220>
<223>  Description of Artificial Sequence: Synthetic oligonucleotide

<400>  1755
gagcugcugg ucucauuug                                                    19


<210>  1756
<211>  19
<212>  RNA
<213>  Artificial Sequence

<220>
<223>  Description of Artificial Sequence: Synthetic oligonucleotide

<400>  1756
gggggcguuc gggagguuu                                                    19


<210>  1757
<211>  19
<212>  RNA
<213>  Artificial Sequence

<220>
<223>  Description of Artificial Sequence: Synthetic oligonucleotide

<400>  1757
   uggggggcau caacccccc                                                      19


<210>  1758
<211>  19
<212>  RNA
<213>  Artificial Sequence

<220>
<223>  Description of Artificial Sequence: Synthetic oligonucleotide

<400>  1758
cgacugugcu gcucgcgaa                                                    19


<210>  1759
<211>  19
<212>  RNA
<213>  Artificial Sequence

<220>
<223>  Description of Artificial Sequence: Synthetic oligonucleotide

<400>  1759
aggucccaca gcccugaga                                                    19


<210>  1760
<211>  19
<212>  RNA
<213>  Artificial Sequence

<220>
<223>  Description of Artificial Sequence: Synthetic oligonucleotide

<400>  1760
   augggccggc ccccuuccu                                                     19


<210>  1761
<211>  19
<212>  RNA
<213>  Artificial Sequence

<220>
<223>  Description of Artificial Sequence: Synthetic oligonucleotide

<400>  1761
uggccccuca uggcgggac                                                    19


<210>  1762
<211>  19
<212>  RNA
<213>  Artificial Sequence

<220>
<223>  Description of Artificial Sequence: Synthetic oligonucleotide

<400>  1762
cuggagaaau gguccgcuu                                                    19


<210>  1763
<211>  19
<212>  RNA
<213>  Artificial Sequence

<220>
<223>  Description of Artificial Sequence: Synthetic oligonucleotide

<400>  1763
uuccuggagc caauggccc                                                    19


<210>  1764
<211>  19
<212>  RNA
<213>  Artificial Sequence

<220>
<223>  Description of Artificial Sequence: Synthetic oligonucleotide

<400>  1764
cggccccucc ugacucauc                                                    19

<210>  1765
<211>  19
<212>  RNA
<213>  Artificial Sequence

<220>
<223>  Description of Artificial Sequence: Synthetic oligonucleotide

<400>  1765
ccgccuggcc cgggaauga                                                    19

<210>  1766
<211>  19
<212>  RNA
<213>  Artificial Sequence

<220>
<223>  Description of Artificial Sequence: Synthetic oligonucleotide

<400>  1766
   aauggggagg ccgcugaac                                                   19

<210>  1767
<211>  19
<212>  RNA
<213>  Artificial Sequence

<220>
<223>  Description of Artificial Sequence: Synthetic oligonucleotide

<400>  1767
cccacccggc ccauauccc                                                    19

<210>  1768
<211>  19
<212>  RNA
<213>  Artificial Sequence

<220>
<223>  Description of Artificial Sequence: Synthetic oligonucleotide

<400>  1768
cugguugccu cauggccag                                                    19

<210>  1769
<211>  19
<212>  RNA
<213>  Artificial Sequence

<220>
<223>  Description of Artificial Sequence: Synthetic oligonucleotide

<400>  1769

gcgccccuca gccucugcc                                                    19

<210>  1770
<211>  19
<212>  RNA
<213>  Artificial Sequence

<220>
<223>  Description of Artificial Sequence: Synthetic oligonucleotide

<400>  1770
cacugugaac cggcuccca                                                    19

<210>  1771
<211>  19
<212>  RNA
<213>  Artificial Sequence

<220>
<223>  Description of Artificial Sequence: Synthetic oligonucleotide

<400>  1771
acccucaagg ugcggggag                                                    19

<210>  1772
<211>  19
<212>  RNA
<213>  Artificial Sequence

<220>
<223>  Description of Artificial Sequence: Synthetic oligonucleotide

<400>  1772
gaagaagcgg ccaggcggg                                                    19

<210>  1773
<211>  19
<212>  RNA
<213>  Artificial Sequence

<220>
<223>  Description of Artificial Sequence: Synthetic oligonucleotide

<400>  1773
ggcgccccaa gagcccaaa                                                    19

<210>  1774
<211>  19
<212>  RNA
<213>  Artificial Sequence

<220>
<223>  Description of Artificial Sequence: Synthetic oligonucleotide

<400>  1774
aagagggcac accgccauc                                                    19

<210> 1775
<211> 19
<212> RNA
<213> Artificial Sequence

<220>
<223> Description of Artificial Sequence: Synthetic oligonucleotide

<400> 1775
ccucugccuc aaauucugc 19


<210> 1776
<211> 19
<212> RNA
<213> Artificial Sequence

<220>
<223> Description of Artificial Sequence: Synthetic oligonucleotide

<400> 1776
cguuuuuggu uuuaauguu 19


<210> 1777
<211> 19
<212> RNA
<213> Artificial Sequence

<220>
<223> Description of Artificial Sequence: Synthetic oligonucleotide

<400> 1777
uauaucugau gcugcuaua 19


<210> 1778
<211> 19
<212> RNA
<213> Artificial Sequence

<220>
<223> Description of Artificial Sequence: Synthetic oligonucleotide

<400> 1778
uauauccacu guccaacgg 19


<210> 1779
<211> 25
<212> RNA
<213> Artificial Sequence

<220>
<223> Description of Artificial Sequence: Synthetic oligonucleotide

<400> 1779
agcucccagg gcccggcccc ccccg 25

<210>  1780
<211>  25
<212>  RNA
<213>  Artificial Sequence

<220>
<223>  Description of Artificial Sequence: Synthetic oligonucleotide

<400>  1780
ggcgcucacg cucucggggc ggacu                                          25


<210>  1781
<211>  25
<212>  RNA
<213>  Artificial Sequence

<220>
<223>  Description of Artificial Sequence: Synthetic oligonucleotide

<400>  1781
ucccggcccu ccgcgcccuc ucgcg                                          25


<210>  1782
<211>  25
<212>  RNA
<213>  Artificial Sequence

<220>
<223>  Description of Artificial Sequence: Synthetic oligonucleotide

<400>  1782
gcggcgaugg ccccacucgg auacu                                          25


<210>  1783
<211>  25
<212>  RNA
<213>  Artificial Sequence

<220>
<223>  Description of Artificial Sequence: Synthetic oligonucleotide

<400>  1783
uucuuacucc ucugcagccu gaagc                                          25


<210>  1784
<211>  25
<212>  RNA
<213>  Artificial Sequence

<220>
<223>  Description of Artificial Sequence: Synthetic oligonucleotide

<400>  1784
caggcucugg gcagcuaccc gaucu                                          25

<210>  1785
<211>  25
<212>  RNA
<213>  Artificial Sequence

<220>
<223>  Description of Artificial Sequence: Synthetic oligonucleotide

<400>  1785
ugguggucgc uggcuguugg gccac                                          25


<210>  1786
<211>  25
<212>  RNA
<213>  Artificial Sequence

<220>
<223>  Description of Artificial Sequence: Synthetic oligonucleotide

<400>  1786
caguauuccu cccugggcuc gcagc                                          25


<210>  1787
<211>  25
<212>  RNA
<213>  Artificial Sequence

<220>
<223>  Description of Artificial Sequence: Synthetic oligonucleotide

<400>  1787
cccauccugu gugccagcau cccgg                                          25


<210>  1788
<211>  25
<212>  RNA
<213>  Artificial Sequence

<220>
<223>  Description of Artificial Sequence: Synthetic oligonucleotide

<400>  1788
ggccuggucc ccaagcagcu ccgcu                                          25


<210>  1789
<211>  25
<212>  RNA
<213>  Artificial Sequence

<220>
<223>  Description of Artificial Sequence: Synthetic oligonucleotide

<400>  1789
uucugcagga acuacgugga gauca                                          25


<210>  1790

<211>  25
<212>  RNA
<213>  Artificial Sequence

<220>
<223>  Description of Artificial Sequence: Synthetic oligonucleotide

<400>  1790
augcccagcg uggccgaggg cauca                                          25


<210>  1791
<211>  25
<212>  RNA
<213>  Artificial Sequence

<220>
<223>  Description of Artificial Sequence: Synthetic oligonucleotide

<400>  1791
aagauuggca uccaggagug ccagc                                          25


<210>  1792
<211>  25
<212>  RNA
<213>  Artificial Sequence

<220>
<223>  Description of Artificial Sequence: Synthetic oligonucleotide

<400>  1792
caccaguucc gcggccgccg gugga                                          25


<210>  1793
<211>  25
<212>  RNA
<213>  Artificial Sequence

<220>
<223>  Description of Artificial Sequence: Synthetic oligonucleotide

<400>  1793
aacugcacca ccguccacga cagcc                                          25


<210>  1794
<211>  25
<212>  RNA
<213>  Artificial Sequence

<220>
<223>  Description of Artificial Sequence: Synthetic oligonucleotide

<400>  1794
cuggccaucu ucgggcccgu gcugg                                          25


<210>  1795
<211>  25

<212>  RNA
<213>  Artificial Sequence

<220>
<223>  Description of Artificial Sequence: Synthetic oligonucleotide

<400>  1795
gacaaagcua ccagggaguc ggccu                                          25


<210>  1796
<211>  25
<212>  RNA
<213>  Artificial Sequence

<220>
<223>  Description of Artificial Sequence: Synthetic oligonucleotide

<400>  1796
uuuguccacg ccauugccuc agccg                                          25


<210>  1797
<211>  25
<212>  RNA
<213>  Artificial Sequence

<220>
<223>  Description of Artificial Sequence: Synthetic oligonucleotide

<400>  1797
   gguguggccu uugcagugac acgcu                                          25


<210>  1798
<211>  25
<212>  RNA
<213>  Artificial Sequence

<220>
<223>  Description of Artificial Sequence: Synthetic oligonucleotide

<400>  1798
ucaugugcag aaggcacggc cgcca                                          25


<210>  1799
<211>  25
<212>  RNA
<213>  Artificial Sequence

<220>
<223>  Description of Artificial Sequence: Synthetic oligonucleotide

<400>  1799
aucuguggcu gcagcagccg ccacc                                          25


<210>  1800
<211>  25
<212>  RNA

<213>  Artificial Sequence

<220>
<223>  Description of Artificial Sequence: Synthetic oligonucleotide

<400>  1800
cagggcucac caggcaaggg cugga                                          25


<210>  1801
<211>  25
<212>  RNA
<213>  Artificial Sequence

<220>
<223>  Description of Artificial Sequence: Synthetic oligonucleotide

<400>  1801
aaguggggug gcuguagcga ggaca                                          25


<210>  1802
<211>  25
<212>  RNA
<213>  Artificial Sequence

<220>
<223>  Description of Artificial Sequence: Synthetic oligonucleotide

<400>  1802
aucgaguuug gugggauggu gucuc                                          25


<210>  1803
<211>  25
<212>  RNA
<213>  Artificial Sequence

<220>
<223>  Description of Artificial Sequence: Synthetic oligonucleotide

<400>  1803
cgggaguucg ccgacgcccg ggaga                                          25


<210>  1804
<211>  25
<212>  RNA
<213>  Artificial Sequence

<220>
<223>  Description of Artificial Sequence: Synthetic oligonucleotide

<400>  1804
aaccggccag augcccgcuc agcca                                          25


<210>  1805
<211>  25
<212>  RNA
<213>  Artificial Sequence

<220>
<223>  Description of Artificial Sequence: Synthetic oligonucleotide

<400>  1805
augaaccgcc acaacaacga ggcug                                          25


<210>  1806
<211>  25
<212>  RNA
<213>  Artificial Sequence

<220>
<223>  Description of Artificial Sequence: Synthetic oligonucleotide

<400>  1806
gggcgccagg ccaucgccag ccaca                                          25


<210>  1807
<211>  25
<212>  RNA
<213>  Artificial Sequence

<220>
<223>  Description of Artificial Sequence: Synthetic oligonucleotide

<400>  1807
augcaccuca agugcaagug ccacg                                          25


<210>  1808
<211>  25
<212>  RNA
<213>  Artificial Sequence

<220>
<223>  Description of Artificial Sequence: Synthetic oligonucleotide

<400>  1808
gggcugucgg gcagcugcga gguga                                          25


<210>  1809
<211>  25
<212>  RNA
<213>  Artificial Sequence

<220>
<223>  Description of Artificial Sequence: Synthetic oligonucleotide

<400>  1809
aagacaugcu gguggucgca acccg                                          25


<210>  1810
<211>  25
<212>  RNA
<213>  Artificial Sequence

<220>
<223>  Description of Artificial Sequence: Synthetic oligonucleotide

<400>  1810
gacuuccgcg ccaucgguga cuucc                                          25


<210>  1811
<211>  25
<212>  RNA
<213>  Artificial Sequence

<220>
<223>  Description of Artificial Sequence: Synthetic oligonucleotide

<400>  1811
cucaaggaca aguacgacag cgccu                                          25


<210>  1812
<211>  25
<212>  RNA
<213>  Artificial Sequence

<220>
<223>  Description of Artificial Sequence: Synthetic oligonucleotide

<400>  1812
ucggagaugg ugguggagaa gcacc                                          25


<210>  1813
<211>  25
<212>  RNA
<213>  Artificial Sequence

<220>
<223>  Description of Artificial Sequence: Synthetic oligonucleotide

<400>  1813
cgggaguccc gcggcugggu ggaga                                          25


<210>  1814
<211>  25
<212>  RNA
<213>  Artificial Sequence

<220>
<223>  Description of Artificial Sequence: Synthetic oligonucleotide

<400>  1814
acccugcggc cgcgcuacac cuacu                                          25


<210>  1815
<211>  25
<212>  RNA
<213>  Artificial Sequence

<220>

<223>  Description of Artificial Sequence: Synthetic oligonucleotide

<400>  1815
uucaaggugc ccacggagcg cgacc                                          25


<210>  1816
<211>  25
<212>  RNA
<213>  Artificial Sequence

<220>
<223>  Description of Artificial Sequence: Synthetic oligonucleotide

<400>  1816
cuggucuacu acgaggccuc gccca                                          25


<210>  1817
<211>  25
<212>  RNA
<213>  Artificial Sequence

<220>
<223>  Description of Artificial Sequence: Synthetic oligonucleotide

<400>  1817
aacuucugcg agcccaaccc ugaga                                          25


<210>  1818
<211>  25
<212>  RNA
<213>  Artificial Sequence

<220>
<223>  Description of Artificial Sequence: Synthetic oligonucleotide

<400>  1818
acgggcuccu ucggcacgcg cgacc                                          25


<210>  1819
<211>  25
<212>  RNA
<213>  Artificial Sequence

<220>
<223>  Description of Artificial Sequence: Synthetic oligonucleotide

<400>  1819
cgcaccugca acgucagcuc gcacg                                          25


<210>  1820
<211>  25
<212>  RNA
<213>  Artificial Sequence

<220>
<223>  Description of Artificial Sequence: Synthetic oligonucleotide

<400>  1820
ggcaucgacg gcugcgaccu gcugu                                          25


<210>  1821
<211>  25
<212>  RNA
<213>  Artificial Sequence

<220>
<223>  Description of Artificial Sequence: Synthetic oligonucleotide

<400>  1821
ugcugcggcc gcggccacaa cgcgc                                          25


<210>  1822
<211>  25
<212>  RNA
<213>  Artificial Sequence

<220>
<223>  Description of Artificial Sequence: Synthetic oligonucleotide

<400>  1822
cgagcggagc ggcgccggga gaagu                                          25


<210>  1823
<211>  25
<212>  RNA
<213>  Artificial Sequence

<220>
<223>  Description of Artificial Sequence: Synthetic oligonucleotide

<400>  1823
ugccgcugcg uguuccacug gugcu                                          25


<210>  1824
<211>  25
<212>  RNA
<213>  Artificial Sequence

<220>
<223>  Description of Artificial Sequence: Synthetic oligonucleotide

<400>  1824
ugcuacguca gcugccagga gugca                                          25


<210>  1825
<211>  25
<212>  RNA
<213>  Artificial Sequence

<220>
<223>  Description of Artificial Sequence: Synthetic oligonucleotide

<400>  1825
acgcgcgucu acgacgugca caccu                                            25


<210>  1826
<211>  25
<212>  RNA
<213>  Artificial Sequence

<220>
<223>  Description of Artificial Sequence: Synthetic oligonucleotide

<400>  1826
ugcaaguagg caccggccgc ggcuc                                            25


<210>  1827
<211>  25
<212>  RNA
<213>  Artificial Sequence

<220>
<223>  Description of Artificial Sequence: Synthetic oligonucleotide

<400>  1827
cccccuggac ggggcgggcc cugcc                                            25


<210>  1828
<211>  25
<212>  RNA
<213>  Artificial Sequence

<220>
<223>  Description of Artificial Sequence: Synthetic oligonucleotide

<400>  1828
cugagggugg gcuuuucccu gggug                                            25


<210>  1829
<211>  25
<212>  RNA
<213>  Artificial Sequence

<220>
<223>  Description of Artificial Sequence: Synthetic oligonucleotide

<400>  1829
ggagcaggac ucccaccuaa acggg                                            25


<210>  1830
<211>  25
<212>  RNA
<213>  Artificial Sequence

<220>
<223>  Description of Artificial Sequence: Synthetic oligonucleotide

<400>  1830

ggcaguacuc cucccugggg gcggg                                          25

<210>  1831
<211>  25
<212>  RNA
<213>  Artificial Sequence

<220>
<223>  Description of Artificial Sequence: Synthetic oligonucleotide

<400>  1831
gacuccuccc ugggggugggg gcucc                                          25

<210>  1832
<211>  25
<212>  RNA
<213>  Artificial Sequence

<220>
<223>  Description of Artificial Sequence: Synthetic oligonucleotide

<400>  1832
cuaccugggg gcagaacucc uaccu                                          25

<210>  1833
<211>  25
<212>  RNA
<213>  Artificial Sequence

<220>
<223>  Description of Artificial Sequence: Synthetic oligonucleotide

<400>  1833
ugaaggcagg gcuccucccu ggagc                                          25

<210>  1834
<211>  25
<212>  RNA
<213>  Artificial Sequence

<220>
<223>  Description of Artificial Sequence: Synthetic oligonucleotide

<400>  1834
cuagugucuc cucucuggug gcugg                                          25

<210>  1835
<211>  25
<212>  RNA
<213>  Artificial Sequence

<220>
<223>  Description of Artificial Sequence: Synthetic oligonucleotide

<400>  1835
ggcugcuccu gaaugaggcg gagcu                                          25

<210> 1836
<211> 25
<212> RNA
<213> Artificial Sequence

<220>
<223> Description of Artificial Sequence: Synthetic oligonucleotide

<400> 1836
uccaggaugg ggaggggcuc ugcgu 25


<210> 1837
<211> 25
<212> RNA
<213> Artificial Sequence

<220>
<223> Description of Artificial Sequence: Synthetic oligonucleotide

<400> 1837
uuggcuucuc ccuggggacg gggcu 25


<210> 1838
<211> 25
<212> RNA
<213> Artificial Sequence

<220>
<223> Description of Artificial Sequence: Synthetic oligonucleotide

<400> 1838
uccccuggac agaggcgggg cuaca 25


<210> 1839
<211> 25
<212> RNA
<213> Artificial Sequence

<220>
<223> Description of Artificial Sequence: Synthetic oligonucleotide

<400> 1839
agauugggcg gggcuucucu ugggu 25


<210> 1840
<211> 25
<212> RNA
<213> Artificial Sequence

<220>
<223> Description of Artificial Sequence: Synthetic oligonucleotide

<400> 1840
ugggacaggg cuucuccugc ggggg 25

<210>  1841
<211>  25
<212>  RNA
<213>  Artificial Sequence

<220>
<223>  Description of Artificial Sequence: Synthetic oligonucleotide

<400>  1841
gcgaggcccc ucccaguaag ggcgu                                          25


<210>  1842
<211>  25
<212>  RNA
<213>  Artificial Sequence

<220>
<223>  Description of Artificial Sequence: Synthetic oligonucleotide

<400>  1842
uggcucuggg ugggcggggc acuag                                          25


<210>  1843
<211>  25
<212>  RNA
<213>  Artificial Sequence

<220>
<223>  Description of Artificial Sequence: Synthetic oligonucleotide

<400>  1843
gguaggcuuc uaccugcagg cgggg                                          25


<210>  1844
<211>  25
<212>  RNA
<213>  Artificial Sequence

<220>
<223>  Description of Artificial Sequence: Synthetic oligonucleotide

<400>  1844
gcuccuccug aaggaggcgg ggcuc                                          25


<210>  1845
<211>  25
<212>  RNA
<213>  Artificial Sequence

<220>
<223>  Description of Artificial Sequence: Synthetic oligonucleotide

<400>  1845
cuaggauggg gcacggcucu ggggu                                          25

<210>  1846
<211>  25
<212>  RNA
<213>  Artificial Sequence

<220>
<223>  Description of Artificial Sequence: Synthetic oligonucleotide

<400>  1846
uaggcugcuc ccugagggcg gagcg                                          25


<210>  1847
<211>  25
<212>  RNA
<213>  Artificial Sequence

<220>
<223>  Description of Artificial Sequence: Synthetic oligonucleotide

<400>  1847
   gccuccuuag gagugggguu uuaug                                           25


<210>  1848
<211>  25
<212>  RNA
<213>  Artificial Sequence

<220>
<223>  Description of Artificial Sequence: Synthetic oligonucleotide

<400>  1848
gguggaugag gcuucuuccu ggaug                                          25


<210>  1849
<211>  25
<212>  RNA
<213>  Artificial Sequence

<220>
<223>  Description of Artificial Sequence: Synthetic oligonucleotide

<400>  1849
ggggcagagc uucuccugac caggg                                          25


<210>  1850
<211>  25
<212>  RNA
<213>  Artificial Sequence

<220>
<223>  Description of Artificial Sequence: Synthetic oligonucleotide

<400>  1850
gcaaggcccc uuccacgggg gcugu                                          25


<210>  1851

<211>  25
<212>  RNA
<213>  Artificial Sequence

<220>
<223>  Description of Artificial Sequence: Synthetic oligonucleotide

<400>  1851
uggcucuggg ugggcguggc cugca                                          25


<210>  1852
<211>  25
<212>  RNA
<213>  Artificial Sequence

<220>
<223>  Description of Artificial Sequence: Synthetic oligonucleotide

<400>  1852
auaggcuccu uccugugggu ggggc                                          25


<210>  1853
<211>  25
<212>  RNA
<213>  Artificial Sequence

<220>
<223>  Description of Artificial Sequence: Synthetic oligonucleotide

<400>  1853
cuucucuggg accaggcucc aaugg                                          25


<210>  1854
<211>  25
<212>  RNA
<213>  Artificial Sequence

<220>
<223>  Description of Artificial Sequence: Synthetic oligonucleotide

<400>  1854
gggcggggcu ucucuccgcg ggugg                                          25


<210>  1855
<211>  25
<212>  RNA
<213>  Artificial Sequence

<220>
<223>  Description of Artificial Sequence: Synthetic oligonucleotide

<400>  1855
ggacucuucc cugggaaccg cccuc                                          25


<210>  1856
<211>  25

<212>  RNA
<213>  Artificial Sequence

<220>
<223>  Description of Artificial Sequence: Synthetic oligonucleotide

<400>  1856
ccugauuaag gcguggcuuc ugcag                                          25


<210>  1857
<211>  25
<212>  RNA
<213>  Artificial Sequence

<220>
<223>  Description of Artificial Sequence: Synthetic oligonucleotide

<400>  1857
ggaaucccgg cuccagagca ggaaa                                          25


<210>  1858
<211>  25
<212>  RNA
<213>  Artificial Sequence

<220>
<223>  Description of Artificial Sequence: Synthetic oligonucleotide

<400>  1858
auucagccca ccagccaccu caucc                                          25


<210>  1859
<211>  25
<212>  RNA
<213>  Artificial Sequence

<220>
<223>  Description of Artificial Sequence: Synthetic oligonucleotide

<400>  1859
cccaaccccc uguaagguuc caucc                                          25


<210>  1860
<211>  25
<212>  RNA
<213>  Artificial Sequence

<220>
<223>  Description of Artificial Sequence: Synthetic oligonucleotide

<400>  1860
caccccugcg ucgagcuggg aaggu                                          25


<210>  1861
<211>  25
<212>  RNA

<213>  Artificial Sequence

<220>
<223>  Description of Artificial Sequence: Synthetic oligonucleotide

<400>  1861
uuccaugaag cgagucgggu cccca                                          25


<210>  1862
<211>  25
<212>  RNA
<213>  Artificial Sequence

<220>
<223>  Description of Artificial Sequence: Synthetic oligonucleotide

<400>  1862
aacccgugcc ccugggaucc gaggg                                          25


<210>  1863
<211>  25
<212>  RNA
<213>  Artificial Sequence

<220>
<223>  Description of Artificial Sequence: Synthetic oligonucleotide

<400>  1863
gccccucucc aagcgccugg cuuug                                          25


<210>  1864
<211>  25
<212>  RNA
<213>  Artificial Sequence

<220>
<223>  Description of Artificial Sequence: Synthetic oligonucleotide

<400>  1864
ggaaugcucc aggcgcgccg acgcc                                          25


<210>  1865
<211>  25
<212>  RNA
<213>  Artificial Sequence

<220>
<223>  Description of Artificial Sequence: Synthetic oligonucleotide

<400>  1865
cugugccacc ccuuccucag ccugg                                          25


<210>  1866
<211>  25
<212>  RNA
<213>  Artificial Sequence

<220>
<223>  Description of Artificial Sequence: Synthetic oligonucleotide

<400>  1866
ggguuugacc acccaccuga ccagg                                          25


<210>  1867
<211>  25
<212>  RNA
<213>  Artificial Sequence

<220>
<223>  Description of Artificial Sequence: Synthetic oligonucleotide

<400>  1867
gggcccuacc uggggaaagc cugaa                                          25


<210>  1868
<211>  25
<212>  RNA
<213>  Artificial Sequence

<220>
<223>  Description of Artificial Sequence: Synthetic oligonucleotide

<400>  1868
agggccuccc agcccccaac cccaa                                          25


<210>  1869
<211>  25
<212>  RNA
<213>  Artificial Sequence

<220>
<223>  Description of Artificial Sequence: Synthetic oligonucleotide

<400>  1869
agaccaagcu uaguccuggg agagg                                          25


<210>  1870
<211>  25
<212>  RNA
<213>  Artificial Sequence

<220>
<223>  Description of Artificial Sequence: Synthetic oligonucleotide

<400>  1870
gacagggacu ucgcagaggc aagcg                                          25


<210>  1871
<211>  25
<212>  RNA
<213>  Artificial Sequence

<220>
<223>  Description of Artificial Sequence: Synthetic oligonucleotide

<400>  1871
gaccgaggcc cucccaaaga ggccc                                          25


<210>  1872
<211>  25
<212>  RNA
<213>  Artificial Sequence

<220>
<223>  Description of Artificial Sequence: Synthetic oligonucleotide

<400>  1872
cgcccugccc gggcucccac accgu                                          25


<210>  1873
<211>  25
<212>  RNA
<213>  Artificial Sequence

<220>
<223>  Description of Artificial Sequence: Synthetic oligonucleotide

<400>  1873
ucagguacuc cugccaggga acugg                                          25


<210>  1874
<211>  25
<212>  RNA
<213>  Artificial Sequence

<220>
<223>  Description of Artificial Sequence: Synthetic oligonucleotide

<400>  1874
gccugcugcg ccccaggccc cgccc                                          25


<210>  1875
<211>  25
<212>  RNA
<213>  Artificial Sequence

<220>
<223>  Description of Artificial Sequence: Synthetic oligonucleotide

<400>  1875
cgucucugcu cugcucagcu gcgcc                                          25


<210>  1876
<211>  25
<212>  RNA
<213>  Artificial Sequence

<220>

<223>  Description of Artificial Sequence: Synthetic oligonucleotide

<400>  1876
ccccuucuuu gcagcugccc agccc                                          25


<210>  1877
<211>  25
<212>  RNA
<213>  Artificial Sequence

<220>
<223>  Description of Artificial Sequence: Synthetic oligonucleotide

<400>  1877
ccuccucccu gcccucgggu cuccc                                          25


<210>  1878
<211>  25
<212>  RNA
<213>  Artificial Sequence

<220>
<223>  Description of Artificial Sequence: Synthetic oligonucleotide

<400>  1878
ccaccugcac uccauccagc uacag                                          25


<210>  1879
<211>  25
<212>  RNA
<213>  Artificial Sequence

<220>
<223>  Description of Artificial Sequence: Synthetic oligonucleotide

<400>  1879
ggagagauag aagccucucg ucccg                                          25


<210>  1880
<211>  25
<212>  RNA
<213>  Artificial Sequence

<220>
<223>  Description of Artificial Sequence: Synthetic oligonucleotide

<400>  1880
gucccucccu uuccuccgcc ugucc                                          25


<210>  1881
<211>  25
<212>  RNA
<213>  Artificial Sequence

<220>
<223>  Description of Artificial Sequence: Synthetic oligonucleotide

<400>  1881
cacagccccu uaagggaaag guagg                                          25


<210>  1882
<211>  25
<212>  RNA
<213>  Artificial Sequence

<220>
<223>  Description of Artificial Sequence: Synthetic oligonucleotide

<400>  1882
gaagagaggu ccagcccccc aggcu                                          25


<210>  1883
<211>  25
<212>  RNA
<213>  Artificial Sequence

<220>
<223>  Description of Artificial Sequence: Synthetic oligonucleotide

<400>  1883
ugcccagagc ugcuggucuc auuug                                          25


<210>  1884
<211>  25
<212>  RNA
<213>  Artificial Sequence

<220>
<223>  Description of Artificial Sequence: Synthetic oligonucleotide

<400>  1884
gggggcguuc gggagguuug ggggg                                          25


<210>  1885
<211>  25
<212>  RNA
<213>  Artificial Sequence

<220>
<223>  Description of Artificial Sequence: Synthetic oligonucleotide

<400>  1885
gcaucaaccc cccgacugug cugcu                                          25


<210>  1886
<211>  25
<212>  RNA
<213>  Artificial Sequence

<220>
<223>  Description of Artificial Sequence: Synthetic oligonucleotide

<400>  1886
ucgcgaaggu cccacagccc ugaga                                          25


<210>  1887
<211>  25
<212>  RNA
<213>  Artificial Sequence

<220>
<223>  Description of Artificial Sequence: Synthetic oligonucleotide

<400>  1887
augggccggc ccccuuccug gcccc                                          25


<210>  1888
<211>  25
<212>  RNA
<213>  Artificial Sequence

<220>
<223>  Description of Artificial Sequence: Synthetic oligonucleotide

<400>  1888
cucauggcgg gacuggagaa auggu                                          25


<210>  1889
<211>  25
<212>  RNA
<213>  Artificial Sequence

<220>
<223>  Description of Artificial Sequence: Synthetic oligonucleotide

<400>  1889
uccgcuuucc uggagccaau ggccc                                          25


<210>  1890
<211>  25
<212>  RNA
<213>  Artificial Sequence

<220>
<223>  Description of Artificial Sequence: Synthetic oligonucleotide

<400>  1890
cggccccucc ugacucaucc gccug                                          25


<210>  1891
<211>  25
<212>  RNA
<213>  Artificial Sequence

<220>
<223>  Description of Artificial Sequence: Synthetic oligonucleotide

<400>  1891

ggcccgggaa ugaaugggga ggccg                                          25

<210>  1892
<211>  25
<212>  RNA
<213>  Artificial Sequence

<220>
<223>  Description of Artificial Sequence: Synthetic oligonucleotide

<400>  1892
gcugaaccca cccggcccau auccc                                          25

<210>  1893
<211>  25
<212>  RNA
<213>  Artificial Sequence

<220>
<223>  Description of Artificial Sequence: Synthetic oligonucleotide

<400>  1893
cugguugccu cauggccagc gcccc                                          25

<210>  1894
<211>  25
<212>  RNA
<213>  Artificial Sequence

<220>
<223>  Description of Artificial Sequence: Synthetic oligonucleotide

<400>  1894
cucagccucu gccacuguga accgg                                          25

<210>  1895
<211>  25
<212>  RNA
<213>  Artificial Sequence

<220>
<223>  Description of Artificial Sequence: Synthetic oligonucleotide

<400>  1895
gcucccaccc ucaaggugcg gggag                                          25

<210>  1896
<211>  25
<212>  RNA
<213>  Artificial Sequence

<220>
<223>  Description of Artificial Sequence: Synthetic oligonucleotide

<400>  1896
gaagaagcgg ccaggcgggg cgccc                                          25

<210>  1897
<211>  25
<212>  RNA
<213>  Artificial Sequence

<220>
<223>  Description of Artificial Sequence: Synthetic oligonucleotide

<400>  1897
ccaagagccc aaaagagggc acacc                                          25


<210>  1898
<211>  25
<212>  RNA
<213>  Artificial Sequence

<220>
<223>  Description of Artificial Sequence: Synthetic oligonucleotide

<400>  1898
cgccauccuc ugccucaaau ucugc                                          25


<210>  1899
<211>  25
<212>  RNA
<213>  Artificial Sequence

<220>
<223>  Description of Artificial Sequence: Synthetic oligonucleotide

<400>  1899
cguuuuuggu uuuaauguua uaucu                                          25


<210>  1900
<211>  25
<212>  RNA
<213>  Artificial Sequence

<220>
<223>  Description of Artificial Sequence: Synthetic oligonucleotide

<400>  1900
ugaugcugcu auauccacug uccaa                                          25


<210>  1901
<211>  25
<212>  RNA
<213>  Artificial Sequence

<220>
<223>  Description of Artificial Sequence: Synthetic oligonucleotide

<400>  1901
ugcugcuaua uccacugucc aacgg                                          25

## Description

### CROSS-REFERENCE TO RELATED APPLICATIONS

The present application claims priority to U.S. Provisional Patent Application Nos. 60/934,940, filed March 2, 2007; and 60/934,930, filed March 16, 2007, each of which is incorporated by reference in its entirety.

### TECHNICAL FIELD

The present disclosure relates generally to compounds for use in treating hyperproliferative or degenerative disorders by gene silencing and, more specifically, to a nicked or gapped double-stranded RNA (dsRNA) comprising at least three strands that decreases expression of a Wingless-Type MMTV (Murine Mammary Tumor Virus) Integration Site (WNT) gene, and to uses of such dsRNA to treat or prevent cancer, schizophrenia, type II diabetes, or other disease associated with inappropriate WNT gene expression. The dsRNA that decreases WNT gene expression may optionally have at least one uridine that is a 5-methyluridine.

### BACKGROUND

RNA interference (RNAi) refers to the cellular process of sequence specific, post-transcriptional gene silencing in animals mediated by small inhibitory nucleic acid molecules, such as a double-stranded RNA (dsRNA) that is homologous to a portion of a targeted messenger RNA (Fire et al., Nature 391:806, 1998; Hamilton et al., Science 286:950, 1999). RNAi has been observed in a variety of organisms, including mammalians (Fire et al., Nature 391:806, 1998; Bahramian and Zarbl, Mol. Cell. Biol. 19:274, 1999; Wianny and Goetz, Nature Cell Biol. 2:70, 1999). RNAi can be induced by introducing an exogenous 21-nucleotide RNA duplex into cultured mammalian cells (Elbashir et al., Nature 411:494, 2001a).

The mechanism by which dsRNA mediates targeted gene-silencing can be described as involving two steps. The first step involves degradation of long dsRNAs by a ribonuclease III-like enzyme, referred to as Dicer, into short interfering RNAs (siRNAs) having from 21 to 23 nucleotides with double-stranded regions of about 19 base pairs and a two nucleotide, generally, overhang at each 3'-end (Berstein et al., Nature 409:363, 2001; Elbashir et al., Genes Dev. 15:188, 2001b; and Kim et al., Nature Biotech. 23:222, 2005). The second step of RNAi gene-silencing involves activation of a multi-component nuclease having one strand (guide or antisense strand) from the siRNA and an Argonaute protein to form an RNA-induced silencing complex ("RISC") (Elbashir et al., Genes Dev. 15:188, 2001). Argonaute initially associates with a double-stranded siRNA and then endonucleolytically cleaves the non-incorporated strand (passenger or sense strand) to facilitate its release due to resulting thermodynamic instability of the cleaved duplex (Leuschner et al., EMBO 7:314, 2006). The guide strand is now able to bind a complementary target mRNA and the activated RISC cleaves the mRNA to promote gene silencing. Cleavage of the target RNA occurs in the middle of the target region that is complementary to the guide strand (Elbashir *et al.,* 2001b).

Signaling proteins encoded by members of the Wingless-Type MMTV Integration Site (WNT) gene family (Wnt proteins) are a large family of secreted cysteine-rich glycoproteins that locally activate receptor-mediated signaling cascades *(*Luo et al., J. Lab. Inv. 87:97, 2007). Wingless-Type MMTV (Murine Mammary Tumor Virus) Integration Site Family, Member 1 gene (WNT1, also known as Oncogene INT1), Member 2 gene (WNT2) and Member 3A gene (WNT3A) are involved in the canonical Wtn/beta-catentin signaling pathway. The signaling cascade of these WNT gene family members is initiated via the Frizzled-LRP transmembrane receptor, and affects cell proliferation and differentiation, embryonic development, and other critical regulatory functions at a variety of cellular and ontogenetic levels and in a large variety of tissue types (Logan and Nusse, Ann. Rev. Cell Dev. Biol. 20:781, 2004; Giles et al., Biochemica et Biophysica Acta, 1653:1, 2003).

A variety of WTN inhibitors have been described. For example, secreted frizzled-related proteins (sFRPs) compete with the Fz proteins for binding to secreted Wnt ligands to inhibit Wtn protein function *(*Rattner et al., Proc. Nat'l Acad. Sci. U.S.A. 94:2859, 1997). Other inhibitors of Wtn protein activity include Wnt-inhibitory factor-1 (WIF-1), which binds to Wnt and prevents its association with its cell surface receptor *(*Hsieh et al., Nature 398:431, 1999). Another group of secreted antagonists includes the Dickkopf ("fat head") (Dkk) proteins, which interact with the Wnt co-receptor (LRP) to prevent formation of an active Wnt-Fz-LRP complex (Krupnik et al., Gene 238:301, 1999).

There continues to be a need for alternative effective therapeutic modalities useful for treating or preventing WNT1-, WNT2-, or WNT3A-associated diseases or disorders in which reduced WNT1, WNT2, or WNT3A gene expression (gene silencing) would be beneficial. The present disclosure meets such needs, and further provides other related advantages.

### BRIEF SUMMARY

Briefly, the present disclosure provides nicked or gapped double-stranded RNA (dsRNA) comprising at least three strands that is suitable as a substrate for Dicer or as a RISC activator to modify expression of a Wingless-Type MMTV (Murine Mammary Tumor Virus) Integration Site Family, Member 1, 2, or 3A (WNT1, WNT2, or WNT3A) gene messenger RNA (mRNA).

In one aspect, the instant disclosure provides a meroduplex mdRNA molecule, comprising a first strand that is complementary to any one of human WNT 1 mRNA as set forth in SEQ ID NO:1158, human WNT2 mRNA as set forth in SEQ ID NO:1390, or human WNT3A mRNA as set forth in SEQ ID NO:1615, and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands, and wherein (a) the mdRNA molecule optionally includes at least one double-stranded region of 5 base pairs to 13 base pairs, or (b) the double-stranded regions combined total about 15 base pairs to about 40 base pairs and the mdRNA molecule optinally has blunt ends. In certain embodiments, the first strand is about 15 to about 40 nucleotides in length, and the second and third strands are each, individually, about 5 to about 20 nucleotides, wherein the combined length of the second and third strands is about 15 nucleotides to about 40 nucleotides. In other embodiments, the first strand is about 15 to about 40 nucleotides in length and is complementary to at least about 15, 16, 17, 18, 19, 20, 21, 22, 23, 24, 25, 26, 27, 28, 29, 30, 31, 32, 33, 34, 35, 36, 37, 38, 39, or 40 contiguous nucleotides of a human WNT1 mRNA as set forth in SEQ ID NO:1158, human WNT2 mRNA as set forth in SEQ ID NO:1390, or human WNT3A mRNA as set forth in SEQ ID NO:1615. In still further embodiments, the first strand is about 15 to about 40 nucleotides in length and is at least about 70%, 75%, 80%, 85%, 90%, 91%, 92%, 93%, 94%, 95%, 96%, 97%, 98%, or 99% identical to a sequence that is complementary to at least about 15, 16, 17, 18, 19, 20, 21, 22, 23, 24, 25, 26, 27, 28, 29, 30, 31, 32, 33, 34, 35, 36, 37, 38, 39, or 40 contiguous nucleotides of a human WNT1 mRNA as set forth in SEQ ID NO:1158, human WNT2 mRNA as set forth in SEQ ID NO: 1390, or human WNT3A mRNA as set forth in SEQ ID NO:1615.

In other embodiments, the mdRNA is a RISC activator (e.g., the first strand has about 15 nucleotides to about 25 nucleotides) or a Dicer substrate (e.g., the first strand has about 26 nucleotides to about 40 nucleotides). In some embodiments, the gap comprises at least one to ten unpaired nucleotides in the first strand positioned between the double-stranded regions formed by the second and third strands when annealed to the first strand, or the gap is a nick. In certain embodiments, the nick or gap is located 10 nucleotides from the 5'-end of the first (antisense) strand or at the Argonaute cleavage site. In another embodiment, the meroduplex nick or gap is positioned such that the thermal stability is maximized for the first and second strand duplex and for the first and third strand duplex as compared to the thermal stability of such meroduplexes having a nick or gap in a different position.

In another aspect, the instant disclosure provides an mdRNA molecule having a first strand that is complementary to human WNT1 mRNA as set forth in SEQ ID NO:1158, WNT2 mRNA as set forth in SEQ ID NO: 1390, or WNT3A mRNA as set forth in SEQ ID NO:1615, and a second strand and a third strand that is each complementary to non-overlapping regions of the first strand, wherein the second strand and third strand can anneal with the first strand to form at least two double-stranded regions spaced apart by up to 10 nucleotides and thereby forming a gap between the second and third strands, and wherein (a) the mdRNA molecule optionally includes at least one double-stranded region of 5 base pairs to 13 base pairs, or (b) the double-stranded regions combined total about 15 base pairs to about 40 base pairs and the mdRNA molecule optinally has blunt ends; and wherein at least one pyrimidine nucleoside of the mdRNA is according to Formula I or II: 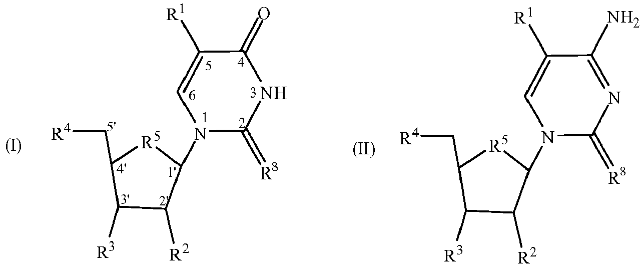 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C=N, or heterocyclo group; R³ and R⁴ are each independently a hydroxyl, a protected hydroxyl, a phosphate, or an internucleoside linking group; and R⁵ and R⁸ are independently O or S. In certain embodiments, at least one nucleoside is according to Formula I in which R¹ is methyl and R² is -OH. In certain related embodiments, at least one uridine of the dsRNA molecule is replaced with a nucleoside according to Formula I in which R¹ is methyl and R² is - OH, or R¹ is methyl, R² is -OH, and R⁸ is S. In some embodiments, the at least one R¹ is a C₁-C₅ alkyl, such as methyl. In some embodiments, at least one R² is selected from 2'-O-(C₁-C₅) alkyl, 2'-O-methyl, 2'-OCH₂OCH₂CH₃, 2'-OCH₂CH₂OCH₃, 2'-O-allyl, or fluoro. In some embodiments, at least one pyrimidine nucleoside of the mdRNA molecule is a locked nucleic acid (LNA) in the form of a bicyclic sugar, wherein R² is oxygen, and the 2'-O and 4'-C form an oxymethylene bridge on the same ribose ring (*e.g*., a 5-methyluridine LNA) or is a G clamp. In other embodiments, one or more of the nucleosides are according to Formula I in which R¹ is methyl and R² is a 2'-O-(C₁-C₅) alkyl, such as 2'-O-methyl. In some embodiments, the gap comprises at least one unpaired nucleotide in the first strand positioned between the double-stranded regions formed by the second and third strands when annealed to the first strand, or the gap is a nick. In certain embodiments, the nick or gap is located 10 nucleotides from the 5'-end of the first strand or at the Argonaute cleavage site. In another embodiment, the meroduplex nick or gap is positioned such that the thermal stability is maximized for the first and second strand duplex and for the first and third strand duplex as compared to the thermal stability of such meroduplexes having a nick or gap in a different position.

In still another aspect, the instant disclosure provides a method for reducing the expression of a human WNT1, WNT2, or WNT3A gene in a cell, comprising administering an mdRNA molecule to a cell expressing a WNT1, WNT2, or WNT3A gene, wherein the mdRNA molecule is capable of specifically binding to a WNT1, WNT2, or WNT3A mRNA and thereby reducing the gene's level of expression in the cell. In a related aspect, there is provided a method of treating or preventing a disease associated with WNT1, WNT2, or WNT3A expression in a subject by administering an mdRNA molecule of this disclosure. In certain embodiments, the cell or subject is human. In certain embodiments, the disease is atherosclerosis, diabetes mellitus, or cerebrovascular disease.

In any of the aspects of this disclosure, some embodiments provide an mdRNA molecule having a 5-methyluridine (ribothymidine), 2-thioribothymidine, or 2'-O-methyl-5-methyluridine in place of at least one uridine on the first, second, or third strand, or in place of each and every uridine on the first, second, or third strand. In further embodiments, the mdRNA further comprises one or more non-standard nucleoside, such as a deoxyuridine, locked nucleic acid (LNA) molecule, or a universal-binding nucleotide, or a G clamp. Exemplary universal-binding nucleotides include C-phenyl, C-naphthyl, inosine, azole carboxamide, 1-β-D-ribofuranosyl-4-nitroindole, 1-β-D-ribofuranosyl-5-nitroindole, 1-β-D-ribofuranosyl-6-nitroindole, or 1-β-D-ribofuranosyl-3-nitropyrrole. In some embodiments, the mdRNA molecule further comprises a 2'-sugar substitution, such as a 2'-O-methyl, 2'-O-methoxyethyl, 2'-O-2-methoxyethyl, 2'-O-allyl, or halogen (*e.g*., 2'-fluoro). In certain embodiments, the mdRNA molecule further comprises a terminal cap substituent on one or both ends of one or more of the first strand, second strand, or third strand, such as independently an alkyl, abasic, deoxy abasic, glyceryl, dinucleotide, acyclic nucleotide, or inverted deoxynucleotide moiety. In other embodiments, the mdRNA molecule further comprises at least one modified internucleoside linkage, such as independently a phosphorothioate, chiral phosphorothioate, phosphorodithioate, phosphotriester, aminoalkylphosphotriester, methyl phosphonate, alkyl phosphonate, 3'-alkylene phosphonate, 5'-alkylene phosphonate, chiral phosphonate, phosphonoacetate, thiophosphonoacetate, phosphinate, phosphoramidate, 3'-amino phosphoramidate, aminoalkylphosphoramidate, selenophosphate, thionophosphoramidate, thionoalkylphosphonate, thionoalkylphosphotriester, or boranophosphate linkage.

In any aspect of this disclosure, some embodiments provide an mdRNA comprising an overhang of one to four nucleotides on at least one 3'-end that is not part of the gap, such as at least one deoxynucleotide or two deoxynucleotides (*e.g*., thymidine). In some embodiments, at least one or two 5'-terminal ribonucleotide of the second strand within the double-stranded region comprises a 2'-sugar substitution. In related embodiments, at least one or two 5'-terminal ribonucleotide of the first strand within the double-stranded region comprises a 2'-sugar substitution. In other related embodiments, at least one or two 5'-terminal ribonucleotide of the second strand and at least one or two 5'-terminal ribonucleotide of the first strand within the double-stranded regions comprise independent 2'-sugar substitutions. In other embodiments, the mdRNA molecule comprises at least three 5-methyluridines within at least one double-stranded region. In some embodiments, the mdRNA molecule has a blunt end at one or both ends. In other embodiments, the 5'-terminal of the third strand is a hydroxyl or a phosphate.

### BRIEF DESCRIPTION OF THE DRAWINGS

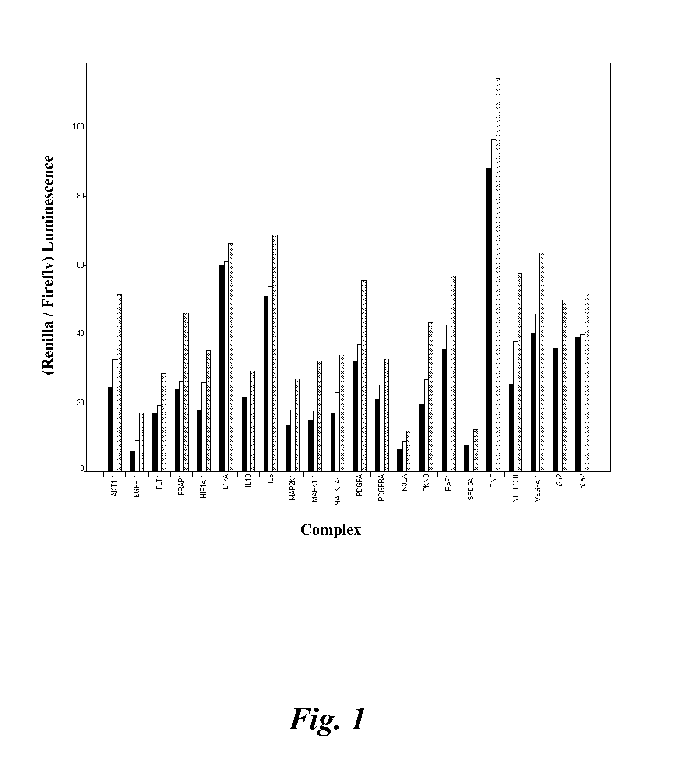

Figure 1 shows the average gene silencing activity of intact (first bar), nicked (middle bar), and gapped (last bar) dsRNA Dicer substrate specific for each of 22 different targets (AKT, EGFR, FLT1, FRAP1, HIF1A, IL17A, IL18, IL6, MAP2K1, MAPK1, MAPK14, PDGFA, PDGFRA, PIKC3A, PKN3, RAF1, SRD5A1, TNF, TNFSF13B, VEGFA, BCR-ABL [b2a2], and BCR-ABL [b3a2]). Each bar is a graphical representation of an average activity of ten different sequences for each target, which is calculated from the data found in Table 1.

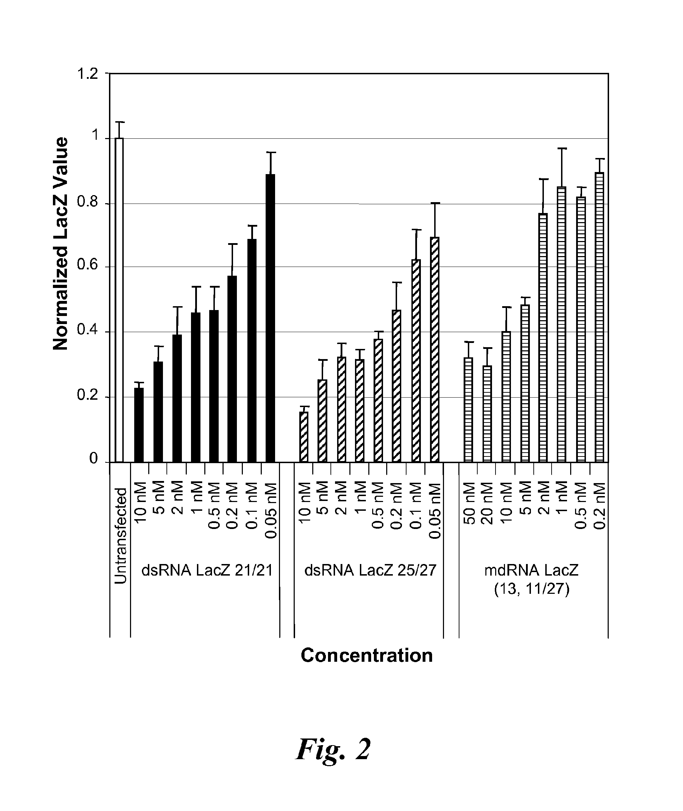

Figure 2 shows knockdown activity for RISC activator lacZ dsRNA (21 nucleotide sense strand/21 nucleotide antisense strand; 21/21), Dicer substrate lacZ dsRNA (25 nucleotide sense strand/27 nucleotide antisense strand; 25/27), and meroduplex lacZ mdRNA (13 nucleotide sense strand and 11 nucleotide sense strand/27 nucleotide antisense strand; 13, 11/27 - the sense strand is missing one nucleotide so that a single nucleotide gap is left between the 13 nucleotide and 11 nucleotide sense strands when annealed to the 27 nucleotide antisense strand. Knockdown activities were normalized to a Qneg control dsRNA and presented as a normalized value of Qneg *(i.e.,* Qneg represents 100% or "normal" gene expression levels). A smaller value indicates a greater knockdown effect.

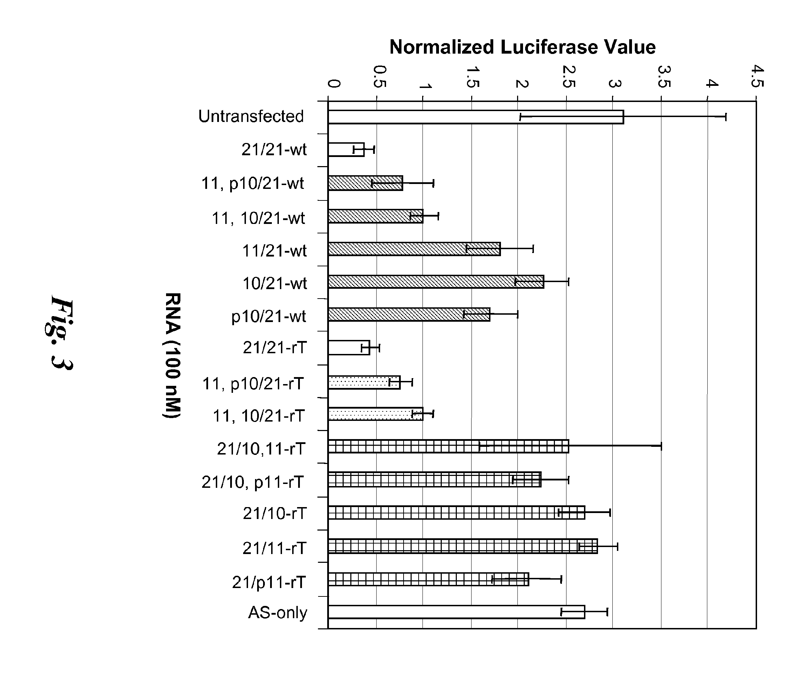

Figure 3 shows knockdown activity of a RISC activator influenza dsRNA G1498 (21/21) and nicked dsRNA (10, 11/21) at 100 nM. The "wt" designation indicates an unsubstituted RNA molecule; "rT" indicates RNA having each uridine substituted with a ribothymidine; and "p" indicates that the 5'-nucleotide of that strand was phosphorylated. The 21 nucleotide sense and antisense strands of G1498 were individually nicked between the nucleotides 10 and 11 as measured from the 5'-end, and is referred to as 11, 10/21 and 21/10, 11, respectively. The G1498 single stranded 21 nucleotide antisense strand alone (designated AS-only) was used as a control.
Figure 4 shows knockdown activity of a lacZ dicer substrate (25/27) having a nick in one of each of positions 8 to 14 and a one nucleotide gap at position 13 of the sense strand (counted from the 5'-end). A dideoxy guanosine (ddG) was incorporated at the 5'-end of the 3'-most strand of the nicked or gapped sense sequence at position 13.
Figure 5 shows knockdown activity of a dicer substrate influenza dsRNA G1498DS (25/27) and this sequence nicked at one of each of positions 8 to 14 of the sense strand, and shows the activity of these nicked molecules that are also phosphorylated or have a locked nucleic acid substitution.
Figure 6 shows a dose dependent knockdown activity a dicer substrate influenza dsRNA G1498DS (25/27) and this sequence nicked at position 13 of the sense strand.
Figure 7 shows knockdown activity of a dicer substrate influenza dsRNA G1498DS having a nick or a gap of one to six nucleotides that begins at any one of positions 8 to 12 of the sense strand.
Figure 8 shows knockdown activity of a LacZ RISC dsRNA having a nick or a gap of one to six nucleotides that begins at any one of positions 8 to 14 of the sense strand.
Figure 9 shows knockdown activity of an influenza RISC dsRNA having a nick at any one of positions 8 to 14 of the sense strand and further having one or two locked nucleic acids (LNA) per sense strand. The inserts on the right side of the graph provides a graphic depiction of the meroduplex structures (for clarity, a single antisense strand is shown at the bottom of the grouping with each of the different nicked sense strands above the antisense) having different nick positions with the relative positioning of the LNAs on the sense strands.
Figure 10 shows knockdown activity of a LacZ dicer substrate dsRNA having a nick at any one of positions 8 to 14 of the sense strand as compared to the same nicked dicer substrates but having a locked nucleic acid substitution.
Figure 11 shows the percent knockdown in influenza viral titers using influenza specific mdRNA against influenza strain WSN.
Figure 12 shows the *in vivo* reduction in PR8 influenza viral titers using influenza specific mdRNA as measured by TCID₅₀.

### DETAILED DESCRIPTION

The instant disclosure is predicated upon the unexpected discovery that a nicked or gapped double-stranded RNA (dsRNA) comprising at least three strands is a suitable substrate for Dicer or RISC and, therefore, may be advantageously employed for gene silencing via, for example, the RNA interference pathway. That is, partially duplexed dsRNA molecules described herein (also referred to as meroduplexes having a nick or gap in at least one strand) are capable of initiating an RNA interference cascade that modifies (e.g., reduces) expression of a target messenger RNA (mRNA), such as a human Wingless-Type MMTV (Murine Mammary Tumor Virus) Integration Site Family, Member 1 (WNT1), or a human Wingless-Type MMTV (Murine Mammary Tumor Virus) Integration Site Family, Member 2 (WNT2), or a human Wingless-Type MMTV (Murine Mammary Tumor Virus) Integration Site Family, Member 3A (WNT3A) mRNA. This is surprising because a person of skill in the art would expect the thermodynamically less stable nicked or gapped dsRNA passenger strand (as compared to an intact dsRNA) to fall apart before any gene silencing effect would result *(see, e.g.,* Leuschner et al., EMBO 7:314, 2006).

Meroduplex ribonucleic acid (mdRNA) molecules described herein include a first (antisense) strand that is complementary to a human WNT1 mRNA as set forth in SEQ ID NO:1158, WNT2 mRNA as set forth in SEQ ID NO:1390, or WNT3A mRNA as set forth in SEQ ID NO:1615, along with second and third strands (together forming a gapped sense strand) that are each complementary to non-overlapping regions of the first strand, wherein the second and third strands can anneal with the first strand to form at least two double-stranded regions separated by a gap, and wherein at least one double-stranded region is optionally from about 5 base pairs to about 15 base pairs, or the combined double-stranded regions total about 5 base pairs to about 40 base pairs and the mdRNA is blunt-ended. The gap can be from 0 nucleotides *(i.e.,* a nick in which only a phosphodiester bond between two nucleotides is broken in a polynucleotide molecule) up to about 10 nucleotides (*i.e*., the first strand will have at least one unpaired nucleotide). In certain embodiments, the nick or gap is located 10 nucleotides from the 5'-end of the first (antisense) strand or at the Argonaute cleavage site. In another embodiment, the meroduplex nick or gap is positioned such that the thermal stability is maximized for the first and second strand duplex and for the first and third strand duplex as compared to the thermal stability of such meroduplexes having a nick or gap in a different position. Also provided herein are methods of using such dsRNA to reduce expression of a WNT1, WNT2, or WNT3A gene in a cell or to treat or prevent diseases or disorders associated with WNT1, WNT2, or WNT3A gene expression, including cardiovascular disease (*e.g*., venous malformation) or cancer.

Prior to introducing more detail to this disclosure, it may be helpful to an appreciation thereof to provide definitions of certain terms to be used herein.

In the present description, any concentration range, percentage range, ratio range, or integer range is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated. As used herein, "about" or "consisting essentially of" mean ± 20% of the indicated range, value, or structure, unless otherwise indicated. As used herein, the terms "include" and "comprise" are open ended and are used synonymously. It should be understood that the terms "a" and "an" as used herein refer to "one or more" of the enumerated components. The use of the alternative (*e.g*., "or") should be understood to mean either one, both, or any combination thereof of the alternatives.

As used herein, the term "isolated" means that the referenced material (e.g., nucleic acid molecules of the instant disclosure), is removed from its original environment, such as being separated from some or all of the co-existing materials in a natural environment (e.g., a natural environment may be a cell).

As used herein, "complementary" refers to a nucleic acid molecule that can form hydrogen bond(s) with another nucleic acid molecule or itself by either traditional Watson-Crick base pairing or other non-traditional types of pairing (*e.g*., Hoogsteen or reversed Hoogsteen hydrogen bonding) between complementary nucleosides or nucleotides. In reference to the nucleic molecules of the present disclosure, the binding free energy for a nucleic acid molecule with its complementary sequence is sufficient to allow the relevant function of the nucleic acid molecule to proceed, for example, RNAi activity, and there is a sufficient degree of complementarity to avoid non-specific binding of the nucleic acid molecule (*e.g*., dsRNA) to non-target sequences under conditions in which specific binding is desired, *i.e.,* under physiological conditions in the case of *in vivo* assays or therapeutic treatment, or under conditions in which the assays are performed in the case of *in vitro* assays (*e.g*., hybridization assays). Determination of binding free energies for nucleic acid molecules is well known in the art *(see, e.g.,* Turner et al., CSH Symp. Quant. Biol. LII:123, 1987; Frier et al., Proc. Nat'l. Acad. Sci. USA 83:9373, 1986; Turner et al., J. Am. Chem. Soc. 109:3783, 1987). Thus, "complementary" or "specifically hybridizable" or "specifically binds" are terms that indicate a sufficient degree of complementarity or precise pairing such that stable and specific binding occurs between a nucleic acid molecule (*e.g*., dsRNA) and a DNA or RNA target. It is understood in the art that a nucleic acid molecule need not be 100% complementary to a target nucleic acid sequence to be specifically hybridizable or to specifically bind. That is, two or more nucleic acid molecules may be less than fully complementary and is indicated by a percentage of contiguous residues in a nucleic acid molecule that can form hydrogen bonds with a second nucleic acid molecule.

For example, a first nucleic acid molecule may have 10 nucleotides and a second nucleic acid molecule may have 10 nucleotides, then base pairing of 5, 6, 7, 8, 9, or 10 nucleotides between the first and second nucleic acid molecules, which may or may not form a contiguous double-stranded region, represents 50%, 60%, 70%, 80%, 90%, and 100% complementarity, respectively. In certain embodiments, complementary nucleic acid molecules may have wrongly paired bases - that is, bases that cannot form a traditional Watson-Crick base pair or other non-traditional types of pair (*i.e*., "mismatched" bases). For instance, complementary nucleic acid molecules may be identified as having a certain number of "mismatches," such as zero or about 1, about 2, about 3, about 4 or about 5.

"Perfectly" or "fully" complementary nucleic acid molecules means those in which a certain number of nucleotides of a first nucleic acid molecule hydrogen bond (anneal) with the same number of residues in a second nucleic acid molecule to form a contiguous double-stranded region. For example, two or more fully complementary nucleic acid molecule strands can have the same number of nucleotides (*i.e*., have the same length and form one double-stranded region, with or without an overhang) or have a different number of nucleotides (*e.g*., one strand may be shorter than but fully contained within another strand or one strand may overhang the other strand).

By "ribonucleic acid" or "RNA" is meant a nucleic acid molecule comprising at least one ribonucleotide molecule. As used herein, "ribonucleotide" refers to a nucleotide with a hydroxyl group at the 2'-position of a β-D-ribofuranose moiety. The term RNA includes double-stranded (ds) RNA, single-stranded (ss) RNA, isolated RNA (such as partially purified RNA, essentially pure RNA, synthetic RNA, recombinantly produced RNA), altered RNA (which differs from naturally occurring RNA by the addition, deletion, substitution or alteration of one or more nucleotides), or any combination thereof. For example, such altered RNA can include addition of non-nucleotide material, such as at one or both ends of an RNA molecule, internally at one or more nucleotides of the RNA, or any combination thereof. Nucleotides in RNA molecules of the instant disclosure can also comprise non-standard nucleotides, such as naturally occurring nucleotides, non-naturally occurring nucleotides, chemically-modified nucleotides, deoxynucleotides, or any combination thereof. These altered RNAs may be referred to as analogs or analogs of RNA containing standard nucleotides (*i.e*., standard nucleotides, as used herein, are considered to be adenine, cytidine, guanidine, thymidine, and uridine).

The term "dsRNA" as used herein, which is interchangeable with "mdRNA," refers to any nucleic acid molecule comprising at least one ribonucleotide molecule and capable of inhibiting or down regulating gene expression, for example, by promoting RNA interference ("RNAi") or gene silencing in a sequence-specific manner. The dsRNAs (mdRNAs) of the instant disclosure may be suitable substrates for Dicer or for association with RISC to mediate gene silencing by RNAi. Examples of dsRNA molecules of this disclosure are provided in the Sequence Listing identified herein. One or both strands of the dsRNA can further comprise a terminal phosphate group, such as a 5'-phosphate or 5', 3'-diphosphate. As used herein, dsRNA molecules, in addition to at least one ribonucleotide, can further include substitutions, chemically-modified nucleotides, and non-nucleotides. In certain embodiments, dsRNA molecules comprise ribonucleotides up to about 100% of the nucleotide positions.

In addition, as used herein, the term dsRNA is meant to be equivalent to other terms used to describe nucleic acid molecules that are capable of mediating sequence specific RNAi, for example, meroduplex RNA (mdRNA), nicked dsRNA (ndsRNA), gapped dsRNA (gdsRNA), short interfering nucleic acid (siNA), siRNA, micro-RNA (miRNA), short hairpin RNA (shRNA), short interfering oligonucleotide, short interfering substituted oligonucleotide, short interfering modified oligonucleotide, chemically-modified dsRNA, post-transcriptional gene silencing RNA (ptgsRNA), or the like. The term "large double-stranded RNA" ("large dsRNA") refers to any double-stranded RNA longer than about 40 base pairs (bp) to about 100 bp or more, particularly up to about 300 bp to about 500 bp. The sequence of a large dsRNA may represent a segment of an mRNA or an entire mRNA. A double-stranded structure may be formed by a self-complementary nucleic acid molecule or by annealing of two or more distinct complementary nucleic acid molecule strands.

In one aspect, a dsRNA comprises two separate oligonucleotides, comprising a first strand (antisense) and a second strand (sense), wherein the antisense and sense strands are self-complementary (*i.e*., each strand comprises a nucleotide sequence that is complementary to a nucleotide sequence in the other strand and the two separate strands form a duplex or double-stranded structure, for example, wherein the double-stranded region is about 15 to about 24 base pairs or about 26 to about 40 base pairs); the antisense strand comprises a nucleotide sequence that is complementary to a nucleotide sequence in a target nucleic acid molecule or a portion thereof (*e.g*., a human WNT1 mRNA of SEQ ID NO: 1158, WNT2 mRNA as set forth in SEQ ID NO: 1390, or WNT3A mRNA as set forth in SEQ ID NO:1615); and the sense strand comprises a nucleotide sequence corresponding (*i.e*., homologous) to the target nucleic acid sequence or a portion thereof (*e.g*., a sense strand of about 15 to about 25 nucleotides or about 26 to about 40 nucleotides corresponds to the target nucleic acid or a portion thereof).

In another aspect, the dsRNA is assembled from a single oligonucleotide in which the self-complementary sense and antisense strands of the dsRNA are linked together by a nucleic acid based-linker or a non-nucleic acid-based linker. In certain embodiments, the first (antisense) and second (sense) strands of the dsRNA molecule are covalently linked by a nucleotide or non-nucleotide linker as described herein and known in the art. In other embodiments, a first dsRNA molecule is covalently linked to at least one second dsRNA molecule by a nucleotide or non-nucleotide linker known in the art, wherein the first dsRNA molecule can be linked to a plurality of other dsRNA molecules that can be the same or different, or any combination thereof. In another embodiment, the linked dsRNA may include a third strand that forms a meroduplex with the linked dsRNA.

In still another aspect, dsRNA molecules described herein form a meroduplex RNA (mdRNA) having three or more strands such as, for example, an'A' (first or antisense) strand, 'S1' (second) strand, and 'S2' (third) strand in which the 'S1' and 'S2' strands are complementary to and form base pairs (bp) with non-overlapping regions of the'A' strand (*e.g*., an mdRNA can have the form of A:S1S2). The double-stranded region formed by the annealing of the 'S1 and 'A' strands is distinct from and non-overlapping with the double-stranded region formed by the annealing of the 'S2' and 'A' strands. An mdRNA molecule is a "gapped" molecule, *i.e.,* it contains a "gap" ranging from 0 nucleotides up to about 10 nucleotides (or a gap of 11, 12, 13, 14, 15, 16, 17, 18, 19, 20, 21, 22, 23, 24, 25, 26, 27, 28, 29, 30, 31, 32, 33, 34 or 35 nucleotides). In one embodiment, the A:S1 duplex is separated from the A:S2 duplex by a gap resulting from at least one unpaired nucleotide (up to about 10 unpaired nucleotides) in the 'A' strand that is positioned between the A:S1 duplex and the A: S2 duplex and that is distinct from any one or more unpaired nucleotide at the 3'-end of one or more of the 'A', 'S1', or 'S2' strands. In another embodiment, the A:S1 duplex is separated from the A:S2 duplex by a gap of zero nucleotides *(i.e.,* a nick in which only a phosphodiester bond between two nucleotides is broken or missing in the polynucleotide molecule) between the A:S1 duplex and the A:S2 duplex - which can also be referred to as nicked dsRNA (ndsRNA). For example, A:S1S2 may be comprised of a dsRNA having at least two double-stranded regions that combined total about 14 base pairs to about 40 base pairs and the double-stranded regions are separated by a gap of 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 nucleotides, optionally having blunt ends, or A:S1S2 may comprise a dsRNA having at least two double-stranded regions spaced apart by up to 10 nucleotides and thereby forming a gap between the second and third strands wherein at least one of the double-stranded regions optionally has from 5 base pairs to 13 base pairs.

A dsRNA or large dsRNA may include a substitution or modification in which the substitution or modification may be in a phosphate backbone bond, a sugar, a base, or a nucleoside. Such nucleoside substitutions can include natural non-standard nucleosides (*e.g*., 5-methyluridine or 5-methylcytidine or a 2-thioribothymidine), and such backbone, sugar, or nucleoside modifications can include an alkyl or heteroatom substitution or addition, such as a methyl, alkoxyalkyl, halogen, nitrogen or sulfur, or other modifications known in the art.

In addition, as used herein, the term "RNAi" is meant to be equivalent to other terms used to describe sequence specific RNA interference, such as post transcriptional gene silencing, translational inhibition, or epigenetics. For example, dsRNA molecules of this disclosure can be used to epigenetically silence genes at the post-transcriptional level or the pre-transcriptional level or any combination thereof.

As used herein, "target nucleic acid" refers to any nucleic acid sequence whose expression or activity is to be altered (*e.g*., WNT1, WNT2, or WNT3A). The target nucleic acid can be DNA, RNA, or analogs thereof, and includes single, double, and multi-stranded forms. By "target site" or "target sequence" is meant a sequence within a target nucleic acid (*e.g*., mRNA) that, when present in an RNA molecule, is "targeted" for cleavage by RNAi and mediated by a dsRNA construct of this disclosure containing a sequence within the antisense strand that is complementary to the target site or sequence.

As used herein, "off-target effect" or "off-target profile" refers to the observed altered expression pattern of one or more genes in a cell or other biological sample not targeted, directly or indirectly, for gene silencing by an mdRNA or dsRNA. For example, an off-target effect can be quantified by using a DNA microarray to determine how many non-target genes have an expression level altered by about two-fold or more in the presence of a candidate mdRNA or dsRNA, or analog thereof specific for a target sequence, such as a WNT1, WNT2, or WNT3A mRNA. A "minimal off-target effect" means that an mdRNA or dsRNA affects expression by about two-fold or more of about 25% to about 1% of the non-target genes examined or it means that the off-target effect of substituted or modified mdRNA or dsRNA (*e.g*., having at least one uridine substituted with a 5-methyluridine or 2-thioribothymidine and optionally having at least one nucleotide modified at the 2'-position), is reduced by at least about 1% to about 80% or more as compared to the effect on non-target genes of an unsubstituted or unmodified mdRNA or dsRNA.

By "sense region" or "sense strand" is meant one ore more nucleotide sequences of a dsRNA molecule having complementarity to one or more antisense regions of the dsRNA molecule. In addition, the sense region of a dsRNA molecule comprises a nucleic acid sequence having homology or identity to a target sequence, such as WNT1, WNT2, or WNT3A. By "antisense region" or "antisense strand" is meant a nucleotide sequence of a dsRNA molecule having complementarity to a target nucleic acid sequence, such as WNT1, WNT2, or WNT3A. In addition, the antisense region of a dsRNA molecule can comprise nucleic acid sequence region having complementarity to one or more sense strands of the dsRNA molecule.

"Analog" as used herein refers to a compound that is structurally similar to a parent compound (*e.g*., a nucleic acid molecule), but differs slightly in composition (*e.g*., one atom or functional group is different, added, or removed). The analog may or may not have different chemical or physical properties than the original compound and may or may not have improved biological or chemical activity. For example, the analog may be more hydrophilic or it may have altered activity as compared to a parent compound. The analog may mimic the chemical or biological activity of the parent compound (*i.e*., it may have similar or identical activity), or, in some cases, may have increased or decreased activity. The analog may be a naturally or non-naturally occurring (*e.g*., chemically-modified or recombinant) variant of the original compound. An example of an RNA analog is an RNA molecule having a non-standard nucleotide, such as 5-methyuridine or 5-methylcytidine or 2-thioribothymidine, which may impart certain desirable properties (*e.g*., improve stability, bioavailability, minimize off-target effects or interferon response).

As used herein, the term "universal base" refers to nucleotide base analogs that form base pairs with each of the standard DNA/RNA bases with little discrimination between them. A universal base is thus interchangeable with all of the standard bases when substituted into a nucleotide duplex *(see, e.g.,* Loakes et al., J. Mol. Bio. 270:426, 1997). Examplary universal bases include C-phenyl, C-naphthyl and other aromatic derivatives, inosine, azole carboxamides, or nitroazole derivatives such as 3-nitropyrrole, 4-nitroindole, 5-nitroindole, and 6-nitroindole *(see, e.g.,* Loakes, Nucleic Acids Res. 29:2437, 2001).

The term "gene" as used herein, especially in the context of "target gene" or "gene target" for RNAi, means a nucleic acid molecule that encodes an RNA or a transcription product of such gene, including a messenger RNA (mRNA, also referred to as structural genes that encode for a polypeptide), an mRNA splice variant of such gene, a functional RNA (fRNA), or non-coding RNA (ncRNA), such as small temporal RNA (stRNA), microRNA (miRNA), small nuclear RNA (snRNA), short interfering RNA (siRNA), small nucleolar RNA (snRNA), ribosomal RNA (rRNA), transfer RNA (tRNA) and precursor RNAs thereof. Such non-coding RNAs can serve as target nucleic acid molecules for dsRNA mediated RNAi to alter the activity of the target RNA involved in functional or regulatory cellular processes.

As used herein, "gene silencing" refers to a partial or complete loss-of-function through targeted inhibition of gene expression in a cell, which may also be referred to as RNAi "knockdown," "inhibition," "down-regulation," or "reduction" of expression of a target gene, such as a human WNT1, WNT2, or WNT3A gene. Depending on the circumstances and the biological problem to be addressed, it may be preferable to partially reduce gene expression. Alternatively, it might be desirable to reduce gene expression as much as possible. The extent of silencing may be determined by methods described herein and known in the art *(see, e.g.,* PCT Publication No. WO 99/32619; Elbashir et al., EMBO J. 20:6877, 2001). Depending on the assay, quantification of gene expression permits detection of various amounts of inhibition that may be desired in certain embodiments of this disclosure, including prophylactic and therapeutic methods, which will be capable of knocking down target gene expression, in terms of mRNA level or protein level or activity, for example, by equal to or greater than 10%, 30%, 50%, 75% 90%, 95% or 99% of baseline (*i.e*., normal) or other control levels, including elevated expression levels as may be associated with particular disease states or other conditions targeted for therapy.

As used herein, the term "therapeutically effective amount" means an amount of dsRNA that is sufficient to result in a decrease in severity of disease symptoms, an increase in frequency or duration of disease symptom-free periods, or a prevention of impairment or disability due to the disease, in the subject (*e.g*., human) to which it is administered. For example, a therapeutically effective amount of dsRNA directed against an mRNA of WNT1, WNT2, or WNT3A (*e.g*., SEQ ID NO:1158, SEQ ID NO:1390, or SEQ ID NO:1615, respectively) can inhibit the deposition of lipoproteins in the walls of arteries by at least about 20%, at least about 40%, at least about 60%, or at least about 80% relative to untreated subjects. A therapeutically effective amount of a therapeutic compound can decrease, for example, atheromatous plaque size or otherwise ameliorate symptoms in a subject. One of ordinary skill in the art would be able to determine such therapeutically effective amounts based on such factors as the subject's size, the severity of symptoms, and the particular composition or route of administration selected. The nucleic acid molecules of the instant disclosure, individually, or in combination or in conjunction with other drugs, can be used to treat diseases or conditions discussed herein. For example, to treat a particular disease, disorder, or condition, the dsRNA molecules can be administered to a patient or can be administered to other appropriate cells evident to those skilled in the art, individually or in combination with one or more drugs, under conditions suitable for treatment.

In addition, one or more dsRNA may be used to knockdown expression of a WNT1 mRNA as set forth in SEQ ID NO:1158, WNT2 mRNA as set forth in SEQ ID NO: 1390, or WNT3A mRNA as set forth in SEQ ID NO:1615, or a related mRNA splice variant. In this regard it is noted that a WNT1, WNT2, or WNT3A gene may be transcribed into two or more mRNA splice variants; and thus, for example, in certain embodiments, knockdown of one mRNA splice variant without affecting the other mRNA splice variant may be desired, or vice versa; or knockdown of all transcription products may be targeted.

In addition, it should be understood that the individual compounds, or groups of compounds, derived from the various combinations of the structures and substituents described herein, are disclosed by the present application to the same extent as if each compound or group of compounds was set forth individually. Thus, selection of particular structures or particular substituents is within the scope of the present disclosure. As described herein, all value ranges are inclusive over the indicated range. Thus, a range of C₁-C₄ will be understood to include the values of 1, 2, 3, and 4, such that C₁, C₂, C₃ and C₄ are included.

The term "alkyl" as used herein refers to saturated straight- or branched-chain aliphatic groups containing from 1-20 carbon atoms, preferably 1-8 carbon atoms and most preferably 1-4 carbon atoms. This definition applies as well to the alkyl portion of alkoxy, alkanoyl and aralkyl groups. The alkyl group may be substituted or unsubstituted. In certain embodiments, the alkyl is a (C₁-C₄) alkyl or methyl.

The term "cycloalkyl" as used herein refers to a saturated cyclic hydrocarbon ring system containing from 3 to 12 carbon atoms that may be optionally substituted. Exemplary embodiments include, but are not limited to, cyclopropyl, cyclobutyl, cyclopentyl and cyclohexyl. In certain embodiments, the cycloalkyl group is cyclopropyl. In another embodiment, the (cycloalkyl)alkyl groups contain from 3 to 12 carbon atoms in the cyclic portion and 1 to 6 carbon atoms in the alkyl portion. In certain embodiments, the (cycloalkyl)alkyl group is cyclopropylmethyl. The alkyl groups are optionally substituted with from one to three substituents selected from the group consisting of halogen, hydroxy and amino.

The terms "alkanoyl" and "alkanoyloxy" as used herein refer, respectively, to -C(O)-alkyl groups and -O-C(=O)- alkyl groups, each optionally containing 2 to 10 carbon atoms. Specific embodiments of alkanoyl and alkanoyloxy groups are acetyl and acetoxy, respectively.

The term "alkenyl" refers to an unsaturated branched, straight-chain or cyclic alkyl group having 2 to 15 carbon atoms and having at least one carbon-carbon double bond derived by the removal of one hydrogen atom from a single carbon atom of a parent alkene. The group may be in either the *cis* or *trans* conformation about the double bond(s). Certain embodiments include ethenyl, 1-propenyl, 2-propenyl, 1-methylethenyl, 1-butenyl, 2-butenyl, 3-butenyl, 2-methyl-2-propenyl, 1-pentenyl, 2-pentenyl, 4-pentenyl, 3-methyl-2-butenyl, 1-hexenyl, 2-hexenyl, 1-heptenyl, 2-heptenyl, 1-octenyl, 2-octenyl, 1,3-octadienyl, 2-nonenyl, 1,3-nonadienyl, 2-decenyl, *etc.,* or the like. The alkenyl group may be substituted or unsubstituted.

The term "alkynyl" as used herein refers to an unsaturated branched, straight-chain, or cyclic alkyl group having 2 to 10 carbon atoms and having at least one carbon-carbon triple bond derived by the removal of one hydrogen atom from a single carbon atom of a parent alkyne. Exemplary alkynyls include ethynyl, 1-propynyl, 2-propynyl, 1-butynyl, 2-butynyl, 3-butynyl, 1-pentynyl, 2-pentynyl, 4-pentynyl, 1-octynyl, 6-methyl-1-heptynyl, 2-decynyl, or the like. The alkynyl group may be substituted or unsubstituted.

The term "hydroxyalkyl" alone or in combination, refers to an alkyl group as previously defined, wherein one or several hydrogen atoms, preferably one hydrogen atom has been replaced by a hydroxyl group. Examples include hydroxymethyl, hydroxyethyl and 2-hydroxyethyl.

The term "aminoalkyl" as used herein refers to the group -NRR', where R and R' may independently be hydrogen or (C₁-C₄) alkyl.

The term "alkylaminoalkyl" refers to an alkylamino group linked via an alkyl group (*i.e.,* a group having the general structure -alkyl-NH-alkyl or -alkyl-N(alkyl)(alkyl)). Such groups include, but are not limited to, mono- and di-(C₁-C₈ alkyl)aminoC₁-C₈ alkyl, in which each alkyl may be the same or different.

The term "dialkylaminoalkyl" refers to alkylamino groups attached to an alkyl group. Examples include, but are not limited to, N,N-dimethylaminomethyl, N,N-dimethylaminoethyl N,N-dimethylaminopropyl, and the like. The term dialkylaminoalkyl also includes groups where the bridging alkyl moiety is optionally substituted.

The term "haloalkyl" refers to an alkyl group substituted with one or more halo groups, for example chloromethyl, 2-bromoethyl, 3-iodopropyl, trifluoromethyl, perfluoropropyl, 8-chlorononyl, or the like.

The term "carboxyalkyl" as used herein refers to the substituent -R¹⁰-COOH, wherein R¹⁰ is alkylene; and "carbalkoxyalkyl" refers to -R¹⁰-C(=O)OR¹¹, wherein R¹⁰ and R¹¹ are alkylene and alkyl respectively. In certain embodiments, alkyl refers to a saturated straight- or branched-chain hydrocarbyl radical of 1 to 6 carbon atoms such as methyl, ethyl, n-propyl, isopropyl, n-butyl, t-butyl, n-pentyl, 2-methylpentyl, n-hexyl, and so forth. Alkylene is the same as alkyl except that the group is divalent.

The term "alkoxy" includes substituted and unsubstituted alkyl, alkenyl, and alkynyl groups covalently linked to an oxygen atom. In one embodiment, the alkoxy group contains 1 to about 10 carbon atoms. Embodiments of alkoxy groups include, but are not limited to, methoxy, ethoxy, isopropyloxy, propoxy, butoxy, and pentoxy groups. Embodiments of substituted alkoxy groups include halogenated alkoxy groups. In a further embodiment, the alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Exemplary halogen substituted alkoxy groups include, but are not limited to, fluoromethoxy, difluoromethoxy, trifluoromethoxy, chloromethoxy, dichloromethoxy, and trichloromethoxy.

The term "alkoxyalkyl" refers to an alkylene group substituted with an alkoxy group. For example, methoxyethyl (CH_{3O}CH₂CH₂-) and ethoxymethyl (CH₃CH₂OCH₂-) are both C₃ alkoxyalkyl groups.

The term "aryl" as used herein refers to monocyclic or bicyclic aromatic hydrocarbon groups having from 6 to 12 carbon atoms in the ring portion, for example, phenyl, naphthyl, biphenyl and diphenyl groups, each of which may be substituted with, for example, one to four substituents such as alkyl; substituted alkyl as defined above, halogen, trifluoromethyl, trifluoromethoxy, hydroxy, alkoxy, cycloalkyloxy, alkanoyl, alkanoyloxy, amino, alkylamino, dialkylamino, nitro, cyano, carboxy, carboxyalkyl, carbamyl, carbamoyl and aryloxy. Specific embodiments of aryl groups in accordance with the present disclosure include phenyl, substituted phenyl, naphthyl, biphenyl, and diphenyl.

The term "aroyl," as used alone or in combination herein, refers to an aryl radical derived from an aromatic carboxylic acid, such as optionally substituted benzoic or naphthoic acids.

The term "aralkyl" as used herein refers to an aryl group bonded to the 2-pyridinyl ring or the 4-pyridinyl ring through an alkyl group, preferably one containing 1 to 10 carbon atoms. A preferred aralkyl group is benzyl.

The term "carboxy," as used herein, represents a group of the formula -C(=O)OH or - C(=O)O⁻.

The term "carbonyl" as used herein refers to a group in which an oxygen atom is double-bonded to a carbon atom -C=O.

The term "trifluoromethyl" as used herein refers to -CF₃.

The term "trifluoromethoxy" as used herein refers to -OCF₃.

The term "hydroxyl" as used herein refers to -OH or -O⁻.

The term "nitrile" or "cyano" as used herein refers to the group -CN.

The term "nitro," as used herein alone or in combination refers to a -NO₂ group.

The term "amino" as used herein refers to the group NR⁹R⁹, wherein R⁹ may independently be hydrogen, alkyl, aryl, alkoxy, or heteroaryl. The term "aminoalkyl" as used herein represents a more detailed selection as compared to "amino" and refers to the group -NR'R', wherein R' may independently be hydrogen or (C₁-C₄) alkyl. The term "dialkylamino" refers to an amino group having two attached alkyl groups that can be the same or different.

The term "alkanoylamino" refers to alkyl, alkenyl or alkynyl groups containing the group -C(=O)- followed by -N(H)-, for example acetylamino, propanoylamino and butanoylamino and the like.

The term "carbonylamino" refers to the group -NR'-CO-CH₂-R', wherein R' may be independently selected from hydrogen or (C₁-C₄) alkyl.

The term "carbamoyl" as used herein refers to -O-C(O)NH₂.

The term "carbamyl" as used herein refers to a functional group in which a nitrogen atom is directly bonded to a carbonyl, *i.e*., as in -NR"C(=O)R" or -C(=O)NR"R", wherein R" can be independently hydrogen, alkyl, substituted alkyl, alkenyl, substituted alkenyl, alkoxy, cycloalkyl, aryl, heterocyclo, or heteroaryl.

The term "alkylsulfonylamino" refers to the group -NHS(O)₂R¹², wherein R¹² is alkyl.

The term "halogen" as used herein refers to bromine, chlorine, fluorine or iodine. In one embodiment, the halogen is fluorine. In another embodiment, the halogen is chlorine.

The term "heterocyclo" refers to an optionally substituted, unsaturated, partially saturated, or fully saturated, aromatic or nonaromatic cyclic group that is a 4 to 7 membered monocyclic, or 7 to 11 membered bicyclic ring system that has at least one heteroatom in at least one carbon atom-containing ring. The substituents on the heterocyclo rings may be selected from those given above for the aryl groups. Each ring of the heterocyclo group containing a heteroatom may have 1, 2, or 3 heteroatoms selected from nitrogen, oxygen or sulfur. Plural heteroatoms in a given heterocyclo ring may be the same or different.

Exemplary monocyclic heterocyclo groups include pyrrolidinyl, pyrrolyl, indolyl, pyrazolyl, imidazolyl, oxazolyl, isoxazolyl, thiazolyl, furyl, tetrahydrofuryl, thienyl, piperidinyl, piperazinyl, azepinyl, pyrimidinyl, pyridazinyl, tetrahydropyranyl, morpholinyl, dioxanyl, triazinyl and triazolyl. Preferred bicyclic heterocyclo groups include benzothiazolyl, benzoxazolyl, benzothienyl, quinolinyl, tetrahydroisoquinolinyl, benzimidazolyl, benzofuryl, indazolyl, benzisothiazolyl, isoindolinyl and tetrahydroquinolinyl. In more detailed embodiments heterocyclo groups may include indolyl, imidazolyl, furyl, thienyl, thiazolyl, pyrrolidyl, pyridyl and pyrimidyl.

"Substituted" refers to a group in which one or more hydrogen atoms are each independently replaced with the same or different substituent(s). Representative substituents include -X, -R⁶, -O-, =O, -OR, -SR⁶, -S-, =S, -NR⁶R⁶, =NR⁶, -CX₃, -CF₃, -CN, -OCN, -SCN, -NO, -NO₂, =N₂, -N₃, -S(=O)₂O-, -S(=O) ₂OH, -S(=O)₂R⁶, -OS(=O)₂O-, -OS(=O)₂OH, -OS(=O)₂R⁶, -P(=O)(O⁻)₂, -P(=O)(OH)(O⁻), -OP(=O)₂(O⁻), -C(-O)R⁶, -C(=S)R⁶, -C(=O)OR⁶, -C(=O)O⁻, -C(=S)OR⁶, -NR⁶-C(=O)-N(R⁶)₂, -NR⁶-C(=S)-N(R⁶)₂, and -C(=NR⁶)NR⁶R⁶, wherein each X is independently a halogen; and each R⁶ is independently hydrogen, halogen, alkyl, aryl, arylalkyl, arylaryl, arylheteroalkyl, heteroaryl, heteroarylalkyl, NR⁷R⁷, -C(=O)R⁷, and -S(=O)₂R⁷; and each R⁷ is independently hydrogen, alkyl, alkanyl, alkynyl, aryl, arylalkyl, arylheteralkyl, arylaryl, heteroaryl or heteroarylalkyl. Aryl containing substituents, whether or not having one or more substitutions, may be attached in a para (*p*-), meta *(m-)* or ortho (*o*-) conformation, or any combination thereof.

### Wingless-Type MMTV (Murine Mammary Tumor Virus) Integration Site Family (WNT1) and Exemplary dsRNA Molecules

The products of the Wingless-Type MMTV (Murine Mammary Tumor Virus) Integration Site Family, Member 1 gene (WNT1; also known as Oncogene INT1), the Wingless-Type MMTV (Murine Mammary Tumor Virus) Integration Site Family, Member 2 (WNT2; also known as Oncogene INT1-like or Int-1 related protein), or Wingless-Type MMTV (Murine Mammary Tumor Virus) Integration Site Family, Member 3 (WNT3A; also known as MGC119418, MGC119419, MGC119420) are central players in cell proliferation and differentiation, embryonic development, and other critical regulatory functions at a variety of cellular and ontogenetic levels and in a large variety of tissue types. Mutation or overexpression of WNT1, WNT2, or WNT3A that increases activity is associated with a variety of disorders including, for example, cancers (colorectal, hepatocellular, bone marrow, melanomas, uterine, ovarian, prostate, endometrial, esophageal, and medulloblastomas) as well as other hypoproliferative disorders, cardiac and other tissue hypertrophy, skeletal and cartilaginous disorders, regulatory disorders, degenerative diseases, developmental disorders, schizophrenia, type II diabetes, and dupuytren skin disease.

More detail regarding WNT1, WNT2, or WNT3A and related disorders are described at *www.ncbi.nlm.nih.gov*/*entrez*/*query.jcgi?db=OMIM,* which is in the Online Mendelian Inheritance in Man database (WNT1 OMIM Accession No. 164820; WNT2 OMIM Accession No. 147870; and WNT3A OMIM Accession No. 606359). The complete mRNA sequence for human WNT1 has Genbank accession number NM 005430.2 (SEQ ID NO: 1158). The complete mRNA sequence for human WNT2 has Genbank accession number NM 003391.2 (SEQ ID NO:1390). The complete mRNA sequence for human WNT3A has Genbank accession number NM 033131.2 (SEQ ID NO:1615). As used herein, reference to WNT1, WNT2, and WNT3A mRNA or RNA sequences or sense strands means a WNT1, WNT2, and WNT3A RNA as set forth in SEQ ID NOS:1158, 1390, and 1615, respectively, as well as variants and homologs having at least 80% or more identity with human WNT1, WNT2, or WNT3A mRNA sequence as set forth in SEQ ID NO:1158, 1390, or 1615, respectively.

The "percent identity" between two or more nucleic acid sequences is a function of the number of identical positions shared by the sequences (i.e., % identity = number of identical positions / total number of positions x 100), taking into account the number of gaps, and the length of each gap that needs to be introduced to optimize alignment of two or more sequences. The comparison of sequences and determination of percent identity between two or more sequences can be accomplished using a mathematical algorithm, such as BLAST and Gapped BLAST programs at their default parameters (*e.g*., BLASTN, *see* www.ncbi.nlm.nih.gov/BLAST; see also Altschul et al., J. Mol. Biol. 215:403-410, 1990).

In one aspect, the instant disclosure provides an mdRNA molecule, comprising a first strand that is complementary to any one of human WNT1 mRNA as set forth in SEQ ID NO:1158, human WNT2 RNA as set forth in SEQ ID NO: 1390, or human WNT3A RNA as set forth in SEQ ID NO:1615, and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands, and wherein (a) the mdRNA molecule optionally has at least one double-stranded region of 5 base pairs to 13 base pairs, or (b) the combined double-stranded regions total about 15 base pairs to about 40 base pairs and the mdRNA molecule optinally has blunt ends; wherein at least one pyrimidine of the mdRNA is a pyrimidine nucleoside according to Formula I or II: 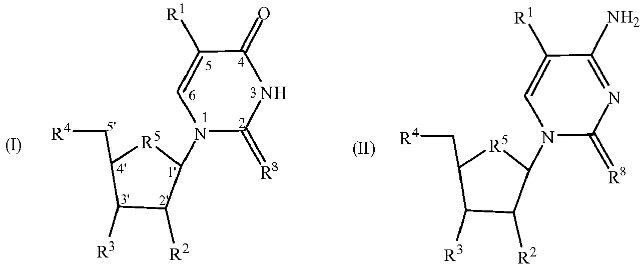 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C=N, or heterocyclo group; R³ and R⁴ are each independently a hydroxyl, a protected hydroxyl, a phosphate, or an internucleoside linking group; and R⁵ and R⁸ are each independently O or S. In certain embodiments, at least one nucleoside is according to Formula I in which R¹ is methyl and R² is -OH, or R¹ is methyl, R² is -OH, and R⁸ is S. In other embodiments, the internucleoside linking group covalently links from about 5 to about 40 nucleosides. In some embodiments, the gap comprises at least one unpaired nucleotide in the first strand positioned between the double-stranded regions formed by the second and third strands when annealed to the first strand, or the gap is a nick. In certain embodiments, the nick or gap is located 10 nucleotides from the 5'-end of the first (antisense) strand or at the Argonaute cleavage site. In another embodiment, the meroduplex nick or gap is positioned such that the thermal stability is maximized for the first and second strand duplex and for the first and third strand duplex as compared to the thermal stability of such meroduplexes having a nick or gap in a different position.

In still another aspect, the instant disclosure provides an mdRNA molecule, comprising a first strand that is complementary to Wingless-Type MMTV (Murine Mammary Tumor Virus) Integration Site Family, Member 1 (WNT1) mRNA as set forth in SEQ ID NO: 1158, a human Wingless-Type MMTV (Murine Mammary Tumor Virus) Integration Site Family, Member 2 (WNT2) mRNA as set forth in SEQ ID NO:1390, or a human Wingless-Type MMTV (Murine Mammary Tumor Virus) Integration Site Family, Member 3A (WNT3A) mRNA as set forth in SEQ ID NO:1615, and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands, and wherein the mdRNA molecule optionally includes at least one double-stranded region of 5 base pairs to 13 base pairs. In a further aspect, the instant disclosure provides an mdRNA molecule having a first strand that is complementary to any one of human WNT1 mRNA as set forth in SEQ ID NO:1158, human WNT2 mRNA as set forth in SEQ ID NO:1390, or human WNT3A mRNA as set forth in SEQ ID NO:1615, and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands, and wherein the combined double-stranded regions total about 15 base pairs to about 40 base pairs and the mdRNA molecule optinally has blunt ends. In some embodiments, the gap comprises at least one unpaired nucleotide in the first strand positioned between the double-stranded regions formed by the second and third strands when annealed to the first strand, or the gap is a nick. In certain embodiments, the nick or gap is located 10 nucleotides from the 5'-end of the first (antisense) strand or at the Argonaute cleavage site. In another embodiment, the meroduplex nick or gap is positioned such that the thermal stability is maximized for the first and second strand duplex and for the first and third strand duplex as compared to the thermal stability of such meroduplexes having a nick or gap in a different position.

As provided herein, any of the aspects or embodiments disclosed herein would be useful in treating WNT1-, WNT2-, or WNT3A-associated diseases or disorders, such as atherosclerosis, diabetes mellitus, and cerebrovascular disease.

In some embodiments, the dsRNA comprises at least three strands in which the first strand comprises about 5 nucleotides to about 40 nucleotides, and the second and third strands include each, individually, about 5 nucleotides to about 20 nucleotides, wherein the combined length of the second and third strands is about 15 nucleotides to about 40 nucleotides. In other embodiments, the dsRNA comprises at least two strands in which the first strand comprises about 15 nucleotides to about 24 nucleotides or about 25 nucleotides to about 40 nucleotides. In yet other embodiments, the first strand comprises about 15 to about 24 nucleotides or about 25 nucleotides to about 40 nucleotides and is complementary to at least about 15, 16, 17, 18, 19, 20, 21, 22, 23, 24, 25, 26, 27, 28, 29, 30, 31, 32, 33, 34, 35, 36, 37, 38, 39, or 40 contiguous nucleotides of a human WNT1 mRNA as set forth in SEQ ID NO: 1158, WNT2 mRNA as set forth in SEQ ID NO: 1390, or WNT3A mRNA as set forth in SEQ ID NO: 1615. In alternative embodiments, the first strand comprises about 15 to about 24 nucleotides or about 25 nucleotides to about 40 nucleotides and is at least about 70%, 75%, 80%, 85%, 90%, 91%, 92%, 93%, 94%, 95%, 96%, 97%, 98%, or 99% identical to a sequence that is complementary to at least about 15, 16, 17, 18, 19, 20, 21, 22, 23, 24, 25, 26, 27, 28, 29, 30, 31, 32, 33, 34, 35, 36, 37, 38, 39, or 40 contiguous nucleotides of a human WNT1 mRNA as set forth in SEQ ID NO:1158, WNT2 mRNA as set forth in SEQ ID NO:1390, or WNT3A mRNA as set forth in SEQ ID NO:1615.

In further embodiments, the first strand will be complementary to a second strand or a second and third strand or to a plurality of strands. The first strand and its complements will be able to form dsRNA and mdRNA molecules of this disclosure, but only about 19 to about 25 nucleotides of the first strand comprise a sequence complementary to a WNT1, WNT2, or WNT3A mRNA. For example, a Dicer substrate dsRNA can have about 25 nucleotides to about 40 nucleotides, but with only 19 nucleotides of the antisense (first) strand being complementary to a WNT1, WNT2, or WNT3A mRNA. In further embodiments, the first strand having complementarity to a WNT 1 mRNA in about 19 nucleotides to about 25 nucleotides will have one, two, or three mismatches with a WNT1 mRNA, such as a sequence set forth in SEQ ID NO:1158, or the first strand of 19 nucleotides to about 25 nucleotides, that for example activates or is capable of loading into RISC, will have at least 80% identity with the corresponding nucleotides found in a WNT1 mRNA, such as the sequence set forth in SEQ ID NO:1158. In further embodiments, the first strand having complementarity to a WNT2 mRNA in about 19 nucleotides to about 25 nucleotides will have one, two, or three mismatches with a WNT2 mRNA, such as a sequence set forth in SEQ ID NO:1390, or the first strand of 19 nucleotides to about 25 nucleotides, that for example activates or is capable of loading into RISC, will have at least 80% identity with the corresponding nucleotides found in a WNT2 mRNA, such as the sequence set forth in SEQ ID NOS:1390. In further embodiments, the first strand having complementarity to a WNT3A mRNA in about 19 nucleotides to about 25 nucleotides will have one, two, or three mismatches with a WNT3A mRNA, such as a sequence set forth in SEQ ID NO:1615, or the first strand of 19 nucleotides to about 25 nucleotides, that for example activates or is capable of loading into RISC, will have at least 80% identity with the corresponding nucleotides found in a WNT3A mRNA, such as the sequence set forth in SEQ ID NO:1615.

Certain illustrative dsRNA molecules, which can be used to design mdRNA molecules and can optionally include substitutions or modifications as described herein are provided in the Sequence Listings as attached herewith, which is herein incorporated by reference (text file named "07-R079PCT_Sequence_Listing," created February 20, 2008 and having a size of 452 kilobytes). In addition, the content of Table B as disclosed in U.S. Provisional Patent Application No. 60/934,930 (filed March 16, 2007), which was submitted with that application as a separate text file named "Table_B_Human_RefSeq_Accession_Numbers.txt" (created March 16, 2007 and having a size of 3,604 kilobytes), is incorporated herein by reference in its entirety.

### Substituting and Modifying WNT dsRNA Molecules

The introduction of substituted and modified nucleotides into mdRNA and dsRNA molecules of this disclosure provides a powerful tool in overcoming potential limitations of *in vivo* stability and bioavailability inherent to native RNA molecules *(i.e.,* having standard nucleotides) that are exogenously delivered. For example, the use of dsRNA molecules of this disclosure can enable a lower dose of a particular nucleic acid molecule for a given therapeutic effect (*e.g*., reducing or silencing WNT1, WNT2, or WNT3A expression) since dsRNA molecules of this disclosure tend to have a longer half-life in serum. Furthermore, certain substitutions and modifications can improve the bioavailability of dsRNA by targeting particular cells or tissues or improving cellular uptake of the dsRNA molecules. Therefore, even if the activity of a dsRNA molecule of this disclosure is reduced as compared to a native RNA molecule, the overall activity of the substituted or modified dsRNA molecule can be greater than that of the native RNA molecule due to improved stability or delivery of the molecule. Unlike native unmodified dsRNA, substituted and modified dsRNA can also minimize the possibility of activating the interferon response in, for example, humans.

In certain embodiments, a dsRNA molecule of this disclosure has at least one uridine, at least three uridines, or each and every uridine *(i.e.,* all uridines) of the first (antisense) strand of that is a 5-methyluridine, 2-thioribothymidine, 2'-O-methyl-5-methyluridine, or any combination thereof. In a related embodiment, the dsRNA molecule or analog thereof of this disclosure has at least one uridine, at least three uridines, or each and every uridine of the second (sense) strand of the dsRNA is a 5-methyluridine, 2-thioribothymidine, 2'-O-methyl-5-methyluridine, or any combination thereof. In a related embodiment, the dsRNA molecule of this disclosure has at least one uridine, at least three uridines, or each and every uridine of the third (sense) strand of the dsRNA is a 5-methyluridine, 2-thioribothymidine, 2'-O-methyl-5-methyluridine, or any combination thereof. In still another embodiment, the dsRNA molecule of this disclosure has at least one uridine, at least three uridines, or each and every uridine of both the first (antisense) and second (sense) strands; of both the first (antisense) and third (sense) strands; of both the second (sense) and third (sense) strands; or all of the first (antisense), second (sense) and third (sense) strands of the dsRNA are a 5-methyluridine, 2-thioribothymidine, 2'-O-methyl-5-methyluridine, or any combination thereof. In some embodiments, the double-stranded region of a dsRNA molecule has at least three 5-methyluridines, 2-thioribothymidine, 2'-O-methyl-5-methyluridine, or any combination thereof. In certain embodiments, dsRNA molecules comprise ribonucleotides at about 5% to about 95% of the nucleotide positions in one strand, both strands, or any combination thereof.

In further embodiments, a dsRNA molecule that decreases expression of a WNT1, WNT2, or WNT3A gene by RNAi according to the instant disclosure further comprises one or more natural or synthetic non-standard nucleoside. In related embodiments, the non-standard nucleoside is one or more deoxyuridine, locked nucleic acid (LNA) molecule, a modified base (e.g., 5-methyluridine), a universal-binding nucleotide, a 2'-O-methyl nucleotide, a modified internucleoside linkage (*e.g*., phosphorothioate), a G clamp, or any combination thereof. In certain embodiments, the universal-binding nucleotide can be C-phenyl, C-naphthyl, inosine, azole carboxamide, 1-β-*D*-ribofuranosyl-4-nitroindole, 1-β-*D*-ribofuranosyl-5-nitroindole, 1-β-*D-*ribofuranosyl-6-nitroindole, or 1-β-*D*-ribofuranosyl-3-nitropyrrole.

Substituted or modified nucleotides present in dsRNA molecules, preferably in the sense or antisense strand, but also optionally in both the antisense and sense strands, comprise modified or substituted nucleotides according to this disclosure having properties or characteristics similar to natural or standard ribonucleotides. For example, this disclosure features dsRNA molecules including nucleotides having a Northern conformation (*e.g*., Northern pseudorotation cycle; *see, e.g.,* Saenger, Principles of Nucleic Acid Structure, Springer-Verlag ed., 1984). As such, chemically modified nucleotides present in dsRNA molecules of this disclosure, preferably in the antisense strand, but also optionally in the sense or both the antisense and sense strands, are resistant to nuclease degradation while at the same time maintaining the capacity to mediate RNAi. Exemplary nucleotides having a Northern configuration include locked nucleic acid (LNA) nucleotides (*e.g*., 2'-O, 4'-C-methylene-(*D-*ribofuranosyl) nucleotides), 2'-methoxyethyl (MOE) nucleotides, 2'-methyl-thio-ethyl, 2'-deoxy-2'-fluoro nucleotides, 2'-deoxy-2'-chloro nucleotides, 2'-azido nucleotides, 5-methyluridines, or 2'-O-methyl nucleotides. In certain embodiments, the LNA is a 5-methyluridine LNA or 2-thio-5-methyluridine LNA. In any of these embodiments, one or more substituted or modified nucleotides can be a G clamp (*e.g*., a cytosine analog that forms an additional hydrogen bond to guanine, such as 9-(aminoethoxy)phenoxazine; *see, e.g.,* Lin and Mateucci, J. Am. Chem. Soc. 120:8531, 1998).

As described herein, the first and one or more second strands of a dsRNA molecule or analog thereof provided by this disclosure can anneal or hybridize together (*i.e*., due to complementarity between the strands) to form at least one double-stranded region having a length of about 4 to about 10 base pairs, about 5 to about 13 base pairs, or about 15 to about 40 base pairs. In some embodiments, the dsRNA has at least one double-stranded region ranging in length from about 15 to about 24 base pairs or about 19 to about 23 base pairs. In other embodiments, the dsRNA has at least one double-stranded region ranging in length from about 26 to about 40 base pairs or about 27 to about 30 base pairs or about 30 to about 35 base pairs. In other embodiments, the two or more strands of a dsRNA molecule of this disclosure may optionally be covalently linked together by nucleotide or non-nucleotide linker molecules.

In certain embodiments, the dsRNA molecule or analog thereof comprises an overhang of one to four nucleotides on one or both 3'-ends of the dsRNA, such as an overhang comprising a deoxyribonucleotide or two deoxyribonucleotides (*e.g*., thymidine, adenine). In certain embodiments, the 3'-end comprising one or more deoxyribonucleotide is in an mdRNA molecule and is either in the gap, not in the gap, or any combination thereof. In some embodiments, dsRNA molecules or analogs thereof have a blunt end at one or both ends of the dsRNA. In certain embodiments, the 5'-end of the first or second strand is phosphorylated. In any of the embodiments of dsRNA molecules described herein, the 3'-terminal nucleotide overhangs can comprise ribonucleotides or deoxyribonucleotides that are chemically-modified at a nucleic acid sugar, base, or backbone. In any of the embodiments of dsRNA molecules described herein, the 3'-terminal nucleotide overhangs can comprise one or more universal base ribonucleotides. In any of the embodiments of dsRNA molecules described herein, the 3'-terminal nucleotide overhangs can comprise one or more acyclic nucleotides. In any of the embodiments of dsRNA molecules described herein, the dsRNA can further comprise a terminal phosphate group, such as a 5'-phosphate *(see* Martinez et al., Cell. 110:563-574, 2002; and Schwarz et al., Molec. Cell 10:537-568, 2002) or a 5',3'-diphosphate.

As set forth herein, the terminal structure of dsRNAs of this disclosure that decrease expression of a WNT1, WNT2, or WNT3A gene by, for example, RNAi may either have blunt ends or one or more overhangs. In certain embodiments, the overhang may be at the 3'-end or the 5'-end. The total length of dsRNAs having overhangs is expressed as the sum of the length of the paired double-stranded portion together with the overhanging nucleotides. For example, if a 19 base pair dsRNA has a two nucleotide overhang at both ends, the total length is expressed as 21-mer. Furthermore, since the overhanging sequence may have low specificity to a WNT1, WNT2, or WNT3A gene, it is not necessarily complementary (antisense) or identical (sense) to a WNT1, WNT2, or WNT3A gene sequence. In further embodiments, a dsRNA of this disclosure that decreases expression of a WNT1, WNT2, or WNT3A gene by RNAi may further comprise a low molecular weight structure (*e.g*., a natural RNA molecule such as a tRNA, rRNA or viral RNA, or an artificial RNA molecule) at, for example, one or more overhanging portion of the dsRNA.

In further embodiments, a dsRNA molecule that decreases expression of a human WNT1, human WNT2, or human WNT3A gene by RNAi according to the instant disclosure further comprises a 2'-sugar substitution, such as 2'-deoxy, 2'-O-methyl, 2'-O-methoxyethyl, 2'-O-2-methoxyethyl, halogen, 2'-fluoro, 2'-O-allyl, or the like, or any combination thereof. In still further embodiments, a dsRNA molecule that decreases expression of a WNT1, WNT2, or WNT3A gene by RNAi according to the instant disclosure further comprises a terminal cap substituent on one or both ends of the first strand or one or more second strands, such as an alkyl, abasic, deoxy abasic, glyceryl, dinucleotide, acyclic nucleotide, inverted deoxynucleotide moiety, or any combination thereof. In certain embodiments, at least one or two 5'-terminal ribonucleotides of the sense strand within the double-stranded region have a 2'-sugar substitution. In certain other embodiments, at least one or two 5'-terminal ribonucleotides of the antisense strand within the double-stranded region have a 2'-sugar substitution. In certain embodiments, at least one or two 5'-terminal ribonucleotides of the sense strand and the antisense strand within the double-stranded region have a 2'-sugar substitution.

In other embodiments, a dsRNA molecule that decreases expression of one or more target gene by RNAi according to the instant disclosure comprises one or more substitutions in the sugar backbone, including any combination of ribosyl, 2'-deoxyribosyl, a tetrofuranosyl (*e.g*., L-α-threofuranosyl), a hexopyranosyl (*e.g*., β-allopyranosyl, β-altropyranosyl, and β-glucopyranosyl), a pentopyranosyl (*e.g*., β-ribopyranosyl, α-lyxopyranosyl, β-xylopyranosyl, and α-arabinopyranosyl), a carbocyclic (carbon only ring) analog, a pyranose, a furanose, a morpholino, or analogs or derivatives thereof.

In yet other embodiments, a dsRNA molecule that decreases expression of a human WNT1, WNT2, or WNT3A gene (including a mRNA splice variant thereof) by RNAi according to the instant disclosure further comprises at least one modified internucleoside linkage, such as independently a phosphorothioate, chiral phosphorothioate, phosphorodithioate, phosphotriester, aminoalkylphosphotriester, methyl phosphonate, alkyl phosphonate, 3'-alkylene phosphonate, 5'-alkylene phosphonate, chiral phosphonate, phosphonoacetate, thiophosphonoacetate, phosphinate, phosphoramidate, 3'-amino phosphoramidate, aminoalkylphosphoramidate, thionophosphoramidate, thionoalkylphosphonate, thionoalkylphosphotriester, selenophosphate, boranophosphate linkage, or any combination thereof.

A modified internucleotide linkage, as described herein, can be present in one or more strands of a dsRNA molecule of this disclosure, for example, in the sense strand, the antisense strand, both strands, or a plurality of strands (*e.g*., in an mdRNA). The dsRNA molecules of this disclosure can comprise one or more modified internucleotide linkages at the 3'-end, the 5'-end, or both of the 3'- and 5'-ends of the second sense strand, the third sense strand, the antisense strand or any combination of the antisense strand and one or more of the sense strands. In one embodiment, a dsRNA molecule capable of decreasing expression of a human WNT1, WNT2, or WNT3A gene (including a specific or selected mRNA splice variant thereof) by RNAi has one modified internucleotide linkage at the 3'-end, such as a phosphorothioate linkage. For example, this disclosure provides a dsRNA molecule capable of decreasing expression of a WNT 1, WNT2, or WNT3A gene by RNAi having about 1 to about 8 or more phosphorothioate internucleotide linkages in one dsRNA strand. In yet another embodiment, this disclosure provides a dsRNA molecule capable of decreasing expression of a human WNT1, WNT2, or WNT3A gene by RNAi having about 1 to about 8 or more phosphorothioate internucleotide linkages in the dsRNA strands. In other embodiments, an exemplary dsRNA molecule of this disclosure can comprise from about 1 to about 5 or more consecutive phosphorothioate internucleotide linkages at the 5'-end of the sense strand, the antisense strand, both strands, or a plurality of strands. In another example, a dsRNA molecule of this disclosure can comprise one or more pyrimidine phosphorothioate internucleotide linkages in the sense strand, the antisense strand, either strand, or a plurality of strands. In yet another example, an exemplary dsRNA molecule of this disclosure comprises one or more purine phosphorothioate internucleotide linkages in the sense strand, the antisense strand, either strand, or a plurality of strands.

Many exemplary modified nucleotide bases or analogs thereof useful in the dsRNA of the instant disclosure include 5-methylcytosine; 5-hydroxymethylcytosine; xanthine; hypoxanthine; 2-aminoadenine; 6-methyl, 2-propyl, or other alkyl derivatives of adenine and guanine; 8-substituted adenines and guanines (such as 8-aza, 8-halo, 8-amino, 8-thiol, 8-thioalkyl, 8-hydroxyl, or the like); 7-methyl, 7-deaza, and 3-deaza adenines and guanines; 2-thiouracil; 2-thiothymine; 2-thiocytosine; 5-methyl, 5-propynyl, 5-halo (such as 5-bromo or 5-fluoro), 5-trifluoromethyl, or other 5-substituted uracils and cytosines; and 6-azouracil. Further useful nucleotide bases can be found in Kurreck, Eur. J. Biochem. 270:1628, 2003; Herdewijn, Antisense Nucleic Acid Develop. 10:297, 2000; Concise Encyclopedia of Polymer Science and Engineering, pages 858-859, Kroschwitz, J. I., ed. John Wiley & Sons, 1990; U.S. Patent No. 3,687,808, and similar references.

Certain nucleotide base moieties are particularly useful for increasing the binding affinity of the dsRNA molecules of this disclosure to complementary targets. These include 5-substituted pyrimidines; 6-azapyrimidines; and N-2, N-6, or O-6 substituted purines (including 2-aminopropyladenine, 5-propynyluracil and 5-propynylcytosine). For example, 5-methyluridine and 5-methylcytosine substitutions are known to increase nucleic acid duplex stability, which can be combined with 2'-sugar modifications (such as 2'-methoxy or 2'-methoxyethyl) or internucleoside linkages (*e.g*., phosphorothioate) that provide nuclease resistance to the modified or substituted dsRNA.

In another aspect of the instant disclosure, there is provided a dsRNA that decreases expression of a WNT1, WNT2, or WNT3A gene, comprising a first strand that is complementary to any one of human WNT1 mRNA set forth in SEQ ID NO:1158, human WNT2 mRNA as set forth in SEQ ID NO: 1390, or human WNT3A mRNA as set forth in SEQ ID NO:1615 and a second strand that is complementary to the first strand, wherein the first and second strands form a double-stranded region of about 5 to about 40 base pairs; wherein at least one pyrimidine of the dsRNA is a pyrimidine nucleoside according to Formula I or II: 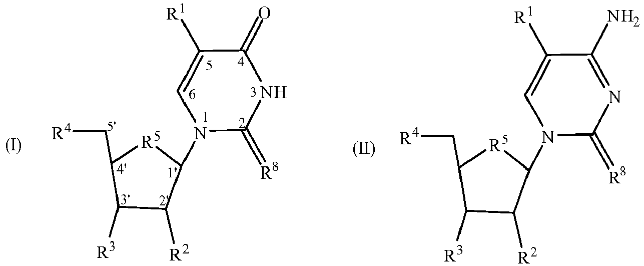 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C=N, or heterocyclo group; R³ and R⁴ are each independently a hydroxyl, a protected hydroxyl, or an internucleoside linking group; and R⁵ and R⁸ are each independently O or S. In certain embodiments, at least one nucleoside is according to Formula I in which R¹ is methyl and R² is -OH, or R¹ is methyl, R² is -OH, and R⁸ is S. In certain embodiments, at least one nucleoside is according to Formula I in which R¹ is methyl and R² is -O-methyl, or R¹ is methyl, R² is -O-methyl, and R⁸ is O. In other embodiments, the internucleoside linking group covalently links from about 2 to about 40 nucleosides.

In certain embodiments, the first and one or more second strands of a dsRNA, which decreases expression of a WNT1, WNT2, or WNT3A gene by RNAi and has at least one pyrimidine substituted with a pyrimidine nucleoside according to Formula I or II, can anneal or hybridize together (*i.e.*, due to complementarity between the strands) to form at least one double-stranded region having a length or a combined length of about 15 to about 40 base pairs. In some embodiments, the dsRNA has at least one double-stranded region ranging in length from about 4 base pairs to about 10 base pairs or about 5 to about 13 base pairs or about 15 to about 25 base pairs or about 19 to about 23 base pairs. In other embodiments, the dsRNA has at least one double-stranded region ranging in length from about 26 to about 40 base pairs or about 27 to about 30 base pairs or about 30 to about 35 base pairs. In certain embodiments, the dsRNA molecule or analog thereof has an overhang of one to four nucleotides on one or both 3'-ends, such as an overhang comprising a deoxyribonucleotide or two deoxyribonucleotides (*e.g.*, thymidine). In some embodiments, dsRNA molecule or analog thereof has a blunt end at one or both ends of the dsRNA. In certain embodiments, the 5'-end of the first or second strand is phosphorylated.

In certain embodiments, at least one R¹ is a C₁-C₅ alkyl, such as methyl or ethyl. Within other exemplary embodiments of this disclosure, compounds of Formula I are a 5-alkyluridine (*i.e.,* R¹ is alkyl, R² is -OH, and R³, R⁴, and R⁵ are as defined herein) or compounds of Formula II are a 5-alkylcytidine (*i.e.*, R¹ is alkyl, R² is -OH, and R³, R⁴, and R⁵ are as defined herein). In related embodiments, the 5-alkyluridine is a 5-methyluridine (also referred to as ribothymidine or T^{r} - *i.e.*, R¹ is methyl and R² is -OH), and the 5-alkylcytidine is a 5-methylcytidine. In other embodiments, at least one, at least three, or all uridines of the first strand of the dsRNA are replaced with 5-methyluridine, or at least one, at least three, or all uridines of the second strand of the dsRNA are replaced with 5-methyluridine, or any combination thereof (*e.g.*, such changes are made on more than one strand). In certain embodiments, at least one pyrimidine nucleoside of Formula I or Formula II has an R⁵ that is S or R⁸ that is S.

In further embodiments, at least one pyrimidine nucleoside of the dsRNA is a locked nucleic acid (LNA) in the form of a bicyclic sugar, wherein R² is oxygen, and the 2'-O and 4'-C form an oxymethylene bridge on the same ribose ring. In a related embodiment, the LNA comprises a base substitution, such as a 5-methyluridine LNA or 2-thio-5-methyluridine LNA. In other embodiments, at least one, at least three, or all uridines of the first strand of the dsRNA are replaced with 5-methyluridine or 2-thioribothymidine or 5-methyluridine LNA or 2-thio-5-methyluridine LNA, or at least one, at least three, or all uridines of the second strand of the dsRNA are replaced with 5-methyluridine, 2-thioribothymidine, 5-methyluridine LNA, 2-thio-5-methyluridine LNA, or any combination thereof (*e.g.*, such changes are made on both strands, or some substitutions include 5-methyluridine only, 2-thioribothymidine only, 5-methyluridine LNA only, 2-thio-5-methyluridine LNA only, or one or more 5-methyluridine or 2-thioribothymidine with one or more 5-methyluridine LNA or 2-thio-5-methyluridine LNA).

In further embodiments, a ribose of the pyrimidine nucleoside or the internucleoside linkage can be optionally modified. For example, compounds of Formula I or II are provided wherein R² is alkoxy, such as a 2'-O-methyl substitution (*e.g.*, which may be in addition to a 5-alkyluridine or a 5-alkylcytidine, respectively). In certain embodiments, R² is selected from 2'-O-(C₁-C₅) alkyl, 2'-O-methyl, 2'-OCH₂OCH₂CH₃, 2'-OCH₂CH₂OCH₃, 2'-O-allyl, or 2'-fluoro. In further embodiments, one or more of the pyrimidine nucleosides are according to Formula I in which R¹ is methyl and R² is a 2'-O-(C₁-C₅) alkyl (*e.g.*, 2'-O-methyl), or in which R¹ is methyl, R² is a 2'O-(C₁-C₅) alkyl (*e.g.*, 2'O-methyl), and R² is S, or any combination thereof. In other embodiments, one or more, or at least two, pyrimidine nucleosides according to Formula I or II have an R² that is not -H or -OH and is incorporated at a 3'-end or 5'-end and not within the gap of one or more strands within the double-stranded region of the dsRNA molecule.

In further embodiments, a dsRNA molecule or analog thereof comprising a pyrimidine nucleoside according to Formula I or Formula II in which R² is not -H or -OH and an overhang, further comprises at least two of pyrimidine nucleosides that are incorporated either at a 3'-end or a 5'-end or both of one strand or two strands within the double-stranded region of the dsRNA molecule. In a related embodiment, at least one of the at least two pyrimidine nucleosides in which R² is not -H or -OH is located at a 3'-end or a 5'-end within the double-stranded region of at least one strand of the dsRNA molecule, and wherein at least one of the at least two pyrimidine nucleosides in which R² is not -H or -OH is located internally within a strand of the dsRNA molecule. In still further embodiments, a dsRNA molecule or analog thereof that has an overhang has a first of the two or more pyrimidine nucleosides in which R² is not -H or -OH that is incorporated at a 5'-end within the double-stranded region of the sense strand of the dsRNA molecule and a second of the two or more pyrimidine nucleosides is incorporated at a 5'-end within the double-stranded region of the antisense strand of the dsRNA molecule. In any of these embodiments, one or more substituted or modified nucleotides can be a G clamp (*e.g*., a cytosine analog that forms an additional hydrogen bond to guanine, such as 9-(aminoethoxy)phenoxazine; *see, e.g.,* Lin and Mateucci, 1998). In any of these embodiments, provided the one or more modified pyrimidine nucleosides are not within the gap.

In yet other embodiments, a dsRNA molecule or analog thereof of Formula I or II according to the instant disclosure that has an overhang that comprises four or more independent pyrimidine nucleosides or four or more independent pyrimidine nucleosides in which R² is not - H or -OH, wherein (a) a first pyrimidine nucleoside is incorporated into a 3'-end within the double-stranded region of the sense (second) strand of the dsRNA, (b) a second pyrimidine nucleoside is incorporated into a 5'-end within the double-stranded region of the sense (second) strand, (c) a third pyrimidine nucleoside is incorporated into a 3'-end within the double-stranded region of the antisense (first) strand of the dsRNA, and (d) a fourth pyrimidine nucleoside is incorporated into a 5'-end within the double-stranded region of the antisense (first) strand. In any of these embodiments, provided the one or more modified pyrimidine nucleosides are not within the gap.

In further embodiments, a dsRNA molecule or analog thereof comprising a pyrimidine nucleoside according to Formula I or Formula II in which R² is not -H or -OH and is blunt-ended, further comprises at least two of pyrimidine nucleosides that are incorporated either at a 3'-end or a 5'-end or both of one strand or two strands of the dsRNA molecule. In a related embodiment, at least one of the at least two pyrimidine nucleosides in which R² is not -H or -OH is located at a 3'-end or a 5'-end of at least one strand of the dsRNA molecule, and wherein at least one of the at least two pyrimidine nucleosides in which R² is not -H or -OH is located internally within a strand of the dsRNA molecule. In still further embodiments, a dsRNA molecule or analog thereof that is blunt-ended has a first of the two or more pyrimidine nucleosides in which R² is not -H or -OH that is incorporated at a 5'-end of the sense strand of the dsRNA molecule and a second of the two or more pyrimidine nucleosides is incorporated at a 5'-end of the antisense strand of the dsRNA molecule. In any of these embodiments, provided the one or more modified pyrimidine nucleosides are not within the gap.

In yet other embodiments, a dsRNA molecule comprising a pyrimidine nucleoside according to Formula I or Formula II and that is blunt-ended comprises four or more independent pyrimidine nucleosides or four or more independent pyrimidine nucleosides in which R² is not -H or -OH, wherein (a) a first pyrimidine nucleoside is incorporated into a 3'-end within the double-stranded region of the sense (second) strand of the dsRNA, (b) a second pyrimidine nucleoside is incorporated into a 5'-end within the double-stranded region of the sense (second) strand, (c) a third pyrimidine nucleoside is incorporated into a 3'-end within the double-stranded region of the antisense (first) strand of the dsRNA, and (d) a fourth pyrimidine nucleoside is incorporated into a 5'-end within the double-stranded region of the antisense (first) strand. In any of these embodiments, provided the one or more modified pyrimidine nucleosides are not within the gap.

In still further embodiments, a dsRNA molecule or analog thereof of Formula I or II according to the instant disclosure further comprises a terminal cap substituent on one or both ends of the first strand or second strand, such as an alkyl, abasic, deoxy abasic, glyceryl, dinucleotide, acyclic nucleotide, inverted deoxynucleotide moiety, or any combination thereof. In further embodiments, one or more internucleoside linkage can be optionally modified. For example, a dsRNA molecule or analog thereof of Formula I or II according to the instant disclosure wherein at least one internucleoside linkage is modified to a phosphorothioate, chiral phosphorothioate, phosphorodithioate, phosphotriester, aminoalkylphosphotriester, methyl phosphonate, alkyl phosphonate, 3'-alkylene phosphonate, 5'-alkylene phosphonate, chiral phosphonate, phosphonoacetate, thiophosphonoacetate, phosphinate, phosphoramidate, 3'-amino phosphoramidate, aminoalkylphosphoramidate, selenophosphate, thionophosphoramidate, thionoalkylphosphonate, thionoalkylphosphotriester, boranophosphate linkage, or any combination thereof.

In another exemplary of this disclosure, the dsRNAs comprise at least two or more substituted pyrimidine nucleosides can each be independently selected wherein R¹ comprises any chemical modification or substitution as contemplated herein, for example an alkyl (*e.g.*, methyl), halogen, hydroxy, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, carbonyl, alkanoylamino, carbamoyl, carbonylamino, alkylsulfonylamino, or heterocyclo group. When two or more modified ribonucleotides are present, each modified ribonucleotide can be independently modified to have the same, or different, modification or substitution at R¹ or R².

In other detailed embodiments, one or more substituted pyrimidine nucleosides according to Formula I or II can be located at any ribonucleotide position, or any combination of ribonucleotide positions, on either or both of the sense and antisense strands of a dsRNA molecule of this disclosure, including at one or more multiple terminal positions as noted above, or at any one or combination of multiple non-terminal ("internal") positions. In this regard, each of the sense and antisense strands can incorporate about 1 to about 6 or more of the substituted pyrimidine nucleosides.

In certain embodiments, when two or more substituted pyrimidine nucleosides are incorporated within a dsRNA of this disclosure, at least one of the substituted pyrimidine nucleosides will be at a 3'- or 5'-end of one or both strands, and in certain embodiments at least one of the substituted pyrimidine nucleosides will be at a 5'-end of one or both strands. In other embodiments, the substituted pyrimidine nucleosides are located at a position corresponding to a position of a pyrimidine in an unmodified dsRNA that is constructed as a homologous sequence for targeting a cognate mRNA, as described herein.

In addition, the terminal structure of the dsRNAs of this disclosure may have a stem-loop structure in which ends of one side of the dsRNA molecule are connected by a linker nucleic acid, *e.g*., a linker RNA. The length of the double-stranded region (stem-loop portion) can be, for example, about 15 to about 49 bp, about 15 to about 35 bp, or about 21 to about 30 bp long. Alternatively, the length of the double-stranded region that is a final transcription product of dsRNAs to be expressed in a target cell may be, for example, approximately about 15 to about 49 bp, about 15 to about 35 bp, or about 21 to about 30 bp long. When linker segments are employed, there is no particular limitation in the length of the linker as long as it does not hinder pairing of the stem portion. For example, for stable pairing of the stem portion and suppression of recombination between DNAs coding for this portion, the linker portion may have a clover-leaf tRNA structure. Even if the linker has a length that would hinder pairing of the stem portion, it is possible, for example, to construct the linker portion to include introns so that the introns are excised during processing of a precursor RNA into mature RNA, thereby allowing pairing of the stem portion. In the case of a stem-loop dsRNA, either end (head or tail) of RNA with no loop structure may have a low molecular weight RNA. As described above, these low molecular weight RNAs may include a natural RNA molecule, such as tRNA, rRNA or viral RNA, or an artificial RNA molecule.

A dsRNA molecule may be comprised of a circular nucleic acid molecule, wherein the dsRNA is about 38 to about 70 nucleotides in length having from about 18 to about 23 base pairs (*e.g.*, about 19 to about 21 bp) wherein the circular oligonucleotide forms a dumbbell shaped structure having about 19 base pairs and two loops. In certain embodiments, a circular dsRNA molecule contains two loop motifs wherein one or both loop portions of the dsRNA molecule is biodegradable. For example, a circular dsRNA molecule of this disclosure is designed such that degradation of the loop portions of the dsRNA molecule *in vivo* can generate a dsRNA molecule with 3'-terminal overhangs, such as 3'-terminal nucleotide overhangs comprising from about 1 to about 4 (unpaired) nucleotides.

Substituting or modifying nucleosides of a dsRNA according to this disclosure can result in increased resistance to enzymatic degradation, such as exonucleolytic degradation, including 5'-exonucleolytic or 3'-exonucleolytic degradation. As such, in some embodiments, the dsRNAs described herein will exhibit significant resistance to enzymatic degradation compared to a corresponding dsRNA having standard nucleotides, and will thereby possess greater stability, increased half-life, and greater bioavailability in physiological environments (*e.g.*, when introduced into a eukaryotic target cell). In addition to increasing resistance of the substituted or modified dsRNAs to exonucleolytic degradation, the incorporation of one or more pyrimidine nucleosides according to Formula I or II will render dsRNAs more resistant to other enzymatic or chemical degradation processes and thus more stable and bioavailable than otherwise identical dsRNAs that do not include the substitutions or modifications. In related aspects of this disclosure, dsRNA substitutions or modifications described herein will often improve stability of a modified dsRNA for use within research, diagnostic and treatment methods wherein the modified dsRNA is contacted with a biological sample, for example, a mammalian cell, intracellular compartment, serum or other extracellular fluid, tissue, or other *in vitro* or *in vivo* physiological compartment or environment. In one embodiment, diagnosis is performed on an isolated biological sample. In another embodiment, the diagnostic method is performed *in vitro.* In a further embodiment, the diagnostic method is not performed (directly) on a human or animal body.

In addition to increasing stability of substituted or modified dsRNAs, incorporation of one or more pyrimidine nucleosides according to Formula I or II in a dsRNA designed for gene silencing can provide additional desired functional results, including increasing a melting point of a substituted or modified dsRNA compared to a corresponding unmodified dsRNA. In another aspect of this disclosure, certain substitutions or modifications of dsRNAs described herein can reduce "off-target effects" of the substituted or modified dsRNA molecules when they are contacted with a biological sample (*e.g.*, when introduced into a target eukaryotic cell having specific, and non-specific mRNA species present as potential specific and non-specific targets). In yet another aspect of this disclosure, the dsRNA substitutions or modifications described herein can reduce interferon activation by the dsRNA molecule when the dsRNA is contacted with a biological sample, *e.g*., when introduced into a eukaryotic cell.

In further embodiments, dsRNAs of this disclosure can comprise one or more sense (second) strand that is homologous or corresponds to a sequence of a target gene (*e.g.*, WNT1, WNT2, or WNT3A) and an antisense (first) strand that is complementary to the sense strand and a sequence of the target gene (*e.g.*, WNT1, WNT2, or WNT3A). In exemplary embodiments, at least one strand of the dsRNA incorporates one or more pyrimidines substituted according to Formula I or II (*e.g*., wherein the pyrimidine is a 5-methyluridine, 2-thioribothymidine, or 2-O-methyl-5-methylurindine, the ribose is modified to incorporate one or more 2'-O-methyl substitutions, or any combination thereof). These and other multiple substitutions or modifications according to Formula I or II can be introduced into one or more pyrimidines, or into any combination and up to all pyrimidines present in one or more strands of a dsRNA of the instant disclosure, so long as the dsRNA has or retains RNAi activity similar to or better than the activity of an unmodified dsRNA. In one embodiment, the dsRNA comprises one or more 2'O-methyl-5-methyluridine.

In any of the embodiments described herein, the dsRNA may include multiple modifications. For example, a dsRNA having at least one ribothymidine or 2'-O-methyl-5-methyluridine may further comprise at least one LNA, 2'-methoxy, 2'-fluoro, 2'-deoxy, phosphorothioate linkage, an inverted base terminal cap, or any combination thereof. In certain embodiments, a dsRNA will have from one to all ribothymidines and have up to 75% LNA. In other embodiments, a dsRNA will have from one to all ribothymidines and have up to 75% 2'-methoxy (*e.g.*, not at the Argonaute cleavage site). In still other embodiments, a dsRNA will have from one to all ribothymidines and have up to 100% 2'-fluoro. In further embodiments, a dsRNA will have from one to all ribothymidines and have up to 75% 2'-deoxy. In further embodiments, a dsRNA will have up to 75% LNA and have up to 75% 2'-methoxy. In still other embodiments, a dsRNA will have up to 75% LNA and have up to 100% 2'-fluoro. In further embodiments, a dsRNA will have up to 75% LNA and have up to 75% 2'-deoxy. In other embodiments, a dsRNA will have up to 75% 2'-methoxy and have up to 100% 2'-fluoro. In more embodiments, a dsRNA will have up to 75% 2'-methoxy and have up to 75% 2'-deoxy. In further embodiments, a dsRNA will have up to 100% 2'-fluoro and have up to 75% 2'-deoxy.

In further multiple modification embodiments, a dsRNA will have from one to all ribothymidines, up to 75% LNA, and up to 75% 2'-methoxy. In still further embodiments, a dsRNA will have from one to all ribothymidines, up to 75% LNA, and up to 100% 2'-fluoro. In further embodiments, a dsRNA will have from one to all ribothymidines, up to 75% LNA, and up to about 75% 2'-deoxy. In further embodiments, a dsRNA will have from one to all ribothymidines, up to 75% 2'-methoxy, and up to 75% 2'-fluoro. In further embodiments, a dsRNA will have from one to all ribothymidines, up to 75% 2'-methoxy, and up to 75% 2'-deoxy. In further embodiments, a dsRNA will have from one to all ribothymidines, up to 100% 2'-fluoro, and up to 75% 2'-deoxy. In yet further embodiments, a dsRNA will have from one to all ribothymidines, up to 75% LNA substitutions, up to 75% 2'-methoxy, up to 100% 2'-fluoro, and up to 75% 2'-deoxy. In other embodiments, a dsRNA will have up to 75% LNA, up to 75% 2'-methoxy, and up to 100% 2'-fluoro. In further embodiments, a dsRNA will have up to 75% LNA, up to 75% 2'-methoxy, and up to about 75% 2'-deoxy. In further embodiments, a dsRNA will have up to 75% LNA, up to 100% 2'-fluoro, and up to 75% 2'-deoxy. In still further embodiments, a dsRNA will have up to 75% 2'-methoxy, up to 100% 2'-fluoro, and up to 75% 2'-deoxy.

In any of these exemplary methods for using multiply modified dsRNA, the dsRNA may further comprise up to 100% phosphorothioate internucleoside linkages, from one to ten or more inverted base terminal caps, or any combination thereof. Additionally, any of these dsRNA may have these multiple modifications on one strand, two strands, three strands, a plurality of strands, or all strands, or on the same or different nucleoside within a dsRNA molecule. Finally, in any of these multiple modification dsRNA, the dsRNA must have gene silencing activity.

Within certain aspects, the present disclosure provides dsRNA that decreases expression of a WNT1, WNT2, or WNT3A gene by RNAi (*e.g.*, a WNT1 of SEQ ID NO:1158, WNT2 of SEQ ID NO:1390, or WNT3A of SEQ ID NO:1615), and compositions comprising one or more dsRNA, wherein at least one dsRNA comprises one or more universal-binding nucleotide(s) in the first, second or third position in the anti-codon of the antisense or sense strand of the dsRNA and wherein the dsRNA is capable of specifically binding to a WNT1, WNT2, or WNT3A sequence, such as an RNA expressed by a target cell. In cases wherein the sequence of a target WNT1, WNT2, or WNT3A RNA includes one or more single nucleotide substitutions, dsRNA comprising a universal-binding nucleotide retains its capacity to specifically bind a target WNT1, WNT2, or WNT3A RNA, thereby mediating gene silencing and, as a consequence, overcoming escape of the target WNT1, WNT2, or WNT3A from dsRNA-mediated gene silencing. Examplary universal-binding nucleotides that may be suitably employed in the compositions and methods disclosed herein include inosine, 1-β-*D*-ribofuranosyl-5-nitroindole, or 1-β-*D*-ribofuranosyl-3-nitropyrrole.

In certain aspects, dsRNA disclosed herein can include between about 1 universal-binding nucleotide and about 10 universal-binding nucleotides. Within other aspects, the presently disclosed dsRNA may comprise a sense strand that is homologous to a sequence of a WNT1, WNT2, or WNT3A gene and an antisense strand that is complementary to the sense strand, with the proviso that at least one nucleotide of the antisense or sense strand of the otherwise complementary dsRNA duplex has one or more universal-binding nucleotide.

### Synthesis of Nucleic Acid Molecules

Exemplary molecules of the instant disclosure are recombinantly produced, chemically synthesized, or a combination thereof. Oligonucleotides (*e.g.*, certain modified oligonucleotides or portions of oligonucleotides lacking ribonucleotides) are synthesized using protocols known in the art, for example as described in Caruthers et al., Methods in Enzymol. 211:3-19, 1992; Thompson et al., PCT Publication No. WO 99/54459, Wincott et al., Nucleic Acids Res. 23:2677-2684, 1995; Wincott et al., Methods Mol. Bio. 74:59, 1997; Brennan et al., Biotechnol Bioeng. 61:33-45, 1998; and Brennan, U.S. Patent No. 6,001,311. Synthesis of RNA, including certain dsRNA molecules and analogs thereof of this disclosure, can be made using the procedure as described in Usman et al., J. Am. Chem. Soc. 109:7845, 1987; Scaringe et al., Nucleic Acids Res. 18:5433, 1990; and Wincott et al., Nucleic Acids Res. 23:2677-2684, 1995; Wincott et al., Methods Mol. Bio. 74:59, 1997.

In certain embodiments, the nucleic acid molecules of the present disclosure can be synthesized separately and joined together post-synthetically, for example, by ligation (Moore et al., Science 256:9923, 1992; Draper et al., PCT Publication No. WO 93/23569; Shabarova et al., Nucleic Acids Res. 19:4247, 1991; Bellon et al., Nucleosides & Nucleotides 16:951, 1997; Bellon et al., Bioconjugate Chem. 8:204, 1997), or by hybridization following synthesis or deprotection.

In further embodiments, dsRNAs of this disclosure that decrease expression of a WNT1, WNT2, or WNT3A gene by RNAi can be made as single or multiple transcription products expressed by a polynucleotide vector encoding one or more dsRNAs and directing their expression within host cells. In these embodiments the double-stranded portion of a final transcription product of the dsRNAs to be expressed within the target cell can be, for example, about 5 to about 40 bp, about 15 to about 24 bp, or about 25 to about 40 bp long. Within exemplary embodiments, double-stranded portions of dsRNAs, in which two or more strands pair up, are not limited to completely paired nucleotide segments, and may contain non-pairing portions due to a mismatch (the corresponding nucleotides are not complementary), bulge (lacking in the corresponding complementary nucleotide on one strand), overhang, or the like. Non-pairing portions can be contained to the extent that they do not interfere with dsRNA formation and function. In certain embodiments, a "bulge" may comprise 1 to 2 non-pairing nucleotides, and the double-stranded region of dsRNAs in which two strands pair up may contain from about 1 to 7, or about 1 to 5 bulges. In addition, "mismatch" portions contained in the double-stranded region of dsRNAs may include from about 1 to 7, or about 1 to 5 mismatches. In other embodiments, the double-stranded region of dsRNAs of this disclosure may contain both bulge and mismatched portions in the approximate numerical ranges specified herein.

A dsRNA or analog thereof of this disclosure may be further comprised of a nucleotide, non-nucleotide, or mixed nucleotide/non-nucleotide linker that joins the sense region of the dsRNA to the antisense region of the dsRNA. In one embodiment, a nucleotide linker can be a linker of more than about 2 nucleotides length up to about 10 nucleotides in length. In another embodiment, the nucleotide linker can be a nucleic acid aptamer. By "aptamer" or "nucleic acid aptamer" as used herein is meant a nucleic acid molecule that binds specifically to a target molecule wherein the nucleic acid molecule has sequence that comprises a sequence recognized by the target molecule in its natural setting. Alternately, an aptamer can be a nucleic acid molecule that binds to a target molecule wherein the target molecule does not naturally bind to a nucleic acid. The target molecule can be any molecule of interest. For example, the aptamer can be used to bind to a ligand-binding domain of a protein, thereby preventing interaction of the naturally occurring ligand with the protein. This is a non-limiting example and those in the art will recognize that other embodiments can be readily generated using techniques generally known in the art (*see, e.g.,* Gold et al., Annu. Rev. Biochem. 64:763, 1995; Brody and Gold, J. Biotechnol. 74:5, 2000; Sun, Curr. Opin. Mol. Ther. 2:100, 2000; Kusser, J. Biotechnol. 74:27, 2000; Hermann and Patel, Science 287:820, 2000; and Jayasena, Clinical Chem. 45:1628, 1999).

A non-nucleotide linker may be comprised of an abasic nucleotide, polyether, polyamine, polyamide, peptide, carbohydrate, lipid, polyhydrocarbon, or other polymeric compounds (*e.g.*, polyethylene glycols such as those having between 2 and 100 ethylene glycol units). Specific examples include those described by Seela and Kaiser, Nucleic Acids Res. 18:6353, 1990, and Nucleic Acids Res. 15:3113, 1987; Cload and Schepartz, J. Am. Chem. Soc. 113:6324, 1991; Richardson and Schepartz, J. Am. Chem. Soc. 113:5109, 1991; Ma et al., Nucleic Acids Res. 21:2585, 1993, and Biochemistry 32:1751, 1993; Durand et al., Nucleic Acids Res. 18:6353, 1990; McCurdy et al., Nucleosides & Nucleotides 10:287, 1991; Jaschke et al., Tetrahedron Lett. 34:301, 1993; Ono et al., Biochemistry 30:9914, 1991; Arnold et al., PCT Publication No. WO 89/02439; Usman et al., PCT Publication No. WO 95/06731; Dudycz et al., PCT Publication No. WO 95/11910 and Ferentz and Verdine, J. Am. Chem. Soc. 113:4000, 1991. The synthesis of a dsRNA molecule of this disclosure, which can be further modified, comprises: (a) synthesis of a first (antisense) strand and synthesis of a second (sense) strand and a third (sense) strand that are each complementary to non-overlapping regions of the first strand; and (b) annealing the first, second and third strands together under conditions suitable to obtain a dsRNA molecule. In another embodiment, synthesis of the first, second and thirdstrands of a dsRNA molecule is by solid phase oligonucleotide synthesis. In yet another embodiment, synthesis of the first, second ,and third strands of a dsRNA molecule is by solid phase tandem oligonucleotide synthesis.

Chemically synthesizing nucleic acid molecules with substitutions or modifications (base, sugar, phosphate, or any combination thereof) can prevent their degradation by serum ribonucleases, which may lead to increased potency. *See, e.g.,* Eckstein et al., PCT Publication No. WO 92/07065; Perrault et al., Nature 344:565, 1990; Pieken et al., Science 253:314, 1991; Usman and Cedergren, Trends in Biochem. Sci. 17:334, 1992; Usman et al., Nucleic Acids Symp. Ser. 31:163, 1994; Beigelman et al., J. Biol. Chem. 270:25702, 1995; Burgin et al., Biochemistry 35:14090, 1996; Burlina et al., Bioorg. Med. Chem. 5:1999, 1997; Thompson et al., Karpeisky et al., Tetrahedron Lett. 39:1131, 1998; Earnshaw and Gait, Biopolymers (Nucleic Acid Sciences) 48:39-55, 1998; Verma and Eckstein, Annu. Rev. Biochem. 67:99-134, 1998; Herdewijn, Antisense Nucleic Acid Drug Dev. 10:297, 2000; Kurreck, Eur. J. Biochem. 270:1628, 2003*;* Dorsett and Tuschl, Nature Rev. Drug Discov. 3:318, 2004; PCT Publication Nos. WO 91/03162; WO 93/15187; WO 97/26270; WO 98/13526; U.S. Patent Nos. 5,334,711; 5,627,053; 5,716,824; 5,767, 264; 6,300,074. Each of the above references discloses various substitutions and chemical modifications to the base, phosphate, or sugar moieties of nucleic acid molecules, which can be used in the dsRNAs described herein. For example, oligonucleotides can be modified at the sugar moiety to enhance stability or prolong biological activity by increasing nuclease resistance. Representative sugar modifications include 2'-amino, 2'-C-allyl, 2'-fluoro, 2'-O-methyl, 2'-O-allyl, or 2'-H. Other modifications to enhance stability or prolong biological activity can be internucleoside linkages, such as phosphorothioate, or base-modifications, such as locked nucleic acids *(see, e.g.,* U.S. Patent Nos. 6,670,461; 6,794,499; 6,268,490), or 5-methyluridine or 2'-O-methyl-5-methyluridine in place of uridine (*see, e.g.,* U.S. Patent Application Publication No. 2006/0142230). Hence, dsRNA molecules of the instant disclosure can be modified to increase nuclease resistance or duplex stability while substantially retaining or having enhanced RNAi activity as compared to unmodified dsRNA.

In one embodiment, this disclosure features substituted or modified dsRNA molecules, such as phosphate backbone modifications comprising one or more phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, or alkylsilyl substitutions. For a review of oligonucleotide backbone modifications, *see* Hunziker and Leumann, Nucleic Acid Analogues: Synthesis and Properties, in Modern Synthetic Methods, VCH, 331-417, 1995; and Mesmaeker et al., ACS, 24-39, 1994.

In another embodiment, a conjugate molecule can be optionally attached to a dsRNA or analog thereof that decreases expression of a WNT1, WNT2, or WNT3A gene by RNAi. For example, such conjugate molecules may be polyethylene glycol, human serum albumin, polyarginine, Gln-Asn polymer, or a ligand for a cellular receptor that can, for example, mediate cellular uptake (*e.g.*, HIV TAT, *see* Vocero-Akbani et al., Nature Med. 5:23, 1999; *see also* U.S. Patent Application Publication No. 2004/0132161).. Examples of specific conjugate molecules contemplated by the instant disclosure that can be attached to a dsRNA or analog thereof of this disclosure are described in Vargeese et al., U.S. Patent Application Publication No. 2003/0130186, and U.S. Patent Application Publication No. 2004/0110296. In another embodiment, a conjugate molecule is covalently attached to a dsRNA or analog thereof that decreases expression of a WNT1, WNT2, or WNT3A gene by RNAi via a biodegradable linker. In certain embodiments, a conjugate molecule can be attached at the 3'-end of either the sense strand, the antisense strand, or both strands of a dsRNA molecule provided herein. In another embodiment, a conjugate molecule can be attached at the 5'-end of either the sense strand, the antisense strand, or both strands of the dsRNA or analog thereof. In yet another embodiment, a conjugate molecule is attached at both the 3'-end and 5'-end of either the sense strand, the antisense strand, or both strands of a dsRNA molecule, or any combination thereof. In further embodiments, a conjugate molecule of this disclosure comprises a molecule that facilitates delivery of a dsRNA or analog thereof into a biological system, such as a cell. A person of skill in the art can screen dsRNA of this disclosure having various conjugates to determine whether the dsRNA-conjugate possesses improved properties (*e.g.*, pharmacokinetic profiles, bioavailability, stability) while maintaining the ability to mediate RNAi in, for example, an animal model as described herein or generally known in the art.

### Methods for Selecting dsRNA Molecules Specific for WNT1, WNT2, or WNT3A

As indicated herein, the present disclosure also provides methods for selecting dsRNA and analogs thereof that are capable of specifically binding to a WNT1, WNT2, or WNT3A gene (including a mRNA splice variant thereof) while being incapable of specifically binding or minimally binding to non-WNT1, non-WNT2, or non-WNT3A genes. The selection process disclosed herein is useful, for example, in eliminating dsRNAs analogs that are cytotoxic due to non-specific binding to, and subsequent degradation of, one or more non-WNT1, non-WNT2, or non-WNT3A genes.

Methods of the present disclosure do not require *a priori* knowledge of the nucleotide sequence of every possible gene variant (including mRNA splice variants) targeted by the dsRNA or analog thereof. In one embodiment, the nucleotide sequence of the dsRNA is selected from a conserved region or consensus sequence of a WNT1, WNT2, or WNT3A gene. In another embodiment, the nucleotide sequence of the dsRNA may be selectively or preferentially targeted to a certain sequence contained in an mRNA splice variant of a WNT1, WNT2, or WNT3A gene.

In certain embodiments, methods are provided for selecting one or more dsRNA molecule that decreases expression of a WNT1, WNT2, or WNT3A gene by RNAi, comprising a first strand that is complementary to a WNT1 mRNA set forth in SEQ ID NO:1158, WNT2 mRNA set forth in SEQ ID NO:1390, or WNT3A mRNA set forth in SEQ ID NO:1615, and a second strand that is complementary to the first strand, wherein the first and second strands form a double-stranded region of about 15 to about 40 base pairs *(see, e.g.,* WNT1, WNT2, or WNT3A sequences in the Sequence Listing identified herein), and wherein at least one uridine of the dsRNA molecule is replaced with a 5-methyluridine or 2-thioribothymidine or 2'-O-methyl-5-methyluridine, which methods employ "off-target" profiling whereby one or more dsRNA provided herein is contacted with a cell, either *in vivo* or *in vitro,* and total mRNA is collected for use in probing a microarray comprising oligonucleotides having one or more nucleotide sequence from a panel of known genes, including non-WNT1, non-WNT2, or non-WNT3A genes (*e.g.*, interferon). Within related embodiments, one or more dsRNA molecule that decreases expressioni of a WNT1, WNT2, or WNT3A gene by RNAi may further comprise a third strand that is complementary to the first strand, wherein the first and third strands form a double-stranded region wherein the double-stranded region formed by the first and third strands is non-overlapping with a double-stranded region formed by the first and second strands. The "off-target" profile of the dsRNA provided herein is quantified by determining the number of non-WNT1, non-WNT2, or non-WNT3A genes having reduced expression levels in the presence of the candidate dsRNAs. The existence of "off target" binding indicates a dsRNA is capable of specifically binding to one or more non-WNT1, non-WNT2, or non-WNT3A gene messages. In certain embodiments, a dsRNA as provided herein *(see, e.g.,* sequences in the Sequence Listing identified herein) applicable to therapeutic use will exhibit a greater stability, minimal interferon response, and little or no "off-target" binding.

Still further embodiments provide methods for selecting more efficacious dsRNA by using one or more reporter gene constructs comprising a constitutive promoter, such as a cytomegalovirus (CMV) or phosphoglycerate kinase (PGK) promoter, operably fused to, and capable of altering the expression of one or more reporter genes, such as a luciferase, chloramphenicol (CAT), or β-galactosidase, which, in turn, is operably fused in-frame with a dsRNA (such as one having a length between about 15 base-pairs and about 40 base-pairs or from about 5 nucleotides to about 24 nucleotides, or about 25 nucleotides to about 40 nucleotides) that contains a WNT1, WNT2, or WNT3A sequence, as provided herein.

Individual reporter gene expression constructs may be co-transfected with one or more dsRNA or analog thereof. The capacity of a given dsRNA to reduce the expression level of WNT1, WNT2, or WNT3A may be determined by comparing the measured reporter gene activity in cells transfected with or without a dsRNA molecule of interest.

Certain embodiments disclosed herein provide methods for selecting one or more modified dsRNA molecule(s) that employ the step of predicting the stability of a dsRNA duplex. In some embodiments, such a prediction is achieved by employing a theoretical melting curve wherein a higher theoretical melting curve indicates an increase in dsRNA duplex stability and a concomitant decrease in cytotoxic effects. Alternatively, stability of a dsRNA duplex may be determined empirically by measuring the hybridization of a single RNA analog strand as described herein to a complementary target gene within, for example, a polynucleotide array. The melting temperature (*i.e*., the T*ₘ* value) for each modified RNA and complementary RNA immobilized on the array can be determined and, from this T*ₘ* value, the relative stability of the modified RNA pairing with a complementary RNA molecule determined.

For example, Kawase et al. (Nucleic Acids Res. 14:7727, 1986) have described an analysis of the nucleotide-pairing properties of Di (inosine) to A, C, G , and T, which was achieved by measuring the hybridization of oligonucleotides (ODNs) with Di in various positions to complementary sets of ODNs made as an array. The relative strength of nucleotide-pairing is I-C > I-A > I-G ≈ I-T. Generally, Di containing duplexes showed lower T*ₘ* values when compared to the corresponding wild type (WT) nucleotide pair. The stabilization of Di by pairing was in order of Dc > Da > Dg > Dt > Du. As a person of skill in the art would understand, although universal-binding nucleotides are used herein as an example of determining duplex stability (*i.e.*, the T*ₘ* value), other nucleotide substitutions (*e.g.,* 5-methyluridine for uridine) or further modifications (*e.g*., a ribose modification at the 2'-position) can also be evaluated by these or similar methods.

In still further embodiments of the presently disclosed methods, one or more anti-codon within an antisense strand of a dsRNA molecule or analog thereof is substituted with a universal-binding nucleotide in a second or third position in the anti-codon of the antisense strand. By substituting a universal-binding nucleotide for a first or second position, the one or more first or second position nucleotide-pair substitution allows the substituted dsRNA molecule to specifically bind to mRNA wherein a first or a second position nucleotide-pair substitution has occurred, wherein the one or more nucleotide-pair substitution results in an amino acid change in the corresponding gene product.

Any of these methods of identifying dsRNA of interest can also be used to examine a dsRNA that decreases expression of a WNT1, WNT2, or WNT3A gene by RNA interference, comprising a first strand that is complementary to a WNT1 mRNA set forth in SEQ ID NO:1158, WNT2 mRNA set forth in SEQ ID NO: 1390, or WNT3A mRNA set forth in SEQ ID NO:1615, and a second and third strand that have non-overlapping complementarity to the first strand, wherein the first and at least one of the second or third strand optionally form a double-stranded region of about 5 to about 13 base pairs; wherein at least one pyrimidine nucleoside of the dsRNA is according to Formula I or II: 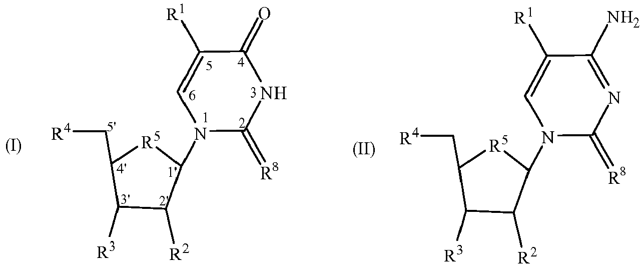 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C=N, or heterocyclo group; R³ and R⁴ are each independently a hydroxyl, a protected hydroxyl, or an internucleoside linking group; and R⁵ and R⁸ are independently O or S. In certain embodiments, at least one nucleoside is according to Formula I in which R¹ is methyl and R² is -OH, or R¹ is methyl, R² is -OH, and R⁸ is S. In certain embodiments, at least one nucleoside is according to Formula I in which R¹ is methyl and R² is -O-methyl, or R¹ is methyl, R² is -O-methyl, and R⁸ is O. In other embodiments, the internucleoside linking group covalently links from about 5 to about 40 nucleosides.

### Compositions and Methods of Use

As set forth herein, dsRNA of the instant disclosure are designed to target a WNT1, WNT2, or WNT3A gene (including one or more mRNA splice variant thereof) that is expressed at an elevated level or continues to be expressed when it should not, and is a causal or contributing factor associated with, for example, atherosclerosis, diabetes mellitus, and cerebrovascular disease, state, or adverse condition. In this context, a dsRNA or analog thereof of this disclosure will effectively downregulate expression of a WNT1, WNT2, or WNT3A gene to levels that prevent, alleviate, or reduce the severity or recurrence of one or more associated disease symptoms. Alternatively, for various distinct disease models in which expression of a WNT1, WNT2, or WNT3A gene is not necessarily elevated as a consequence or sequel of disease or other adverse condition, down regulation of a WNT1, WNT2, or WNT3A gene will nonetheless result in a therapeutic result by lowering gene expression (*i.e.*, to reduce levels of a selected mRNA or protein product of a WNT1, WNT2, or WNT3A gene). Furthermore, dsRNAs of this disclosure may be targeted to lower expression of WNT1, WNT2, or WNT3A, which can result in upregulation of a "downstream" gene whose expression is negatively regulated, directly or indirectly, by a WNT1, WNT2, or WNT3A protein. The dsRNA molecules of the instant disclosure comprise useful reagents and can be used in methods for a variety of therapeutic, diagnostic, target validation, genomic discovery, genetic engineering, and pharmacogenomic applications.

In certain embodiments, aqueous suspensions contain dsRNA of this disclosure in admixture with suitable excipients, such as suspending agents or dispersing or wetting agents. Exemplary suspending agents include sodium carboxymethylcellulose, methylcellulose, hydropropyl-methylcellulose, sodium alginate, polyvinylpyrrolidone, gum tragacanth and gum acacia. Representative dispersing or wetting agents include naturally-occurring phosphatides (*e.g.*, lecithin), condensation products of an alkylene oxide with fatty acids (*e.g.*, polyoxyethylene stearate), condensation products of ethylene oxide with long chain aliphatic alcohols (*e.g.*, heptadecaethyleneoxycetanol), condensation products of ethylene oxide with partial esters derived from fatty acids and hexitol (*e.g.*, polyoxyethylene sorbitol monooleate), or condensation products of ethylene oxide with partial esters derived from fatty acids and hexitol anhydrides (*e.g.*, polyethylene sorbitan monooleate). In certain embodiments, the aqueous suspensions can optionally contain one or more preservatives (*e.g.*, ethyl or *n*-propyl-*p-*hydroxybenzoate), one or more coloring agents, one or more flavoring agents, or one or more sweetening agents (*e.g.*, sucrose, saccharin). In additional embodiments, dispersible powders and granules suitable for preparation of an aqueous suspension by the addition of water provide dsRNA of this disclosure in admixture with a dispersing or wetting agent, suspending agent and optionally one or more preservative, coloring agent, flavoring agent, or sweetening agent.

The present disclosure includes dsRNA compositions prepared for storage or administration that include a pharmaceutically effective amount of a desired compound in a pharmaceutically acceptable carrier or diluent. Acceptable carriers or diluents for therapeutic use are well known in the pharmaceutical art, and are described, for example, in Remington's Pharmaceutical Sciences, Mack Publishing Co., A.R. Gennaro edit., 1985, hereby incorporated by reference herein. In certain embodiments, pharmaceutical compositions of this disclosure can optionally include preservatives, antioxidants, stabilizers, dyes, flavoring agents, or any combination thereof. Exemplary preservatives include sodium benzoate, sorbic acid, chlorobutanol, and esters of p-hydroxybenzoic acid.

The dsRNA compositions of the instant disclosure can be effectively employed as pharmaceutically-acceptable formulations. Pharmaceutically-acceptable formulations prevent, alter the occurrence or severity of, or treat (alleviate one or more symptom(s) to a detectable or measurable extent) of a disease state or other adverse condition in a subject. A pharmaceutically acceptable formulation includes salts of the above compounds, *e.g*., acid addition salts, such as salts of hydrochloric acid, hydrobromic acid, acetic acid, or benzene sulfonic acid. A pharmaceutical composition or formulation refers to a composition or formulation in a form suitable for administration into a cell, or a subject such as a human (*e.g.*, systemic administration). The formulations of the present disclosure, having an amount of dsRNA sufficient to treat or prevent a disorder associated with WNT1, WNT2, or WNT3A gene expression are, for example, suitable for topical (*e.g.*, creams, ointments, skin patches, eye drops, ear drops) application or administration. Other routes of administration include oral, parenteral, sublingual, bladder wash-out, vaginal, rectal, enteric, suppository, nasal, and inhalation. The term parenteral, as used herein, includes subcutaneous, intravenous, intramuscular, intraarterial, intraabdominal, intraperitoneal, intraarticular, intraocular or retrobulbar, intraaural, intrathecal, intracavitary, intracelial, intraspinal, intrapulmonary or transpulmonary, intrasynovial, and intraurethral injection or infusion techniques. The pharmaceutical compositions of the present disclosure are formulated to allow the dsRNA contained therein to be bioavailable upon administration to a subject.

In further embodiments, dsRNA of this disclosure can be formulated as oily suspensions or emulsions (*e.g.*, oil-in-water) by suspending dsRNA in, for example, a vegetable oil (*e.g.,* arachis oil, olive oil, sesame oil or coconut oil) or a mineral oil (*e.g.*, liquid paraffin). Suitable emulsifying agents can be naturally-occurring gums (*e.g.*, gum acacia or gum tragacanth), naturally-occurring phosphatides (*e.g.*, soy bean, lecithin, esters or partial esters derived from fatty acids and hexitol), anhydrides (*e.g.*, sorbitan monooleate), or condensation products of partial esters with ethylene oxide (*e.g.*, polyoxyethylene sorbitan monooleate). In certain embodiments, the oily suspensions or emulsions can optionally contain a thickening agent, such as beeswax, hard paraffin or cetyl alcohol. In related embodiments, sweetening agents and flavoring agents can optionally be added to provide palatable oral preparations. In yet other embodiments, these compositions can be preserved by optionally adding an anti-oxidant, such as ascorbic acid.

In further embodiments, dsRNA of this disclosure can be formulated as syrups and elixirs with sweetening agents (*e.g.*, glycerol, propylene glycol, sorbitol, glucose or sucrose). Such formulations can also contain a demulcent, preservative, flavoring, coloring agent, or any combination thereof. In other embodiments, pharmaceutical compositions comprising dsRNA of this disclosure can be in the form of a sterile, injectable aqueous or oleaginous suspension. The sterile injectable preparation can also be a sterile, injectable solution or suspension in a nontoxic parenterally acceptable diluent or solvent (*e.g.*, as a solution in 1,3-butanediol). Among the exemplary acceptable vehicles and solvents useful in the compositions of this disclosure is water, Ringer's solution, or isotonic sodium chloride solution. In addition, sterile, fixed oils may be employed as a solvent or suspending medium for the dsRNA of this disclosure. For this purpose, any bland fixed oil can be employed including synthetic mono- or diglycerides. In addition, fatty acids such as oleic acid find use in the preparation of parenteral formulations.

Within certain embodiments of this disclosure, pharmaceutical compositions and methods are provided that feature the presence or administration of one or more dsRNA or analogs thereof of this disclosure, combined, complexed, or conjugated with a polypeptide, optionally formulated with a pharmaceutically-acceptable carrier, such as a diluent, stabilizer, buffer, or the like. The negatively charged dsRNA molecules of this disclosure may be administered to a patient by any standard means, with or without stabilizers, buffers, or the like, to form a composition suitable for treatment. When it is desired to use a liposome delivery mechanism, standard protocols for formation of liposomes can be followed. The compositions of the present disclosure may also be formulated and used as a tablet, capsule or elixir for oral administration, suppository for rectal administration, sterile solution, or suspension for injectable administration, either with or without other compounds known in the art. Thus, dsRNAs of the present disclosure may be administered in any form, such as nasally, transdermally, parenterally, or by local injection.

In accordance with this disclosure, dsRNA molecules (optionally substituted or modified or conjugated), compositions thereof, and methods for inhibiting expression of a WNT1, WNT2, or WNT3A gene in a cell or organism are provided. In certain embodiments, this disclosure provides methods and dsRNA compositions for treating a subject, including a human cell, tissue or individual, having a disease or at risk of developing a disease caused by or associated with the expression of a WNT1, WNT2, or WNT3A gene. In one embodiment, the method includes administering a dsRNA of this disclosure or a pharmaceutical composition containing the dsRNA to a cell or an organism, such as a mammal, such that expression of the target gene is silenced. Subjects (*e.g.*, mammalian, human) amendable for treatment using the dsRNA molecules (optionally substituted or modified or conjugated), compositions thereof, and methods of the present disclosure include those suffering from one or more disease or condition mediated, at least in part, by overexpression or inappropriate expression of a WNT1, WNT2, or WNT3A gene, or which are amenable to treatment by reducing expression of a WNT1, WNT2, or WNT3A protein, including coronary artery disease (*i.e.*, coronary heart disease, ischaemic heart disease), atherosclerosis, diabetes mellitus, dyslipidemia (*e.g.*, hyperlipidemia), peripheral vascular and ischemic cerebrovascular disease, and risk of ischemic stroke (cerebral thrombosis and cerebral embolisms) and hemorrhagic stroke (cerebral hemorrhage and subarachnoid hemorrhage). Within exemplary embodiments, the compositions and methods of this disclosure are also useful as therapeutic tools to regulate expression of WNT1, WNT2, or WNT3A to treat or prevent symptoms of, for example, the conditions listed herein.

In any of the methods disclosed herein there may be used with one or more dsRNA, or substituted or modified dsRNA, as described herein, comprising a first strand that is complementary to a human Wingless-Type MMTV (Murine Mammary Tumor Virus) Integration Site Family, Member 1 (WNT1) mRNA as set forth in SEQ ID NO:1158, a human Wingless-Type MMTV (Murine Mammary Tumor Virus) Integration Site Family, Member 2 (WNT2) mRNA as set forth in SEQ ID NO:1390, or a human Wingless-Type MMTV (Murine Mammary Tumor Virus) Integration Site Family, Member 3A (WNT3A) mRNA as set forth in SEQ ID NO:1615, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the mdRNA molecule optionally includes at least one double-stranded region of 5 base pairs to 13 base pairs. In other embodiments, subjects can be effectively treated, prophylactically or therapeutically, by administering an effective amount of one or more dsRNA having a first strand that is complementary to a human Wingless-Type MMTV (Murine Mammary Tumor Virus) Integration Site Family, Member 1 (WNT1) mRNA as set forth in SEQ ID NO:1158, a human Wingless-Type MMTV (Murine Mammary Tumor Virus) Integration Site Family, Member 2 (WNT2) mRNA as set forth in SEQ ID NO:1390, or a human Wingless-Type MMTV (Murine Mammary Tumor Virus) Integration Site Family, Member 3A (WNT3A) mRNA as set forth in SEQ ID NO:1615, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the mdRNA molecule optionally includes at least one double-stranded region of 5 to 13 base pairs and at least one pyrimidine nucleoside of the mdRNA is according to Formula I or II: 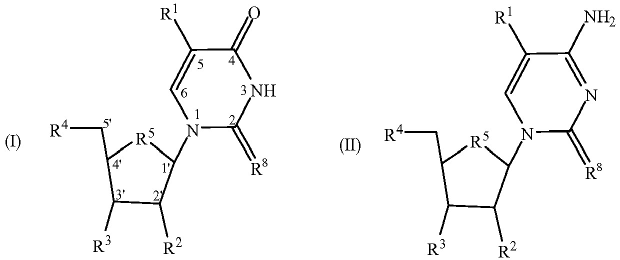 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C=N, or heterocyclo group; R³ and R⁴ are each independently a hydroxyl, a protected hydroxyl, or an internucleoside linking group; and R⁵ and R⁸ are independently O or S. In certain embodiments, at least one nucleoside is according to Formula I in which R¹ is methyl and R² is -OH, or R¹ is methyl, R² is -OH, and R⁸ is S. In certain embodiments, at least one nucleoside is according to Formula I in which R¹ is methyl and R² is -O-methyl, or R¹ is methyl, R² is -O-methyl, and R⁸ is O. In other embodiments, the internucleoside linking group covalently links from about 5 to about 40 nucleosides.

In any of the methods described herein, the dsRNA used may include multiple modifications. For example, a dsRNA can have at least one 5-methyluridine, 2'-O-methyl-5-methyluridine, LNA, 2'-methoxy, 2'-fluoro, 2'-deoxy, phosphorothioate linkage, inverted base terminal cap, or any combination thereof. In certain exemplary methods, a dsRNA will have from one to all 5-methyluridines and have up to about 75% LNA. In other exemplary methods, a dsRNA will have from one to all 5-methyluridines and have up to about 75% 2'-methoxy provided the 2'-methoxy are not at the Argonaute cleavage site. In still other exemplary methods, a dsRNA will have from one to all 5-methyluridines and have up to about 100% 2'-fluoro substitutions. In further exemplary methods, a dsRNA will have from one to all 5-methyluridines and have up to about 75% 2'-deoxy. In further exemplary methods, a dsRNA will have up to about 75% LNA and have up to about 75% 2'-methoxy. In still other embodiments, a dsRNA will have up to about 75% LNA and have up to about 100% 2'-fluoro. In further exemplary methods, a dsRNA will have up to about 75% LNA and have up to about 75% 2'-deoxy. In further exemplary methods, a dsRNA will have up to about 75% 2'-methoxy and have up to about 100% 2'-fluoro. In further exemplary methods, a dsRNA will have up to about 75% 2'-methoxy and have up to about 75% 2'-deoxy. In further embodiments, a dsRNA will have up to about 100% 2'-fluoro and have up to about 75% 2'-deoxy.

In other exemplary methods for using multiply modified dsRNA, a dsRNA will have from one to all uridines substituted with 5-methyluridine, up to about 75% LNA, and up to about 75% 2'-methoxy. In still further exemplary methods, a dsRNA will have from one to all 5-methyluridines, up to about 75% LNA, and up to about 100% 2'-fluoro. In further exemplary methods, a dsRNA will have from one to all 5-methyluridines, up to about 75% LNA, and up to about 75% 2'-deoxy. In further exemplary methods, a dsRNA will have from one to all 5-methyluridines, up to about 75% 2'-methoxy, and up to about 75% 2'-fluoro. In further exemplary methods, a dsRNA will have from one to all 5-methyluridines, up to about 75% 2'-methoxy, and up to about 75% 2'-deoxy. In more exemplary methods, a dsRNA will have from one to all 5-methyluridines, up to about 100% 2'-fluoro, and up to about 75% 2'-deoxy. In yet other exemplary methods, a dsRNA will have from one to all 5-methyluridines, up to about 75% LNA, up to about 75% 2'-methoxy, up to about 100% 2'-fluoro, and up to about 75% 2'-deoxy. In other exemplary methods, a dsRNA will have up to about 75% LNA, up to about 75% 2'-methoxy, and up to about 100% 2'-fluoro. In further exemplary methods, a dsRNA will have up to about 75% LNA, up to about 75% 2'-methoxy, and up to about 75% 2'-deoxy. In more exemplary methods, a dsRNA will have up to about 75% LNA, up to about 100% 2'-fluoro, and up to about 75% 2'-deoxy. In still further exemplary methods, a dsRNA will have up to about 75% 2'-methoxy, up to about 100% 2'-fluoro, and up to about 75% 2'-deoxy.

In any of these exemplary methods for using multiply modified dsRNA, the dsRNA may further comprise up to 100% phosphorothioate internucleoside linkages, from one to ten or more inverted base terminal caps, or any combination thereof. Additionally, any of these dsRNA may have these multiple modifications on one strand, two strands, three strands, a plurality of strands, or all strands, or on the same or different nucleoside within a dsRNA molecule. Finally, in any of these multiple modification dsRNA, the dsRNA must have gene silencing activity.

In further embodiments, subjects can be effectively treated, prophylactically or therapeutically, by administering an effective amount of one or more dsRNA, or substituted or modified dsRNA as described herein, having a first strand that is complementary to a WNT1 mRNA as set forth in SEQ ID NO:1158, WNT2 mRNA as set forth in SEQ ID NO:1390, or WNT3A mRNA as set forth in SEQ ID NO:1615, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the combined double-stranded regions total about 15 base pairs to about 40 base pairs and the mdRNA molecule optinally has blunt ends. In still further embodiments, methods disclosed herein there may be used with one or more dsRNA that comprises a first strand that is complementary to a WNT1 mRNA as set forth in SEQ ID NO:1158, WNT2 mRNA as set forth in SEQ ID NO: 1390, or WNT3A mRNA as set forth in SEQ ID NO:1615, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein the mdRNA has a combined double-stranded region totaling about 15 to about 40 base pairs, optionally has at least one double-stranded region of 5 base pairs to 13 base pairs, optionally has one or more blunt ends, or any combination thereof, and at least one pyrimidine nucleoside of the mdRNA is according to Formula I or II: 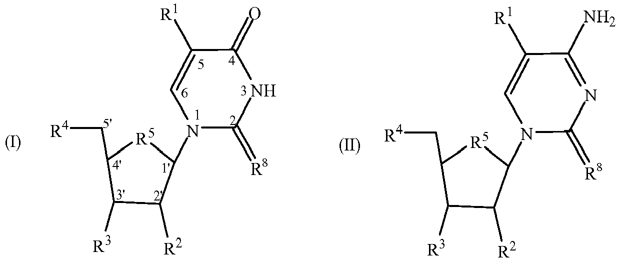 wherein R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C=N, or heterocyclo group; R³ and R⁴ are each independently a hydroxyl, a protected hydroxyl, or an internucleoside linking group; and R⁵ and R⁸ are independently O or S. In certain embodiments, at least one nucleoside is according to Formula I in which R¹ is methyl and R² is -OH, or R¹ is methyl, R² is -OH, and R⁸ is S. In certain embodiments, at least one nucleoside is according to Formula I in which R¹ is methyl and R² is -O-methyl, or R¹ is methyl, R² is -O-methyl, and R⁸ is O. In other embodiments, the internucleoside linking group covalently links from about 5 to about 40 nucleosides.

Within additional aspects of this disclosure, combination formulations and methods are provided comprising an effective amount of one or more dsRNA of the present disclosure in combination with one or more secondary or adjunctive active agents that are formulated together or administered coordinately with the dsRNA of this disclosure to control a WNT1-, WNT2-, or WNT3A-associated disease or condition as described herein. Useful adjunctive therapeutic agents in these combinatorial formulations and coordinate treatment methods include, for example, enzymatic nucleic acid molecules, allosteric nucleic acid molecules, antisense, decoy, or aptamer nucleic acid molecules, antibodies such as monoclonal antibodies, small molecules and other organic or inorganic compounds including metals, salts and ions, and other drugs and active agents indicated for treating a WNT1-, WNT2-, or WNT3A-associated disease or condition, including chemotherapeutic agents used to treat cancer, steroids, non-steroidal anti-inflammatory drugs (NSAIDs), tyrosine kinase inhibitors, or the like.

Exemplary chemotherapeutic agents include alkylating agents (*e.g.*, cisplatin, oxaliplatin, carboplatin, busulfan, nitrosoureas, nitrogen mustards, uramustine, temozolomide), antimetabolites (*e.g.*, aminopterin, methotrexate, mercaptopurine, fluorouracil, cytarabine), taxanes (*e.g.*, paclitaxel, docetaxel), anthracyclines (*e.g.*, doxorubicin, daunorubicin, epirubicin, idaruicin, mitoxantrone, valrubicin), bleomycin, mytomycin, actinomycin, hydroxyurea, topoisomerase inhibitors (*e.g.*, camptothecin, topotecan, irinotecan, etoposide, teniposide), monoclonal antibodies (*e.g.*, alemtuzumab, bevacizumab, cetuximab, gemtuzumab, panitumumab, rituximab, tositumomab, trastuzumab), vinca alkaloids (*e.g.*, vincristine, vinblastine, vindesine, vinorelbine), cyclophosphamide, prednisone, leucovorin, oxaliplatin.

Some adjunctive therapies may be directed at targets that interact or associate with WNT1, WNT2, or WNT3A or affect specific WNT1, WNT2, or WNT3A biological activities. Adjunctive therapies include statins (*e.g.*, rosuvastatin, lovastatin, atorvastatin, cerivastatin, fluvastatin, mevastatin, pitavastatin, pravastatin, simvastatin), frizzled-related proteins (sFRPs), Wnt-inhibitor factor-1 (WIF-1), Dickkofp (Dkk), diuretics, pentoxifylline, tumor necrosis factor alpha inhibitors, small molecules inhibitors of WNT1, WNT2, or WNT3A, antisense molecules, niacin, additional small molecules, rationally designed peptides, and antibodies or fragments thereof.

To practice the coordinate administration methods of this disclosure, a dsRNA is administered, simultaneously or sequentially, in a coordinated treatment protocol with one or more of the secondary or adjunctive therapeutic agents contemplated herein. The coordinate administration may be done in any order, and there may be a time period while only one or both (or all) active therapeutic agents, individually or collectively, exert their biological activities. A distinguishing aspect of all such coordinate treatment methods is that the dsRNA present in a composition elicits some favorable clinical response, which may or may not be in conjunction with a secondary clinical response provided by the secondary therapeutic agent. For example, the coordinate administration of the dsRNA with a secondary therapeutic agent as contemplated herein can yield an enhanced (synergistic) therapeutic response beyond the therapeutic response elicited by either or both the purified dsRNA or secondary therapeutic agent alone.

In another embodiment, a dsRNA of this disclosure can include a conjugate member on one or more of the terminal nucleotides of a dsRNA. The conjugate member can be, for example, a lipophile, a terpene, a protein binding agent, a vitamin, a carbohydrate, or a peptide. For example, the conjugate member can be naproxen, nitroindole (or another conjugate that contributes to stacking interactions), folate, ibuprofen, or a C5 pyrimidine linker. In other embodiments, the conjugate member is a glyceride lipid conjugate (*e.g.*, a dialkyl glyceride derivatives), vitamin E conjugates, or thio-cholesterols. Additional conjugate members include peptides that function, when conjugated to a modified dsRNA of this disclosure, to facilitate delivery of the dsRNA into a target cell, or otherwise enhance delivery, stability, or activity of the dsRNA when contacted with a biological sample (*e.g.*, a target cell expressing WNT1, WNT2, or WNT3A). Exemplary peptide conjugate members for use within these aspects of this disclosure, include peptides PN27, PN28, PN29, PN58, PN61, PN73, PN158, PN159, PN173, PN182, PN183, PN202, PN204, PN250, PN361, PN365, PN404, PN453, PN509, and PN963, described, for example, in U.S. Patent Application Publication Nos. 2006/0040882 and 2006/0014289, and U.S. Provisional Patent Application Nos. 60/822,896 and 60/939,578; and PCT Application PCT/US2007/075744, which are all incorporated herein by reference. In certain embodiments, when peptide conjugate partners are used to enhance delivery of dsRNA of this disclosure, the resulting dsRNA formulations and methods will often exhibit further reduction of an interferon response in target cells as compared to dsRNAs delivered in combination with alternate delivery vehicles, such as lipid delivery vehicles (*e.g.*, Lipofectamine^{™}).

In still another embodiment, a dsRNA or analog thereof of this disclosure may be conjugated to the polypeptide and admixed with one or more non-cationic lipids or a combination of a non-cationic lipid and a cationic lipid to form a composition that enhances intracellular delivery of the dsRNA as compared to delivery resulting from contacting the target cells with a naked dsRNA. In more detailed aspects of this disclosure, the mixture, complex or conjugate comprising a dsRNA and a polypeptide can be optionally combined with (*e.g.*, admixed or complexed with) a cationic lipid, such as Lipofectine^{™}. To produce these compositions comprised of a polypeptide, dsRNA and a cationic lipid, the dsRNA and peptide may be mixed together first in a suitable medium such as a cell culture medium, after which the cationic lipid is added to the mixture to form a dsRNA/delivery peptide/cationic lipid composition. Optionally, the peptide and cationic lipid can be mixed together first in a suitable medium such as a cell culture medium, followed by the addition of the dsRNA to form the dsRNA/delivery peptide/cationic lipid composition.

This disclosure also features the use of dsRNA compositions comprising surface-modified liposomes containing, for example, poly(ethylene glycol) lipids (PEG-modified, or long-circulating liposomes or stealth liposomes) (Lasic et al., Chem. Rev. 95:2601, 1995; Ishiwata et al., Chem. Pharm. Bull. 43:1005, 1995; Lasic et al., Science 267:1275, 1995; Oku et al., Biochim. Biophys. Acta 1238:86, 1995; Liu et al., J. Biol. Chem. 42:24864, 1995; PCT Publication Nos. WO 96/10391; WO 96/10390; WO 96/10392).

In another embodiment, compositions are provided for targeting dsRNA molecules of this disclosure to specific cell types, such as hepatocytes. For example, dsRNA can be complexed or conjugated glycoproteins or synthetic glycoconjugates glycoproteins or synthetic glycoconjugates having branched galactose (*e.g.*, asialoorosomucoid), *N*-acetyl-*D-*galactosamine, or mannose *(see, e.g.,* Wu and Wu, J. Biol. Chem. 262:4429, 1987; Baenziger and Fiete, Cell 22: 611, 1980; Connolly et al., J. Biol. Chem. 257:939, 1982; Lee and Lee, Glycoconjugate J. 4:317, 1987; Ponpipom et al., J. Med. Chem. 24:1388, 1981) for a targeted delivery to, for example, the liver.

A pharmaceutically effective dose is that dose required to prevent, inhibit the occurrence of, or treat (alleviate a symptom to some extent, preferably all of the symptoms) a disease state. The pharmaceutically effective dose depends on the type of disease, the composition used, the route of administration, the type of subject being treated, the physical characteristics of the specific subject under consideration for treatment, concurrent medication, and other factors that those skilled in the medical arts will recognize. For example, an amount between 0.1 mg/kg and 100 mg/kg body weight/day of active ingredients may be administered depending on the potency of a dsRNA of this disclosure.

A specific dose level for any particular patient depends upon a variety of factors including the activity of the specific compound employed, age, body weight, general health, sex, diet, time of administration, route of administration, rate of excretion, drug combination, and the severity of the particular disease undergoing therapy. Following administration of dsRNA compositions as disclosed herein, test subjects will exhibit about a 10% up to about a 99% reduction in one or more symptoms associated with the disease or disorder being treated, as compared to placebo-treated or other suitable control subjects.

Dosage levels in the order of about 0.1 mg to about 140 mg per kilogram of body weight per day can be useful in the treatment of the above-indicated conditions (about 0.5 mg to about 7 g per patient per day). The amount of active ingredient that can be combined with the carrier materials to produce a single dosage form varies depending upon the host treated and the particular mode of administration. Dosage unit forms generally contain between from about 1 mg to about 500 mg of an active ingredient.

A dosage form of a dsRNA or composition thereof of this disclosure can be liquid, an emulsion, or a micelle, or in the form of an aerosol or droplets. A dosage form of a dsRNA or composition thereof of this disclosure can be solid, which can be reconstituted in a liquid prior to administration. The solid can be administered as a powder. The solid can be in the form of a capsule, tablet, or gel. In addition to *in vivo* gene inhibition, a skilled artisan will appreciate that the dsRNA and analogs thereof of the present disclosure are useful in a wide variety of *in vitro* applications, such as scientific and commercial research (*e.g.*, elucidation of physiological pathways, drug discovery and development), and medical and veterinary diagnostics.

Nucleic acid molecules and polypeptides can be administered to cells by a variety of methods known to those of skill in the art, including administration within formulations that comprise a dsRNA alone, a dsRNA and a polypeptide complex / conjugate alone, or that further comprise one or more additional components, such as a pharmaceutically acceptable carrier, diluent, excipient, adjuvant, emulsifier, stabilizer, preservative, or the like. Other exemplary substances used to approximate physiological conditions include pH adjusting and buffering agents, tonicity adjusting agents, and wetting agents, for example, sodium acetate, sodium lactate, sodium chloride, potassium chloride, calcium chloride, sorbitan monolaurate, triethanolamine oleate, and mixtures thereof. For solid compositions, conventional nontoxic pharmaceutically acceptable carriers can be used which include, for example, pharmaceutical grades of mannitol, lactose, starch, magnesium stearate, sodium saccharin, talcum, cellulose, glucose, sucrose, magnesium carbonate, and the like.

In certain embodiments, the dsRNA and compositions thereof can be encapsulated in liposomes, administered by iontophoresis, or incorporated into other vehicles, such as hydrogels, cyclodextrins, biodegradable nanocapsules, bioadhesive microspheres, or proteinaceous vectors *(see, e.g.,* PCT Publication No. WO 00/53722). In certain embodiments of this disclosure, the dsRNA may be administered in a time release formulation, for example, in a composition that includes a slow release polymer. The dsRNA can be prepared with carriers that will protect against rapid release, for example, a controlled release vehicle such as a polymer, microencapsulated delivery system, or bioadhesive gel. Prolonged delivery of the dsRNA, in various compositions of this disclosure can be brought about by including in the composition agents that delay absorption, for example, aluminum monosterate hydrogels and gelatin.

Alternatively, a dsRNA composition of this disclosure can be locally delivered by direct injection or by use of, for example, an infusion pump. Direct injection of dsRNAs of this disclosure, whether subcutaneous, intramuscular, or intradermal, can be done by using standard needle and syringe methodologies or by needle-free technologies, such as those described in Conry et al., Clin. Cancer Res. 5:2330, 1999 and PCT Publication No. WO 99/31262.

The dsRNA of this disclosure and compositions thereof may be administered to subjects by a variety of mucosal administration modes, including oral, rectal, vaginal, intranasal, intrapulmonary, or transdermal delivery, or by topical delivery to the eyes, ears, skin, or other mucosal surfaces. In one embodiment, the mucosal tissue layer includes an epithelial cell layer, which can be pulmonary, tracheal, bronchial, alveolar, nasal, buccal, epidermal, or gastrointestinal. Compositions of this disclosure can be administered using conventional actuators, such as mechanical spray devices, as well as pressurized, electrically activated, or other types of actuators. The dsRNAs can also be administered in the form of suppositories, *e.g*., for rectal administration. For example, these compositions can be mixed with an excipient that is solid at room temperature but liquid at the rectal temperature so that the dsRNA is released. Such materials include, for example, cocoa butter and polyethylene glycols.

Further methods for delivery of nucleic acid molecules, such as the dsRNAs of this disclosure, are described, for example, in Boado et al., J. Pharm. Sci. 87:1308, 1998; Tyler et al., FEBS Lett. 421:280, 1999; Pardridge et al., Proc. Nat'l Acad. Sci. USA 92:5592, 1995; Boado, Adv. Drug Delivery Rev. 15:73, 1995; Aldrian-Herrada et al., Nucleic Acids Res. 26:4910, 1998; Tyler et al., Proc. Nat'l Acad. Sci. USA 96:7053-7058, 1999; Akhtar et al., Trends Cell Bio. 2:139, 1992; "Delivery Strategies for Antisense Oligonucleotide Therapeutics," ed. Akhtar, 1995, Maurer et al., Mol. Membr. Biol. 16:129, 1999; Hofland and Huang, Handb. Exp. Pharmacol 137:165, 1999; and Lee et al., ACS Symp. Ser. 752:184, 2000; PCT Publication No. WO 94/02595.

All U.S. patents, U.S. patent application publications, U.S. patent applications, foreign patents, foreign patent applications, non-patent publications, figures, tables, and websites referred to in this specification are expressly incorporated herein by reference, in their entirety.

### EXAMPLES

### EXAMPLE 1

### KNOCKDOWN OF GENE EXPRESSION BY MDRNA

The gene silencing activity of dsRNA as compared to nicked or gapped versions of the same dsRNA was examined using a dual fluorescence assay. A total of 22 different genes were targeted at ten different sites each (*see* Table 1).

A Dicer substrate dsRNA molecule was used, which has a 25 nucleotide sense strand, a 27 nucleotide antisense strand, and a two deoxynucleotide overhang at the 3'-end of the antisense strand (referred to as a 25/27 dsRNA). The nicked version of each dsRNA Dicer substrate has a nick at one of positions 9 to 16 on the sense strand as measured from the 5'-end of the sense strand. For example, an ndsRNA having a nick at position 11 has three strands - a 5'-sense strand of 11 nucleotides, a 3'-sense strand of 14 nucleotides, and an antisense strand of 27 nucleotides (which is also referred to as an N11-14/27 mdRNA). In addition, each of the sense strands of the ndsRNA have three locked nucleic acids (LNAs) evenly distributed along each sense fragment. If the nick is at position 9, then the LNAs can be found at positions 2, 6, and 9 of the 5' sense strand fragment and at positions 11, 18, and 23 of the 3' sense strand fragment. If the nick is at position 10, then the LNAs can be found at positions 2, 6, and 10 of the 5' sense strand fragment and at positions 12, 18, and 23 of the 3' sense strand fragment. If the nick is at position 11, then the LNAs can be found at positions 2, 6, and 11 of the 5' sense strand fragment and at positions 13, 18, and 23 of the 3' sense strand fragment. If the nick is at position 12, then the LNAs can be found at positions 2, 6, and 12 of the 5' sense strand fragment and at positions 14, 18, and 23 of the 3' sense strand fragment. If the nick is at position 13, then the LNAs can be found at positions 2, 7, and 13 of the 5' sense strand fragment and at positions 15, 18, and 23 of the 3' sense strand fragment. If the nick is at position 14, then the LNAs can be found at positions 2, 7, and 14 of the 5' sense strand fragment and at positions 16, 18, and 23 of the 3' sense strand fragment. If the nick is at position 15, then the LNAs can be found at positions 2, 8, and 15 of the 5' sense strand fragment and at positions 17, 19, and 23 of the 3' sense strand fragment. If the nick is at position 16, then the LNAs can be found at positions 2, 8, and 16 of the 5' sense strand fragment and at positions 18, 19, and 23 of the 3' sense strand fragment. Similarly, a gapped version of each dsRNA Dicer substrate has a single nucleotide missing at one of positions 10 to 17 on the sense strand as measured from the 5'-end of the sense strand. For example, a gdsRNA having a gap at position 11 has three strands - a 5'-sense strand of 11 nucleotides, a 3'-sense strand of 13 nucleotides, and an antisense strand of 27 nucleotides (which is also referred to as G11-(1)-13/27 mdRNA). In addition, each of the sense strands of the gdsRNA contain three locked nucleic acids (LNAs) evenly distributed along each sense fragment (as described for the nicked counterparts).

In sum, three dsRNA were tested at each of the ten different sites per gene - an unmodified dsRNA, a nicked mdRNA with three LNAs per sense strand fragment, and a single nucleotide gapped mdRNA with three LNAs per sense strand fragment. In other words, 660 different dsRNA were examined.

Briefly, multiwell plates were seeded with about 7-8 x 10⁵ HeLa cells/well in DMEM having 10% fetal bovine serum, and incubated overnight at 37°C / 5% CO₂. The HeLa cell medium was changed to serum-free DMEM just prior to transfection. The psiCHECK™-2 vector, containing about a 1,000 basepair insert of a target gene, diluted in serum-free DMEM was mixed with diluted GenJet^{™} transfection reagent (SignalDT Biosystems, Hayward, California) according to the manufacturer's instructions and then incubated at room temperature for 10 minutes. The GenJet/ psiCHECK™-2-[target gene insert] solution was added to the HeLa cells and then incubated at 37°C, 5% CO₂ for 4.5 hours. After the vector transfection, cells were trypsinized and suspended in antibiotic-free DMEM containing 10% FBS at a concentration of 10⁵ cells per mL.

To transfect the dsRNA, the dsRNA was formulated in OPTI-MEM I reduced serum medium (Gibco® Invitrogen, Carlsbad, California) and placed in multiwell plates. Then Lipofectamine^{™} RNAiMAX (Invitrogen) was mixed with OPTI-MEM per manufacture's specifications, added to each well containing dsRNA, mixed manually, and incubated at room temperature for 10-20 minutes. Then 30 µL of vector-transfected HeLa cells at 10⁵ cells per mL were added to each well (final dsRNA concentration of 25 nM), the plates were spun for 30 seconds at 1,000 rpm, and then incubated at 37°C / 5% CO₂ for 2 days. The Cell Titer Blue (CTB) reagent (Promega, Madison, Wisconson) was used to assay for cell viability and proliferation - none of the dsRNA showed any substantial toxicity.

After transfecting, the media and CTB reagent were removed and the wells washed once with 100 PBS. Cells were assayed for firefly and *Renilla* luciferase reporter activity by first adding Dual-Glo^{™} Luciferase Reagent (Promega, Madison, WI) for 10 minutes with shaking, and then quantitating the luminescent signal on a VICTOR³™ 1420 Multilabel Counter (PerkinElmer). After measuring the firefly luminescence, Stop & Glo^{®} Reagent (Promega, Madison, WI) was added for 10 minutes with shaking to simultaneously quench the firefly reaction and initiate the *Renilla* luciferase reaction, which was then quantitated on a VICTOR³™ 1420 Multilabel Counter (PerkinElmer). The results are presented in Table 1.

**Table 1. Gene Silencing Activity* of dsRNA Dicer Substrate and mdRNA (nicked or gapped) Dicer Substrate**

| **Set** | **Target** | **Pos†** | **Dicer SEQ ID NOS‡** | **Dicer Mean (%)** | **Dicer 95% CI** | **Nicked SEQ ID NOS** | **Nicked Mean (%)** | **Nicked 95% CI** | **Gapped SEQ ID NOS** | **Gapped Mean (%)** | **Gapped 95% CI** | **Length 5'-S^** |
|---|---|---|---|---|---|---|---|---|---|---|---|---|
| 1 | AKT1 | 1862 | 63,283 | 20.6 | 4.0% | 503, 723, 283 | 23.5 | 5.7% | 503,940,283 | 54.3 | 12.0% | 14 |
| 2 | AKT1 | 1883 | 64,284 | 29.7 | 7.3% | 504,724,284 | 51.4 | 6.7% | 504,941,284 | 76.9 | 19.5% | 12 |
| 3 | AKT1 | 2178 | 65,285 | 15.4 | 2.4% | 505,725,285 | 22.3 | 6.4% | 505,942,285 | 24.4 | 5.1% | 14 |
| 4 | AKT1 | 2199 | 66,286 | 26.4 | 3.6% | 506,726,286 | 62.7 | 6.6% | 506,943,286 | 66.8 | 10.8% | 15 |
| 5 | AKT1 | 2264 | 67,287 | 35.2 | 7.3 % | 507,727,287 | 34.1 | 7.3% | 507,944,287 | 31.3 | 5.2% | 12 |
| 6 | AKT1 | 2580 | 68,288 | 27.6 | 5.7% | 508,728,288 | 40.1 | 8.3% | 508,945,288 | 91.5 | 17.0% | 12 |
| 7 | AKT1 | 2606 | 69,289 | 14.0 | 2.6% | 509,729,289 | 14.9 | 3.2% | 509,946,289 | 33.4 | 6.9% | 11 |
| 8 | AKT1 | 2629 | 70,290 | 21.0 | 10.1% | 510, 730, 290 | 13.5 | 2.4% | 510,947,290 | 13.6 | 2.1% | 12 |
| 9 | AKT1 | 2661 | 71,291 | 37.4 | 6.6% | 511,731,291 | 41.0 | 12.1% | 511,948,291 | 71.6 | 11.9% | 15 |
| 10 | AKT1 | 2663 | 72,292 | 18.1 | 4.3% | 512,732,292 | 23.0 | 5.9% | 512,949,292 | 51.4 | 9.2% | 14 |
| 11 | BCR-ABL (b2a2) | 66 | 73,293 | 16.9 | 5.9% | 513,733,293 | 30.4 | 10.5% | 513,950,293 | 38.2 | 11.7% | 13 |
| 12 | BCR-ABL (b2a2) | 190 | 74,294 | 40.0 | 11.6% | 514,734,294 | 22.0 | 6.4% | 514,951,294 | 34.6 | 12.0% | 14 |
| 13 | BCR-ABL (b2a2) | 282 | 75,295 | 24.2 | 5.2% | 515,735,295 | 37.6 | 8.2% | 515,952,295 | 34.6 | 8.6% | 13 |
| 14 | BCR-ABL (b2a2) | 284 | 76,296 | 50.9 | 6.9% | 516,736,296 | 38.3 | 7.8% | 516,953,296 | 68.3 | 18.0% | 13 |
| 15 | BCR-ABL (b2a2) | 287 | 77,297 | 45.5 | 13.2% | 517,737,297 | 39.6 | 11.5% | 517,954,297 | 75.2 | 17.2% | 14 |
| 16 | BCR-ABL (b2a2) | 289 | 78,298 | 36.9 | 7.7% | 518,738,298 | 40.0 | 8.9% | 518,955,298 | 60.9 | 12.3 % | 14 |
| 17 | BCR-ABL (b2a2) | 293 | 79,299 | 55.9 | 9.8% | 519,739,299 | 58.6 | 14.7% | 519,956,299 | 87.0 | 14.3% | 13 |
| 18 | BCR-ABL (b2a2) | 461 | 80,300 | 38.4 | 9.4% | 520,740,300 | 35.9 | 12.1% | 520,957,300 | 28.6 | 10.2% | 13 |
| 19 | BCR-ABL (b2a2) | 462 | 81,301 | 31.1 | 13.7% | 521,741,301 | 26.5 | 5.5% | 521,958,301 | 35.8 | 10.7% | 14 |
| 20 | BCR-ABL (b2a2) | 561 | 82,302 | 17.7 | 3.4% | 522,742,302 | 20.7 | 3.4% | 522,959,302 | 35.5 | 10.6% | 12 |
| 21 | BCR-ABL (b3a2) | 352 | 83,303 | 45.4 | 7.0% | 523,743,303 | 39.8 | 8.3% | 523,960,303 | 45.5 | 11.0% | 12 |
| 22 | BCR-ABL (b3a2) | 353 | 84,304 | 22.6 | 1.8% | 524,744,304 | 20.5 | 5.1% | 524,961,304 | 66.1 | 17.8% | 12 |
| 23 | BCR-ABL (b3a2) | 356 | 85,305 | 11.9 | 2.5% | 525, 745, 305 | 28.4 | 5.8% | 525,962,305 | 56.0 | 10.6% | 13 |
| 24 | BCR-ABL (b3a2) | 357 | 86,306 | 24.5 | 6.0% | 526, 746, 306 | 25.6 | 7.5% | 526,963,306 | 39.2 | 10.0% | 13 |
| 25 | BCR-ABL (b3a2) | 359 | 87,307 | 56.8 | 9.3% | 527, 747, 307 | 42.4 | 7.3% | 527,964,307 | 46.4 | 9.5% | 13 |
| 26 | BCR-ABL (b3a2) | 360 | 88,308 | 32.3 | 5.0% | 528, 748, 308 | 37.2 | 7.3% | 528,965,308 | 55.3 | 13.8% | 13 |
| 27 | BCR-ABL (b3a2) | 362 | 89, 309 | 12.4 | 3.2% | 529, 737, 309 | 26.3 | 9.8% | 529,954,309 | 46.2 | 8.3% | 14 |
| 28 | BCR-ABL (b3a2) | 410 | 90,310 | 66.2 | 12.2% | 530, 749, 310 | 55.9 | 11.2% | 530, 966, 310 | 58.4 | 16.4% | 12 |
| 29 | BCR-ABL (b3a2) | 629 | 91,311 | 35.0 | 11.7% | 531, 750, 311 | 46.5 | 10.1% | 531,967,311 | 41.0 | 9.0% | 13 |
| 30 | BCR-ABL (b3a2) | 727 | 92,312 | 83.4 | 13.6% | 532, 751, 312 | 76.7 | 22.5% | 532,968,312 | 62.9 | 10.9% | 12 |
| 31 | EGFR | 4715 | 93,313 | 15.3 | 2.2% | 533, 752, 313 | 9.4 | 0.9% | 533,969,313 | 11.3 | 1.7% | 11 |
| 32 | EGFR | 4759 | 94,314 | 3.8 | 0.4% | 534, 753, 314 | 6.3 | 0.8% | 534,970,314 | 8.4 | 1.1% | 12 |
| 33 | EGFR | 4810 | 95,315 | 5.2 | 0.6% | 535, 754, 315 | 5.8 | 0.7% | 535,971,315 | 7.2 | 1.0% | 13 |
| 34 | EGFR | 5249 | 96,316 | 2.6 | 0.4% | 536, 755, 316 | 16.6 | 1.8% | 536,972,316 | 42.9 | 3.5% | 14 |
| 35 | EGFR | 5279 | 97,317 | 7.6 | 1.0% | 537, 756, 317 | 10.6 | 1.1% | 537,973,317 | 11.8 | 1.7% | 13 |
| 36 | EGFR | 5374 | 98,318 | 9.6 | 1.0% | 538, 757, 318 | 8.7 | 0.9% | 538,974,318 | 34.7 | 4.3% | 12 |
| 37 | EGFR | 5442 | 99,319 | 4.1 | 0.8% | 539, 758, 319 | 15.1 | 1.8% | 539,975,319 | 19.7 | 2.4% | 12 |
| 38 | EGFR | 5451 | 100, 320 | 5.1 | 0.3 % | 540, 759, 320 | 11.5 | 1.3% | 540,976,320 | 16.5 | 3.0% | 13 |
| 39 | EGFR | 5469 | 101,321 | 5.6 | 0.8% | 541, 760, 321 | 5.1 | 0.5% | 541,977,321 | 12.2 | 2.5% | 13 |
| 40 | EGFR | 5483 | 102, 322 | 2.2 | 0.4% | 542, 761, 322 | 2.4 | 0.5% | 542, 978, 322 | 6.1 | 0.7% | 9 |
| 41 | FLT1 | 863 | 103,323 | 7.6 | 1.1% | 543, 762, 323 | 10.2 | 3.3% | 543, 979, 323 | 29.2 | 8.1% | 12 |
| 42 | FLT1 | 906 | 104, 324 | 10.0 | 2.4% | 544, 763, 324 | 10.8 | 0.8% | 544, 980, 324 | 12.4 | 2.1% | 12 |
| 43 | FLT1 | 993 | 105, 325 | 12.2 | 2.5% | 545, 764, 325 | 13.7 | 2.8% | 545, 981, 325 | 20.0 | 11.3% | 13 |
| 44 | FLT1 | 1283 | 106, 326 | 19.6 | 4.5% | 546, 765, 326 | 25.8 | 7.3% | 546, 982, 326 | 18.7 | 6.5% | 12 |
| 45 | FLT1 | 1289 | 107, 327 | 15.5 | 2.0% | 547, 766, 327 | 13.5 | 1.6% | 547, 983, 327 | 22.5 | 5.0% | 12 |
| 46 | FLT1 | 1349 | 108, 328 | 36.8 | 4.2% | 548, 767, 328 | 22.9 | 4.0% | 548, 984, 328 | 52.7 | 5.4% | 14 |
| 47 | FLT1 | 1354 | 109, 329 | 36.6 | 4.0% | 549, 768, 329 | 49.7 | 5.9% | 549, 985, 329 | 45.8 | 9.3% | 14 |
| 48 | FLT1 | 1448 | 110, 330 | 9.3 | 2.5% | 550, 769, 330 | 16.1 | 2.9% | 550, 986, 330 | 24.2 | 3.6% | 13 |
| 49 | FLT1 | 1459 | 111, 331 | 13.7 | 3.6% | 551, 770, 331 | 20.0 | 8.7% | 551, 987, 331 | 22.4 | 4.4% | 12 |
| 50 | FLT1 | 1700 | 112, 332 | 7.9 | 2.2% | 552, 771, 332 | 11.2 | 3.7% | 552, 988, 332 | 36.4 | 8.0% | 13 |
| 51 | FRAP1 | 7631 | 113, 333 | 9.5 | 2.7% | 553, 772, 333 | 23.3 | 4.9% | 553, 989, 333 | 61.8 | 18.3% | 13 |
| 52 | FRAP1 | 7784 | 114, 334 | 15.1 | 1.7% | 554, 773, 334 | 19.9 | 2.8% | 554, 990, 334 | 29.3 | 3.4% | 12 |
| 53 | FRAP1 | 7812 | 115, 335 | 11.9 | 2.9% | 555, 774, 335 | 14.4 | 3.2% | 555, 991, 335 | 28.3 | 12.7% | 11 |
| 54 | FRAP1 | 7853 | 116, 336 | 16.8 | 3.3% | 556, 775, 336 | 24.1 | 3.7% | 556, 992, 336 | 67.5 | 9.2% | 11 |
| 55 | FRAP1 | 8018 | 117, 337 | 41.1 | 9.1% | 557, 776, 337 | 19.8 | 3.3% | 557, 993, 337 | 41.8 | 9.6% | 12 |
| 56 | FRAP1 | 8102 | 118, 338 | 35.7 | 5.1% | 558, 777, 338 | 30.2 | 6.3% | 558, 994, 338 | 39.5 | 9.9% | 12 |
| 57 | FRAP1 | 8177 | 119, 339 | 21.2 | 3.9% | 559, 778, 339 | 33.2 | 9.3% | 559, 995, 339 | 47.3 | 12.3% | 14 |
| 58 | FRAP1 | 8348 | 120, 340 | 25.8 | 3.6% | 560, 779, 340 | 26.8 | 4.4% | 560, 996, 340 | 37.4 | 4.7% | 11 |
| 59 | FRAP1 | 8435 | 121, 341 | 41.1 | 6.7% | 561, 780, 341 | 54.1 | 9.5% | 561, 997, 341 | 74.9 | 8.5% | 12 |
| 60 | FRAP1 | 8542 | 122, 342 | 23.1 | 4.8% | 562, 781, 342 | 16.5 | 5.5% | 562, 998, 342 | 33.6 | 6.4% | 10 |
| 61 | HIF1A | 1780 | 123, 343 | 76.6 | 14.9% | 563, 782, 343 | 89.2 | 11.9% | 563, 999, 343 | 86.3 | 9.3% | 12 |
| 62 | HIF1A | 1831 | 124, 344 | 9.0 | 0.6% | 564, 783, 344 | 14.0 | 2.3% | 564, 1000, 344 | 38.2 | 8.5% | 12 |
| 63 | HIF1A | 1870 | 125, 345 | 21.4 | 4.5% | 565, 784, 345 | 21.2 | 3.3% | 565, 1001, 345 | 19.6 | 2.2% | 13 |
| 64 | HIF1A | 1941 | 126,346 | 8.9 | 2.1% | 566, 785,346 | 11.4 | 2.2% | 566, 1002, 346 | 11.7 | 2.5% | 12 |
| 65 | HIF1A | 2068 | 127,347 | 7.8 | 1.5% | 567, 786, 347 | 7.0 | 1.4% | 567, 1003, 347 | 16.9 | 3.9% | 12 |
| 66 | HIF1A | 2133 | 128,348 | 13.0 | 20% | 568, 787, 348 | 16.7 | 3.1% | 568, 1004, 348 | 16.3 | 3.1% | 10 |
| 67 | HIF1A | 2232 | 129, 349 | 8.6 | 2.0% | 569, 788, 349 | 17.4 | 3.6% | 569, 1005, 349 | 37.8 | 9.6% | 13 |
| 68 | HIF1A | 2273 | 130, 350 | 19.1 | 5.3% | 570, 789, 350 | 23.4 | 4.4% | 570, 1006, 350 | 20.3 | 3.4% | 12 |
| 69 | HIF1A | 2437 | 131, 351 | 8.2 | 1.4% | 571, 790, 351 | 47.7 | 11.5% | 571, 1007, 351 | 72.4 | 14.3% | 13 |
| 70 | HIF1A | 2607 | 132,352 | 8.0 | 2.1% | 572, 791, 352 | 11.0 | 1.2% | 572, 1008, 352 | 33.6 | 6.0% | 13 |
| 71 | IL17A | 923 | 133,353 | 5.0 | 0.6% | 573, 792, 353 | 7.3 | 0.7% | 573, 1009, 353 | 26.3 | 2.5% | 12 |
| 72 | IL17A | 962 | 134,354 | 6.7 | 0.8% | 574, 793, 354 | 7.7 | 0.9% | 574, 1010, 354 | 8.9 | 2.0% | 13 |
| 73 | IL17A | 969 | 135,355 | 8.9 | 1.7% | 575, 794, 355 | 17.1 | 1.6% | 575, 1011, 355 | 49.5 | 4.3 % | 14 |
| 74 | IL17A | 1098 | 136,356 | 7.2 | 1.3% | 576, 795, 356 | 10.0 | 2.4% | 576, 1012, 356 | 15.4 | 2.8% | 12 |
| 75 | IL17A | 1201 | 137,357 | 14.1 | 2.2% | 577, 796, 357 | 13.4 | 1.1% | 577, 1013, 357 | 17.2 | 2.8% | 12 |
| 76 | IL17A | 1433 | 138,358 | 107.1 | 9.7% | 578, 797, 358 | 111.5 | 10.4% | 578, 1014, 358 | 108.1 | 8.8% | 13 |
| 77 | IL17A | 1455 | 139,359 | 115.4 | 11.1% | 579, 798, 359 | 120.8 | 8.7% | 579, 1015, 359 | 120.3 | 9.9% | 12 |
| 78 | IL17A | 1478 | 140,360 | 82.7 | 6.3% | 580, 799, 360 | 87.6 | 5.0% | 580, 1016, 360 | 95.9 | 5.6% | 14 |
| 79 | IL17A | 1663 | 141,361 | 140.2 | 7.8% | 581, 800, 361 | 125.9 | 9.8% | 581, 1017, 361 | 114.7 | 10.1% | 14 |
| 80 | IL17A | 1764 | 142,362 | 114.3 | 9.2% | 582, 801, 362 | 109.4 | 2.9% | 582, 1018, 362 | 105.7 | 8.1% | 15 |
| 81 | IL18 | 210 | 143,363 | 13.8 | 2.8% | 583,802,363 | 23.9 | 5.8% | 583,1019,363 | 21.4 | 5.7% | 14 |
| 82 | IL18 | 368 | 144,364 | 22.5 | 1.8% | 584,803,364 | 21.0 | 2.0% | 584, 1020, 364 | 29.7 | 3.7% | 13 |
| 83 | IL18 | 479 | 145,365 | 88.1 | 12.9% | 585,804,365 | 66.3 | 9.8% | 585, 1021, 365 | 80.0 | 16.8% | 14 |
| 84 | IL18 | 508 | 146,366 | 8.0 | 1.9% | 586,805,366 | 15.7 | 3.5% | 586, 1022, 366 | 17.0 | 5.7% | 12 |
| 85 | IL18 | 521 | 147,367 | 9.9 | 2.1% | 587, 806, 367 | 10.8 | 2.1% | 587, 1023, 367 | 18.4 | 3.3% | 11 |
| 86 | IL18 | 573 | 148,368 | 18.6 | 4.7% | 588, 807, 368 | 24.8 | 7.6% | 588, 1024, 368 | 48.8 | 7.7% | 14 |
| 87 | IL18 | 605 | 149,369 | 27.5 | 6.1% | 589, 808, 369 | 21.3 | 3.9% | 589, 1025, 369 | 14.9 | 2.7% | 13 |
| 88 | IL18 | 663 | 150,370 | 5.3 | 1.0% | 590, 809, 370 | 8.2 | 1.5% | 590, 1026, 370 | 11.7 | 3.4% | 12 |
| 89 | IL18 | 785 | 151,371 | 8.6 | 1.0% | 591,810,371 | 11.7 | 2.8% | 591, 1027, 371 | 21.1 | 9.1% | 12 |
| 90 | IL18 | 918 | 152,372 | 13.9 | 1.6% | 592,811,372 | 15.0 | 3.0% | 592, 1028, 372 | 30.4 | 3.6% | 11 |
| 91 | IL6 | 24 | 153,373 | 22.6 | 1.7% | 593,812,373 | 45.7 | 7.8% | 593, 1029, 373 | 47.8 | 4.5% | 13 |
| 92 | IL6 | 74 | 154,374 | 52.5 | 12.6% | 594,813,374 | 56.4 | 7.1% | 594, 1030, 374 | 88.3 | 15.5% | 12 |
| 93 | IL6 | 160 | 155,375 | 49.8 | 7.8% | 595,814,375 | 50.6 | 6.1% | 595, 1031, 375 | 68.3 | 9.4% | 14 |
| 94 | IL6 | 370 | 156,376 | 44.7 | 8.2% | 596,815,376 | 52.5 | 4.2% | 596, 1032, 376 | 74.3 | 9.3% | 13 |
| 95 | IL6 | 451 | 157,377 | 39.3 | 5.0% | 597,816,377 | 35.6 | 4.1% | 597, 1033, 377 | 66.6 | 7.1% | 13 |
| 96 | IL6 | 481 | 158,378 | 68.3 | 8.1% | 598,817,378 | 78.7 | 15.6% | 598, 1034, 378 | 63.2 | 6.2% | 11 |
| 97 | IL6 | 710 | 159,379 | 29.2 | 4.2% | 599,818,379 | 32.0 | 4.1% | 599, 1035, 379 | 77.3 | 11.4% | 12 |
| 98 | IL6 | 822 | 160,380 | 73.7 | 11.0% | 600,819,380 | 72.2 | 11.6% | 600, 1036, 380 | 85.2 | 13.3% | 12 |
| 99 | IL6 | 836 | 161,381 | 98.8 | 21.8% | 601,820,381 | 95.0 | 13.2% | 601, 1037, 381 | 90.5 | 15.6% | 13 |
| 100 | IL6 | 960 | 162,382 | 31.1 | 4.4% | 602,821,382 | 20.5 | 6.1% | 602, 1038, 382 | 25.6 | 2.4% | 12 |
| 101 | MAP2K1 | 1237 | 163, 383 | 21.0 | 3.3% | 603,822,383 | 27.9 | 3.8% | 603, 1039, 383 | 50.0 | 8.8% | 11 |
| 102 | MAP2K1 | 1342 | 164,384 | 3.9 | 0.5% | 604,823,384 | 8.7 | 1.5% | 604, 1040, 384 | 11.4 | 1.3% | 13 |
| 103 | MAP2K1 | 1501 | 165,385 | 12.9 | 1.9% | 605,824,385 | 19.4 | 2.9% | 605, 1041, 385 | 19.7 | 5.3% | 12 |
| 104 | MAP2K1 | 1542 | 166,386 | 7.2 | 1.3% | 606,825,386 | 11.7 | 2.1% | 606, 1042, 386 | 18.7 | 3.2% | 11 |
| 105 | MAP2K1 | 1544 | 167,387 | 13.1 | 2.1% | 607, 826, 387 | 11.1 | 1.1% | 607, 1043, 387 | 16.5 | 3.0% | 10 |
| 106 | MAP2K1 | 1728 | 168,388 | 11.9 | 1.7% | 608, 827, 388 | 11.9 | 1.0% | 608, 1044, 388 | 27.9 | 4.3% | 13 |
| 107 | MAP2K1 | 1777 | 169,389 | 18.3 | 2.8% | 609, 828, 389 | 37.2 | 4.3% | 609, 1045, 389 | 64.5 | 8.5% | 13 |
| 108 | MAP2K1 | 1892 | 170,390 | 34.5 | 4.7% | 610,829,390 | 37.6 | 6.8% | 610, 1046, 390 | 42.4 | 7.3% | 12 |
| 109 | MAP2K1 | 1954 | 171,391 | 4.6 | 0.5% | 611, 830, 391 | 4.2 | 0.5% | 611, 1047, 391 | 6.5 | 1.1% | 13 |
| 110 | MAP2K1 | 2062 | 172, 392 | 10.2 | 0.8% | 612,831,392 | 10.4 | 2.9% | 612, 1048, 392 | 12.2 | 2.0% | 12 |
| 111 | MAPK1 | 3683 | 173,393 | 7.0 | 0.9% | 613,614,393 | 24.4 | 17.3% | 613, 1049, 393 | 25.2 | 2.6% | 12 |
| 112 | MAPK1 | 3695 | 174,394 | 32.9 | 4.6% | 614,832,394 | 30.9 | 4.0% | 614, 1050, 394 | 33.8 | 3.1% | 13 |
| 113 | MAPK1 | 3797 | 175,395 | 7.4 | 1.1% | 615,833,395 | 6.4 | 1.3% | 615, 1051, 395 | 40.4 | 5.8% | 11 |
| 114 | MAPK1 | 3905 | 176,396 | 8.0 | 1.0% | 616,834,396 | 8.1 | 0.5% | 616, 1052, 396 | 14.8 | 1.4% | 12 |
| 115 | MAPK1 | 3916 | 177,397 | 11.0 | 1.7% | 617,835,397 | 16.0 | 3.3% | 617, 1053, 397 | 45.5 | 8.1% | 10 |
| 116 | MAPK1 | 3943 | 178,398 | 6.8 | 0.8% | 618,836,398 | 6.6 | 0.7% | 618, 1054, 398 | 11.0 | 2.3% | 10 |
| 117 | MAPK1 | 4121 | 179,399 | 7.6 | 1.1% | 619,837,399 | 12.7 | 1.6% | 619, 1055, 399 | 25.1 | 3.1% | 12 |
| 118 | MAPK1 | 4256 | 180,400 | 27.6 | 2.5% | 620,838,400 | 36.8 | 4.0% | 620, 1056, 400 | 57.7 | 7.0% | 13 |
| 119 | MAPK1 | 4294 | 181,401 | 31.0 | 3.0% | 621, 839, 401 | 22.3 | 3.6% | 621, 1057, 401 | 50.9 | 4.6% | 12 |
| 120 | MAPK1 | 4375 | 182,402 | 10.9 | 1.1% | 622,840,402 | 12.4 | 1.4% | 622, 1058, 402 | 16.9 | 2.7% | 11 |
| 121 | MAPK14 | 2715 | 183,403 | 11.4 | 2.8% | 623,841,403 | 16.5 | 4.1% | 623, 1059, 403 | 16.6 | 2.4% | 12 |
| 122 | MAPK14 | 2737 | 184,404 | 7.5 | 0.8% | 624,842,404 | 10.3 | 1.1% | 624, 1060, 404 | 13.1 | 1.2% | 11 |
| 123 | MAPK14 | 2750 | 185,405 | 8.7 | 1.0% | 625,843,405 | 12.2 | 1.8% | 625, 1061, 405 | 15.8 | 1.9% | 13 |
| 124 | MAPK14 | 2817 | 186,406 | 6.4 | 0.8% | 626,844,406 | 14.6 | 1.7% | 626, 1062, 406 | 19.4 | 2.0% | 11 |
| 125 | MAPK14 | 3091 | 187,407 | 9.9 | 0.6% | 627,845,407 | 10.3 | 1.3% | 627, 1063, 407 | 24.7 | 1.5% | 11 |
| 126 | MAPK14 | 3312 | 188,408 | 20.4 | 1.8% | 628, 846, 408 | 30.5 | 2.9% | 628, 1064, 408 | 38.5 | 3.4% | 13 |
| 127 | MAPK14 | 3346 | 189,409 | 20.9 | 1.6% | 629, 847, 409 | 23.0 | 2.6% | 629, 1065, 409 | 58.3 | 6.7% | 11 |
| 128 | MAPK14 | 3531 | 190,410 | 42.4 | 3.2% | 630,848,410 | 55.1 | 5.0% | 630, 1066, 410 | 61.9 | 3.6% | 12 |
| 129 | MAPK14 | 3621 | 191,411 | 28.6 | 1.9% | 631, 849, 411 | 42.4 | 13.5% | 631, 1067, 411 | 71.9 | 5.2% | 11 |
| 130 | MAPK14 | 3680 | 192,412 | 15.6 | 1.3% | 632,850,412 | 15.5 | 1.9% | 632, 1068, 412 | 19.8 | 2.1% | 12 |
| 131 | PDGFA | 1322 | 193,413 | 23.7 | 3.6% | 633,851,413 | 31.6 | 4.3% | 633, 1069, 413 | 38.4 | 3.3% | 12 |
| 132 | PDGFA | 1332 | 194,414 | 35.5 | 5.4% | 634,852,414 | 48.4 | 3.0% | 634, 1070, 414 | 65.4 | 10.5% | 14 |
| 133 | PDGFA | 1395 | 195,415 | 25.9 | 3.3% | 635,853,415 | 40.2 | 6.0% | 635, 1071, 415 | 55.2 | 9.8% | 14 |
| 134 | PDGFA | 1669 | 196,416 | 40.4 | 5.1% | 636,854,416 | 29.5 | 4.3% | 636, 1072, 416 | 33.9 | 5.9% | 12 |
| 135 | PDGFA | 1676 | 197,417 | 27.1 | 2.5% | 637,855,417 | 36.8 | 4.5% | 637, 1073, 417 | 47.4 | 3.4% | 13 |
| 136 | PDGFA | 1748 | 198,418 | 27.4 | 4.7% | 638, 856, 418 | 34.5 | 5.0% | 638, 1074, 418 | 47.5 | 4.7% | 11 |
| 137 | PDGFA | 2020 | 199,419 | 31.6 | 6.6% | 639, 857, 419 | 37.5 | 4.3% | 639, 1075, 419 | 51.9 | 5.0% | 13 |
| 138 | PDGFA | 2021 | 200,420 | 16.7 | 1.0% | 640,858,420 | 24.2 | 3.1% | 640, 1076, 420 | 62.6 | 6.9% | 14 |
| 139 | PDGFA | 2030 | 201,421 | 38.7 | 6.2% | 641, 859, 421 | 47.0 | 10.5% | 641, 1077, 421 | 80.5 | 7.6% | 13 |
| 140 | PDGFA | 2300 | 202,422 | 55.3 | 7.7% | 642,860,422 | 41.2 | 4.7% | 642, 1078, 422 | 71.7 | 9.1% | 15 |
| 141 | PDGFRA | 4837 | 203,423 | 16.9 | 3.1% | 643,861,423 | 21.1 | 5.1% | 643, 1079, 423 | 23.1 | 4.8% | 12 |
| 142 | PDGFRA | 4900 | 204,424 | 23.8 | 3.8% | 644,862,424 | 40.9 | 8.4% | 644, 1080, 424 | 62.5 | 12.5% | 16 |
| 143 | PDGFRA | 5007 | 205,425 | 52.6 | 9.4% | 645,863,425 | 49.6 | 7.7% | 645, 1081, 425 | 47.0 | 9.5% | 12 |
| 144 | PDGFRA | 5043 | 206,426 | 30.1 | 7.9% | 646,864,426 | 30.0 | 5.4% | 646, 1082, 426 | 57.3 | 7.8% | 11 |
| 145 | PDGFRA | 5082 | 207,427 | 8.3 | 1.1% | 647,865,427 | 11.9 | 1.8% | 647, 1083, 427 | 18.2 | 4.0% | 13 |
| 146 | PDGFRA | 5352 | 208,428 | 6.3 | 1.4% | 648,866,428 | 8.2 | 1.6% | 648, 1084, 428 | 7.9 | 1.1% | 12 |
| 147 | PDGFRA | 5367 | 209,429 | 19.1 | 5.6% | 649,867,429 | 10.9 | 1.6% | 649, 1085, 429 | 25.1 | 2.9% | 14 |
| 148 | PDGFRA | 5496 | 210,430 | 18.9 | 5.4% | 650,868,430 | 17.0 | 2.9% | 650, 1086, 430 | 17.8 | 4.0% | 12 |
| 149 | PDGFRA | 5706 | 211,431 | 24.5 | 4.0% | 651,869,431 | 47.8 | 4.3% | 651, 1087, 431 | 50.6 | 5.5% | 13 |
| 150 | PDGFRA | 5779 | 212,432 | 13.0 | 1.4% | 652,870,432 | 14.0 | 2.1% | 652, 1088, 432 | 17.2 | 4.3 % | 14 |
| 151 | PIK3CA | 213 | 213,433 | 4.3 | 1.0% | 653,871,433 | 3.7 | 0.6% | 653, 1089, 433 | 5.7 | 0.9% | 12 |
| 152 | PIK3CA | 389 | 214,434 | 5.3 | 1.0% | 654,872,434 | 7.0 | 1.5% | 654, 1090, 434 | 5.6 | 1.5% | 10 |
| 153 | PIK3CA | 517 | 215,435 | 9.6 | 1.1% | 655,873,435 | 11.5 | 2.1% | 655, 1091, 435 | 13.5 | 1.6% | 11 |
| 154 | PIK3CA | 630 | 216,436 | 6.1 | 1.2% | 656,874,436 | 8.9 | 2.6% | 656, 1092, 436 | 9.3 | 1.8% | 12 |
| 155 | PIK3CA | 680 | 217,437 | 3.8 | 0.3 % | 657,875,437 | 5.9 | 0.6% | 657, 1093, 437 | 6.9 | 1.0% | 11 |
| 156 | PIK3CA | 732 | 218,438 | 5.7 | 1.7% | 658,876,438 | 15.3 | 1.5% | 658, 1094, 438 | 17.4 | 4.0% | 11 |
| 157 | PIK3CA | 736 | 219,439 | 5.9 | 0.9% | 659,877,439 | 7.8 | 1.1% | 659, 1095, 439 | 6.5 | 1.4% | 12 |
| 158 | PIK3CA | 923 | 220,440 | 5.0 | 0.7% | 660,878,440 | 8.5 | 1.5% | 660, 1096, 440 | 7.4 | 0.6% | 12 |
| 159 | PIK3CA | 1087 | 221,441 | 8.1 | 2.3 % | 661, 879, 441 | 8.5 | 1.6% | 661, 1097, 441 | 17.5 | 4.9% | 12 |
| 160 | PIK3CA | 1094 | 222,442 | 13.0 | 3.8% | 662,880,442 | 13.0 | 2.5% | 662, 1098, 442 | 30.1 | 6.4% | 11 |
| 161 | PKN3 | 2408 | 223,443 | 9.4 | 2.1% | 663,881,443 | 15.2 | 3.7% | 663,665,443 | 32.1 | 6.6% | 12 |
| 162 | PKN3 | 2420 | 224,444 | 14.5 | 1.7% | 664,882,444 | 30.4 | 7.5% | 664, 1099, 444 | 40.1 | 6.7% | 12 |
| 163 | PKN3 | 2421 | 225,445 | 15.2 | 2.0% | 665,883,445 | 20.6 | 2.7% | 665, 1100,445 | 50.8 | 7.8% | 12 |
| 164 | PKN3 | 2425 | 226,446 | 28.4 | 3.8% | 666,884,446 | 27.0 | 6.9% | 666, 1101,446 | 36.2 | 4.8% | 15 |
| 165 | PKN3 | 2682 | 227,447 | 30.0 | 4.6% | 667,885,447 | 27.1 | 2.8% | 667,1102,447 | 37.1 | 6.2% | 11 |
| 166 | PKN3 | 2683 | 228,448 | 22.4 | 2.8% | 668,886,448 | 34.8 | 2.2% | 668,1103,448 | 51.9 | 7.4% | 12 |
| 167 | PKN3 | 2931 | 229,449 | 35.1 | 4.4% | 669,887,449 | 57.3 | 7.8% | 669,1104,449 | 88.6 | 7.1% | 13 |
| 168 | PKN3 | 3063 | 230,450 | 21.8 | 3.1 % | 670,888,450 | 28.6 | 8.5% | 670, 1105,450 | 40.5 | 6.2% | 12 |
| 169 | PKN3 | 3314 | 231,451 | 9.7 | 1.8% | 671, 889, 451 | 12.0 | 1.4% | 671, 1106, 451 | 17.3 | 1.3% | 10 |
| 170 | PKN3 | 3315 | 232,452 | 10.1 | 1.3% | 672,890,452 | 15.3 | 2.8% | 672, 1107,452 | 3 7.4 | 3.6% | 11 |
| 171 | RAF 1 | 1509 | 233,453 | 46.2 | 9.4% | 673,891,453 | 51.3 | 10.7% | 673,1108,453 | 61.3 | 4.4% | 12 |
| 172 | RAF1 | 1512 | 234,454 | 40.1 | 9.7% | 674,892,454 | 34.5 | 5.6% | 674, 1109,454 | 62.4 | 8.6% | 13 |
| 173 | RAF1 | 1628 | 235,455 | 48.3 | 7.9% | 675,893,455 | 47.4 | 7.1% | 675,1110,455 | 41.1 | 5.1% | 12 |
| 174 | RAF1 | 1645 | 236,456 | 38.9 | 2.3% | 676,894,456 | 62.1 | 9.0% | 676, 1111,456 | 85.0 | 9.3% | 13 |
| 175 | RAF1 | 1780 | 237,457 | 22.6 | 4.9% | 677,895,457 | 24.8 | 5.3% | 677, 1112,457 | 37.6 | 10.4% | 12 |
| 176 | RAF1 | 1799 | 238,458 | 23.2 | 3.1% | 678,896,458 | 43.6 | 7.6% | 678, 1113,458 | 50.7 | 6.2% | 12 |
| 177 | RAF1 | 1807 | 239,459 | 28.0 | 5.4% | 679,897,459 | 34.8 | 5.8% | 679, 1114,459 | 37.0 | 5.3% | 15 |
| 178 | RAF1 | 1863 | 240,460 | 28.2 | 3.1% | 680,898,460 | 38.1 | 4.5% | 680, 1115,460 | 35.7 | 4.2% | 14 |
| 179 | RAF1 | 2157 | 241,461 | 68.8 | 6.5% | 681,899,461 | 64.1 | 8.0% | 681, 1116,461 | 86.7 | 12.6% | 14 |
| 180 | RAF1 | 2252 | 242,462 | 11.4 | 1.7% | 682,900,462 | 25.8 | 5.4% | 682, 1117,462 | 71.2 | 10.7% | 13 |
| 181 | SRD5A1 | 1150 | 243,463 | 3.7 | 0.5% | 683,901,463 | 4.4 | 0.7% | 683, 1118,463 | 3.8 | 0.4% | 12 |
| 182 | SRD5A1 | 1153 | 244,464 | 3.2 | 0.4% | 684,902,464 | 5.2 | 0.5% | 684, 1119,464 | 7.0 | 0.9% | 12 |
| 183 | SRD5A1 | 1845 | 245,465 | 3.9 | 0.5% | 685, 903, 465 | 4.5 | 0.6% | 685, 1120,465 | 7.4 | 0.8% | 13 |
| 184 | SRD5A1 | 1917 | 246,466 | 9.4 | 0.8% | 686,904,466 | 10.2 | 1.3% | 686, 1121,466 | 22.0 | 2.8% | 12 |
| 185 | SRD5A1 | 1920 | 247,467 | 4.6 | 0.3% | 687,905,467 | 4.9 | 1.0% | 687,1122,467 | 6.4 | 0.5% | 11 |
| 186 | SRD5A1 | 1964 | 248,468 | 6.2 | 0.7% | 688,906,468 | 10.4 | 0.7% | 688, 1123, 468 | 21.0 | 4.6% | 10 |
| 187 | SRD5A1 | 1981 | 249,469 | 6.5 | 1.0% | 689,907,469 | 7.1 | 0.7% | 689,1124,469 | 8.8 | 1.5% | 12 |
| 188 | SRD5A1 | 2084 | 250,470 | 16.9 | 1.1% | 690,908,470 | 15.7 | 1.5% | 690, 1125, 470 | 13.3 | 1.5% | 12 |
| 189 | SRD5A1 | 2085 | 251,471 | 17.3 | 1.6% | 691,909,471 | 19.4 | 1.7% | 691, 1126,471 | 20.8 | 2.6% | 12 |
| 190 | SRD5A1 | 2103 | 252,472 | 7.5 | 1.3% | 692,910,472 | 10.9 | 1.2% | 692, 1127,472 | 12.3 | 1.7% | 12 |
| 191 | TNF | 32 | 253,473 | 71.4 | 13.2% | 693,911,473 | 93.7 | 14.9% | 693, 1128,473 | 122.6 | 21.1% | 12 |
| 192 | TNF | 649 | 254,474 | 100.0 | 16.3% | 694,912,474 | 127.7 | 12.6% | 694,1129,474 | 147.9 | 21.7% | 12 |
| 193 | TNF | 802 | 255,475 | 67.2 | 10.7% | 695,913,475 | 64.0 | 6.6% | 695, 1130,475 | 116.4 | 21.0% | 12 |
| 194 | TNF | 875 | 256,476 | 101.7 | 19.9% | 696,914,476 | 99.3 | 15.5% | 696,1131,476 | 108.8 | 14.2% | 12 |
| 195 | TNF | 983 | 257,477 | 94.5 | 7.0% | 697,915,477 | 83.1 | 7.3% | 697, 1132,477 | 140.6 | 20.4% | 11 |
| 196 | TNF | 987 | 258,478 | 82.0 | 10.9% | 698,916,478 | 139.4 | 8.2% | 698, 1133,478 | 143.8 | 9.2% | 10 |
| 197 | TNF | 992 | 259,479 | 126.7 | 15.8% | 699,700,479 | 121.7 | 10.8% | 699, 1134,479 | 115.9 | 16.4% | 11 |
| 198 | TNF | 1003 | 260,480 | 123.4 | 16.7% | 700,917,480 | 114.4 | 47.8% | 700, 1135,480 | 98.5 | 17.2% | 14 |
| 199 | TNF | 1630 | 261,481 | 58.0 | 5.7% | 701, 918, 481 | 56.1 | 9.4% | 701, 1136,481 | 71.0 | 17.2% | 11 |
| 200 | TNF | 1631 | 262,482 | 54.2 | 13.4% | 702,919,482 | 63.9 | 10.1% | 702, 1137,482 | 73.8 | 14.8% | 11 |
| 201 | TNFSF13B | 188 | 263,483 | 20.4 | 3.2% | 703,920,483 | 46.2 | 11.9% | 703, 1138,483 | 58.4 | 12.7% | 13 |
| 202 | TNFSF13B | 313 | 264,484 | 15.9 | 5.1% | 704,921,484 | 18.9 | 7.4% | 704, 1139,484 | 48.0 | 8.1% | 12 |
| 203 | TNFSF13B | 337 | 265,485 | 22.3 | 4.6% | 705,922,485 | 37.1 | 11.0% | 705, 1140,485 | 63.6 | 10.4% | 12 |
| 204 | TNFSF13B | 590 | 266,486 | 35.8 | 8.7% | 706,923,486 | 49.4 | 11.0% | 706, 1141,486 | 50.7 | 10.3% | 10 |
| 205 | TNFSF13B | 652 | 267,487 | 21.3 | 7.2% | 707,924,487 | 57.6 | 16.7% | 707, 1142,487 | 78.8 | 5.6% | 14 |
| 206 | TNFSF13B | 661 | 268,488 | 28.8 | 3.0% | 708,925,488 | 38.3 | 8.4% | 708, 1143,488 | 56.5 | 16.3% | 12 |
| 207 | TNFSF13B | 684 | 269,489 | 46.3 | 7.2% | 709,926,489 | 43.8 | 9.7% | 709, 1144,489 | 54.5 | 4.6% | 12 |
| 208 | TNFSF13B | 905 | 270,490 | 18.5 | 5.0% | 710,927,490 | 27.9 | 3.1% | 710, 1145,490 | 51.7 | 10.9% | 12 |
| 209 | TNFSF13B | 961 | 271,491 | 21.4 | 4.0% | 711,928,491 | 3 7.5 | 10.1% | 711, 1146,491 | 77.6 | 11.2% | 14 |
| 210 | TNFSF13B | 1150 | 272,492 | 24.1 | 7.0% | 712,929,492 | 23.4 | 5.7% | 712, 1147,492 | 35.9 | 8.0% | 13 |
| 211 | VEGFA | 1426 | 273,493 | 14.5 | 2.2% | 713,930,493 | 18.1 | 3.2% | 713, 1148,493 | 21.0 | 3.8% | 13 |
| 212 | VEGFA | 1428 | 274,494 | 18.5 | 2.6% | 714,931,494 | 32.1 | 5.8% | 714, 1149,494 | 46.7 | 9.4% | 12 |
| 213 | VEGFA | 1603 | 275,495 | 14.6 | 2.1 % | 715,932,495 | 36.6 | 17.5% | 715, 1150,495 | 65.6 | 6.9% | 13 |
| 214 | VEGFA | 1685 | 276,496 | 17.1 | 1.3% | 716,933,496 | 20.2 | 5.5% | 716,1151,496 | 23.4 | 3.8% | 13 |
| 215 | VEGFA | 1792 | 277,497 | 17.0 | 1.8% | 717,934,497 | 21.2 | 3.2% | 717, 1152,497 | 39.5 | 6.3 % | 12 |
| 216 | VEGFA | 2100 | 278,498 | 116.9 | 11.5% | 718,935,498 | 103.6 | 7.5% | 718, 1153,498 | 101.5 | 12.9% | 12 |
| 217 | VEGFA | 2102 | 279,499 | 116.3 | 9.1% | 719,936,499 | 110.2 | 9.3% | 719,1154,499 | 105.0 | 8.0% | 12 |
| 218 | VEGFA | 2196 | 280,500 | 24.2 | 2.7% | 720,937,500 | 26.6 | 3.1% | 720, 1155,500 | 43.5 | 3.5% | 12 |
| 219 | VEGFA | 2261 | 281,501 | 15.6 | 2.2% | 721,938,501 | 44.2 | 6.2% | 721, 1156,501 | 109.0 | 9.8% | 12 |
| 220 | VEGFA | 2292 | 282,502 | 48.4 | 4.3 % | 722,939,502 | 45.1 | 7.2% | 722, 1157,502 | 80.7 | 6.7% | 15 |

| | | | | | | | | | | | | |
|---|---|---|---|---|---|---|---|---|---|---|---|---|
| * All samples were normalized to the respective dsRNA QNeg (Qiagen) negative control samples run in the same experiment. That is, QNeg values were set as 100% active (*i.e.,* no knockdown), with 95% confidence intervals (CI) ranging from 6.3-22.5%. As a positive control, an siRNA specific for rLuc was used, which samples showed on average expression levels that varied from 1.2% to 16.8% *(i.e.,* about 83% to about 99% knockdown activity and a 95% CI ranging from 0.3% to 13.7%). † "Pos" refers to the position on the target gene mRNA message that aligns with the 5'-end of the dsRNA sense strand. The mRNA numbering is based on the GenBank accession numbers as described herein. ‡ The SEQ ID NOS. are provided in the following order: (1) Dicer: sense strand, antisense strand; (2) Nicked: 5'-sense strand fragment, 3'-sense strand fragment, and antisense strand; and (3) Gapped: 5'-sense strand fragment, 3'-sense strand fragment, and antisense strand. The Dicer dsRNA has two strands, while ndsRNA and gdsRNA have three strands each. The nicked or gapped sense strand fragments have three locked nucleic acids each. ^ "Length 5'-S" refers to the length of the 5'-sense strand fragment of the nicked or gapped mdRNA, which indicates the position of the nick (*e.g.*, 10 means the nick is between position 10 and 11, so the 5'sense strand fragment is 10 nucleotides long and the 3'-sense strand fragment is 15 nucelotides long) or one nucleotide gap (*e.g.,* 10 means the missing nucleotide is number 11, so the 5'sense strand fragment is 10 nucleotides long and the 3'-sense strand fragment is 14 nucelotides long). | | | | | | | | | | | | |

### EXAMPLE 2

### KNOCKDOWN OF β-GALACTOSIDASE ACTIVITY BY GAPPED DSRNA DICER SUBSTRATE

The activity of a Dicer substrate dsRNA containing a gap in the double-stranded structure in silencing LacZ mRNA as compared to the normal Dicer substrate dsRNA *(i.e.,* not having a gap) was examined.

### Nucleotide Sequences of dsRNA and mdRNA Targeting LacZ mRNA

The nucleic acid sequence of the one or more sense strands, and the antisense strand of the dsRNA and gapped dsRNA (also referred to herein as a meroduplex or mdRNA) are shown below and were synthesized using standard techniques. The RISC activator LacZ dsRNA comprises a 21 nucleotide sense strand and a 21 nucleotide antisense strand, which can anneal to form a double-stranded region of 19 base pairs with a two deoxythymidine overhang on each strand (referred to as 21/21 dsRNA).

LacZ dsRNA (21/21) - RISC Activator

| | | |
|---|---|---|
| Sense | 5'-CUACACAAAUCAGCGAUUUdTdT-3' | (SEQ ID NO:1) |
| Antisense | 3'-dTdTGAUGUGUUUAGUCGCUAAA-5' | (SEQ ID NO:2) |

The Dicer substrate LacZ dsRNA comprises a 25 nucleotide sense strand and a 27 nucleotide antisense strand, which can anneal to form a double-stranded region of 25 base pairs with one blunt end and a cytidine and uridine overhang on the other end (referred to as 25/27 dsRNA).

LacZ dsRNA (25/27) - Dicer Substrate

| | | |
|---|---|---|
| Sense | 5'-CUACACAAAUCAGCGAUUUCCAUdGdT-3' | (SEQ ID NO:3) |
| Antisense | 3'-CUGAUGUGUUUAGUCGCUAAAGGUA C A - 5' | (SEQ ID NO:4) |

The LacZ mdRNA comprises two sense strands of 13 nucleotides (5'-portion) and 11 nucleotides (3'-portion) and a 27 nucleotide antisense strand, which three strands can anneal to form two double-stranded regions of 13 and 11 base pairs separated by a single nucleotide gap (referred to as a 13, 11/27 mdRNA). The 5'-end of the 11 nucleotide sense strand fragment may be optionally phosphorylated. The "*" indicates a gap - in this case, a single nucleotide gap (*i.e*., a cytidine is missing).

LacZ mdRNA (13, 11/27) - Dicer Substrate

| | | |
|---|---|---|
| Sense | 5'-CUACACAAAUCAG*GAUUUCCAUdGdT-3' | (SEQ ID NOS:5, 6) |
| Antisense | 3'-CUGAUGUGUUUAGUCGCUAAAGGUA C A - 5' | (SEQ ID NO:4) |

Each of the LacZ dsRNA or mdRNA was used to transfect 9lacZ/R cells.

### Transfection

Six well collagen-coated plates were seeded with 5 x 10⁵ 9lacZ/R cells/well in a 2 ml volume per well, and incubated overnight at 37°C / 5% CO₂ in DMEM/high glucose media. Preparation for transfection: 250 µl of OPTIMEM media without serum was mixed with 5 µl of 20 pmol/µl dsRNA and 5µl of HIPERFECT transfection solution (Qiagen) was mixed with another 250 µl OPTIMEM media. After both mixtures were allowed to equilibrate for 5 minutes, the RNA and transfection solutions were combined and left at room temperature for 20 minutes to form transfection complexes. The final concentration of HIPERFECT was 50 µM, and the dsRNAs were tested at 0.05nM, 0.1nM, 0.2nM, 0.5nM, 1nM, 2nM, 5nM, and 10nM, while the mdRNA was tested at 0.2nM, 0.5nM, 1nM, 2nM, 5nM, 10nM, 20nM, and 50nM. Complete media was removed, the cells were washed with incomplete OPTIMEM, and then 500 µl transfection mixture was applied to the cells, which were incubated with gentle shaking at 37°C for 4 hours. After transfecting, the transfection media was removed, cells were washed once with complete DMEM/high glucose media, fresh media added, and the cells were then incubated for 48 hours at 37°C, 5% CO₂.

### β-Galactosidase Assay

Transfected cells were washed with PBS, and then detached with 0.5 ml trypsin/EDTA. The detached cells were suspended in 1 ml complete DMEM/high glucose and transferred to a clean tube. The cells were harvested by centrifugation at 250 x g for 5 minutes, and then resuspended in 50 µl 1x lysis buffer at 4°C. The lysed cells were subjected to two freeze-thaw cycles on dry ice and a 37°C water bath. The lysed samples were centrifuged for 5 minutes at 4°C and the supernatant was recovered. For each sample, 1.5 µl and 10 µl of lysate was transferred to a clean tube and sterile water added to a final volume of 30 µl followed by the addition of 70 µl *o*-nitrophenyl-β-*D*-galactopyranose (ONPG) and 200 µl 1x cleavage buffer with β-mercaptoethanol. The samples were mixed briefly, incubated for 30 minutes at 37°C, and then 500 µl stop buffer was added (final volume 800 µl). β-Galactosidase activity for each sample was measured in disposable cuvettes at 420 nm. Protein concentration was determined by the BCA (bicinchoninic acid) method. For the purpose of the instant example, the level of measured LacZ activity was correlated with the quantity of LacZ transcript within 9L/LacZ cells. Thus, a reduction in β-galactosidase activity after dsRNA transfection, absent a negative impact on cell viability, was attributed to a reduction in the quantity of LacZ transcripts resulting from targeted degradation mediated by the LacZ dsRNA.

### Results

Knockdown activity in transfected and untransfected cells was normalized to a Qneg control dsRNA and presented as a normalized value of the Qneg control *(i.e.,* Qneg represented 100% or "normal" gene expression levels). Both the lacZ RISC activator and Dicer substrate dsRNAs molecule showed good knockdown of β-galactosidase activity at concentration as low as 0.1 nM (Figure 2), while the Dicer substrate antisense strand alone (single stranded 27mer) had no silencing effect. Surprisingly, a gapped mdRNA showed good knockdown although somewhat lower than that of intact RISC activator and Dicer substrate dsRNAs (Figure 2). The presence of the gapmer cytidine (*i.e*., the missing nucleotide) at various concentrations (0.1 µM to 50 µM) had no effect on the activity of the mdRNA (data not shown). None of the dsRNA or mdRNA solutions showed any detectable toxicity in the transfected 9L/LacZ cells. The IC₅₀ of the lacZ mdRNA was calculated to be 3.74 nM, which is about 10 fold lower than what had been previously measured for lacZ dsRNA 21/21 (data not shown). These results show that a meroduplex (gapped dsRNA) is capable of inducing gene silencing.

### EXAMPLE 3

### KNOCKDOWN OF INFLUENZA GENE EXPRESSION BY NICKED DSRNA

The activity of a nicked dsRNA (21/21) in silencing influenza gene expression as compared to a normal dsRNA (*i.e*., not having a nick) was examined.

### Nucleotide Sequences of dsRNA and mdRNA Targeting Influenza mRNA

The dsRNA and nicked dsRNA (another form of meroduplex, referred to herein as ndsRNA) are shown below and were synthesized using standard techniques. The RISC activator influenza G1498 dsRNA comprises a 21 nucleotide sense strand and a 21 nucleotide antisense strand, which can anneal to form a double-stranded region of 19 base pairs with a two deoxythymidine overhang on each strand.

G1498-wt dsRNA (21/21)

| | | |
|---|---|---|
| Sense | 5'-GGAUCUUAUUUCUUCGGAGdTdT-3' | (SEQ ID NO:7) |
| Antisense | 3'-dTdTCCUAGAAUAAAGAAGCCUC-5' | (SEQ ID NO:8) |

The RISC activator influenza G1498 dsRNA was nicked on the sense strand after nucleotide 11 to produce a ndsRNA having two sense strands of 11 nucleotides (5'-portion, italic) and 10 nucleotides (3'-portion) and a 21 nucleotide antisense strand, which three strands can anneal to form two double-stranded regions of 11 (shown in italics) and 10 base pairs separated by a one nucleotide gap (which may be referred to as G1498 11, 10/21 ndsRNA-wt). The 5'-end of the 10 nucleotide sense strand fragment may be optionally phosphorylated, as depicted by a "p" preceding the nucleotide (*e.g.*, pC).

G1498 ndsRNA-wt (11, 10/21)

| | | |
|---|---|---|
| Sense | 5'-*GGAUCUUAUUU*CUUCGGAGdTdT-3' | (SEQ ID NO:9, 10) |
| Antisense | 3'-dTdTCCUAGAAUAAAGAAGCCUC-5' | (SEQ ID NO:8) |

G1498 ndsRNA-wt (11, 10/21)

| | | |
|---|---|---|
| Sense | 5'-*GGAUCUUAUUU*pCUUCGGAGdTdT-3' | (SEQ ID NOS:9, 10) |
| Antisense | 3'-dTdTCCUAGAAUAAAGAAGCCUC-5' | (SEQ ID NO:8) |

In addition, each of these G1498 dsRNAs were made with each U substituted with a 5-methyluridine (ribothymidine) and are referred to as G1498 dsRNA-rT. Each of the G1498 dsRNA or ndsRNA (meroduplex), with or without the 5-methyluridine substitution, was used to transfect HeLa S3 cells having an influenza target sequence associated with a luciferase gene. Also, the G1498 antisense strand alone or the antisense strand annealed to the 11 nucleotide sense strand portion alone or the 10 nucleotide sense strand portion alone were examined for activity.

### Transfection and Dual Luciferase Assay

The reporter plasmid psiCHECK^{™}-2 (Promega, Madison, WI), which constitutively expresses both firefly *luc2 (Photinus pyralis)* and *Renilla* (*Renilla reniformis,* also known as sea pansy) luciferases, was used to clone in a portion of the influenza NP gene downstream of the *Renilla* translational stop codon that results in a *Renilla*-influenza NP fusion mRNA. The firefly luciferase in the psiCHECK™-2 vector is used to normalize Renilla luciferase expression and serves as a control for transfection efficiency.

Multi-well plates were seeded with HeLa S3 cells/well in 100 µl Ham's F12 medium and 10% fetal bovine serum, and incubated overnight at 37°C / 5% CO₂. The HeLa S3 cells were transfected with the psiCHECK™-influenza plasmid (75 ng) and G1498 dsRNA or ndsRNA (final concentration of 10 nM or 100 nM) formulated in Lipofectamine™ 2000 and OPTIMEM reduced serum medium. The transfection mixture was incubated with the HeLa S3 cells with gentle shaking at 37°C for about 18 to 20 hours.

After transfecting, firefly luciferase reporter activity was measured first by adding Dual-Glo^{™} Luciferase Reagent (Promega, Madison, WI) for 10 minutes with shaking, and then quantitating the luminescent signal using a VICTOR³™ 1420 Multilabel Counter (PerkinElmer, Waltham, MA). After measuring the firefly luminescence, Stop & Glo^{®} Reagent (Promega, Madison, WI) was added for 10 minutes with shaking to simultaneously quench the firefly reaction and initiate the *Renilla* luciferase reaction, and then the *Renilla* luciferase luminescent signal was quantitated VICTOR³™ 1420 Multilabel Counter (PerkinElmer, Waltham, MA).

### Results

Knockdown activity in transfected and untransfected cells was normalized to a Qneg control dsRNA and presented as a normalized value of the Qneg control *(i.e.,* Qneg represented 100% or "normal" gene expression levels). Thus, a smaller value indicates a greater knockdown effect. The G1498 dsRNA-wt and dsRNA-rT showed similar good knockdown at a 100 nM concentration (Figure 3). Surprisingly, the G1498 ndsRNA-rT, whether phosphorylated or not, showed good knockdown although somewhat lower than the G1498 dsRNA-wt (Figure 3). Similar results were obtained with dsRNA or ndsRNA at 10 nM (data not shown). None of the G1498 dsRNA or ndsRNA solutions showed any detectable toxicity in HeLa S3 cells at either 10 nM or 100 nM. Even the presence of only half a nicked sense strand (an 11 nucleotide or 10 nucleotide strand alone) with a G1498 antisense strand showed some detectable activity. These results show that a nicked-type meroduplex dsRNA molecule is unexpectedly capable of promoting gene silencing.

### EXAMPLE 4

### KNOCKDOWN ACTIVITY OF NICKED MDRNA

In this example, the activity of a dicer substrate LacZ dsRNA of Example 1 having a sense strand with a nick at various positions was examined. In addition, a dideoxy nucleotide *(i.e.,* ddG) was incorporated at the 5'-end of the 3'-most strand of a sense sequence having a nick or a single nucleotide gap to determine whether the *in vivo* ligation of the nicked sense strand is "rescuing" activity. The ddG is not a substrate for ligation. Also examined was the influenza dicer substrate dsRNA of Example 7 having a sense strand with a nick at one of positions 8 to 14. The "p" designation indicates that the 5'-end of the 3'-most strand of the nicked sense influenza sequence was phosphorylated. The "L" designation indicates that the G at position 2 of the 5'-most strand of the nicked sense influenza sequence was substituted for a locked nucleic acid G. The Qneg is a negative control dsRNA.

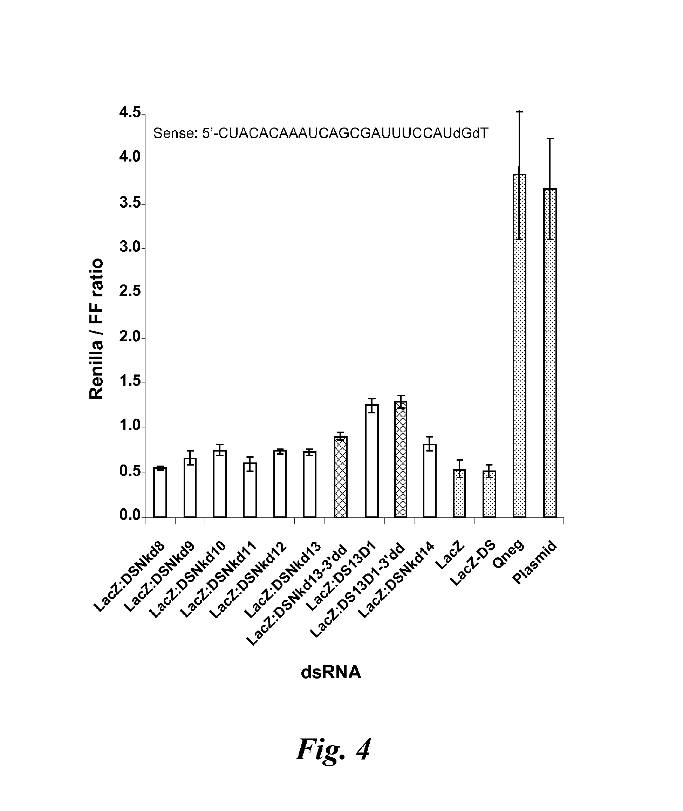

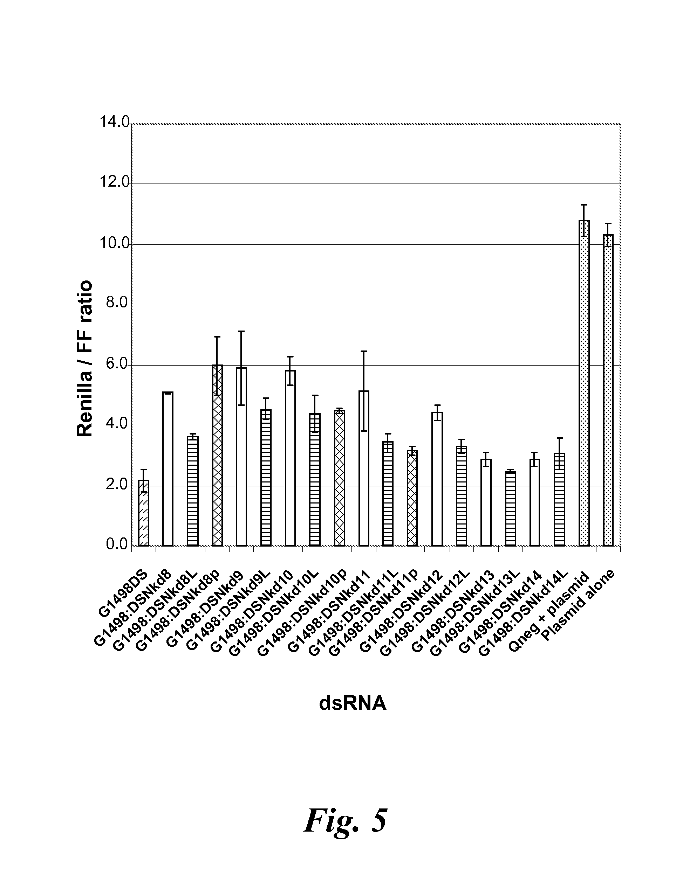

The dual fluorescence assay of Example 3 was used to measure knockdown activity with 5 nM of the LacZ sequences and 0.5 nM of the influenza sequences. The lacZ dicer substrate (25/27, LacZ-DS) and lacZ RISC activator (21/21, LacZ) are equally active, and the LacZ-DS can be nicked in any position between 8 and 14 without affecting activity (Figure 3). In addition, the inclusion of a ddG on the 5'-end of the 3'-most LacZ sense sequence having a nick (LacZ:DSNkd13-3'dd) or a one nucleotide gap (LacZ:DSNkd13D1-3'dd) was essentially as active as the unsubstituted sequence (Figure 4). The influenza dicer substrate (G1498DS) nicked at any one of positions 8 to 14 was also highly active (Figure 5). Phosphorylation of the 5'-end of the 3'-most strand of the nicked sense influenza sequence had essentially no effect on activity, but addition of a locked nucleic acid appears to improve activity.

### EXAMPLE 5

### MEAN INHIBITORY CONCENTRATION OF MDRNA

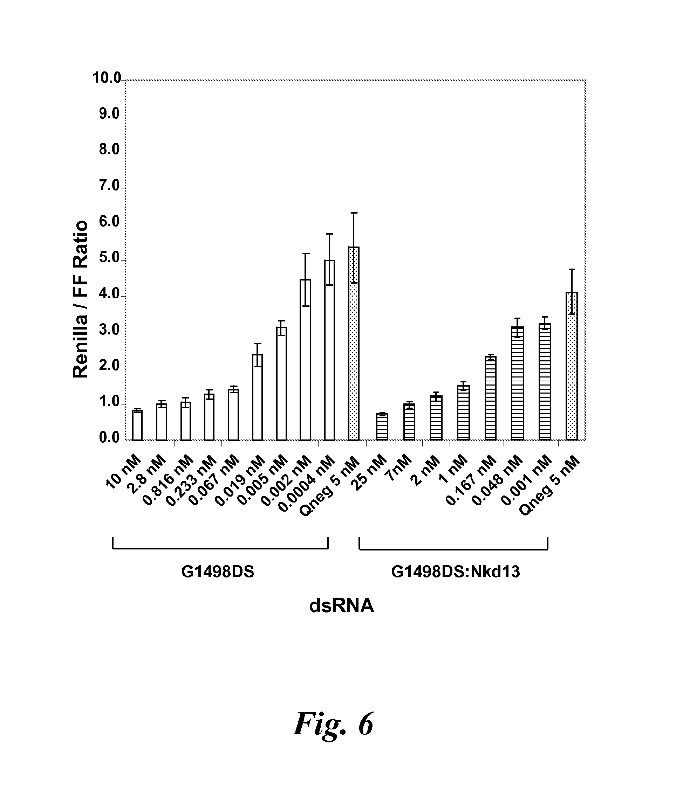

In this example, a dose response assay was performed to measure the mean inhibitory concentration (IC₅₀) of the influenza dicer substrate dsRNA of Example 8 having a sense strand with a nick at position 12, 13, or 14, including or not a locked nucleic acid. The dual luciferase assay of Example 2 was used. The influenza dicer substrate dsRNA (G1498DS) was tested at 0.0004 nM, 0.002 nM, 0.005 nM, 0.019 nM, 0.067 nM, 0.233 nM, 0.816 nM, 2.8 nM, and 10nM, while the mdRNA with a nick at position 13 (G1498DS:Nkd13) was tested at 0.001 nM, 0.048 nM, 0.167 nM, 1 nM, 2 nM, 7 nM, and 25 nM *(see* Figure 6). Also tested were RISC activator molecules (21/21) with or without a nick at various positions (including G1498DS:Nkd11, G1498DS:Nkd12, and G1498DS:Nkd14), each of the nicked versions with a locked nucleic acid as described above (data not shown). The Qneg is a negative control dsRNA.

The IC₅₀ of the RISC activator G1498 was calculated to be about 22 pM, while the dicer substrate G1498DS IC₅₀ was calculated to be about 6 pM. The IC₅₀ of RISC and Dicer mdRNAs range from about 200 pM to about 15 nM. The inclusion of a single locked nucleic acid reduced the IC₅₀ of Dicer mdRNAs by up 4 fold (data not shown). These results show that a meroduplex dsRNA having a nick or gap in any position is capable of inducing gene silencing.

### EXAMPLE 6

### KNOCKDOWN ACTIVITY OF GAPPED MDRNA

The activity of an influenza dicer substrate dsRNA having a sense strand with a gap of differing sizes and positions was examined. The influenza dicer substrate dsRNA of Example 8 was generated with a sense strand having a gap of 0 to 6 nucleotides at position 8, a gap of 4 nucleotides at position 9, a gap of 3 nucleotides at position 10, a gap of 2 nucleotides at position 11, and a gap of 1 nucleotide at position 12 *(see* Table 2). The Qneg is a negative control dsRNA. Each of the mdRNAs was tested at a concentration of 5 nM (data not shown) and 10 nM. The mdRNAs have the following antisense strand 5'-CAUUGUCUCCGAAGAAAUAAGAUCCUU (SEQ ID NO:11), and nicked or gapped sense strands as shown in Table 2.

**Table 2.**

| mdRNA | 5' Sense* (SEQ ID NO.) | 3' Sense (SEQ ID NO.) | Gap Pos | Gap Size | % KD^{†} |
|---|---|---|---|---|---|
| G1498:DSNkd8 | G**G**AUCUUA (12) | UUUCUUCGGAGACAAdTdG (13) | 8 | 0 | 67.8 |
| G1498:DSNkd8D1 | G**G**AUCUUA (12) | UUCUUCGGAGACAAdTdG (14) | 8 | 1 | 60.9 |
| G1498:DSNkd8D2 | G**G**AUCUUA (12) | UCUUCGGAGACAAdTdG (15) | 8 | 2 | 48.2 |
| G1498:DSNkd8D3 | G**G**AUCUUA (12) | CUUCGGAGACAAdTdG (16) | 8 | 3 | 44.1 |
| G1498:DSNkd8D4 | G**G**AUCUUA (12) | UUCGGAGACAAdTdG (17) | 8 | 4 | 30.8 |
| G1498:DSNkd8D5 | G**G**AUCUUA (12) | UCGGAGACAAdTdG (18) | 8 | 5 | 10.8 |
| G1498:DSNkd8D6 | G**G**AUCUUA (12) | CGGAGACAAdTdG (19) | 8 | 6 | 17.9 |
| G1498:DSNkd9D4 | G**G**AUCUUAU (20) | UCGGAGACAAdTdG (18) | 9 | 4 | 38.9 |
| G1498:DSNkd10D3 | G**G**AUCUUAUU (21) | UCGGAGACAAdTdG (18) | 10 | 3 | 38.4 |
| G1498:DSNkd11D2 | G**G**AUCUUAUUU (22) | UCGGAGACAAdTdG (18) | 11 | 2 | 46.2 |
| G1498:DSNkd12D1 | G**G**AUCUUAUUUC (23) | UCGGAGACAAdTdG (18) | 12 | 1 | 49.6 |
| Plasmid | - | - | - | - | 5.3 |

| | | | | | |
|---|---|---|---|---|---|
| * **G** indicates a locked nucleic acid G m the 5' sense strand. ^{†} % KD means percent knockdown activity. | | | | | |

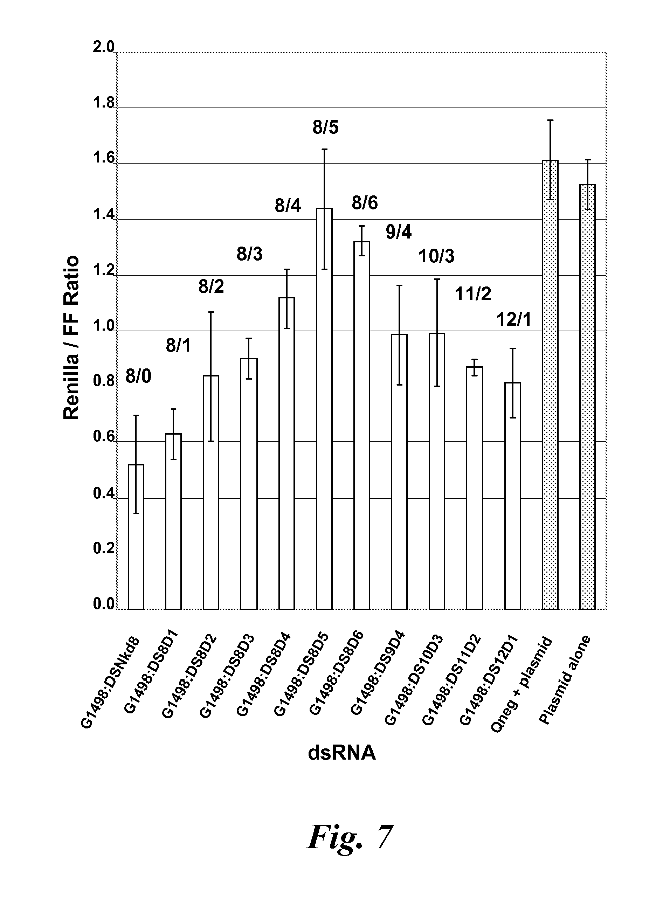

The dual fluorescence assay of Example 2 was used to measure knockdown activity. Similar results were obtained at both the 5 nM and 10 nM concentrations. These data show that an mdRNA having a gap of up to 6 nucleotides still has activity, although having four or fewer missing nucleotides shows the best activity (*see, also,* Figure 7). Thus, mdRNA having various sizes gaps that are in various different positions have knockdown activity.

To examine the general applicability of a sequence having a sense strand with a gap of differing sizes and positions, a different dsRNA sequence was tested. The lacZ RISC dsRNA of Example 1 was generated with a sense strand having a gap of 0 to 6 nucleotides at position 8, a gap of 5 nucleotides at position 9, a gap of 4 nucleotides at position 10, a gap of 3 nucleotides at position 11, a gap of 2 nucleotides at position 12, a gap of 1 nucleotide at position 12, and a nick (gap of 0) at position 14 *(see* Table 3). The Qneg is a negative control dsRNA. Each of the mdRNAs was tested at a concentration of 5 nM (data not shown) and 25 nM. The lacZ mdRNAs have the following antisense strand 5'-AAAUCGCUGAUUUGUGUAGdTdTUAAA (SEQ ID NO:2) and nicked or gapped sense strands as shown in Table 3.

**Table 3.**

| mdRNA | 5' Sense* (SEQ ID NO.) | 3' Sense* (SEQ ID NO.) | Gap Pos | Gap Size |
|---|---|---|---|---|
| LacZ:Nkd8 | CU**A**CACAA (24) | AUCAGCG**A**UUUdTdT(25) | 8 | 0 |
| LacZ:Nkd8D1 | CU**A**CACAA (24) | UCAGCG**A**UUUdTdT (26) | 8 | 1 |
| LacZ:Nkd8D2 | CU**A**CACAA (24) | CAGCG**A**UUUdTdT(27) | 8 | 2 |
| LacZ:Nkd8D3 | CU**A**CACAA (24) | AGCG**A**UUUdTdT (28) | 8 | 3 |
| LacZ:Nkd8D4 | CU**A**CACAA (24) | GCG**A**UUUdTdT (29) | 8 | 4 |
| LacZ:Nkd8D5 | CU**A**CACAA (24) | G**A**UUUdTdT (30) | 8 | 5 |
| LacZ:Nkd8D6 | CU**A**CACAA (24) | G**A**UUUdTdT (31) | 8 | 6 |
| LacZ:Nkd9D5 | CU**A**CACAAA (32) | G**A**UUUdTdT (31) | 9 | 5 |
| LacZ:Nkd10D4 | CU**A**CACAAAU (33) | G**A**UUUdTdT (31) | 10 | 4 |
| LacZ:Nkd11D3 | CU**A**CACAAAUC (34) | G**A**UUUdTdT (31) | 11 | 3 |
| LacZ:Nkd12D2 | CU**A**CACAAAUCA (35) | G**A**UUUdTdT (31) | 12 | 2 |
| LacZ:Nkd13D1 | CU**A**CACAAAUCAG (36) | G**A**UUUdTdT (31) | 13 | 1 |
| LacZ:Nkd14 | CU**A**CACAAAUCAGC (37) | G**A**UUUdTdT (31) | 14 | 0 |

| | | | | |
|---|---|---|---|---|
| * **A** Indicates a locked nucleic acid A m each sense strand. | | | | |

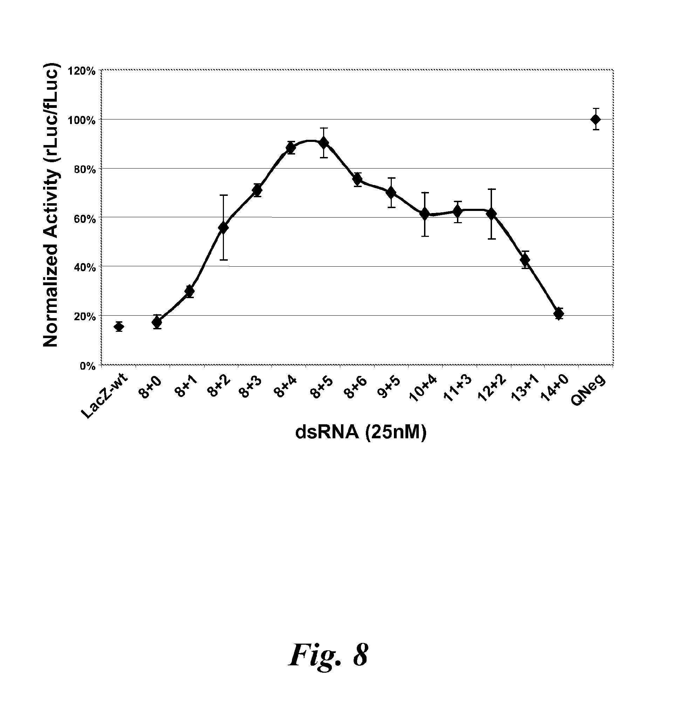

The dual fluorescence assay of Example 3 was used to measure knockdown activity. Figure 8 shows that an mdRNA having a gap of up to 6 nucleotides has substantial activity and the position of the gap may affect the potency of knockdown. Thus, mdRNA having various sizes gaps that are in various different positions and in different mdRNA sequences have knockdown activity.

### EXAMPLE 7

### KNOCKDOWN ACTIVITY OF SUBSTITUTED MDRNA

The activity of an influenza dsRNA RISC sequences having a nicked sense strand and the sense strands having locked nucleic acid substitutions were examined. The influenza RISC sequence G1498 of Example 3 was generated with a sense strand having a nick at positions 8 to 14 counting from the 5'-end. Each sense strand was substituted with one or two locked nucleic acids as shown in Table 4. The Qneg and Plasmid are negative controls. Each of the mdRNAs was tested at a concentration of 5 nM. The antisense strand used was 5'-CUCCGAAGAAAUAAGAUCCdTdT (SEQ ID NO:8).

**Table 4.**

| mdRNA | 5' Sense* (SEQ ID NO.) | 3' Sense* (SEQ ID NO.) | Nick Pos | % KD |
|---|---|---|---|---|
| G1498-wt | GGAUCUUAUUUCUUCGGAGdTdT (7) | - | - | 85.8 |
| G1498-L | G**G**AUCUUAUUUCUUC**G**GAGdTdT (61) | - | - | 86.8 |
| G1498:Nkd8-1 | G**G**AUCUUA(12) | UUUCUUC**G**GAGdTdT (47) | 8 | 36.0 |
| G1498:Nkd8-2 | G**G**AUCUU**A** (40) | **U**UUCUUC**G**GAGdTdT (54) | 8 | 66.2 |
| G1498:Nkd9-1 | G**G**AUCUUAU (20) | UUCUUC**G**GAGdTdT (48) | 9 | 60.9 |
| G1498:Nkd9-2 | G**G**AUCUUA**U** (41) | **U**UCUUC**G**GAGdTdT (55) | 9 | 64.4 |
| G1498:Nkd10-1 | G**G**AUCUUAUU(21) | **U**CUUC**G**GAGdTdT (49) | 10 | 58.2 |
| G1498:Nkd10-2 | G**G**AUCUUAU**U** (42) | **U**CUUC**G**GAGdTdT (56) | 10 | 68.5 |
| G1498:Nkd11-1 | G**G**AUCUUAUUU (22) | CUUC**G**GAGdTdT (50) | 11 | 75.9 |
| G1498:Nkd11-2 | G**G**AUCUUAUU**U** (43) | **C**UUC**G**GAGdTdT (57) | 11 | 67.1 |
| G1498:Nkd12-1 | G**G**AUCUUAUUUC (23) | UUC**G**GAGdTdT (51) | 12 | 59.9 |
| G1498:Nkd12-2 | G**G**AUCUUAUUU**C** (44) | **U**UC**G**GAGdTdT (58) | 12 | 72.8 |
| G1498:Nkd13-1 | G**G**AUCUUAUUUCU (38) | UC**G**GAGdTdT (52) | 13 | 37.1 |
| G1498:Nkd13-2 | G**G**AUCUUAUUUC**U** (45) | **U**C**G**GAGdTdT (59) | 13 | 74.3 |
| G1498:Nkd14-1 | G**G**AUCUUAUUUCUU (39) | C**G**GAGdTdT (53) | 14 | 29.0 |
| G1498:Nkd14-2 | G**G**AUCUUAUUUCU**U** (46) | **CG**GAGdTdT (60) | 14 | 60.2 |
| Qneg | - | - | - | 0 |
| Plasmid | - | - | - | 3.6 |

| | | | | |
|---|---|---|---|---|
| * Nucleotides that are bold and underlined are locked nucleic acids. | | | | |

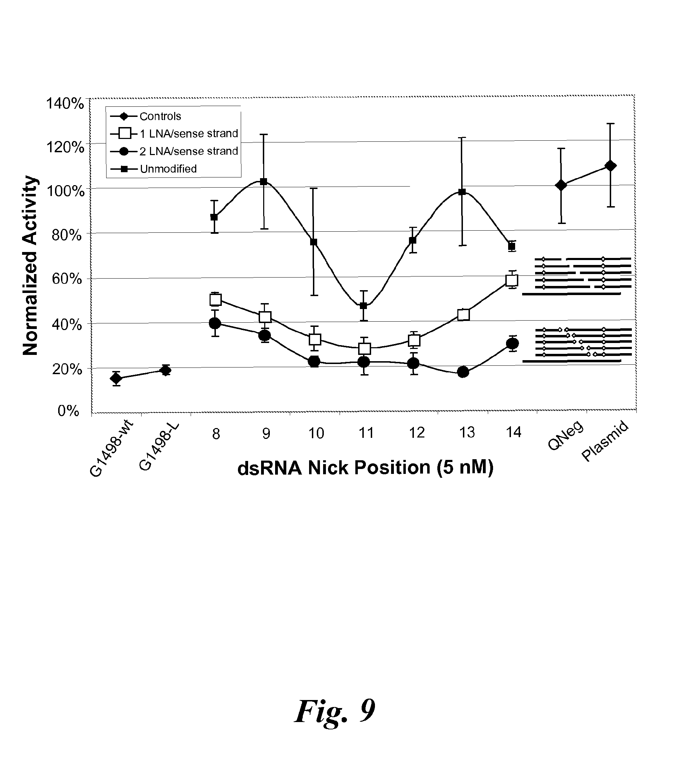

The dual fluorescence assay of Example 3 was used to measure knockdown activity. These data show that increasing the number of locked nucleic acid substitutions tends to increase activity of an mdRNA having a nick at any of a number of positions. The single locked nucleic acid per sense strand appears to be most active when the nick is at position 11 *(see* Figure 9). But, multiple locked nucleic acids on each sense strand make mdRNA having a nick at any position as active as the most optimal nick position with a single substitution (*i.e*., position 11) (Figure 9). Thus, mdRNA having duplex stabilizing modifications make mdRNA essentially equally active regardless of the nick position.

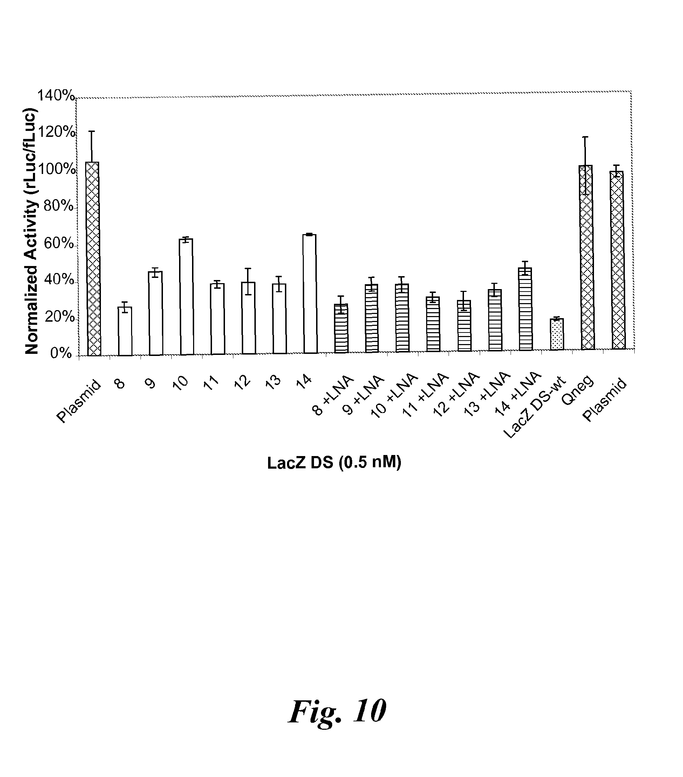

Similar results were observed when locked nucleic acid substitutions were made in the LacZ dicer substrate mdRNA of Example 2 (SEQ ID NOS:3 and 4). The lacZ dicer was nicked at positions 8 to 14, and a duplicate set of nicked LacZ dicer molecules were made with the exception that the A at position 3 (from the 5'-end) of the 5' sense strand was substituted for a locked nucleic acid A (LNA-A). As is evident from Figure 10, most of the nicked lacZ dicer molecules containing LNA-A were as potent in knockdown activity as the unsubstituted lacZ dicer.

### EXAMPLE 7

### MDRNA KNOCKDOWN OF INFLUENZA VIRUS TITER

The activity of a dicer substrate nicked dsRNA in reducing influenza virus titer as compared to a wild-type dsRNA (*i.e*., not having a nick) was examined. The influenza dicer substrate sequence (25/27) is as follows:
Sense 5'-GGAUCUUAUUUCUUCGGAGACAAdTdG (SEQ ID NO:62)
Antisense 5'-CAUUGUCUCCGAAGAAAUAAGAUCCUU (SEQ ID NO:11)

The mdRNA sequences have a nicked sense strand after position 12, 13, and 14, respectively, as counted from the 5'-end, and the G at position 2 is substituted with locked nucleic acid G.

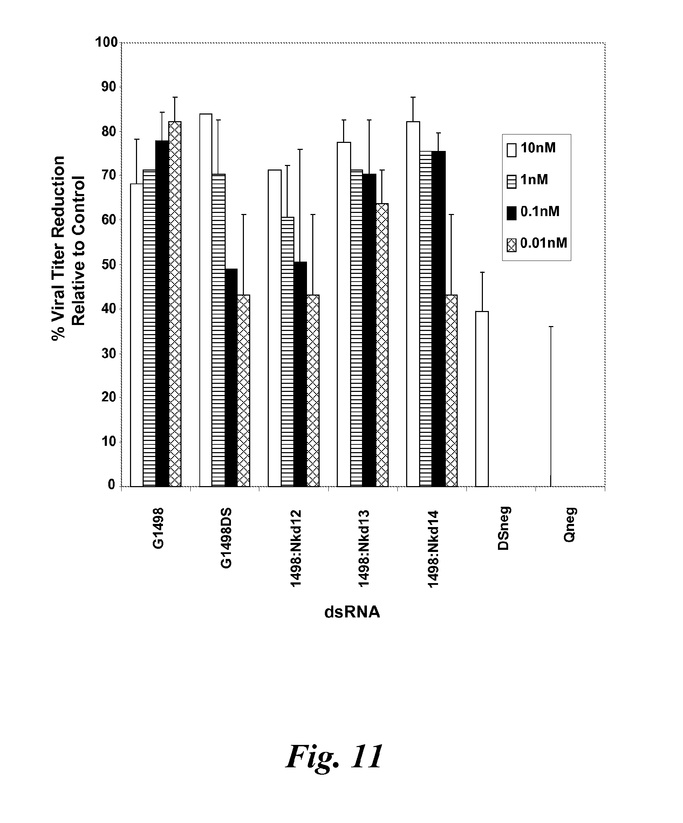

For the viral infectivity assay, Vero cells were seeded at 6.5 x 10⁴ cells/well the day before transfection in 500 µl 10% FBS/DMEM media per well. Samples of 100, 10, 1, 0.1, and 0.01 nM stock of each dsRNA were complexed with 1.0 µl (1 mg/ml stock) of Lipofectamine™ 2000 (Invitrogen, Carlsbad, CA) and incubated for 20 minutes at room temperature in 150 µl OPTIMEM (total volume) (Gibco, Carlsbad, CA). Vero cells were washed with OPTIMEM, and 150 µl of the transfection complex in OPTIMEM was then added to each well containing 150 µl of OPTIMEM media. Triplicate wells were tested for each condition. An additional control well with no transfection condition was prepared. Three hours post transfection, the media was removed. Each well was washed once with 200 µl PBS containing 0.3% BSA and 10 mM HEPES/PS. Cells in each well were infected with WSN strain of influenza virus at an MOI 0.01 in 200 µl of infection media containing 0.3% BSA/10 mM HEPES/PS and 4 µg/ml trypsin. The plate was incubated for 1 hour at 37°C. Unadsorbed virus was washed off with the 200 µl of infection media and discarded, then 400 µl DMEM containing 0.3% BSA/10 mM HEPES/PS and 4 µg/ml trypsin was added to each well. The plate was incubated at 37°C, 5% CO₂ for 48 hours, then 50 µl supernatant from each well was tested in duplicate by TCID₅₀ assays (50% Tissue-Culture Infective Dose, WHO protocol) in MDCK cells and titers were estimated using the Spearman and Karber formula. The results show that these mdRNAs show about a 50% to 60% viral titer knockdown, even at a concentration as low as 10 pM (Figure 11).

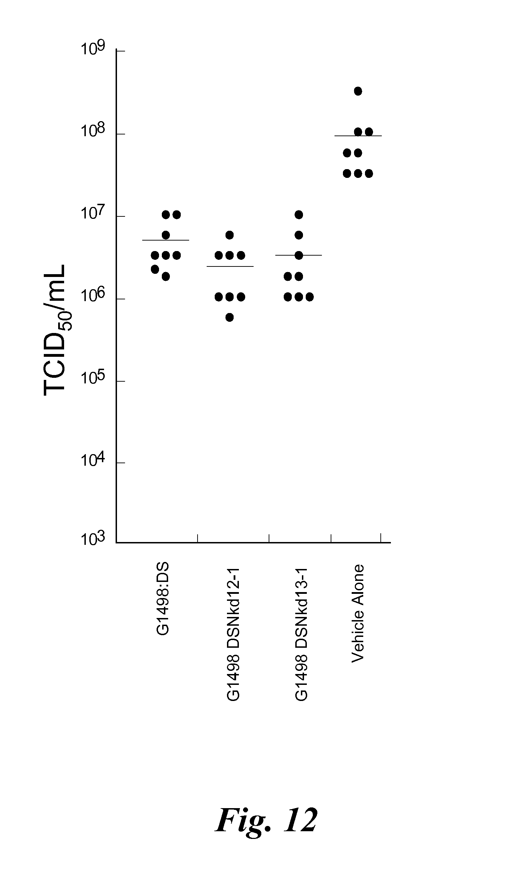

An *in vivo* influenza mouse model was also used to examine the activity of a dicer substrate nicked dsRNA in reducing influenza virus titer as compared to a wild-type dsRNA *(i.e.,* not having a nick). Female BALB/c mice (age 8-10 weeks with 5-10 mice per group) were dosed intranasally with 120 nmol/kg/day dsRNA (formulated in C12-norArg(NH₃+Cl⁻)-C12/DSPE-PEG2000/DSPC/cholesterol at a ratio of 30:1:20:49) for three consecutive days before intranasal challenge with influenza strain PR8 (20 PFU/mouse). Two days after infection, whole lungs are harvested from each mouse and placed in a solution of PBS/0.3% BSA with antibiotics, homogenize, and measure the viral titer (TCID₅₀). Doses were well tolerated by the mice, indicated by less than 2% body weight reduction in any of the dose groups. The mdRNAs tested exhibit similar, if not slightly greater, virus reduction *in vivo* as compared to unmodified and unnicked G1498 dicer substrate (*see* Figure 12). Hence, mdRNA are active *in vivo.*

### EXAMPLE 8

### EFFECT OF MDRNA ON CYTOKINE INDUCTION

The effect of the mdRNA structure on cytokine induction *in vivo* was examined. Female BALB/c mice (age 7-9 weeks) were dosed intranasally with about 50 µM dsRNA (formulated in C12-norArg(NH₃+Cl-)-C12/DSPE-PEG2000/DSPC/cholesterol at a ratio of 30:1:20:49) or with 605 nmol/kg/day naked dsRNA for three consecutive days. About four hours after the final dose is administered, the mice were sacrificed to collect bronchoalveolar fluid (BALF), and collected blood is processed to serum for evaluation of the cytokine response. Bronchial lavage was performed with 0.5 mL ice-cold 0.3% BSA in saline two times for a total of 1 mL. BALF was spun and supernatants collected and frozen until cytokine analysis. Blood was collected from the vena cava immediately following euthanasia, placed into serum separator tubes, and allowed to clot at room temperature for at least 20 minutes. The samples were processed to serum, aliquoted into Millipore ULTRAFREE 0.22 µm filter tubes, spun at 12,000 rpm, frozen on dry ice, and then stored at -70°C until analysis. Cytokine analysis of BALF and plasma were performed using the Procarta™ mouse 10-Plex Cytokine Assay Kit (Panomics, Fremont, CA) on a Bio-Plex™ array reader. Toxicity parameters were also measured, including body weights, prior to the first dose on day 0 and again on day 3 (just prior to euthanasia). Spleens were harvested and weighed (normalized to final body weight). The results are provided in Table 5.

**Table 5. In vivo Cytokine Induction by Naked mdRNA**

| Cytokine | | G1498 | G1498:Nkd 11-1 | G1498:DS | G1498:DSNkd 12-1 | G1498:DSNkd 13-1 | G1498:DSNkd 14-1 |
|---|---|---|---|---|---|---|---|
| IL-6 | Conc (pg/mL) | 90.68 | 10.07 | 77.35 | 17.17 | 18.21 | 38.59 |
| | Fold decrease | - | 9 | - | 5 | 4 | 2 |
| IL-12 (p40) | Conc (pg/mL) | 661.48 | 20.32 | 1403.61 | 25.07 | 37.70 | 57.02 |
| | Fold decrease | - | 33 | - | 56 | 37 | 25 |
| TNFα | Conc (pg/mL) | 264.49 | 25.59 | 112.95 | 20.52 | 29.00 | 64.93 |
| | Fold decrease | - | 10 | - | 6 | 4 | 2 |

The mdRNA (RISC or dicer sized) induced cytokines to lesser extent than the intact (*i.e.,* not nicked) parent molecules. The decrease in cytokine induction was greatest when looking at IL-12(p40), the cytokine with consistently the highest levels of induction of the 10 cytokine multiplex assay. For the mdRNA, the decrease in IL-12 (p40) ranges from 25- to 56-fold, while the reduction in either IL-6 or TNFα induction was more modest (the decrease in these two cytokines ranges from 2- to 10-fold). Thus, the mdRNA structure appears to provide an advantage in vivo in that cytokine induction is minimized compared to unmodified dsRNA.

Similar results were obtained with the formulated mdRNA, although the reduction in induction was not as prominent. In addition, the presence or absence of a locked nucleic acid has no effect on cytokine induction. These results are shown in Table 6.

**Table 6. In vivo Cytokine Induction by Formulated mdRNA**

| Cytokine | | G1498:DS | G1498:Nkd 12-1 | G1498:Nkd 13-1 | G1498:DSNkd 14-1 | G1498:DSNkd 13 |
|---|---|---|---|---|---|---|
| IL-6 | Conc (pg/mL) | 29.04 | 52.95 | 10.28 | 7.79 | 44.29 |
| | Fold decrease | - | -1.8 | 3 | 4 | -1.5 |
| IL-12 (p40) | Conc (pg/mL) | 298.93 | 604.24 | 136.45 | 126.71 | 551.49 |
| | Fold decrease | - | 0 | 2 | 2 | 1 |
| TNFα | Conc (pg/mL) | 13.49 | 21.35 | 3.15 | 3.15 | 18.69 |
| | Fold decrease | - | -1.6 | 4 | 4 | 1.4 |

The teachings of all of references cited herein including patents, patent applications, journal articles, wedpages, tables, and priority documents are incorporated herein in their entirety by reference. Although the foregoing disclosure has been described in detail by way of example for purposes of clarity of understanding, it will be apparent to the artisan that certain changes and modifications may be practiced within the scope of the appended claims which are presented by way of illustration not limitation. In this context, various publications and other references have been cited within the foregoing disclosure for economy of description. It is noted, however, that the various publications discussed herein are incorporated solely for their disclosure prior to the filing date of the present application, and the inventors reserve the right to antedate such disclosure by virtue of prior invention.

### CLAUSES:

1. A meroduplex ribonucleic acid (mdRNA) molecule, comprising a first strand of 15 to 40 nucleotides in length that is complementary to a Wingless-Type MMTV (Murine Mammary Tumor Virus) Integration Site Family, (WNT) Member 1 mRNA as set forth in SEQ ID NO:1158, a human WNT2 mRNA as set forth in SEQ ID NO:1390, or a human WNT3A mRNA as set forth in SEQ ID NO:1615, and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands.
2. The mdRNA molecule of clause1 wherein the first strand is 15 to 25 nucleotides in length or 26 to 40 nucleotides in length.
3. The mdRNA molecule of clause1 wherein the gap is a nick or the gap comprises at least one unpaired nucleotide in the first strand positioned between the double-stranded regions formed by the second and third strands when annealed to the first strand.
4. The mdRNA molecule of clause 1 wherein at least one uridine of the mdRNA molecule is a 5-methyluridine, 2-thioribothymidine, or 2'-O-methy-5-methyuridine.
5. The mdRNA molecule of clause 1 wherein the mdRNA molecule comprises at least one locked nucleic acid (LNA) molecule, deoxy nucleotide, G clamp, 2'-sugar modification, modified internucleoside linkage, or any combination thereof.
6. The mdRNA molecule of clause1 wherein the mdRNA molecule contains at least one 3'-overhang comprising one to four nucleotides that are not part of the gap or wherein the mdRNA has a blunt end at one or both ends of the mdRNA.
7. The mdRNA molecule of clause 1, wherein the third strand comprises a 5'-terminal end comprising a hydroxyl or a phosphate.
8. An mdRNA molecule, comprising a first strand of 15 to 40 nucleotides in length that is complementary to a human WNT1 mRNA as set forth in SEQ ID NO: 1158, WNT2 mRNA as set forth in SEQ ID NO:1390, or WNT3A mRNA as set forth in SEQ ID NO:1615, and a second strand and a third strand that is each complementary to non-overlapping regions of the first strand, wherein the second strand and third strands can anneal with the first strand to form at least two double-stranded regions spaced apart by up to 10 nucleotides and thereby forming a gap between the second and third strands, and wherein at least one pyrimidine of the mdRNA comprises a pyrimidine nucleoside according to Formula I or II: 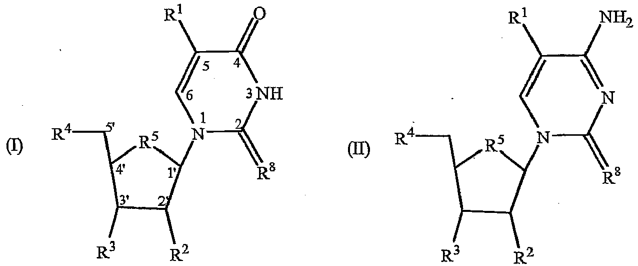 wherein:
   R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, diallcylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C≡N, or heterocyclo group,
   R³ and R⁴ are each independently a hydroxyl, a protected hydroxyl, a phosphate, or an internucleoside linking group, and
   R⁵ and R⁸ are each independently O or S.
9. The mdRNA molecule of clause 8 wherein the first strand is 15 to 25 nucleotides in length or 26 to 40 nucleotides in length.
10. The mdRNA molecule of clause8 wherein the gap is a nick or the gap comprises at least one unpaired nucleotide in the first strand positioned between the double-stranded regions formed by the second and third strands when annealed to the first strand.
11. The mdRNA molecule of clause8 wherein at least one nucleoside is according to Formula I and in which R¹ is methyl and R² is -OH or -O-methyl.
12. The mdRNA molecule of clause 8 wherein at least one R² is selected from the group consisting of 2'-O-(C₁-C₅) alkyl, 2'-O-methyl, 2'-OCH₂OCH₂CH₃, 2'-OCH₂CH₂OCH₃, 2'-O-allyl, and 2'-fluoro.
13. The mdRNA molecule of clause8 wherein at least one uridine of the mdRNA molecule is a 5-methyluridine, 2-thioribothymidine, or 2'-O-methy-5-methyuridine.
14. The mdRNA molecule of clause8 wherein the mdRNA molecule comprises at least one LNA, deoxy nucleotide, G clamp, 2'-sugar modification, modified internucleoside linkage, or any combination thereof.
15. The mdRNA molecule of clause8, wherein the mdRNA contains at least one 3'-overhang comprising one to four nucleotides that is not a part of the gap or wherein the mdRNA molecule has a blunt end on one or both ends of the mdRNA molecule.
16. An mdRNA molecule, comprising a first strand that is complementary to a human WNT1 mRNA as set forth in SEQ ID NO:1158, WNT2 mRNA as set forth in SEQ ID NO:1390, or WNT3A mRNA as set forth in SEQ ID NO:1615, and a second strand and a third strand that are each complementary to non-overlapping regions of the first strand, wherein the second strand and third strands can anneal with the first strand to form at least two double-stranded regions spaced apart by up to 10 nucleotides and thereby forming a gap between the second and third strands, and wherein the combined double-stranded regions total about 15 base pairs to about 40 base pairs.
17. The mdRNA molecule of clause16 wherein the first strand is 15 to 25 nucleotides in length or 26 to 40 nucleotides in length.
18. The mdRNA molecule of clause16 wherein the gap is a nick or the gap comprises at least one unpaired nucleotide in the first strand positioned between the double-stranded regions formed by the second and third strands when annealed to the first strand.
19. The mdRNA molecule of clause16 wherein at least one uridine of the mdRNA molecule is a 5-methyluridine, 2-thioribothymidine, or 2'-O-methy-5-methyuridine.
20. The mdRNA molecule of clause1 wherein the first strand is 19 to 23 nucleotides in length and is complementary to a human WNT1 nucleic acid sequence as set forth in any one of SEQ ID NOS:1159-1389, a human WNT2 nucleic acid sequence as set forth in any one of SEQ ID NOS:1391-1614, or a human WNT3A nucleic acid sequence as set forth in any one of SEQ ID NOS:1616-1901.
21. The mdRNA molecule of clause 1 wherein the first strand is 25 to 29 nucleotides in length and is complementary to a human WNT1 nucleic acid sequence as set forth in any one of SEQ ID NOS:1159-1389, a human WNT2 nucleic acid sequence as set forth in any one of SEQ ID NOS:1391-1614, or a human WNT3A nucleic acid sequence as set forth in any one of SEQ ID NOS:1616-1901.
22. A method for reducing the expression of a human WNT1, WNT2, or WNT3A gene, comprising administering an mdRNA molecule according to any one of clauses 1-21 to a cell expressing a WNT1, WNT2, or WNT3A gene, wherein the mdRNA molecule reduces expression of the WNT1, WNT2, or WNT3A gene in the cell.
23. The method according to clause 22wherein the cell is human.
24. Use of an mdRNA as defined in any one of the precedingclausesfor the manufacture of a medicament for use in the therapy of a metabolic disease.

## Claims

1. A double-stranded ribonucleic acid (dsRNA) molecule that down regulates the expression of a human Wingless-Type MMTV (Murine Mammary Tumor Virus) Integration Site Family, (WNT) Member 1, WNT2, and/or WNT3A mRNA, the dsRNA molecule comprising a first strand of 26 to 40 nucleotides in length, that is complementary to the human WNT1 mRNA as set forth in SEQ ID NO:1158, the human WNT2 mRNA as set forth in SEQ ID NO:1390, or the human WNT3A mRNA as set forth in SEQ ID NO:1615, and a second strand that is complementary to the first strand, and wherein upon annealing of the first strand and the second strand the dsRNA has a 3'-overhang and a blunt end.

2. The dsRNA molecule according to claim 1, wherein the first strand is from 27 to 35 nucleotides in length.

3. The dsRNA molecule according to claim 1, wherein the 3'-overhang has from one to four nucleotides and is on the first strand.

4. The dsRNA molecule according to any of claims 1-3, wherein at least one pyrimidine of the dsRNA molecule is a pyrimidine nucleoside according to Formula I or II: 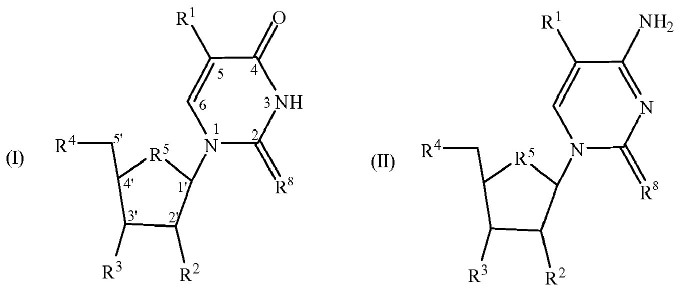 wherein:
R¹ and R² are each independently a -H, -OH, -OCH₃, -OCH₂OCH₂CH₃, -OCH₂CH₂OCH₃, halogen, substituted or unsubstituted C₁-C₁₀ alkyl, alkoxy, alkoxyalkyl, hydroxyalkyl, carboxyalkyl, alkylsulfonylamino, aminoalkyl, dialkylamino, alkylaminoalkyl, dialkylaminoalkyl, haloalkyl, trifluoromethyl, cycloalkyl, (cycloalkyl)alkyl, substituted or unsubstituted C₂-C₁₀ alkenyl, substituted or unsubstituted -O-allyl, -O-CH₂CH=CH₂, -O-CH=CHCH₃, substituted or unsubstituted C₂-C₁₀ alkynyl, carbamoyl, carbamyl, carboxy, carbonylamino, substituted or unsubstituted aryl, substituted or unsubstituted aralkyl, -NH₂, -NO₂, -C=, or heterocyclo group,
R³ and R⁴ are each independently a hydroxyl, a protected hydroxyl, a phosphate, or an internucleoside linking group, and
R⁵ and R⁸ are each independently O or S.

5. The dsRNA molecule of any one of claims 1-4, wherein the molecule comprises at least one 5-methyluridine, 2-thioribothymidine, or 2'-O-methyl-5-methyluridine.

6. The dsRNA molecule of any one of claims 1-4, wherein the molecule comprises at least one locked nucleic acid (LNA) molecule, deoxy nucleotide, G clamp, 2'-sugar modification, modified internucleoside linkage, or any combination thereof.

7. The dsRNA molecule of claim 4, wherein at least one nucleoside is according to Formula I and in which R¹ is methyl and R² is -OH or -O-methyl.

8. The dsRNA molecule of claim 4, wherein at least one R² is selected from the group consisting of 2'-O-(C₁-C₅) alkyl, 2'-O-methyl, 2'-OCH₂OCH₂CH₃, 2'-OCH₂CH₂OCH₃, 2'-O-allyl, and fluoro.

9. The dsRNA molecule of claim 1 wherein the dsRNA molecule has a 5'-terminal end comprising a hydroxyl or a phosphate.

10. A dsRNA molecule according to any preceding claim for reducing the expression of a human WNT1, WNT2 and/or WNT3A gene in a cell expressing the human WNT1, WNT2 and/or WNT3A gene.

11. The dsRNA molecule according to any preceding claim for use according to claim 10, wherein the cell is a human cell.

12. A dsRNA molecule according to any preceding claim for use in treating a metabolic disease.

13. Use of a dsRNA molecule according to any preceding claim for the manufacture of a medicament for use in the therapy of a metabolic disease.

14. Use of a dsRNA molecule according to any preceding claim for the manufacture of a medicament for reducing the expression of a human WNT1, or WNT2 and/or WNT3A gene in a cell expressing a human WNT1, or WNT2 and/or WNT3A gene.

15. Use according to claim 14, wherein the cell is a human cell.
